# EUROPEAN PATENT APPLICATION

(11) **EP 4 745 139 A1**
(43) Date of publication of application: **20.05.2026**
(21) Application number: 24838832.4
(22) Date of filing: 10.07.2024
(51) Int. Cl.: C07D 417/14, C07D 417/04, A61K 31/551, A61K 31/554, A61P 11/00, A61P 31/12

(54) **COMPOUND FOR TREATING AND PREVENTING RESPIRATORY SYNCYTIAL VIRUS INFECTION DISEASES**

(30) Priority: 10.07.2023 CN 202310837957; 08.03.2024 CN 202410267846
(71) Applicant: CSPC Zhongqi Pharmaceutical Technology (Shijiazhuang) Co., Ltd., Shijiazhuang, Hebei 050035 (CN)
(72) Inventor: WANG, Chen, Shijiazhuang, Hebei 050035 (CN); YANG, Jinlu, Shijiazhuang, Hebei 050035 (CN); ZHAO, Chuanwu, Shijiazhuang, Hebei 050035 (CN); ZHANG, Chaozai, Shijiazhuang, Hebei 050035 (CN); WU, Bin, Shijiazhuang, Hebei 050035 (CN); SONG, Yunlong, Shijiazhuang, Hebei 050035 (CN); ZHANG, Xuejiao, Shijiazhuang, Hebei 050035 (CN); GUO, Wenmin, Shijiazhuang, Hebei 050035 (CN); GAO, Na, Shijiazhuang, Hebei 050035 (CN); YANG, Hanyu, Shijiazhuang, Hebei 050035 (CN)
(74) Representative: Gill Jennings & Every LLP
(86) International application number: PCT/CN2024/104752
(87) International publication number: WO 2025/011580

(57) **Abstract**

Provided are a compound represented by formula (I) or a tautomer, a stereoisomer, an isotope derivative or a pharmaceutically acceptable salt thereof. The compound has a good inhibitory effect on RSV viruses.

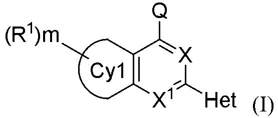

## Description

This application claims priority to two prior applications: one filed with the China National Intellectual Property Administration on July 10, 2023, with the patent application No. 202310837957.0, and the other filed with the China National Intellectual Property Administration on March 8, 2024, with the patent application No. 202410267846.5, both titled "Compound for Treating and Preventing Respiratory Syncytial Virus Infection Diseases". The entire contents of the two applications are incorporated herein by reference.

### THECNICAL FIELD

The present invention relates to the field of medical technology, and in particular, to an RSV inhibitor derivative and its preparation method and use.

### BACKGROUND

Respiratory Syncytial Virus (RSV) is a negative-sense single-stranded RNA virus belonging to the genus Pneumovirus of the family Paramyxoviridae. RSV can cause respiratory tract infections, leading to symptoms such as airway infections, bronchiolitis, and pneumonia from the upper respiratory tract to the lower respiratory tract. In some cases, it may also result in respiratory failure. It is estimated that RSV is the most important viral cause of acute lower respiratory tract infections (ALRTI) in children under the age of 5. Globally, almost all children are infected with RSV by the age of 2, with approximately 40% developing LRTI, and severe cases are observed in infants under 6 months old (which may be related to the immature immune system of infants and young children). In addition to infants, the elderly with underlying cardiopulmonary diseases such as chronic obstructive pulmonary disease are increasingly recognized as a high-risk population for severe infections.

RSV severely impacts human health, but there are still no highly effective therapeutic drugs available. To date, the FDA has only approved Ribavirin, a nucleoside small-molecule drug, for the treatment of severe RSV infections in hospitalized children. However, Ribavirin has poor antiviral efficacy and lacks specificity, leading to significant drug toxicity, and is currently not recommended for clinical use as a therapeutic drug. Other approved drugs such as Palivizumab and Nirsevimab are highly effective monoclonal antibodies that act on Fusion proteins related to viral replication, providing relatively long-acting protection for infants. However, these drugs have complex administration methods and high costs, which hinder their widespread use.

To date, various small-molecule anti-RSV inhibitors acting on different targets have been reported. For the conserved F protein in the viral replication process, Johnson & Johnson reported a class of inhibitors with benzimidazole as the parent nucleus, such as JNJ-53718678 as a representative molecule, while Gilead Sciences reported a class of pyrrolopyridine inhibitors such as GS-5806. However, they both failed in Phase II clinical trials. Roche reported a class of small-molecule inhibitors with quinazoline as the parent nucleus in 2013, which achieved good therapeutic effects in clinical trials in 2022. Several small-molecule inhibitors have also been reported for other conserved proteins in the viral replication process, such as the L protein and N protein. Nevertheless, no small-molecule inhibitors have been approved for marketing so far.

Therefore, developing highly effective small-molecule inhibitors targeting specific conformations of the conserved F protein of RSV to exert potent antiviral effects can provide a new therapeutic regimen for the treatment of RSV infections and related diseases.

Currently, there are no confirmed effective antiviral drugs for the treatment of RSV. RSV inhibitors in preclinical research generally face problems of low *in vivo* exposure and high clearance rate. Thus, the development of RSV inhibitors has broad prospects and can fill the significant clinical gap in the treatment of such diseases.

### SUMMARY

The present invention provides a class of anti-RSV compounds with novel structures, or tautomers, stereoisomers, isotopic derivatives, or pharmaceutically acceptable salts thereof, as well as the uses of such compounds, or the tautomers, stereoisomers, isotopic derivatives, or pharmaceutically acceptable salts thereof, in the medicaments for preventing and/or treating diseases caused by RSV infection.

Specifically, the present invention provides a compound represented by Formula (I), or a tautomer, stereoisomer, isotopic derivative, or pharmaceutically acceptable salt thereof, having the following structure: 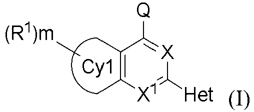 wherein,
Cy1 is independently selected from the group consisting of being absent, C₄₋₁₂ carbocyclyl, 4- to 12-membered heterocyclyl, C₆₋₁₂ aryl, and 5- to 12-membered heteroaryl; when Cy1 is absent, R¹ is connected to the heteroaromatic ring, i.e., 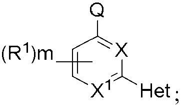
R¹ is independently selected from the group consisting of hydrogen, deuterium, halogen, oxo, hydroxyl, amino, nitro, cyano, -R^{a1}, -OR^{a1}, -SR^{a1}, -N(R^{a1})₂, -S(O)₂R^{a1}, -S(O)₂N(R^{a1})₂, -S(O)R^{a1}, -S(O)(NR^{a1})R^{a1}, -S(O)N(R^{a1})₂, - P(O)(OR^{a1})₂, -P(O)(N(R^{a1})₂)₂, -SC(R^{a1})₃, -C(R^{a1})₂OR^{a1}, -C(R^{a1})₂N(R^{a1})₂, -C(O)R^{a1}, -C(O)OR^{a1}, -C(O)N(R^{a1})₂, - C(O)OR^{a1}-OC(O)R^{a1}, -C(O)N(R^{a1})OR^{a1}, -OC(O)R^{a1}, -OC(O)N(R^{a1})₂, -N(R^{a1})C(O)OR^{a1}, -N(R^{a1})C(O)R^{a1}, - N(R^{a1})C(O)N(R^{a1})₂, -N(R^{a1})S(O)₂R^{a1}, -C(S)R^{a1}, -C(S)OR^{a1}, -C(S)N(R^{a1})₂, -C(S)N(R^{a1})OR^{a1}, -OC(S)R^{a1}, and - OC(S)N(R^{a1})₂;
R^{a1} is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{a2}; or
two R^{a1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{a2} is independently selected from the group consisting of deuterium, halogen, oxo, hydroxyl, amino, nitro, cyano, mercapto, C₁₋₆ alkyl, and C₁₋₆ alkoxy;
X¹ is independently selected from the group consisting of CR² and N;
X is independently selected from the group consisting of CR² and N;
R² is independently selected from the group consisting of hydrogen, deuterium, halogen, hydroxyl, amino, nitro, cyano, -OR^{b1}, -SR^{b1}, -N(R^{b1})₂, -S(O)₂R^{b1}, -S(O)₂N(R^{b1})₂, -S(O)R^{b1}, -S(O)(NR^{b1})R^{b1}, -S(O)N(R^{b1})₂, - P(O)(OR^{b1})₂, -P(O)(N(R^{b1})₂)₂, -C(R^{b1})₂(OR^{b1}), -C(R^{b1})₂N(R^{b1})₂, -C(O)R^{b1}, -C(O)OR^{b1}, -C(O)N(R^{b1})₂,-C(O)N(R^{b1})OR^{b1}, -OC(O)R^{b1}, -OC(O)N(R^{b1})₂, -N(R^{b1})C(O)OR^{b1}, -N(R^{b1})C(O)R^{b1}, -N(R^{b1})C(O)N(R^{b1})₂, - N(R^{b1})S(O)₂R^{b1}, C₁₋₆ alkyl, C₃₋₇ carbocyclyl, 3- to 7-membered heterocyclyl, C₆₋₁₂ aryl, and 5- to 12-membered heteroaryl, wherein each of the C₁₋₆ alkyl, the C₃₋₇ cycloalkyl, the 3- to 7-membered heterocyclyl, the C₆₋₁₂ aryl, and the 5- to 12-membered heteroaryl is optionally substituted with one or more R^{b2};
R^{b1} is independently selected from the group consisting of hydrogen, deuterium, C₁₋₆ alkyl, C₃₋₇ carbocyclyl, 4- to 7-membered heterocyclyl, phenyl, and 5- to 6-membered heteroaryl, wherein each of the C₁₋₆ alkyl, the C₃₋₇ carbocyclyl, the 4- to 7-membered heterocyclyl, the phenyl, and the 5- to 6-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl; or
two R^{b1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl;
R^{b2} is independently selected from the group consisting of deuterium, halogen, oxo, hydroxyl, amino, nitro, cyano, mercapto, C₁₋₆ alkyl, and C₁₋₆ alkoxy;
m is independently selected from 0, 1, 2, 3, or 4;
Q is independently selected from the group consisting of deuterium, halogen, -R³, -OR³, -SR³, -N(R³)-(C₀₋₆ alkylene)-R³, -N(R³)-(C₀₋₆ alkylene)-N(R³)₂, -S(O)₂R³, -S(O)₂N(R³)₂, -S(O)R³, -S(O)(NR³)R³, -S(O)N(R³)₂, - P(O)(OR³)₂, -P(O)(N(R³)₂)₂, -C(R³)₂(OR³), -C(R³)₂N(R³)₂, -C(O)R³, -C(O)OR³, -C(O)N(R³)₂, -C(O)N(R³)OR³, - OC(O)R³, -OC(O)N(R³)₂, -N(R³)C(O)OR³, -N(R³)C(O)R³, -N(R³)C(O)N(R³)₂, and -N(R³)S(O)₂R³, wherein the alkylene is optionally substituted with one or more R^{c1};
R³ is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{c2}; or
two R³s connected to a same nitrogen atom, together with the nitrogen atom to which they are connected, form 3- to 12-membered heterocyclyl or 5- to 12-membered heteroaryl, wherein each of the 3- to 12-membered heterocyclyl and the 5- to 12-membered heteroaryl is optionally substituted with one or more substituents selected from the group consisting of halogen, hydroxyl, amino, -C(O)NH₂, -(CH₂)₁₋₆NH₂, oxo, C₁₋₆ alkyl, C₁₋₆ alkoxy, oxetanylamino, and C₁₋₆ alkylpiperazinyl;
R^{c1} is independently selected from the group consisting of deuterium, halogen, hydroxyl, amino, nitro, cyano, mercapto, C₁₋₆ alkyl, C₁₋₆ alkoxy, C₃₋₆ cycloalkyl, 3- to 6-membered heterocyclyl, phenyl, and 5- to 6-membered heteroaryl;
R^{c2} is independently selected from the group consisting of deuterium, oxo, halogen, hydroxyl, amino, cyano, - OR^{c3}, -SR^{c3}, -N(R^{c3})₂, -S(O)₂R^{c3}, -S(O)₂N(R^{c3})₂, -S(O)R^{c3}, -S(O)(NR^{c3})R^{c3}, -P(O)(OR^{c3})₂, -P(O)(N(R^{c3})₂)₂, - C(R^{c3})₂(OR^{c3}), -C(R^{c3})₂N(R^{c3})₂, -C(O)R^{c3}, -C(O)OR^{c3}, -C(O)N(R^{c3})₂, -C(O)N(R^{c3})OR^{c3}, -OC(O)R^{c3}, - OC(O)N(R^{c3})₂, -N(R^{c3})C(O)OR^{c3}, -N(R^{c3})C(O)R^{c3}, -N(R^{c3})C(O)N(R^{c3})₂, -N(R^{c3})C(O)R^{c3}N(R^{c3})OC(O)R^{c3}, - N(R^{c3})S(O)₂R^{c3}, C₁₋₆ alkyl, C₃₋₆ cycloalkyl, 3- to 6-membered heterocyclyl, C₆₋₈ aryl, and 5- to 8-membered heteroaryl, wherein each of the C₁₋₆ alkyl, the C₃₋₆ cycloalkyl, the 3- to 6-membered heterocyclyl, the C₆₋₈ aryl, and the 5- to 8-membered heteroaryl is optionally substituted with one or more R^{c4};
each of R^{c3} and R^{c4} is independently selected from the group consisting of hydrogen, deuterium, oxo, halogen, hydroxyl, amino, C₁₋₁₈ alkyl, C₁₋₆ alkoxy, C₁₋₆ haloalkyl, C₁₋₆ alkyl optionally substituted with one or more hydroxyl groups, C₁₋₆ alkyl optionally substituted with one or more amino groups, C₃₋₆ cycloalkyl, 3- to 6-membered heterocyclyl, C₆₋₈ aryl, and 5- to 6-membered heteroaryl;
Het has a structure as below: 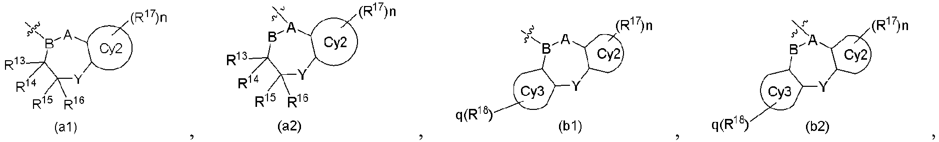 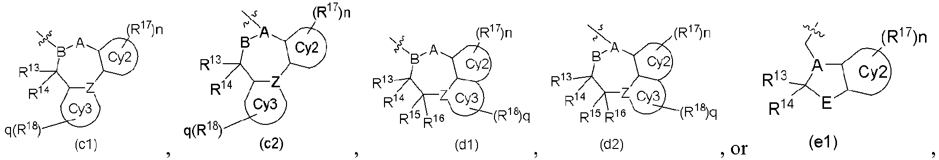
wherein,
when A or B is connected to 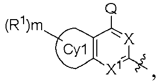 A and B are each independently selected from the group consisting of -C(R^{d1})-, -N-, 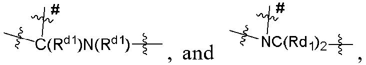 where "#" denotes the end connected to 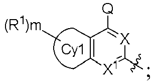
when A or B is not connected to 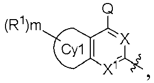 A and B are each independently selected from the group consisting of a bond, -C(R^{d1})₂-, -NR^{d1}-, -C(R^{d1})₂N(R^{d1})-, and -NR^{d1}C(R^{d1})₂-
R^{d1} is independently selected from the group consisting of being absent, a bond, hydrogen, deuterium, hydroxyl, amino, nitro, cyano, and C₁₋₆ alkyl; or two R^{d1}s connected to a same carbon atom together form oxo;
when R^{d1} is absent, 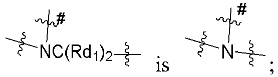
Y is independently selected from the group consisting of -O-, -N(R^{d2})-, -C(R^{d2})₂-, -S-, -C(O)-, -C(=N-O(C₀₋₆ alkyl))-, -S(O)-, -S(O)₂-, and -S(O)(=N-R^{d2})-;
R^{d2} is independently selected from the group consisting of hydrogen, deuterium, halogen, hydroxyl, amino, nitro, cyano, -OR^{d3}, -SR^{d3}, -N(R^{d3})₂, -C(O)R^{d3}, -(C₁₋₆ alkylene)-C(O)R^{d3}, -(C₁₋₆ alkylene)-S(O)₂R^{d3}, -C(O)OR^{d3}, - (C₁₋₆ alkylene)-C(O)OR^{d3}, -C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, -S(O)₂N(R^{d3})₂, -S(O)R^{d3}, -C(O)N(R^{d3})OR^{d3}, -OC(O)R^{d3}, -OC(O)N(R^{d3})₂, -N(R^{d3})C(O)OR^{d3}, -N(R^{d3})C(O)R^{d3},-N(R^{d3})C(O)N(R^{d3})₂, -N(R^{d3})S(O)₂R^{d3}, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₁₋₆ alkylene, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, nitro, cyano, C₁₋₆ alkyl, C₁₋₆ alkoxy, C₃₋₁₂ cycloalkyl, 3- to 12-membered heterocyclyl, C₆₋₁₂ aryl, and 5- to 12-membered heteroaryl;
R^{d3} is independently selected from the group consisting of hydrogen, deuterium, halogen, hydroxyl, amino, nitro, cyano, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy;
R¹³, R¹⁴, R¹⁵, and R¹⁶ are each independently selected from the group consisting of hydrogen, deuterium, oxo, halogen, hydroxyl, amino, imino, sulfinamido, nitro, cyano, C₁₋₆ alkyl, C₁₋₆ haloalkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₁₋₆ haloalkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more R^{d4}; when one of R¹³ and R¹⁴ is selected from the group consisting of oxo and imino, the other of R¹³ and R¹⁴ is absent; when one of R¹⁵ and R¹⁶ is selected from the group consisting of oxo and imino, the other of R¹⁵ and R¹⁶ is absent; or
R¹³ and R¹⁴, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy; or
R¹⁵ and R¹⁶, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy;
R^{d4} is independently selected from the group consisting of deuterium, halogen, oxo, hydroxyl, amino, cyano, sulfonyl, urea, C₂₋₆ alkenyl, C₂₋₆ alkynyl, C₁₋₆ alkyl, C₁₋₆ alkoxy, and C₃₋₈ cycloalkyl, wherein each of the C₁₋₆ alkyl, the C₁₋₆ alkoxy, and the C₃₋₈ cycloalkyl is optionally substituted with one or more halogen;
Cy2 is independently selected from the group consisting of phenyl, 5- to 6-membered heterocyclyl, and 5- to 6-membered heteroaryl;
Cy3 is independently selected from the group consisting of phenyl, 5- to 6-membered heterocyclyl, and 5- to 6-membered heteroaryl;
R¹⁷ and R¹⁸ are each independently selected from the group consisting of deuterium, halogen, hydroxyl, amino, cyano, nitro, carboxyl, oxo, -OR^{d5}, -SR^{d5}, -N(R^{d5})₂, -S(O)₂R^{d5}, -S(O)₂N(R¹⁵)₂, -S(O)R^{d5}, -S(O)(NR^{d5})R^{d5}, - S(O)N(R^{d5})₂, -P(O)(OR^{d5})₂, -P(O)(N(R^{d5})₂)₂, -C(R^{d5})₂(OR^{d5}), -C(R^{d5})₂N(R^{d5})₂, -C(O)R^{d5}, -C(O)OR^{d5}, -C(O)N(R^{d5})₂, -C(O)N(R^{d5})OR^{d5}, -OC(O)R^{d5}, -OC(O)N(R^{d5})₂, -N(R^{d5})C(O)OR^{d5}, -N(R^{d5})C(O)R^{d5}, -N(R^{d5})C(O)N(R^{d5})₂,-N(R^{d5})S(O)₂R^{d5}, -N(R^{a5})S(O)₂N(R^{d5}) ₂, -N(R^{d5})S(O)₂O(R^{d5}), C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, cyano, carboxyl, C₁₋₆ alkyl, C₁₋₆ haloalkyl, C₁₋₆ alkoxy, C₁₋₆ haloalkoxy, C₂₋₆ alkenyl, C₂₋₆ alkynyl, and C₃₋₈ cycloalkyl;
R^{d5} is independently selected from the group consisting of hydrogen, deuterium, halogen, hydroxyl, C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, hydroxyl, amino, cyano, and carboxyl;
Z is independently selected from the group consisting of C and N;
E is independently selected from the group consisting of -C(R^{d6})₂- and -N(R^{d6})-;
R^{d6} is independently selected from the group consisting of hydrogen, deuterium, halogen, hydroxyl, amino, cyano, nitro, sulfinamido, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, carboxyl, and cyano; or two R^{d6}s connected to a same carbon atom together form oxo, thio, or imino; or two R^{d6}s connected to a same carbon atom, together with the carbon atom to which they are connected, form C₃₋₁₅ carbocyclyl or 3- to 15-membered heterocyclyl, wherein each of the C₃₋₁₅ carbocyclyl and the 3- to 15-membered heterocyclyl is optionally substituted with one or more substituents selected from the group consisting of deuterium, halogen, oxo, hydroxyl, amino, carboxyl, and cyano;
n and q are each independently selected from 0, 1, 2, 3, or 4;
when a plurality of R^{a1}s, a plurality of R^{a2}s, a plurality of R^{b1}s, a plurality of R^{b2}s, a plurality of R^{c1}s, a plurality of R^{c2}s, a plurality of R^{c3}s, a plurality of R^{c4}s, a plurality of R^{d1}s, a plurality of R^{d2}s, a plurality of R^{d3}s, a plurality of R^{d4}s, a plurality of R^{dS}s, a plurality of R^{d6}s, a plurality of R¹s, a plurality of R³s, a plurality of R¹⁷s, or a plurality of R¹⁸s are present simultaneously, each R^{a1}, each R^{a2}, each R^{b1}, each R^{b2}, each R^{c1}, each R^{c2}, each R^{c3}, each R^{d1}, each R^{d2}, each R^{d3}, each R^{d4}, each R^{d5}, each R^{d6}, each R¹, each R³, each R¹⁷, or each R¹⁸ may be the same or different.

In some embodiments, provided is a compound represented by Formula (I) below, or a tautomer, a stereoisomer, or a pharmaceutically acceptable salt thereof, or a pharmaceutically acceptable salt of the compound: 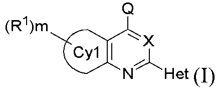 wherein,
Cy1 is independently selected from the group consisting of being absent, C₄₋₁₂ carbocyclyl, 4- to 12-membered heterocyclyl, C₆₋₁₂ aryl, and 5- to 12-membered heteroaryl; when Cy1 is absent, R¹ is connected to the heteroaromatic ring, i.e., 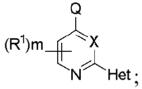
R¹ is independently selected from the group consisting of hydrogen, deuterium, halogen, oxo, hydroxyl, amino, nitro, cyano, -R^{a1}, -OR^{a1}, -SR^{a1}, -N(R^{a1})₂, -S(O)₂R^{a1}, -S(O)₂N(R^{a1})₂, -S(O)R^{a1}, -S(O)(NR^{a1})R^{a1}, -S(O)N(R^{a1})₂, - P(O)(OR^{a1})₂, -P(O)(N(R^{a1})₂)₂, -SC(R^{a1})₃, -C(R^{a1})₂OR^{a1}, -C(R^{a1})₂N(R^{a1})₂, -C(O)R^{a1}, -C(O)OR^{a1}, -C(O)N(R^{a1})₂, - C(O)OR^{a1}-OC(O)R^{a1}, -C(O)N(R^{a1})OR^{a1}, -OC(O)R^{a1}, -OC(O)N(R^{a1})₂, -N(R^{a1})C(O)OR^{a1}, -N(R^{a1})C(O)R^{a1}, - N(R^{a1})C(O)N(R^{a1})₂, -N(R^{a1})S(O)₂R^{a1}, -C(S)R^{a1}, -C(S)OR^{a1}, -C(S)N(R^{a1})₂, -C(S)N(R^{a1})OR^{a1}, -OC(S)R^{a1}, and - OC(S)N(R^{a1})₂;
R^{a1} is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{a2}; or
two R^{a1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{a2} is independently selected from the group consisting of deuterium, halogen, oxo, hydroxyl, amino, nitro, cyano, mercapto, C₁₋₆ alkyl, and C₁₋₆ alkoxy;
X is independently selected from the group consisting of CR² and N;
R² is independently selected from the group consisting of hydrogen, deuterium, halogen, hydroxyl, amino, nitro, cyano, -OR^{b1}, -SR^{b1}, -N(R^{b1})₂, -S(O)₂R^{b1}, -S(O)₂N(R^{b1})₂, -S(O)R^{b1}, -S(O)(NR^{b1})R^{b1}, -S(O)N(R^{b1})₂, - P(O)(OR^{b1})₂, -P(O)(N(R^{b1})₂)₂, -C(R^{b1})₂(OR^{b1}), -C(R^{b1})₂N(R^{b1})₂, -C(O)R^{b1}, -C(O)OR^{b1}, -C(O)N(R^{b1})₂,-C(O)N(R^{b1})OR^{b1}, -OC(O)R^{b1}, -OC(O)N(R^{b1})₂, -N(R^{b1})C(O)OR^{b1}, -N(R^{b1})C(O)R^{b1}, -N(R^{b1})C(O)N(R^{b1})₂, - N(R^{b1})S(O)₂R^{b1}, C₁₋₆ alkyl, C₃₋₇ carbocyclyl, 3- to 7-membered heterocyclyl, C₆₋₁₂ aryl, and 5- to 12-membered heteroaryl, wherein each of the C₁₋₆ alkyl, the C₃₋₇ cycloalkyl, the 3- to 7-membered heterocyclyl, the C₆₋₁₂ aryl, and the 5- to 12-membered heteroaryl is optionally substituted with one or more R^{b2};
R^{b1} is independently selected from the group consisting of hydrogen, deuterium, C₁₋₆ alkyl, C₃₋₇ carbocyclyl, 4- to 7-membered heterocyclyl, phenyl, and 5- to 6-membered heteroaryl, wherein each of the C₁₋₆ alkyl, the C₃₋₇ carbocyclyl, the 4- to 7-membered heterocyclyl, the phenyl, and the 5- to 6-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl; or
two R^{b1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl;
R^{b2} is independently selected from the group consisting of deuterium, halogen, oxo, hydroxyl, amino, nitro, cyano, mercapto, C₁₋₆ alkyl, and C₁₋₆ alkoxy;
m is independently selected from 0, 1, 2, 3, or 4;
Q is independently selected from the group consisting of deuterium, halogen, -R³, -OR³, -SR³, -N(R³)-(C₀₋₆ alkylene)-R³, -N(R³)-(C₀₋₆ alkylene)-N(R³)₂, -S(O)₂R³, -S(O)₂N(R³)₂, -S(O)R³, -S(O)(NR³)R³, -S(O)N(R³)₂, - P(O)(OR³)₂, -P(O)(N(R³)₂)₂, -C(R³)₂(OR³), -C(R³)₂N(R³)₂, -C(O)R³, -C(O)OR³, -C(O)N(R³)₂, -C(O)N(R³)OR³, - OC(O)R³, -OC(O)N(R³)₂, -N(R³)C(O)OR³, -N(R³)C(O)R³, -N(R³)C(O)N(R³)₂, and -N(R³)S(O)₂R³, wherein the alkylene is optionally substituted with one or more R^{c1};
each R³ is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{c2}; or
two R³s connected to a same nitrogen atom, together with the nitrogen atom to which they are connected, form 3- to 12-membered heterocyclyl or 5- to 12-membered heteroaryl, wherein each of the 3- to 12-membered heterocyclyl and the 5- to 12-membered heteroaryl is optionally substituted with one or more substituents selected from the group consisting of halogen, hydroxyl, amino, -C(O)NH₂, -(CH₂)₁₋₆NH₂, oxo, C₁₋₆ alkyl, C₁₋₆ alkoxy, oxetanylamino, and C₁₋₆ alkylpiperazinyl;
R^{c1} is independently selected from the group consisting of deuterium, halogen, hydroxyl, amino, nitro, cyano, mercapto, C₁₋₆ alkyl, C₁₋₆ alkoxy, C₃₋₆ cycloalkyl, 3- to 6-membered heterocyclyl, phenyl, and 5- to 6-membered heteroaryl;
R^{c2} is independently selected from the group consisting of deuterium, oxo, halogen, hydroxyl, amino, -OR^{c3}, - SR^{c3}, -N(R^{c3})₂, -S(O)₂R^{c3}, -S(O)₂N(R^{c3})₂, -S(O)R^{s3}, -S(O)(NR^{c3})R^{c3}, -P(O)(OR^{c3})₂, -P(O)(N(R^{c3})₂)₂, -C(R^{c3})₂(OR^{c3}), -C(R^{c3})₂N(R^{c3})₂, -C(O)R^{c3}, -C(O)OR^{c3}, -C(O)N(R^{c3})₂, -C(O)N(R^{c3})OR^{c3}, -OC(O)R^{c3}, -OC(O)N(R^{c3})₂, - N(R^{c3})C(O)OR^{c3}, -N(R^{c3})C(O)R^{c3}, -N(R^{c3})C(O)N(R^{c3})₂, -N(R^{c3})C(O)R^{c3}N(R^{c3})OC(O)R^{c3}, -N(R^{c3})S(O)₂R^{c3}, C₁₋₆ alkyl, C₃₋₆ cycloalkyl, 3- to 6-membered heterocyclyl, C₆₋₈ aryl, and 5- to 8-membered heteroaryl, wherein each of the C₁₋₆ alkyl, the C₃₋₆ cycloalkyl, the 3- to 6-membered heterocyclyl, the C₆₋₈ aryl, and the 5- to 8-membered heteroaryl is optionally substituted with one or more R^{c4};
each of R^{c3} and R^{c4} is independently selected from the group consisting of hydrogen, deuterium, oxo, halogen, hydroxyl, amino, C₁₋₁₈ alkyl, C₁₋₆ alkoxy, C₁₋₆ haloalkyl, C₁₋₆ alkyl optionally substituted with one or more hydroxyl groups, C₁₋₆ alkyl optionally substituted with one or more amino groups, C₃₋₆ cycloalkyl, 3- to 6-membered heterocyclyl, C₆₋₈ aryl, and 5- to 6-membered heteroaryl;
Het has a structure as below: 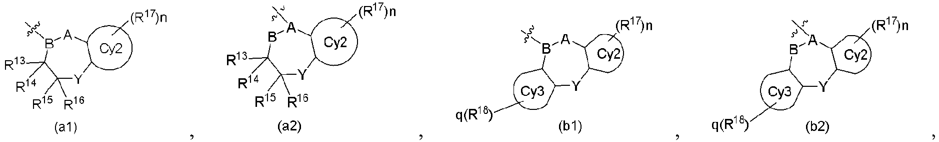 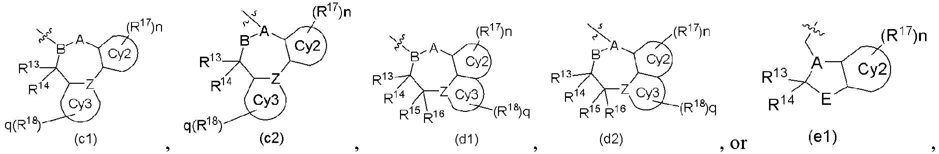
wherein,
when A or B is connected to 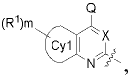 A and B are each independently selected from the group consisting of -C(R^{d1})-, -N-, 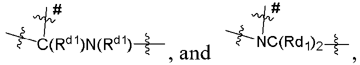 where "#" denotes the end connected to 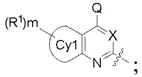
when A or B is not connected to 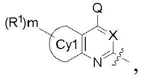 A and B are each independently selected from the group consisting of a bond, -C(R^{d1})₂-, -NR^{d1}-, -C(R^{d1})₂N(R^{d1})-, and -NR^{d1}C(R^{d1})₂-;
R^{d1} is independently selected from the group consisting of being absent, a bond, hydrogen, deuterium, hydroxyl, amino, nitro, cyano, and C₁₋₆ alkyl; or two R^{d1}s connected to a same carbon atom together form oxo;
Y is independently selected from the group consisting of -O-, -N(R^{d2})-, -C(R^{d2})₂-, -S-, -C(O)-, -C(=N-O(C₀₋₆ alkyl))-, -S(O)-, -S(O)₂-, and -S(O)(=N-R^{d2})-;
R^{d2} is independently selected from the group consisting of hydrogen, deuterium, halogen, hydroxyl, amino, nitro, cyano, -OR^{d3}, -SR^{d3}, -N(R^{d3})₂, -C(O)R^{d3}, -(C₁₋₆ alkylene)-C(O)R^{d3}, -C(O)OR^{d3}, -(C₁₋₆ alkylene)-C(O)OR^{d3}, - C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, -S(O)₂N(R^{d3})₂, -S(O)R^{d3}, - C(O)N(R^{d3})OR^{d3}, -OC(O)R^{d3}, -OC(O)N(R^{d3})₂, -N(R^{d3})C(O)OR^{d3}, -N(R^{d3})C(O)R^{d3}, -N(R^{d3})C(O)N(R^{d3})₂, - N(R^{d3})S(O)₂R^{d3}, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₁₋₆ alkylene, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from deuterium, halogen, oxo, hydroxyl, amino, nitro, cyano, C₁₋₆ alkyl, and C₁₋₆ alkoxy;
R^{d3} is independently selected from the group consisting of hydrogen, deuterium, halogen, hydroxyl, amino, nitro, cyano, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy;
R¹³, R¹⁴, R¹⁵, and R¹⁶ are each independently selected from the group consisting of hydrogen, deuterium, oxo, halogen, hydroxyl, amino, imino, sulfinamido, nitro, cyano, C₁₋₆ alkyl, C₁₋₆ haloalkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₁₋₆ haloalkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more R^{d4}; when one of R¹³ and R¹⁴ is selected from the group consisting of oxo and imino, the other of R¹³ and R¹⁴ is absent; when one of R¹⁵ and R¹⁶ is selected from the group consisting of oxo and imino, the other of R¹⁵ and R¹⁶ is absent; or
R¹³ and R¹⁴, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy; or
R¹⁵ and R¹⁶, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy;
R^{d4} is independently selected from the group consisting of deuterium, halogen, oxo, hydroxyl, amino, cyano, sulfonyl, urea, C₂₋₆ alkenyl, C₂₋₆ alkynyl, C₁₋₆ alkyl, C₁₋₆ alkoxy, and C₃₋₈ cycloalkyl, wherein each of the C₁₋₆ alkyl, the C₁₋₆ alkoxy, and the C₃₋₈ cycloalkyl is optionally substituted with one or more halogen atoms;
Cy2 is independently selected from the group consisting of phenyl, 5- to 6-membered heterocyclyl, and 5- to 6-membered heteroaryl;
Cy3 is independently selected from the group consisting of phenyl, 5- to 6-membered heterocyclyl, and 5- to 6-membered heteroaryl;
R¹⁷ and R¹⁸ are each independently selected from the group consisting of deuterium, halogen, hydroxyl, amino, cyano, nitro, carboxyl, oxo, -OR^{d5}, -SR^{d5}, -N(R^{d5})₂, -S(O)₂R^{d5}, -S(O)₂N(R^{d5})₂, -S(O)R^{d5}, -S(O)(NR^{d5})R^{d5}, - S(O)N(R^{d5})₂, -P(O)(OR^{d5})₂, -P(O)(N(R^{d5})₂)₂, -C(R^{d5})₂(OR^{d5}), -C(R^{d5})₂N(R^{d5})₂, -C(O)R^{d5}, -C(O)OR^{d5}, -C(O)N(R^{d5})₂, -C(O)N(R^{d5})OR^{d5}, -OC(O)R^{d5}, -OC(O)N(R^{d5})₂, -N(R^{d5})C(O)OR^{d5}, -N(R^{d5})C(O)R^{d5}, -N(R^{d5})C(O)N(R^{d5} - N(R^{d5})S(O)₂R^{d5}, -N(R^{a5})S(O)₂N(R^{d5}) ₂, -N(R^{d5})S(O)₂O(R^{d5}), C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, cyano, carboxyl, C₁₋₆ alkyl, C₁₋₆ haloalkyl, C₁₋₆ alkoxy, C₁₋₆ haloalkoxy, C₂₋₆ alkenyl, C₂₋₆ alkynyl, and C₃₋₈ cycloalkyl;
R^{d5} is independently selected from the group consisting of hydrogen, deuterium, halogen, hydroxyl, C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, hydroxyl, amino, cyano, and carboxyl;
Z is independently selected from the group consisting of C and N;
E is independently selected from the group consisting of -C(R^{d6})₂- and -N(R^{d6})-;
R^{d6} is independently selected from the group consisting of hydrogen, deuterium, halogen, hydroxyl, amino, cyano, nitro, sulfinamido, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, carboxyl, and cyano; or two R^{d6}s connected to a same carbon atom together form oxo, thio, or imino; or two R^{d6}s connected to a same carbon atom, together with the carbon atom to which they are connected, form C₃₋₁₅ carbocyclyl or 3- to 15-membered heterocyclyl, wherein each of the C₃₋₁₅ carbocyclyl and the 3- to 15-membered heterocyclyl is optionally substituted with one or more substituents selected from the group consisting of deuterium, halogen, oxo, hydroxyl, amino, carboxyl, and cyano;
n and q are each independently selected from 0, 1, 2, 3, or 4;
when a plurality of R^{a1}s, a plurality of R^{a2}s, a plurality of R^{b1}s, a plurality of R^{b2}s, a plurality of R^{c1}s, a plurality of R^{c2}s, a plurality of R^{c3}s, a plurality of R^{c4}s, a plurality of R^{d1}s, a plurality of R^{d2}s, a plurality of R^{d3}s, a plurality of R^{d4}s, a plurality of R^{dS}s, a plurality of R^{d6}s, a plurality of R¹s, a plurality of R³s, a plurality of R¹⁷s, or a plurality of R¹⁸s are present simultaneously, each R^{a1}, each R^{a2}, each R^{b1}, each R^{b2}, each R^{c1}, each R^{c2}, each R^{c3}, each R^{d1}, each R^{d2}, each R^{d3}, each R^{d4}, each R^{d5}, each R^{d6}, each R¹, each R³, each R¹⁷, or each R¹⁸ may be the same or different.

In a preferred embodiment of the present invention, Cy1 is independently selected from the group consisting of 4- to 6-membered heterocyclyl, phenyl, and 5- to 6-membered heteroaryl.

In a preferred embodiment of the present invention, Cy1 is independently selected from the group consisting of 4- to 12-membered heterocyclyl and C₅₋₁₂ aryl.

In a further preferred embodiment, Cy1 is independently selected from the group consisting of 5- to 6-membered heterocyclyl and phenyl.

In a further preferred embodiment, Cy1 is independently selected from the group consisting of 5- to 6-membered heterocyclyl and phenyl, wherein the heteroatom in the heterocyclyl is N, and the number of the heteroatom is 1.

In a further preferred embodiment, Cy1 is independently selected from phenyl.

In a preferred embodiment of the present invention, Cy1 is independently selected from 5- to 6-membered heterocyclyl, wherein a heteroatom in the heterocyclyl is selected from the group consisting of N and S.

In a preferred embodiment of the present invention, Cy1 is selected from the group consisting of the following groups:

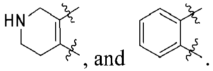

In a preferred embodiment of the present invention, 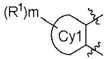 is independently selected from the group consisting of the following groups: 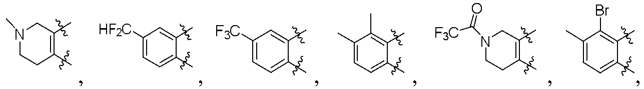 and

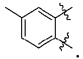

In a further preferred embodiment, 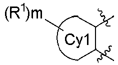 is selected from the group consisting of the following groups:

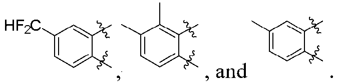

In a further preferred embodiment, 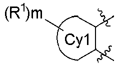 is selected from the group consisting of the following groups:

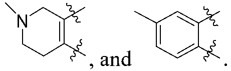

In a preferred embodiment of the present invention, R¹ is independently selected from the group consisting of hydrogen, halogen, oxo, hydroxyl, amino, nitro, cyano, -R^{a1}, -OR^{a1}, -SR^{a1}, -N(R^{a1})₂, -S(O)₂R^{a1}, -S(O)₂N(R^{a1})₂, - C(O)R^{a1}, -C(O)OR^{a1}, -C(O)N(R^{a1})₂, -C(O)OR^{a1}-OC(O)R^{a1}, and -N(R^{a1})C(O)R^{a1}.

In a further preferred embodiment, R¹ is independently selected from the group consisting of hydrogen, halogen, oxo, hydroxyl, amino, cyano, -R^{a1}, -C(O)R^{a1}, and -C(O)OR^{a1}.

In a preferred embodiment of the present invention, R^{a1} is independently selected from the group consisting of hydrogen, C₁₋₆ alkyl, and C₃₋₆ cycloalkyl, wherein each of the C₁₋₆ alkyl and the C₃₋₆ cycloalkyl is optionally substituted with one or more R^{a2}.

In a further preferred embodiment, R^{a1} is independently selected from C₁₋₃ alkyl, wherein the C₁₋₃ alkyl is optionally substituted with one or more R^{a2}.

In a further preferred embodiment, R^{a1} is independently selected from the group consisting of C₁₋₆ alkyl and C₁₋₆ haloalkyl.

In a further preferred embodiment, R^{a1} is independently selected from the group consisting of methyl, ethyl, n-propyl, and isopropyl.

In a preferred embodiment of the present application, R^{a2} is independently selected from the group consisting of deuterium, halogen, oxo, hydroxyl, amino, nitro, cyano, mercapto, C₁₋₄ alkyl, and C₁₋₄ alkoxy.

In a further preferred embodiment, R^{a2} is independently selected from the group consisting of halogen (e.g., fluorine, chlorine, bromine) and hydroxyl.

In a further preferred embodiment, R^{a2} is independently selected from halogen (e.g., fluorine, chlorine, bromine).

In a further preferred embodiment, R^{a2} is independently selected from the group consisting of fluorine and hydroxyl.

In a further preferred embodiment, R^{a2} is independently selected from fluorine.

In a preferred embodiment of the present invention, R¹ is independently selected from the group consisting of hydrogen, fluorine, chlorine, bromine, oxo, hydroxyl, amino, -R^{a1}, -OR^{a1}, -S(O)₂R^{a1}, -C(O)R^{a1}, -C(O)OR^{a1}, - C(O)N(R^{a1})₂, and -C(O)OR^{a1}-OC(O)R^{a1};

R^{a1} is independently selected from the group consisting of hydrogen, C₁₋₆ alkyl, and C₃₋₆ cycloalkyl, wherein each of the C₁₋₆ alkyl and the C₃₋₆ cycloalkyl is optionally substituted with one or more R^{a2};

R^{a2} is independently selected from the group consisting of fluorine, chlorine, bromine, oxo, hydroxyl, and amino.

In a preferred embodiment of the present application, R¹ is independently selected from the group consisting of hydrogen, halogen, oxo, C₁₋₄ alkyl, C₁₋₄ alkoxy, C₃₋₆ cycloalkyl, C₁₋₄ haloalkyl, C₁₋₄ alkyl optionally substituted with one or more hydroxyl groups, -S(O)₂(C₁₋₄ alkyl), -C(O)(C₁₋₄ alkyl), -C(O)(C₁₋₄ haloalkyl), -C(O)O(C₁₋₄ alkyl), -C(O)NH(C₁₋₄ alkyl), and -C(O)O-(C₁₋₄ alkyl)-OC(O)(C₁₋₄ alkyl).

In a further preferred embodiment, R¹ is independently selected from the group consisting of hydrogen, halogen, C₁₋₆ alkyl, C₁₋₆ alkoxy, C₁₋₆ haloalkyl, -C(O)(C₁₋₆ alkyl), -C(O)(C₁₋₆ haloalkyl), and -C(O)O(C₁₋₆ alkyl).

In a further preferred embodiment, R¹ is independently selected from the group consisting of hydrogen, fluorine, chlorine, bromine, C₁₋₃ alkyl, C₁₋₃ alkoxy, C₁₋₃ haloalkyl, -C(O)(C₁₋₃ alkyl), -C(O)(C₁₋₃ haloalkyl), and -C(O)O(C₁₋₃ alkyl).

In a further preferred embodiment, R¹ is independently selected from the group consisting of hydrogen, C₁₋₂ alkyl, C₁₋₂ haloalkyl, and -C(O)(C₁₋₂ haloalkyl).

In a further preferred embodiment, R¹ is independently selected from the group consisting of C₁₋₂ alkyl and C₁₋₂ haloalkyl.

In a further preferred embodiment, R¹ is independently selected from the group consisting of hydrogen, fluorine, chlorine, bromine, oxo, methyl, ethyl, n-propyl, isopropyl, n-butyl, methoxy, ethoxy, n-propoxy, isopropoxy, cyclopropyl, cyclobutyl, cyclopentyl, cyclohexyl, -CH₂F, -CF₂H, -CF₃, -CH₂CF₃, -C(O)CH₃, -C(O)CH₂CH₃, - S(O)₂CH₃, -CH₂OH, -C(O)CF₃, -C(O)CF₂H, -C(O)OCH₃, -C(O)OCH₂CH₃, -C(O)NHCH₃, and -C(O)O-CH(CH₃)-OC(O)CH(CH₃)₂.

In a further preferred embodiment, R¹ is independently selected from the group consisting of methyl and - CF₂H.

In a preferred embodiment of the present invention, R¹ is independently selected from C₁₋₄ alkyl.

In a further preferred embodiment, R¹ is independently selected from methyl.

In a preferred embodiment of the present invention, X¹ is selected from N.

In a preferred embodiment of the present invention, X is selected from N.

In a preferred embodiment of the present invention, X is selected from CR².

In a preferred embodiment of the present invention, X¹ is selected from N; and X is selected from N.

In a preferred embodiment of the present invention, R² is independently selected from the group consisting of halogen (e.g., fluorine, chlorine, bromine, preferably fluorine) and cyano.

In a preferred embodiment of the present invention, X is selected from the group consisting of CR² and N; R² is independently selected from the group consisting of hydrogen, fluorine, chlorine, bromine, hydroxyl, amino, cyano, and C₁₋₄ alkyl (e.g., methyl, ethyl, n-propyl, isopropyl, n-butyl).

In a further preferred embodiment, X is selected from CR²; and R² is independently selected from the group consisting of fluorine, cyano, and methyl.

In a preferred embodiment of the present invention, m is independently selected from 0, 1, 2, or 3.

In a further preferred embodiment, m is independently selected from 0, 1, or 2.

In a further preferred embodiment, m is independently selected from 1 or 2.

In a further preferred embodiment, m is independently selected from 1.

In a preferred embodiment of the present invention, Q is independently selected from the group consisting of -R³, -N(R³)-(C₀₋₆ alkylene)-R³, -N(R³)-(C₀₋₆ alkylene)-N(R³)₂, -N(R³)C(O)OR³, -N(R³)C(O)R³, -N(R³)C(O)N(R³)₂, and -N(R³)S(O)₂R³, wherein the alkylene is optionally substituted with one or more R^{c1}.

In a further preferred embodiment, Q is independently selected from the group consisting of -R³, -N(R³)-(C₀₋₆ alkylene)-R³, and -N(R³)-(C₀₋₆ alkylene)-N(R³)₂, wherein the alkylene is optionally substituted with one or more R^{c1}.

In a further preferred embodiment, Q is independently selected from the group consisting of -R³, -NH-(C₀₋₄ alkylene) -R³, and -NH-(C₀₋₄ alkylene)-NHR³, wherein the alkylene is optionally substituted with one or more R^{c1}.

In a further preferred embodiment, Q is independently selected from the group consisting of -R³, -NH-(C₁₋₄ alkylene) -R³, and -NH-(C₁₋₄ alkylene)-NHR³, wherein the alkylene is optionally substituted with one or more R^{c1}.

In a further preferred embodiment, Q is independently selected from the group consisting of -R³, -NH-(C₁₋₂ alkylene) -R³, and -NH-(C₁₋₄ alkylene)-NHR³, wherein the alkylene is optionally substituted with one or more R^{c1}.

In a preferred embodiment of the present invention, R³ is independently selected from the group consisting of hydrogen, C₁₋₆ alkyl, C₃₋₇ carbocyclyl, 3- to 7-membered heterocyclyl, phenyl, and 5- to 6-membered heteroaryl, wherein each of the C₁₋₆ alkyl, the C₃₋₇ carbocyclyl, the 3- to 7-membered heterocyclyl, the phenyl, and the 5- to 6-membered heteroaryl is optionally substituted with one or more R^{c2}; or
two R³s connected to a same nitrogen atom, together with the nitrogen atom to which they are connected, form 3- to 7-membered heterocyclyl or 5- to 6-membered heteroaryl, wherein each of the 3- to 7-membered heterocyclyl and the 5- to 6-membered heteroaryl is optionally substituted with one or more substituents selected from the group consisting of halogen, hydroxyl, amino, -C(O)NH₂, -(CH₂)₁₋₄NH₂, oxo, C₁₋₄ alkyl, C₁₋₄ alkoxy, oxetanylamino, and C₁₋₄ alkylpiperazinyl.

In a preferred embodiment of the present invention, R³ is independently selected from the group consisting of hydrogen, C₁₋₆ alkyl, C₃₋₁₂ carbocyclyl, and 3- to 12-membered heterocyclyl, wherein each of the C₁₋₆ alkyl, the C₃₋₁₂ carbocyclyl, and the 3- to 12-membered heterocyclyl is substituted with one or more R^{c2}.

In a further preferred embodiment, R³ is independently selected from the group consisting of hydrogen, C₁₋₆ alkyl, C₃₋₁₀ carbocyclyl, and 3- to 10-membered heterocyclyl, wherein each of the C₁₋₆ alkyl, the C₃₋₁₀ carbocyclyl, and the 3- to 10-membered heterocyclyl is substituted with one or more R^{c2}.

In a further preferred embodiment, R³ is independently selected from the group consisting of hydrogen, C₁₋₆ alkyl, C₃₋₆ carbocyclyl, and 4- to 10-membered heterocyclyl, wherein each of the C₁₋₆ alkyl, the C₃₋₆ carbocyclyl, and the 4- to 10-membered heterocyclyl is substituted with one or more R^{c2}.

In a further preferred embodiment, R³ is independently selected from the group consisting of hydrogen, C₁₋₆ alkyl, C₃₋₆ cycloalkyl, C₅₋₆ bridged cyclyl, 3- to 6-membered heterocycloalkyl, a 6- to 10-membered heterospirocyclyl, a 5- to 10-membered bridged heterocyclyl, and a 6- to 10-membered fused heterocyclyl, wherein each of the C₁₋₆ alkyl, the C₃₋₆ cycloalkyl, the C₅₋₆ bridged cyclyl, the 3- to 6-membered heterocycloalkyl, the 6- to 10-membered heterospirocyclyl, the 5- to 10-membered bridged heterocyclyl, and the 6- to 10-membered fused heterocyclyl is optionally substituted with one or more R^{c2}.

In a further preferred embodiment, R³ is independently selected from the group consisting of hydrogen, C₁₋₆ alkyl, C₃₋₆ cycloalkyl, C₅₋₆ bridged cyclyl, 3- to 6-membered heterocycloalkyl, 3-membered/5-membered heterospirocyclyl, 5-membered/3-membered heterospirocyclyl, 4-membered/4-membered heterospirocyclyl, 4-membered/5-membered heterospirocyclyl, 5-membered/4-membered heterospirocyclyl, 5-membered/5-membered heterospirocyclyl, 4-membered/6-membered heterospirocyclyl, 6-membered/4-membered heterospirocyclyl, 7- to 8-membered bridged heterocyclyl, 3-membered/5-membered fused heterocyclyl, 5-membered/3-membered fused heterocyclyl, 4-membered/4-membered fused heterocyclyl, 4-membered/5-membered fused heterocyclyl, 5-membered/4-membered fused heterocyclyl, 5-membered/5-membered fused heterocyclyl, 4-membered/6-membered fused heterocyclyl, 6-membered/4-membered fused heterocyclyl, 5-membered/6-membered fused heterocyclyl, and 6-membered/5-membered fused heterocyclyl, wherein each of the aforementioned groups is optionally substituted with one or more R^{c2}.

In a further preferred embodiment, R³ is independently selected from the group consisting of hydrogen, C₁₋₄ alkyl, C₃₋₆ cycloalkyl, C₅₋₆ bridged cyclyl, 4- to 6-membered heterocycloalkyl, 4-membered/4-membered heterospirocyclyl, 7- to 8-membered bridged heterocyclyl, 3-membered/5-membered fused heterocyclyl, 5-membered/3-membered fused heterocyclyl, and 5-membered/5-membered fused heterocyclyl, wherein each of the aforementioned groups is optionally substituted with one or more R^{c2}.

In a further preferred embodiment, R³ is independently selected from the group consisting of hydrogen, C₁₋₄ alkyl, C₃₋₆ cycloalkyl, 3- to 6-membered heterocycloalkyl, and 6- to 9-membered heterospirocyclyl, wherein each of the C₁₋₄ alkyl, the C₃₋₆ cycloalkyl, the 3- to 6-membered heterocycloalkyl, and the 6- to 9-membered heterospirocyclyl is optionally substituted with one or more R^{c2}.

In a further preferred embodiment, R³ is independently selected from the group consisting of hydrogen, C₁₋₄ alkyl, C₄₋₆ cycloalkyl, 4- to 6-membered heterocycloalkyl, and 7-membered heterospirocyclyl, wherein the heteroatom in the 4- to 6-membered heterocycloalkyl and the 7-membered heterospirocyclyl is N or O, and the number of the heteroatom is 1 or 2; each of the C₁₋₄ alkyl, the C₄₋₆ cycloalkyl, the 4- to 6-membered heterocycloalkyl, and the 7-membered heterospirocyclyl is optionally substituted with one or more R^{c2}.

In a further preferred embodiment, R³ is independently selected from the group consisting of hydrogen, C₁₋₄ alkyl, 4- to 6-membered heterocycloalkyl, and 7-membered heterospirocycloalkyl, wherein the heteroatom in the 4-to 6-membered heterocycloalkyl and the 7-membered heterospirocycloalkyl is N or O, and the number of the heteroatom is 1; and each of the C₁₋₄ alkyl, the 4- to 6-membered heterocycloalkyl, and the 7-membered heterospirocycloalkyl is optionally substituted with one or more R^{c2}.

In a further preferred embodiment, R³ is independently selected from the group consisting of hydrogen, methyl, ethyl, n-propyl, isopropyl, 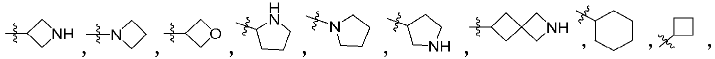 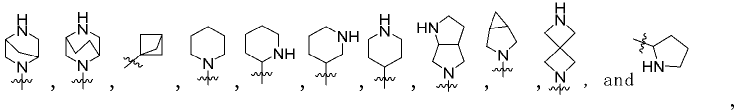 and each of the aforementioned groups is optionally substituted with one or more R^{c2}.

In a further preferred embodiment, R³ is independently selected from the group consisting of hydrogen, methyl, ethyl, n-propyl, isopropyl, 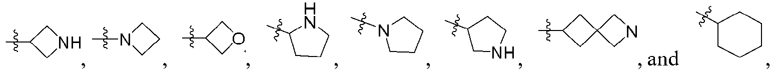 and each of the aforementioned groups is optionally substituted with one or more R^{c2}.

In a further preferred embodiment, R³ is independently selected from the group consisting of hydrogen, methyl, ethyl, n-propyl, isopropyl,

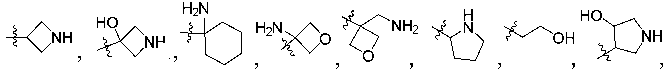

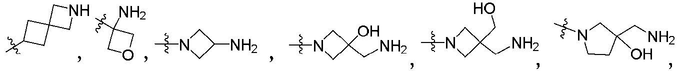

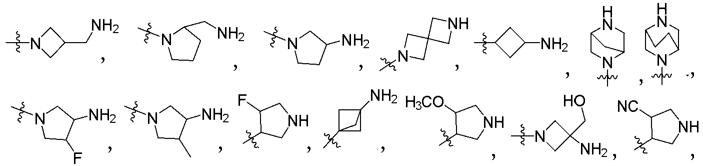

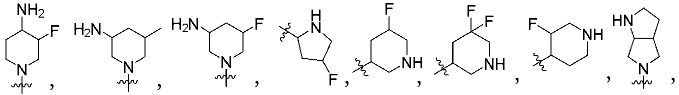

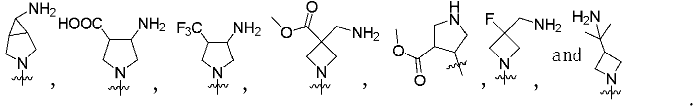

In a preferred embodiment of the present invention, R³ is independently selected from the group consisting of hydrogen, C₁₋₆ alkyl, C₃₋₆ carbocyclyl, and 4- to 10-membered heterocyclyl, wherein each of the C₁₋₆ alkyl, the C₃₋₆ carbocyclyl, and the 4- to 10-membered heterocyclyl is substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), and C₁₋₆ alkyl, wherein the C₁₋₆ alkyl is optionally substituted with one or more substituents selected from the group consisting of halogen, hydroxyl, and amino.

In a further preferred embodiment, R³ is independently selected from the group consisting of hydrogen, C₁₋₆ alkyl, C₃₋₆ cycloalkyl, C₅₋₆ bridged cyclyl, and 4- to 10-membered heterocyclyl, wherein each of the C₁₋₆ alkyl, the C₃₋₆ cycloalkyl, the C₅₋₆ bridged cyclyl, and the 4- to 10-membered heterocyclyl is substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), and C₁₋₆ alkyl, wherein the C₁₋₆ alkyl is optionally substituted with one or more substituents selected from the group consisting of halogen, hydroxyl, and amino.

In a further preferred embodiment, R³ is independently selected from the group consisting of hydrogen, C₁₋₄ alkyl, C₃₋₆ cycloalkyl, C₅₋₆ bridged cyclyl, 4- to 6-membered heterocycloalkyl, 4-membered/4-membered heterospirocyclyl, 7- to 8-membered bridged heterocyclyl, 3-membered/5-membered fused heterocyclyl, 5-membered/3-membered fused heterocyclyl, and 5-membered/5-membered fused heterocyclyl, and each of the aforementioned groups is optionally substituted with one or more R^{c2};
R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl.

In a preferred embodiment of the present invention, R^{c1} is independently selected from the group consisting of deuterium, halogen, hydroxyl, amino, nitro, cyano, mercapto, C₁₋₄ alkyl, and C₁₋₄ alkoxy.

In a further preferred embodiment, R^{c1} is independently selected from the group consisting of fluorine, chlorine, bromine, hydroxyl, amino, methyl, ethyl, n-propyl, and isopropyl.

In a further preferred embodiment, R^{c1} is independently selected from fluorine.

In a preferred embodiment of the present invention, R^{c2} is independently selected from the group consisting of deuterium, oxo, halogen, hydroxyl, amino, -OR^{c3}, -N(R^{c3})₂, -C(R^{c3})₂(OR^{c3}), -C(R^{c3})₂N(R^{c3})₂, -C(O)R^{c3}, -C(O)OR^{c3}, -C(O)N(R^{c3})₂, -C(O)N(R^{c3})OR^{c3}, -OC(O)R^{c3}, -OC(O)N(R^{c3})₂, -N(R^{c3})C(O)OR^{c3}, -N(R^{c3})C(O)R^{c3}, - N(R^{c3})C(O)N(R^{c3})₂, -N(R^{c3})S(O)₂R^{c3}, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more R^{c4}.

In a further preferred embodiment, R^{c2} is independently selected from the group consisting of halogen, hydroxyl, amino, cyano, -OR^{c3}, -C(O)OR^{c3}, and C₁₋₆ alkyl, wherein the C₁₋₆ alkyl is optionally substituted with one or more R^{c4}.

In a further preferred embodiment, R^{c2} is independently selected from the group consisting of halogen, hydroxyl, amino, and C₁₋₄ alkyl, wherein the C₁₋₄ alkyl is optionally substituted with one or more R^{c4}.

In a further preferred embodiment, R^{c2} is independently selected from the group consisting of halogen (e.g., fluorine, chlorine, bromine), hydroxyl, amino, methyl, ethyl, n-propyl, and isopropyl, and each of the aforementioned groups is optionally substituted with one or more substituents selected from the group consisting of halogen (e.g., fluorine, chlorine, bromine), hydroxyl, and amino.

In a preferred embodiment of the present invention, R^{c2} is independently selected from the group consisting of halogen, hydroxyl, amino, cyano, -O(C₁₋₆ alkyl), -C(O)OH, -C(O)O(C₁₋₆ alkyl), and C₁₋₆ alkyl, wherein the C₁₋₆ alkyl is optionally substituted with one or more substituents selected from the group consisting of halogen, hydroxyl, and amino.

In a further preferred embodiment, R^{c2} is independently selected from the group consisting of halogen, hydroxyl, amino, cyano, -O(C₁₋₃ alkyl), -C(O)OH, -C(O)O(C₁₋₃ alkyl), and C₁₋₃ alkyl, wherein the C₁₋₃ alkyl is optionally substituted with one or more substituents selected from the group consisting of halogen(e.g., fluorine, chlorine, bromine), hydroxyl, and amino.

In a further preferred embodiment, R^{c2} is independently selected from the group consisting of fluorine, chlorine, bromine, hydroxyl, amino, cyano, methoxy, ethoxy, -C(O)OH, -C(O)OCH₃, -C(O)OCH₂CH₃, -CH₂OH, - CH₂CH₂OH, trifluoromethyl, difluoromethyl, monofluoromethyl, -CH₂NH₂, -CH₂CH₂NH₂, and -C(CH₃)₂NH₂.

In a preferred embodiment of the present invention, each of R^{c3} and R^{c4} is independently selected from the group consisting of hydrogen, halogen, hydroxyl, amino, C₁₋₆ alkyl, C₁₋₆ haloalkyl, and C₁₋₆ alkyl optionally substituted with one or more hydroxyl groups.

In a preferred embodiment of the present invention, R^{c3} is independently selected from the group consisting of hydrogen, methyl, ethyl, n-propyl, and isopropyl.

In a preferred embodiment of the present invention, R^{c4} is independently selected from the group consisting of halogen, hydroxyl, and amino.

In a further preferred embodiment, R^{c4} is independently selected from the group consisting of fluorine, chlorine, bromine, hydroxyl, and amino.

In a further preferred embodiment, R^{c4} is independently selected from the group consisting of hydroxyl and amino.

In a preferred embodiment of the present invention, Q is independently selected from the group consisting of -R³, -N(R³)-(C₀₋₆ alkylene)-R³, and -N(R³)-(C₀₋₆ alkylene)-N(R³)₂, wherein the alkylene is optionally substituted with one or more substituents selected from the group consisting of halogen, hydroxyl, amino, and C₁₋₆ alkyl;
R³ is independently selected from the group consisting of hydrogen, C₁₋₆ alkyl, C₃₋₆ carbocyclyl, and 3- to 10-membered heterocyclyl, wherein each of the C₁₋₆ alkyl, the C₃₋₆ carbocyclyl, and the 3- to 10-membered heterocyclyl is optionally substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), and C₁₋₆ alkyl, wherein the C₁₋₆ alkyl is optionally substituted with one or more substituents selected from the group consisting of halogen, hydroxyl, and amino.

In a further preferred embodiment, Q is independently selected from the group consisting of 3- to 10-membered heterocyclyl, -NH-(C₀₋₄ alkylene)-(C₃₋₆ carbocyclyl), -NH-(C₀₋₄ alkylene)-(3- to 10-membered heterocyclyl), -NH-(C₀₋₄ alkylene)-NH₂, -NH-(C₀₋₄ alkylene)-NH(C₁₋₆ alkyl), and -NH-(C₀₋₄ alkylene)-N(C₁₋₆ alkyl)₂, wherein the alkylene is optionally substituted with one or more substituents selected from halogen (e.g., fluorine, chlorine, bromine); and each of the C₁₋₆ alkyl, the C₃₋₆ carbocyclyl, and the 3- to 10-membered heterocyclyl is optionally substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), C₁₋₆ alkyl, C₁₋₆ haloalkyl, hydroxyl-substituted C₁₋₆ alkyl, and amino-substituted C₁₋₆ alkyl.

In a further preferred embodiment, Q is independently selected from the group consisting of 3- to 6-membered heterocycloalkyl, 7- to 8-membered heterospirocyclyl, 7- to 8-membered bridged heterocyclyl, 6- to 10-membered fused heterocyclyl, -NH-(C₀₋₄ alkylene)-(C₃₋₆ cycloalkyl), -NH-(C₀₋₄ alkylene)-(C₅₋₆ bridged cyclyl), -NH-(C₀₋₄ alkylene)-(3- to 6-membered heterocyclyl), -NH-(C₀₋₄ alkylene)-(7- to 8-membered heterospirocyclyl), -NH-(C₀₋₄ alkylene)-NH₂, -NH-(C₀₋₄ alkylene)-NH(C₁₋₆ alkyl), and -NH-(C₀₋₄ alkylene)-N(C₁₋₄ alkyl)₂, wherein the alkylene is optionally substituted with one or more substituents selected from halogen (e.g., fluorine, chlorine, bromine); and each of the C₁₋₆ alkyl, the C₃₋₆ cycloalkyl, the 3- to 6-membered heterocycloalkyl, the 7- to 8-membered heterospirocyclyl, the 7- to 8-membered bridged heterocyclyl, the 6- to 10-membered fused heterocyclyl, and the C₅₋₆ bridged cyclyl is optionally substituted with one or more R^{c2};
R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl.

In a further preferred embodiment, Q is independently selected from the group consisting of 3- to 6-membered heterocycloalkyl, 7- to 8-membered heterospirocyclyl, 7- to 8-membered bridged heterocyclyl, 6- to 10-membered fused heterocyclyl, -NH-(C₀₋₂ alkylene)-(C₃₋₆ cycloalkyl), -NH-(C₀₋₂ alkylene)-(C₅₋₆ bridged cyclyl), -NH-(C₀₋₂ alkylene)-(3- to 6-membered heterocyclyl), and -NH-(C₀₋₂ alkylene)-(7- to 8-membered heterospirocyclyl), wherein each of the cycloalkyl, the heterocycloalkyl, the heterospirocyclyl, the bridged heterocyclyl, the fused heterocyclyl, and the bridged cyclyl is optionally substituted with one or more R^{c2}; the heteroatom in the 3- to 6-membered heterocycloalkyl, the 7- to 8-membered heterospirocyclyl, the 7- to 8-membered bridged heterocyclyl, and the 6- to 10-membered fused heterocyclyl is N or O, and the number of the heteroatom is 1 or 2, and 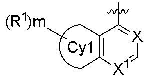 is connected to a N atom in Q;

R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl.
In a preferred embodiment of the present invention, Q is independently selected from the group consisting of -R³, -N(R³)-(C₀₋₆ alkylene)-R³, and -N(R³)-(C₀₋₆ alkylene)-N(R³)₂, wherein the alkylene is optionally substituted with one or more substituents selected from the group consisting of halogen, hydroxyl, amino, and C₁₋₆ alkyl;
R³ is independently selected from the group consisting of hydrogen, C₁₋₆ alkyl, C₃₋₁₀ carbocyclyl, and 3- to 10-membered heterocyclyl, wherein each of the C₁₋₆ alkyl, the C₃₋₁₀ carbocyclyl, and the 3- to 10-membered heterocyclyl is optionally substituted with one or more R^{c2};
R^{c2} is independently selected from the group consisting of halogen, hydroxyl, amino, and C₁₋₆ alkyl, wherein the C₁₋₆ alkyl is optionally substituted with one or more substituents selected from the group consisting of halogen, hydroxyl, and amino.

In a further preferred embodiment, Q is independently selected from the group consisting of C₃₋₆ cycloalkyl, 3- to 6-membered heterocyclyl, -NH-(C₀₋₆ alkylene)-R³, and -NH-(C₀₋₆ alkylene)-N(R³)₂, wherein the alkylene is optionally substituted with one or more substituents selected from the group consisting of halogen, hydroxyl, amino, methyl, and ethyl;
R³ is independently selected from the group consisting of hydrogen, C₁₋₆ alkyl, C₃₋₆ cycloalkyl, 3- to 6-membered heterocyclyl, and 6- to 9-membered heterospirocyclyl, wherein each of the C₁₋₆ alkyl, the C₃₋₆ cycloalkyl, the 3- to 6-membered heterocyclyl, and the 6- to 9-membered heterospirocyclyl is optionally substituted with one or more R^{c2};

R^{c2} is independently selected from the group consisting of halogen, hydroxyl, amino, C₁₋₆ alkyl, amino-substituted C₁₋₆ alkyl, and hydroxyl-substituted C₁₋₆ alkyl.

In a further preferred embodiment, Q is independently selected from the group consisting of C₃₋₆ cycloalkyl, 3- to 6-membered heterocyclyl, -NH-(C₀₋₄ alkylene)-(C₃₋₆ cycloalkyl), -NH-(C₀₋₄ alkylene)-(3- to 6-membered heterocyclyl), -NH-(C₀₋₄ alkylene)-(7-membered heterospirocyclyl), -NH-(C₀₋₄ alkylene)-NH₂, -NH-(C₀₋₄ alkylene)-NH(C₁₋₄ alkyl), and -NH-(C₀₋₄ alkylene)-N(C₁₋₄ alkyl)₂, wherein the alkylene is optionally substituted with one or more substituents selected from halogen (e.g., fluorine, chlorine, bromine); and each of the C₁₋₄ alkyl, the C₃₋₆ cycloalkyl, the 3- to 6-membered heterocyclyl, and the 7-membered heterospirocyclyl is optionally substituted with one or more substituents selected from the group consisting of halogen (e.g., fluorine, chlorine, bromine), hydroxyl, amino, C₁₋₄ alkyl, amino-substituted C₁₋₄ alkyl, and hydroxyl-substituted C₁₋₄ alkyl.

In a preferred embodiment of the present invention, Q is independently selected from the group consisting of 3- to 10-membered nitrogen-containing heterocyclyl (preferably 3- to 8-membered nitrogen-containing heterocyclyl), -NH-(C₀₋₂ alkylene)-(C₃₋₆ cycloalkyl)-NH₂, and -NH-(C₀₋₂ alkylene)-(3- to 6-membered heterocyclyl); the 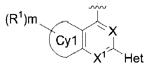 is connected to a nitrogen atom in the 3- to 10-membered nitrogen-containing heterocyclyl, where " 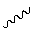 " donates a connecting site; the heteroatom in the 3- to 6-membered heterocyclyl is N or O, and the number of the heteroatom is 1 or 2; each of the 3- to 10-membered nitrogen-containing heterocyclyl, the C₃₋₆ cycloalkyl, and the 3- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl).

In a preferred embodiment of the present invention, Q is independently selected from the group consisting of 3- to 7-membered nitrogen-containing heterocyclyl (more preferably, 4- to 5-membered nitrogen-containing heterocyclyl), -NH-(C₀₋₂ alkylene)-(C₃₋₆ cycloalkyl)-NH₂ (more preferably, -NH-(C₀₋₁ alkylene)-(C₄₋₅ cycloalkyl)-NH₂), and -NH-(C₀₋₂ alkylene)-(3- to 6-membered heterocyclyl) (more preferably, -NH-(C₀₋₁ alkylene)-(4- to 5-membered heterocyclyl)); the 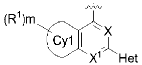 is connected to a nitrogen atom in the 3- to 7-membered nitrogen-containing heterocyclyl, where " 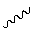 " donates a connecting site; the heteroatom in the 3- to 6-membered heterocyclyl is N or O, and the number of the heteroatom is 1 or 2;each of the 3- to 7-membered nitrogen-containing heterocyclyl, the C₃₋₆ cycloalkyl, and the 3- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl).

In a further preferred embodiment, Q is independently selected from the group consisting of 4- to 6-membered nitrogen-containing heterocyclyl (more preferably, 4- to 5-membered nitrogen-containing heterocyclyl), -NH-(C₄₋₅ cycloalkyl)-NH₂ (more preferably, -NH-cyclobutyl-NH₂), and -NH-(C₀₋₂ alkylene)-(4- to 6-membered heterocyclyl) (more preferably, -NH-(C₀₋₁ alkylene)-(4- to 5-membered heterocyclyl)); the 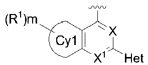 is connected to a nitrogen atom in the 4- to 6-membered nitrogen-containing heterocyclyl, where " 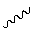 " donates a connecting site; the heteroatom in the 4- to 6-membered heterocyclyl is N or O, and the number of the heteroatom is 1 or 2; each of the 4- to 6-membered nitrogen-containing heterocyclyl and the 4- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl).

In a further preferred embodiment, Q is independently selected from the group consisting of 4- to 6-membered nitrogen-containing heterocyclyl (more preferably, 4- to 5-membered nitrogen-containing heterocyclyl), -NH-(C₄₋₅ cycloalkyl)-NH₂ (more preferably, -NH-cyclobutyl-NH₂), and -NH-(C₀₋₂ alkylene)-(4- to 6-membered heterocyclyl) (more preferably, -NH-(C₀₋₁ alkylene)-(4- to 5-membered heterocyclyl)); the 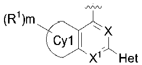 is connected to a nitrogen atom in the 4- to 6-membered nitrogen-containing heterocyclyl, where " 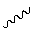 " donates a connecting site; the heteroatom in the 4- to 6-membered heterocyclyl is N or O, and the number of the heteroatom is 1; each of the 4-to 6-membered nitrogen-containing heterocyclyl and the 4- to 6-membered heterocyclyl is optionally substituted with one or more substituents selected from the group consisting of fluorine, chlorine, hydroxyl, amino, -CH₂NH₂, -CH₂OH, and -C(O)OCH₃.

In a preferred embodiment of the present invention, Q is independently selected from the group consisting of 4- to 5-membered nitrogen-containing heterocyclyl, -NH-(C₄₋₅ cycloalkyl)-NH₂, and -NH-(C₀₋₁ alkylene)-(4- to 5-membered heterocyclyl); the 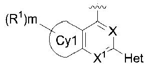 is connected to a nitrogen atom in the 4- to 5-membered nitrogen-containing heterocyclyl, where " 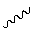 " donates a connecting site; the heteroatom in the 4- to 5-membered heterocyclyl is N or O, and the number of the heteroatom is 1; each of the 4- to 5-membered nitrogen-containing heterocyclyl and the 4- to 5-membered heterocyclyl is optionally substituted with one or more substituents selected from the group consisting of fluorine, hydroxyl, amino, -CH₂NH₂, -CH₂OH, and -C(O)OCH₃.

In a preferred embodiment of the present invention, Q is independently selected from the group consisting of 4- to 6-membered nitrogen-containing heterocyclyl, and -NH-(C₀₋₁ alkylene)-(4- to 5-membered heterocyclyl)); the 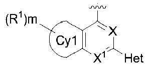 is connected to a nitrogen atom in the 4- to 6-membered nitrogen-containing heterocyclyl, where " 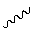 " donoates a connecting site; the heteroatom in the 4- to 5-membered heterocyclyl is N or O, and the number of the heteroatom is 1; each of the 4- to 6-membered nitrogen-containing heterocyclyl and the 4- to 5-membered heterocyclyl is optionally substituted with one or two substituents selected from the group consisting of fluorine, amino, and -CH₂NH₂.

In a preferred embodiment, Q is selected from the group consisting of the following groups:

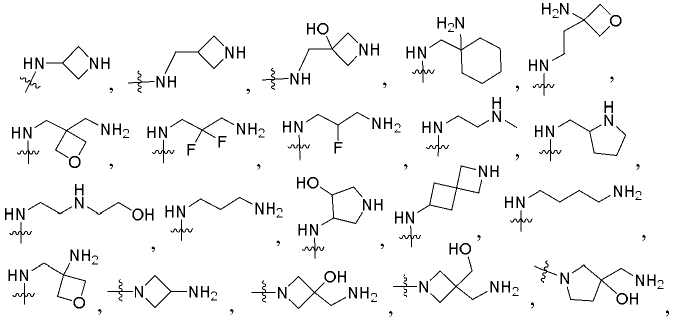

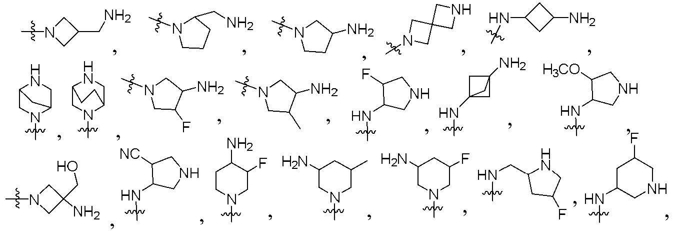

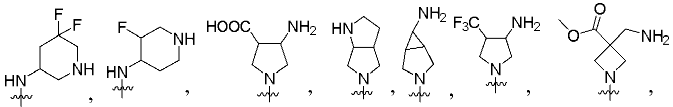

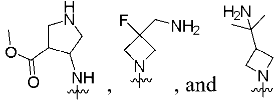

In a further preferred embodiment, Q is selected from the group consisting of

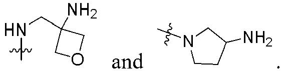

In a preferred embodiment of the present invention, Het is selected from the following structure:

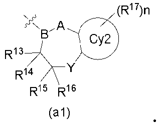

In a further preferred embodiment, Het is selected from 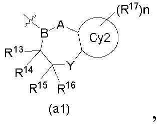 wherein B is -N-; and A is -CH₂-.

In a further preferred embodiment, Het is selected from the following structure: 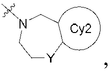 wherein Cy2 is selected from the group consisting of phenyl and pyridyl.

In a preferred embodiment of the present invention, when A or B is connected to 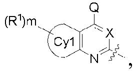 A or B is independently selected from -N-; when A or B is not connected to 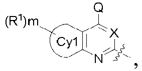 A or B is independently selected from -C(R^{d1})₂-.

In a further preferred embodiment, when A or B is connected to 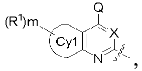 A or B is independently selected from -N-; when A or B is not connected to 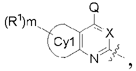 A or B is independently selected from -CH₂-.

In a preferred embodiment of the present invention, R^{d1} is independently selected from the group consisting of hydrogen, hydroxyl, amino, cyano, methyl, and ethyl.

In a further preferred embodiment, R^{d1} is independently selected from hydrogen.

In a preferred embodiment of the present invention, Y is independently selected from the group consisting of - O-, -N(R^{d2})-, -S(O)-, -S(O)₂-, and -S(O)(=N-R^{d2})-.

In a further preferred embodiment, Y is independently selected from the group consisting of -S(O)-, -S(O)₂-, and -S(O)(=N-R^{d2})-.

In a further preferred embodiment, Y is independently selected from the group consisting of -S(O)₂- and - S(O)(=N-R^{d2})-.

In a further preferred embodiment, Y is independently selected from -S(O)₂-.

In a further preferred embodiment, Y is independently selected from -S(O)(=N-R^{d2})-.

In a preferred embodiment of the present invention, R^{d2} is independently selected from the group consisting of hydrogen, deuterium, halogen, hydroxyl, amino, nitro, cyano, -SR^{d3}, -N(R^{d3})₂, -C(O)R^{d3}, -(C₁₋₆ alkylene)-C(O)R^{d3}, -C(O)OR^{d3}, -(C₁₋₆ alkylene)-C(O)OR^{d3}, -C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, -C(O)N(R^{d3})OR^{d3}, -OC(O)R^{d3}, -OC(O)N(R^{d3})₂ C₁₋₆ alkyl, C₃₋₁₀ carbocyclyl, 3- to 10-membered heterocyclyl, C₆₋₈ aryl, and 5- to 10-membered heteroaryl, wherein each of the C₁₋₆ alkyl, the C₁₋₆ alkylene, the C₃₋₁₀ carbocyclyl, the 3- to 10-membered heterocyclyl, the C₆₋₈ aryl, and the 5- to 10-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy.

In a further preferred embodiment, R^{d2} is independently selected from the group consisting of hydrogen, deuterium, halogen, hydroxyl, amino, nitro, cyano, -SR^{d3}, -N(R^{d3})₂, -C(O)R^{d3}, -(C₁₋₄ alkylene)-C(O)R^{d3}, -C(O)OR^{d3}, -(C₁₋₄ alkylene)-C(O)OR^{d3}, -C(O)N(R^{d3})₂, -(C₁₋₄ alkylene)-C(O)N(R^{d3})₂, -C(O)-(C₁₋₄ alkylene)-N(R^{d3})₂, -S(O)₂R^{d3}, C₁₋₄ alkyl, C₃₋₆ carbocyclyl, 3- to 6-membered heterocyclyl, phenyl, and 5- to 6-membered heteroaryl, wherein each of the C₁₋₄ alkyl, the C₁₋₄ alkylene, the C₃₋₆ carbocyclyl, the 3- to 6-membered heterocyclyl, the phenyl, and the 5-to 6-membered heteroaryl is optionally substituted with one or more substituents selected from the group consisting of deuterium, halogen, oxo, hydroxyl, amino, nitro, cyano, C₁₋₄ alkyl, and C₁₋₄ alkoxy.

In a further preferred embodiment, R^{d2} is independently selected from the group consisting of hydrogen, -SR^{d3}, -C(O)R^{d3}, -(C₁₋₄ alkylene)-C(O)R^{d3}, -(C₁₋₄ alkylene)-S(O)₂R^{d3}, -(C₁₋₄ alkylene)-C(O)OR^{d3}, -C(O)N(R^{d3})₂, -(C₁₋₄ alkylene)-C(O)N(R^{d3})₂, -C(O)-(C₁₋₄ alkylene)-N(R^{d3})₂, -S(O)₂R^{d3}, C₁₋₆ alkyl, C₃₋₆ cycloalkyl, 3- to 6-membered monocyclic heterocyclyl, 7- to 8-membered heterospirocyclyl, phenyl, and 5- to 6-membered heteroaryl, wherein the 3- to 6-membered monocyclic heterocyclyl, the 7- to 8-membered heterospirocyclyl, and the heteroatom in the 5- or 6-membered heteroaryl is N or O, and the number of the heteroatom is 1 or 2; each of the C₁₋₆ alkyl, the C₁₋₄ alkylene, the C₃₋₆ cycloalkyl, the 3- to 6-membered monocyclic heterocyclyl, the 7- to 8-membered heterospirocyclyl, the phenyl, and the 5- to 6-membered heteroaryl is optionally substituted with one or more substituents selected from the group consisting of deuterium, halogen (e.g., fluorine, chlorine, bromine, preferably fluorine), oxo, hydroxyl, amino, cyano, C₁₋₃ alkyl, C₁₋₃ alkoxy, C₃₋₆ cycloalkyl, and 3- to 6-membered heterocyclyl.

In a further preferred embodiment, R^{d2} is independently selected from the group consisting of hydrogen, -SR^{d3}, -C(O)R^{d3}, -(C₁₋₄ alkylene )-C(O)Rd³, -(C₁₋₄ alkylene)-C(O)OR^{d3}, -C(O)N(R^{d3})₂, -(C₁₋₄ alkylene)-C(O)N(R^{d3})₂, - C(O)-(C₁₋₄ alkylene)-N(R^{d3})₂, -S(O)₂R^{d3}, C₁₋₄ alkyl, C₃₋₆ cycloalkyl, 3- to 6-membered heterocyclyl, phenyl, and 5-to 6-membered heteroaryl, wherein the heteroatom in the 3- to 6-membered heterocyclyl and the 5- to 6-membered heteroaryl is N or O, and the number of the heteroatom is 1 or 2; each of the C₁₋₄ alkyl, the C₁₋₄ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen (e.g., fluorine, chlorine, bromine, preferably fluorine), oxo, hydroxyl, amino, cyano, methyl, ethyl, n-propyl, isopropyl, n-butyl, methoxy, ethoxy, n-propoxy, and isopropoxy.

In a preferred embodiment of the present invention, R^{d3} is independently selected from the group consisting of hydrogen, deuterium, halogen, hydroxyl, amino, nitro, cyano, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy.

In a further preferred embodiment, R^{d3} is independently selected from the group consisting of hydrogen, halogen, hydroxyl, amino, nitro, cyano, C₁₋₄ alkyl, C₃₋₆ cycloalkyl, and 3- to 6-membered heterocyclyl, wherein each of the C₁₋₄ alkyl, the C₃₋₆ cycloalkyl, and the 3- to 6-membered heterocyclyl is optionally substituted with one or more substituents selected from the group consisting of halogen, oxo, hydroxyl, amino, nitro, cyano, C₁₋₄ alkyl, and C₁₋₄ alkoxy.

In a further preferred embodiment, R^{d3} is independently selected from the group consisting of hydrogen, amino, C₁₋₆ alkyl, and C₃₋₆ cycloalkyl, wherein each of the C₁₋₆ alkyl and the C₃₋₆ cycloalkyl is optionally substituted with one or more substituents selected from the group consisting of halogen and hydroxyl.

In a further preferred embodiment, R^{d3} is independently selected from the group consisting of hydrogen, fluorine, chlorine, bromine, hydroxyl, amino, cyano, methyl, ethyl, n-propyl, isopropyl, n-butyl, cyclopropyl, cyclobutyl, cyclopentyl, and cyclobutyl, and each of the aforementioned groups is optionally substituted with one or more substituents selected from the group consisting of fluorine, chlorine, bromine, hydroxyl, and amino.

In a preferred embodiment of the present invention, R^{d2} is independently selected from the group consisting of hydrogen, -SR^{d3}, -C(O)R^{d3}, -(C₁₋₆ alkylene)-C(O)R^{d3}, -C(O)OR^{d3}, -(C₁₋₆ alkylene)-C(O)OR^{d3}, -C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₁₋₆ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, and C₁₋₆ alkoxy;

R^{d3} is independently selected from the group consisting of hydrogen, halogen, hydroxyl, amino, cyano, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, and C₁₋₆ alkoxy.

In a preferred embodiment of the present invention, R^{d2} is hydrogen.

In a preferred embodiment of the present invention, R^{d2} is independently selected from the group consisting of -S(C₁₋₆ alkyl), -C(O)NH₂, -C(O)NH(C₁₋₆ alkyl), -C(O)N(C₁₋₆ alkyl)₂, -C(O)(C₁₋₆ alkyl), -C(O)(C₃₋₆ cycloalkyl), - C(O)(3- to 6-membered heterocyclyl), -C(O)-(C₁₋₄ alkylene)-NH(C₁₋₆ alkyl), -C(O)-(C₁₋₄ alkylene)-N(C₁₋₆ alkyl)₂, -(C₁₋₄ alkylene)-C(O)O(C₁₋₆ alkyl), -(C₁₋₄ alkylene)-C(O)(C₁₋₆ alkyl), -(C₁₋₄ alkylene)-S(O)₂(C₁₋₆ alkyl), -(C₁₋₄ alkylene)-C(O)NH₂, -(C₁₋₄ alkylene)-C(O)NH(C₁₋₆ alkyl), -(C₁₋₄ alkylene)-C(O)N(C₁₋₆ alkyl)₂, -S(O)₂(C₁₋₆ alkyl), C₁₋₆ alkyl, C₃₋₆ cycloalkyl, 3- to 8-membered heterocyclyl, phenyl, and 5- to 6-membered heteroaryl, wherein each of the C₁₋₆ alkyl, the C₁₋₄ alkylene, the C₃₋₆ cycloalkyl, the 3- to 8-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, C₁₋₆ alkoxy, C₃₋₆ cycloalkyl, and 3- to 6-membered heterocyclyl.

In a preferred embodiment of the present invention, R^{d2} is independently selected from the group consisting of -S(C₁₋₃ alkyl), -C(O)NH₂, -C(O)NH(C₁₋₃ alkyl), -C(O)N(C₁₋₃ alkyl)₂, -C(O)(C₁₋₃ alkyl), -C(O)(C₃₋₆ cycloalkyl), - C(O)(3- to 6-membered heterocyclyl), -C(O)-(C₁₋₂ alkylene)-NH(C₁₋₆ alkyl), -C(O)-(C₁₋₂ alkylene)-N(C₁₋₃ alkyl)₂, -(C₁₋₂ alkylene)-C(O)O(C₁₋₃ alkyl), -(C₁₋₂ alkylene)-C(O)(C₁₋₃ alkyl), -(C₁₋₂ alkylene)-S(O)₂(C₁₋₃ alkyl), -(C₁₋₂ alkylene)-C(O)NH₂, -(C₁₋₂ alkylene)-C(O)NH(C₁₋₃ alkyl), -(C₁₋₂ alkylene)-C(O)N(C₁₋₃ alkyl)₂, -S(O)₂(C₁₋₃ alkyl), C₁₋₆ alkyl, C₃₋₆ cycloalkyl, 3- to 6-membered monocyclic heterocyclyl, 7-membered heterospirocyclyl, 8-membered heterospirocyclyl, phenyl, and 5- to 6-membered heteroaryl, wherein each of the alkyl, the alkylene, the C₃₋₆ cycloalkyl, the 3- to 6-membered monocyclic heterocyclyl, the 7-membered heterospirocyclyl, the 8-membered heterospirocyclyl, the phenyl, and the 5- to 6-membered heteroaryl is optionally substituted with one or more substituents selected from the group consisting of deuterium, halogen, hydroxyl, amino, cyano, C₁₋₃ alkyl, C₁₋₃ alkoxy, and 3- to 6-membered heterocyclyl.

In a further preferred embodiment, R^{d2} is independently selected from the group consisting of -S(C₁₋₃ alkyl), - C(O)NH₂, -C(O)NH(C₁₋₃ alkyl), -C(O)N(C₁₋₃ alkyl)₂, -C(O)(C₁₋₃ alkyl), -C(O)(C₃₋₆ cycloalkyl), -C(O)-(C₁₋₂ alkylene)-NH(C₁₋₃ alkyl), -C(O)-(C₁₋₂ alkylene)-N(C₁₋₃ alkyl)₂, -(C₁₋₂ alkylene)-C(O)O(C₁₋₃ alkyl), -(C₁₋₂ alkylene)-C(O)(C₁₋₃ alkyl), -(C₁₋₂ alkylene)-S(O)₂(C₁₋₃ alkyl), -(C₁₋₂ alkylene)-C(O)NH₂, -(C₁₋₂ alkylene)-C(O)NH(C₁₋₃ alkyl), -(C₁₋₂ alkylene)-C(O)N(C₁₋₃ alkyl)₂, -S(O)₂(C₁₋₃ alkyl), C₁₋₆ alkyl, C₃₋₆ cycloalkyl, 3- to 6-membered monocyclic heterocyclyl, 7-membered heterospirocyclyl (e.g., 4-membered/4-membered heterospirocyclyl), phenyl, and 5- to 6-membered heteroaryl, wherein the heteroatom in the monocyclic heterocyclyl, the heterospirocyclyl, and the heteroaryl is O or N, and the number of the heteroatom is 1 or 2;each of the alkyl, the alkylene, the cycloalkyl, the monocyclic heterocyclyl, the heterospirocyclyl, the phenyl, and the heteroaryl is optionally substituted with one or more substituents selected from the group consisting of deuterium, fluorine, chlorine, bromine, hydroxyl, amino, cyano, methyl, ethyl, methoxy, ethoxy, cyclopropyl, cyclobutyl, oxiranyl, and oxetanyl.

In a further preferred embodiment, R^{d2} is independently selected from the group consisting of hydrogen, -S(C₁₋₄ alkyl), -C(O)NH₂, -C(O)NH(C₁₋₄ alkyl), -C(O)N(C₁₋₄ alkyl)₂, -C(O)(C₁₋₄ alkyl), -C(O)(C₃₋₆ cycloalkyl), -C(O)(3-to 6-membered heterocyclyl), -C(O)-(C₁₋₄ alkylene)-NH(C₁₋₄ alkyl), -C(O)-(C₁₋₄ alkylene)-N(C₁₋₄ alkyl)₂, -(C₁₋₄ alkylene)-C(O)O(C₁₋₄ alkyl), -(C₁₋₄ alkylene)-C(O)(C₁₋₄ alkyl), -(C₁₋₄ alkylene)-C(O)NH₂, -(C₁₋₄ alkylene)-C(O)NH(C₁₋₄ alkyl), -(C₁₋₄ alkylene)-C(O)N(C₁₋₄ alkyl)₂, -S(O)₂(C₁₋₄ alkyl), C₁₋₄ alkyl, C₃₋₆ cycloalkyl, 3- to 6-membered heterocyclyl, phenyl, and 5- to 6-membered heteroaryl, wherein each of the C₁₋₄ alkyl, the C₁₋₄ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, fluorine, chlorine, bromine, hydroxyl, amino, cyano, methyl, ethyl, n-propyl, isopropyl, and n-butyl.

In a further preferred embodiment, R^{d2} is independently selected from C₁₋₃ alkyl, wherein the C₁₋₃ alkyl is optionally substituted with one or more substituents selected from the group consisting of deuterium, fluorine, chlorine, bromine, hydroxyl, amino, cyano, methyl, ethyl, n-propyl, isopropyl, n-butyl, and 3- to 6-membered heterocyclyl.

In a further preferred embodiment, R^{d2} is independently selected from C₁₋₃ alkyl, wherein the C₁₋₃ alkyl is optionally substituted with one or more substituents selected from 3- to 6-membered heterocyclyl; preferably, the 3- to 6-membered heterocyclyl contains 1, 2 or more ring atoms selected from the group consisting of N, O and S; preferably, the 3- to 6-membered heterocyclyl is selected from the group consisting of oxetanyl, thietanyl, and tetrahydrofuranyl.

In a preferred embodiment of the present invention, R^{d2} is independently selected from the group consisting of hydrogen, -C(O)NH₂, -C(O)NH(C₁₋₃ alkyl), -C(O)(C₁₋₃ alkyl), -C(O)(C₃₋₆ cycloalkyl), -(C₁₋₃ alkylene)-S(O)₂(C₁₋₃ alkyl), C₁₋₆ alkyl (more preferably C₁₋₅ alkyl, more preferably C₁₋₃ alkyl), C₃₋₆ cycloalkyl (more preferably C₃₋₄ cycloalkyl), 3- to 7-membered heterocyclyl (more preferably 4- to 5-membered heterocyclyl), and 5- to 6-membered heteroaryl (more preferably 5-membered heteroaryl containing 2 nitrogen atoms), wherein each of the alkyl, the cycloalkyl, the heterocyclyl, and the heteroaryl is optionally substituted with one or more substituents selected from the group consisting of deuterium, halogen (more preferably fluorine, chlorine, bromine, more preferably fluoro), hydroxyl, amino, cyano, C₁₋₃ alkyl (more preferably methyl), C₁₋₃ alkoxy (more preferably methoxy), and 3- to 6-membered heterocyclyl (more preferably 4- to 5-membered oxygen-containing heterocyclyl); and the heteroatom in the heterocyclyl and the heteroaryl is O or N, and the number of the heteroatom is 1 or 2.

In a preferred embodiment of the present invention, R^{d2} is independently selected from the group consisting of hydrogen, -C(O)NH₂, -C(O)NH(C₁₋₂ alkyl), -C(O)(C₁₋₂ alkyl), -C(O)(C₃₋₄ cycloalkyl), -(C₁₋₂ alkylene)-S(O)₂(C₁₋₂ alkyl), C₁₋₆ alkyl (more preferably C₁₋₅ alkyl, more preferably C₁₋₃ alkyl), -(C₁₋₃ alkylene)-(C₁₋₂ alkoxy), -(C₁₋₂ alkylene)-(3- to 6-membered heterocyclyl) (more preferably -(C₁₋₂ alkylene)-(4-5 membered heterocyclyl)), C₃₋₆ cycloalkyl (more preferably C₃₋₄ cycloalkyl), 4- to 6-membered heterocyclyl (more preferably 4- to 5-membered heterocyclyl), and 5- to 6-membered heteroaryl (more preferably 5-membered heteroaryl containing 2 nitrogen atoms), wherein the alkyl is optionally substituted with one or more substituents selected from the group consisting of deuterium, halogen (more preferably fluorine, chlorine, bromine, more preferably fluorine), hydroxyl, and cyano; the cycloalkyl is optionally substituted with one or more substituents selected from halogen (more preferably fluorine, chlorine, bromine, more preferably fluorine); the heteroaryl is optionally substituted with one or more substituents selected from C₁₋₂ alkyl (preferably methyl); and the heteroatom in the heterocyclyl and the heteroaryl is O or N, and the number of the heteroatom is 1 or 2.

In a preferred embodiment of the present invention, R^{d2} is independently selected from the group consisting of -(C₁₋₃ alkylene)-S(O)₂(C₁₋₃ alkyl), C₁₋₆ alkyl (more preferably C₁₋₅ alkyl, more preferably C₁₋₃ alkyl), C₃₋₆ cycloalkyl (more preferably C₃₋₄ cycloalkyl), and 3- to 6-membered heterocyclyl (more preferably 4- to 5-membered heterocyclyl); wherein each of the alkyl, the cycloalkyl, and the heterocyclyl is optionally substituted with one or more substituents selected from the group consisting of deuterium, halogen (more preferably fluorine, chlorine, bromine, more preferably fluorine), hydroxyl, cyano, C₁₋₃ alkyl (more preferably methyl), C₁₋₃ alkoxy (more preferably methoxy), and 3- to 6-membered heterocyclyl (more preferably 4- to 5-membered heterocyclyl); and the heteroatom in the heterocyclyl is O, and the number of the heteroatom is 1.

In a preferred embodiment of the present invention, R^{d2} is independently selected from the group consisting of -(C₁₋₂ alkylene)-S(O)₂(C₁₋₂ alkyl), C₁₋₆ alkyl (more preferably C₁₋₅ alkyl, more preferably C₁₋₃ alkyl), -(C₁₋₃ alkylene)-(C₁₋₂ alkoxy), -(C₁₋₂ alkylene)-(3- to 6-membered heterocyclyl) (more preferably -(C₁₋₂ alkylene)-(4- to 5-membered heterocyclyl)), C₃₋₆ cycloalkyl (more preferably C₃₋₄ cycloalkyl), and 4- to 6-membered heterocyclyl (more preferably 4- to 5-membered heterocyclyl), wherein the alkyl is optionally substituted with one or more substituents selected from the group consisting of deuterium, halogen (more preferably fluorine, chlorine, bromine, more preferably fluorine), hydroxyl, and cyano; and the heteroatom in the heterocyclyl is O, and the number of the heteroatom is 1.

In a preferred embodiment of the present invention, R^{d2} is independently selected from the group consisting of -(C₁₋₂ alkylene)-(3- to 6-membered heterocyclyl) (more preferably -(C₁₋₂ alkylene)-(4- to 5-membered heterocyclyl)), C₃₋₆ cycloalkyl (more preferably C₃₋₄ cycloalkyl), and 3- to 6-membered heterocyclyl (more preferably 4- to 5-membered heterocyclyl), wherein the heteroatom in the heterocyclyl is O, and the number of the heteroatom is 1.

In a preferred embodiment of the present invention, R^{d2} is independently selected from the group consisting of -(C₁₋₂ alkylene)-S(O)₂(C₁₋₂ alkyl), C₁₋₃ alkyl (more preferably C₁₋₂ alkyl), -(C₁₋₂ alkylene)-(3- to 6-membered heterocyclyl) (more preferably -methyl-(4- to 5-membered heterocyclyl)), and C₃₋₆ cycloalkyl (more preferably C₃₋₄ cycloalkyl), wherein the alkyl is optionally substituted with one or more substituents selected from the group consisting of deuterium, halogen (more preferably fluorine), and cyano; and the heteroatom in the heterocyclyl is O, and the number of the heteroatom is 1.

In a further preferred embodiment, R^{d2} is independently selected from the group consisting of - CH₂CH₂SO₂CH₃, C₁₋₂ alkyl, -CD₃, -methyl-(4- to 5-membered heterocyclyl), cyclopropyl, and cyclobutyl, wherein the alkyl is optionally substituted with one or more substituents selected from the group consisting of fluorine and cyano; and the heteroatom in the heterocyclyl is O, and the number of the heteroatom is 1.

In a preferred embodiment of the present invention, R^{d2} is independently selected from the group consisting of -(C₁₋₃ alkylene)-C₁₋₂ alkoxy, -(C₁₋₂ alkylene)-(3- to 6-membered heterocyclyl) (more preferably -methyl-(4- to 5-membered heterocyclyl)), C₃₋₆ cycloalkyl (more preferably C₃₋₄ cycloalkyl), and 3- to 6-membered heterocyclyl (more preferably 4- to 5-membered heterocyclyl), wherein the heteroatom in the heterocyclyl is O, and the number of the heteroatom is 1.

In a further preferred embodiment, R^{d2} is independently selected from the group consisting of -C(O)NH₂, -CD₃, -CF₃, -SCF₃, -C(O)CH₃, -C(O)CH₂OH, -C(O)CH₂N(CH₃)₂, -CH₂CN, -CH₂CH₂CH₂CN, -CH₂CH₂C(CH₃)₂OH, - CH₂CH₂CH₂OH, -CH(CH₃)CH₂OCH₃, -CH₂CH₂CH₂OCH₃, -CH₂-C(O)OCH₃, -C(O)NHCH₃, -CH₂-C(O)NH₂, - S(O)₂CH₃, -C(O)N(CH₃)₂, -CH₂CF₃, -CH₂CH₂F, -CH₂CHF₂, -CH₂CH₂SO₂CH₃, methyl, ethyl, n-propyl, isopropyl, n-butyl, cyclopropyl, cyclobutyl, cyclopentyl, cyclohexyl, phenyl,

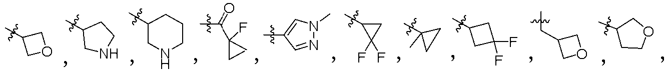

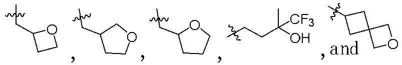

In a further preferred embodiment, R^{d2} is independently selected from the group consisting of hydrogen, - C(O)NH₂, -CD₃, -CF₃, -SCF₃, -C(O)CH₃, -C(O)CH₂OH, -C(O)CH₂N(CH₃)₂, -CH₂CN, -CH₂-C(O)OCH₃, - C(O)NHCH₃, -CH₂-C(O)NH₂, -S(O)₂CH₃, -C(O)N(CH₃)₂, -CH₂CF₃, methyl, ethyl, n-propyl, isopropyl, n-butyl, cyclopropyl, cyclobutyl, cyclopentyl, phenyl,

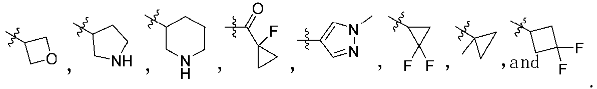

In a preferred embodiment of the present invention, R¹³, R¹⁴, R¹⁵, and R¹⁶ are each independently selected from hydrogen.

In a preferred embodiment of the present invention, Cy2 is independently selected from the group consisting of phenyl and 6-membered heteroaryl.

In a further preferred embodiment, Cy2 is independently selected from the group consisting of phenyl and pyridyl.

In a further preferred embodiment, Cy2 is independently selected from the group consisting of phenyl,

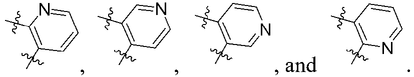

In a further preferred embodiment, Cy2 is independently selected from phenyl.

In a preferred embodiment of the present invention, R¹⁷ is independently selected from the group consisting of fluorine and chlorine.

In a preferred embodiment of the present invention, n and q are each independently selected from 0 or 1.

In a further preferred embodiment, n and q are each independently selected from 0.

The present invention provides a compound represented by Formula (II), or a tautomer, stereoisomer, isotopic derivative, or pharmaceutically acceptable salt thereof: 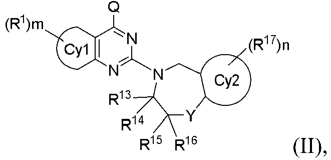 wherein Cy1, Cy2, Y, Q, R¹, R¹³, R¹⁴, R¹⁵, R¹⁶, R¹⁷, m, and n are defined as in the compound represented by Formula (I) of the present invention.

The present invention provides a compound represented by Formula (II-1), or a tautomer, stereoisomer, isotopic derivative, or pharmaceutically acceptable salt thereof: 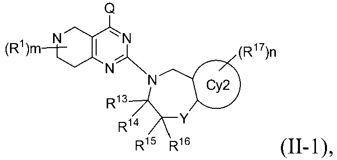 wherein Cy2, Y, Q, R¹, R¹³, R¹⁴, R¹⁵, R¹⁶ R¹⁷, m, and n are defined as in the compound represented by Formula (I) or Formula (II) of the present invention.

The present invention provides a compound represented by Formula (III), or a tautomer, stereoisomer, isotopic derivative, or pharmaceutically acceptable salt thereof: 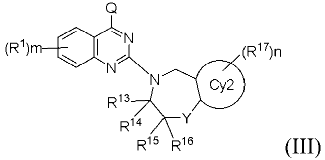 wherein Cy2, Y, Q, R¹, R¹³, R¹⁴, R¹⁵, R¹⁶ R¹⁷, m, and n are defined as in the compound represented by Formula (I) of the present invention.

In a further preferred embodiment, Cy2 is independently selected from the group consisting of phenyl and 6-membered heteroaryl, wherein the heteroatom(s) in the heteroaryl is/are N;

R¹ is independently selected from the group consisting of hydrogen, halogen, oxo, hydroxyl, amino, cyano, C₁₋₆ alkyl, C₁₋₆ alkoxy, C₃₋₆ cycloalkyl, C₁₋₆ haloalkyl, C₁₋₆ alkyl optionally substituted with one or more hydroxyl groups, -S(O)₂(C₁₋₆ alkyl), -C(O)(C₁₋₆ alkyl), -C(O)(C₁₋₆ haloalkyl), -C(O)O(C₁₋₆ alkyl), -C(O)NH(C₁₋₆ alkyl), and -C(O)O-(C₁₋₆ alkyl)-OC(O)(C₁₋₆ alkyl);
Q is selected from 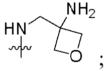
Y is independently selected from -S(O)(=N-R^{d2})-;
R^{d2} is independently selected from the group consisting of hydrogen, -S(C₁₋₄ alkyl), -C(O)NH₂, -C(O)NH(C₁₋₄ alkyl), -C(O)N(C₁₋₄ alkyl)₂, -C(O)(C₁₋₄ alkyl), -C(O)(C₃₋₆ cycloalkyl), -C(O)(3- to 6-membered heterocyclyl), - C(O)-(C₁₋₄ alkylene)-NH(C₁₋₄ alkyl), -C(O)-(C₁₋₄ alkylene)-N(C₁₋₄ alkyl)₂, -(C₁₋₄ alkylene)-C(O)O(C₁₋₄ alkyl), -(C₁₋₄ alkylene)-C(O)(C₁₋₄ alkyl), -(C₁₋₄ alkylene)-C(O)NH₂, -(C₁₋₄ alkylene)-C(O)NH(C₁₋₄ alkyl), -(C₁₋₄ alkylene)-C(O)N(C₁₋₄ alkyl)₂, -S(O)₂(C₁₋₄ alkyl), C₁₋₄ alkyl, C₃₋₆ cycloalkyl, 3- to 6-membered heterocyclyl, phenyl, and 5- to 6-membered heteroaryl, wherein each of the C₁₋₄ alkyl, the C₁₋₄ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, fluorine, chlorine, bromine, hydroxyl, amino, cyano, methyl, ethyl, n-propyl, isopropyl, and n-butyl;
R¹³, R¹⁴, R¹⁵, and R¹⁶ are each independently selected from the group consisting of hydrogen, deuterium, halogen, and oxo; or R¹³ and R¹⁴, together with the carbon atom to which they are connected, form C₃₋₆ cycloalkyl;
R¹⁷ is independently selected from halogen.

The present invention provides a compound represented by Formula (III-1), or a tautomer, stereoisomer, isotopic derivative, or pharmaceutically acceptable salt thereof: 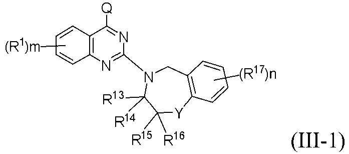 wherein Cy2, Y, Q, R¹, R¹³, R¹⁴, R¹⁵ , R¹⁶ R¹⁷, m, and n are defined as in the compound represented by Formula (I) or Formula (III) of the present invention.

The present invention provides a compound represented by Formula (IV), or a tautomer, stereoisomer, isotopic derivative, or pharmaceutically acceptable salt thereof: 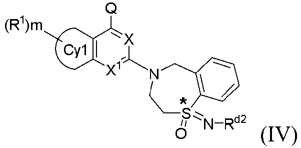 wherein X¹, X, Q, R¹, R^{d2}, and m are defined as in the compound represented by Formula (I) of the present invention; "*" indicates that a chiral center is present at the S atom, with an R configuration, an S configuration, or a mixture of the two.

The present invention provides a compound represented by Formula (IV-a), or a tautomer, stereoisomer, isotopic derivative, or pharmaceutically acceptable salt thereof: 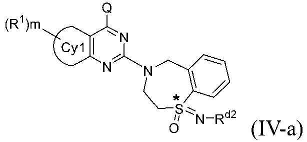 wherein Q, R¹, R^{d2}, and m are defined as in the compound represented by Formula (I) or Formula (IV) of the present invention; "*" indicates that a chiral center is present at the S atom, with an R configuration, an S configuration, or a mixture of the two.

In a preferred embodiment of the present invention, Cy1 is selected from the group consisting of 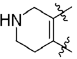 and

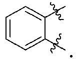

In a preferred embodiment of the present invention, R¹ is independently selected from the group consisting of fluorine, chlorine, bromine, C₁₋₃ alkyl, C₁₋₃ alkoxy, C₁₋₃ haloalkyl, -C(O)(C₁₋₃ alkyl), -C(O)(C₁₋₃ haloalkyl), and - C(O)O(C₁₋₃ alkyl).

In a further preferred embodiment, R¹ is selected from the group consisting of fluorine, chlorine, bromine, C₁₋₃ alkyl, C₁₋₃ alkoxy, C₁₋₃ fluoroalkyl, -C(O)(C₁₋₃ alkyl), -C(O)(C₁₋₃ fluoroalkyl), and -C(O)O(C₁₋₃ alkyl).

In a preferred embodiment of the present invention, R¹ is independently selected from the group consisting of fluorine, chlorine, bromine, methyl, ethyl, n-propyl, isopropyl, n-butyl, methoxy, ethoxy, n-propoxy, isopropoxy, - CH₂F, -CF₂H, -CF₃, -CH₂CF₃, -C(O)CH₃, -C(O)CH₂CH₃, -C(O)CF₃, -C(O)CF₂H, -C(O)OCH₃, and -C(O)OCH₂CH₃.

In a preferred embodiment of the present invention, Q is independently selected from the group consisting of 3- to 10-membered heterocyclyl, -NH-(C₀₋₄ alkylene)-(C₃₋₁₀ carbocyclyl), and -NH-(C₀₋₄ alkylene)-(3- to 10-membered heterocyclyl), wherein the alkylene is optionally substituted with one or more substituents selected from halogen; and each of the C₃₋₁₀ carbocyclyl and the 3- to 10-membered heterocyclyl is optionally substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), C₁₋₆ alkyl, C₁₋₆ haloalkyl, hydroxyl-substituted C₁₋₆ alkyl, and amino-substituted C₁₋₆ alkyl.

In a further preferred embodiment, Q is independently selected from the group consisting of 3- to 8-membered heterocyclyl, -NH-(C₀₋₂ alkylene)-(C₃₋₆ carbocyclyl), and -NH-(C₀₋₂ alkylene)-(3- to 8-membered heterocyclyl), wherein the alkylene is optionally substituted with one or more substituents selected from halogen; and each of the C₃₋₆ carbocyclyl and the 3- to 8-membered heterocyclyl is optionally substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), C₁₋₆ alkyl, C₁₋₆ haloalkyl, hydroxyl-substituted C₁₋₆ alkyl, and amino-substituted C₁₋₆ alkyl.

In a further preferred embodiment, Q is independently selected from the group consisting of 3- to 6-membered heterocycloalkyl, 7- to 8-membered heterospirocyclyl, 7- to 8-membered bridged heterocyclyl, 6- to 10-membered fused heterocyclyl, -NH-(C₀₋₂ alkylene)-(C₃₋₆ cycloalkyl), -NH-(C₀₋₂ alkylene)-(C₅₋₆ bridged cyclyl), -NH-(C₀₋₂ alkylene)-(3- to 6-membered heterocyclyl), and -NH-(C₀₋₂ alkylene)-(7- to 8-membered heterospirocyclyl), wherein each of the cycloalkyl, the heterocycloalkyl, the heterospirocyclyl, the bridged heterocyclyl, the fused heterocyclyl, and the bridged cyclyl is optionally substituted with one or more R^{c2};
R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl.

In a further preferred embodiment, Q is independently selected from the group consisting of 3- to 6-membered heterocycloalkyl, 7- to 8-membered heterospirocyclyl, 7- to 8-membered bridged heterocyclyl, 6- to 10-membered fused heterocyclyl, -NH-(C₀₋₂ alkylene)-(C₃₋₆ cycloalkyl), -NH-(C₀₋₂ alkylene)-(C₅₋₆ bridged cyclyl), -NH-(C₀₋₂ alkylene)-(3- to 6-membered heterocyclyl), and -NH-(C₀₋₂ alkylene)-(7- to 8-membered heterospirocyclyl), wherein each of the cycloalkyl, the heterocycloalkyl, the heterospirocyclyl, the bridged heterocyclyl, the fused heterocyclyl, and the bridged cyclyl is optionally substituted with one or more R^{c2}; the heteroatom in the 3- to 6-membered heterocycloalkyl, the 7- to 8-membered heterospirocyclyl, the 7- to 8-membered bridged heterocyclyl, and the 6- to 10-membered fused heterocyclyl is N or O, the number of the heteroatom is 1 or 2, and 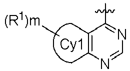 is connected to the N atom in Q;

R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl.

In a further preferred embodiment, Q is independently selected from the group consisting of:

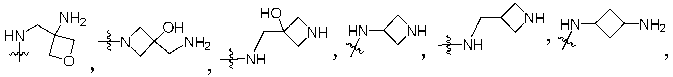

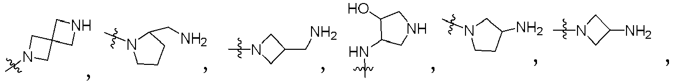

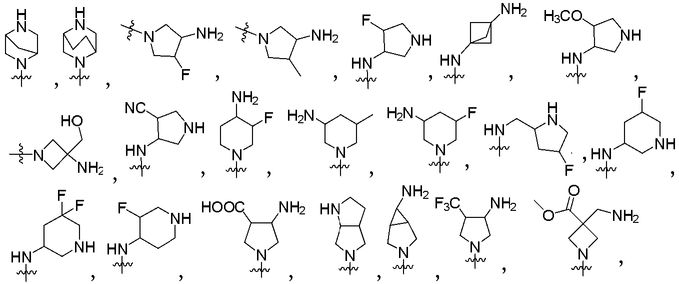

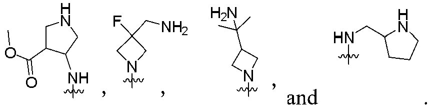

In a preferred embodiment of the present invention, R^{d2} is independently selected from the group consisting of -S(C₁₋₆ alkyl), -C(O)NH₂, -C(O)NH(C₁₋₆ alkyl), -C(O)N(C₁₋₆ alkyl)₂, -C(O)(C₁₋₆ alkyl), -C(O)(C₃₋₆ cycloalkyl), - C(O)(3- to 6-membered heterocyclyl), -C(O)-(C₁₋₆ alkylene)-NH(C₁₋₆ alkyl), -C(O)-(C₁₋₆ alkylene)-N(C₁₋₆ alkyl)₂, -(C₁₋₆ alkylene)-C(O)O(C₁₋₆ alkyl), -(C₁₋₆ alkylene)-C(O)(C₁₋₆ alkyl), -(C₁₋₆ alkylene)-S(O)₂(C₁₋₆ alkyl), -(C₁₋₆ alkylene)-C(O)NH₂, -(C₁₋₆ alkylene)-C(O)NH(C₁₋₆ alkyl), -(C₁₋₆ alkylene)-C(O)N(C₁₋₆ alkyl)₂, -S(O)₂(C₁₋₆ alkyl), C₁₋₆ alkyl, C₃₋₆ cycloalkyl, 3- to 8-membered heterocyclyl, phenyl, and 5- to 6-membered heteroaryl, wherein each of the C₁₋₆ alkyl, the C₁₋₆ alkylene, the C₃₋₆ cycloalkyl, the 3- to 8-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, C₁₋₆ alkoxy, C₃₋₆ cycloalkyl, and 3- to 6-membered heterocyclyl.

In a further preferred embodiment, R^{d2} is independently selected from the group consisting of -S(C₁₋₃ alkyl), - C(O)NH₂, -C(O)NH(C₁₋₃ alkyl), -C(O)N(C₁₋₃ alkyl)₂, -C(O)(C₁₋₃ alkyl), -C(O)(C₃₋₆ cycloalkyl), -C(O)(3- to 6-membered heterocyclyl), -C(O)-(C₁₋₂ alkylene)-NH(C₁₋₆ alkyl), -C(O)-(C₁₋₂ alkylene)-N(C₁₋₃ alkyl)₂, -(C₁₋₂ alkylene)-C(O)O(C₁₋₃ alkyl), -(C₁₋₂ alkylene)-C(O)(C₁₋₃ alkyl), -(C₁₋₂ alkylene)-S(O)₂(C₁₋₃ alkyl), -(C₁₋₂ alkylene)-C(O)NH₂, -(C₁₋₂ alkylene)-C(O)NH(C₁₋₃ alkyl), -(C₁₋₂ alkylene)-C(O)N(C₁₋₃ alkyl)₂, -S(O)₂(C₁₋₃ alkyl), C₁₋₆ alkyl, C₃₋₆ cycloalkyl, 3- to 6-membered monocyclic heterocyclyl, 7-membered heterospirocyclyl, 8-membered heterospirocyclyl, phenyl, and 5- to 6-membered heteroaryl, wherein each of the alkyl, the alkylene, the cycloalkyl, the monocyclic heterocyclyl, the heterospirocyclyl, the phenyl, and the 5- to 6-membered heteroaryl is optionally substituted with one or more substituents selected from the group consisting of deuterium, halogen, hydroxyl, amino, cyano, C₁₋₃ alkyl, C₁₋₃ alkoxy, C₃₋₆ cycloalkyl, and 3- to 6-membered heterocyclyl.

In a further preferred embodiment, R^{d2} is independently selected from the group consisting of -S(C₁₋₃ alkyl), - C(O)NH₂, -C(O)NH(C₁₋₃ alkyl), -C(O)N(C₁₋₃ alkyl)₂, -C(O)(C₁₋₃ alkyl), -C(O)(C₃₋₆ cycloalkyl), -C(O)(3- to 6-membered heterocyclyl), -C(O)-(C₁₋₂ alkylene)-NH(C₁₋₃ alkyl), -C(O)-(C₁₋₂ alkylene)-N(C₁₋₃ alkyl)₂, -(C₁₋₂ alkylene)-C(O)O(C₁₋₃ alkyl), -(C₁₋₂ alkylene)-C(O)(C₁₋₃ alkyl), -(C₁₋₂ alkylene)-S(O)₂(C₁₋₃ alkyl), -(C₁₋₂ alkylene)-C(O)NH₂, -(C₁₋₂ alkylene)-C(O)NH(C₁₋₃ alkyl), -(C₁₋₂ alkylene)-C(O)N(C₁₋₃ alkyl)₂, -S(O)₂(C₁₋₃ alkyl), C₁₋₆ alkyl, C₃₋₆ cycloalkyl, 3- to 6-membered monocyclic heterocyclyl, 7-membered heterospirocyclyl, phenyl, and 5- to 6-membered heteroaryl, wherein the heteroatom in the monocyclic heterocyclyl, the heterospirocyclyl, and the heteroaryl is O or N, the number of the heteroatom is 1 or 2; and each of the alkyl, the alkylene, the cycloalkyl, the monocyclic heterocyclyl, the heterospirocyclyl, the phenyl, and the heteroaryl is optionally substituted with one or more substituents selected from the group consisting of deuterium, fluorine, chlorine, bromine, hydroxyl, amino, cyano, methyl, ethyl, methoxy, ethoxy, cyclopropyl, cyclobutyl, oxiranyl, and oxetanyl.

In a further preferred embodiment, R^{d2} is independently selected from the group consisting of -C(O)NH₂, -CD₃, -CF₃, -SCF₃, -C(O)CH₃, -C(O)CH₂OH, -C(O)CH₂N(CH₃)₂, -CH₂CN, -CH₂CH₂CH₂CN, -CH₂CH₂C(CH₃)₂OH, - CH₂CH₂CH₂OH, -CH(CH₃)CH₂OCH₃, -CH₂CH₂CH₂OCH₃, -CH₂-C(O)OCH₃, -C(O)NHCH₃, -CH₂-C(O)NH₂, - S(O)₂CH₃, -C(O)N(CH₃)₂, -CH₂CF₃, -CH₂CH₂F, -CH₂CHF₂, -CH₂CH₂SO₂CH₃, methyl, ethyl, n-propyl, isopropyl, n-butyl, cyclopropyl, cyclobutyl, cyclopentyl, cyclohexyl, phenyl,

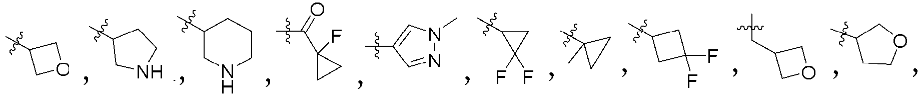

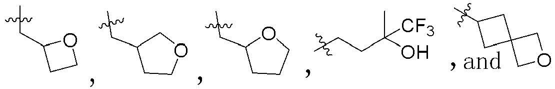

In a preferred embodiment of the present invention, R¹ is independently selected from the group consisting of halogen, C₁₋₆ alkyl, C₁₋₆ alkoxy, C₁₋₆ haloalkyl, -C(O)(C₁₋₆ alkyl), -C(O)(C₁₋₆ haloalkyl), and -C(O)O(C₁₋₆ alkyl);
m is selected from 1 or 2;
Q is independently selected from the group consisting of C₃₋₈ carbocyclyl, 3- to 10-membered heterocyclyl, - NH-(C₀₋₄ alkylene)-(C₃₋₈ carbocyclyl), and -NH-(C₀₋₄ alkylene)-(3- to 10-membered heterocyclyl), wherein the alkylene is optionally substituted with one or more substituents selected from halogen; and each of the C₃₋₈ carbocyclyl and the 3- to 10-membered heterocyclyl is optionally substituted with one or more R^{c2};
R^{c2} is independently selected from the group consisting of halogen, hydroxyl, amino, cyano, -O(C₁₋₆ alkyl), - C(O)OH, -C(O)O(C₁₋₆ alkyl), C₁₋₆ alkyl, C₁₋₆ haloalkyl, hydroxyl-substituted C₁₋₆ alkyl, and amino-substituted C₁₋₆ alkyl;
R^{d2} is independently selected from the group consisting of -S(C₁₋₆ alkyl), -C(O)NH₂, -C(O)NH(C₁₋₆ alkyl), - C(O)N(C₁₋₆ alkyl)₂, -C(O)(C₁₋₆ alkyl), -C(O)(C₃₋₆ cycloalkyl), -C(O)(3- to 6-membered heterocyclyl), -C(O)-(C₁₋₄ alkylene)-NH(C₁₋₆ alkyl), -C(O)-(C₁₋₄ alkylene)-N(C₁₋₆ alkyl)₂, -(C₁₋₄ alkylene)-C(O)O(C₁₋₆ alkyl), -(C₁₋₄ alkylene)-C(O)(C₁₋₆ alkyl), -(C₁₋₄ alkylene)-S(O)₂(C₁₋₆ alkyl), -(C₁₋₄ alkylene)-C(O)NH₂, -(C₁₋₄ alkylene)-C(O)NH(C₁₋₆ alkyl), -(C₁₋₄ alkylene)-C(O)N(C₁₋₆ alkyl)₂, -S(O)₂(C₁₋₆ alkyl), C₁₋₆ alkyl, C₃₋₆ cycloalkyl, 3- to 8-membered heterocyclyl, phenyl, and 5- to 6-membered heteroaryl, wherein each of the C₁₋₆ alkyl, the C₁₋₄ alkylene, the C₃₋₆ cycloalkyl, the 3- to 8-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, C₁₋₆ alkoxy, C₃₋₆ cycloalkyl, and 3-6 membered heterocyclyl.

In a preferred embodiment of the present invention, R¹ is independently selected from the group consisting of fluorine, chlorine, bromine, C₁₋₃ alkyl, C₁₋₃ alkoxy, C₁₋₃ haloalkyl, -C(O)(C₁₋₃ alkyl), -C(O)(C₁₋₃ haloalkyl), and - C(O)O(C₁₋₃ alkyl);
m is selected from 1 or 2;
Q is independently selected from the group consisting of C₃₋₆ cycloalkyl, 3- to 6-membered heterocycloalkyl, 7- to 8-membered heterospirocyclyl, 7- to 8-membered bridged heterocyclyl, 6- to 10-membered fused heterocyclyl, -NH-(C₀₋₂ alkylene)-(C₃₋₆ cycloalkyl), -NH-(C₀₋₂ alkylene)-(C₅₋₆ bridged cyclyl), -NH-(C₀₋₂ alkylene)-(3- to 6-membered heterocyclyl), and -NH-(C₀₋₂ alkylene)-(7- to 8-membered heterospirocyclyl), wherein each of the cycloalkyl, the heterocycloalkyl, the heterospirocyclyl, the bridged heterocyclyl, the fused heterocyclyl, and the bridged cyclyl is optionally substituted with one or more R^{c2};
R^{c2} is independently selected from the group consisting of halogen, hydroxyl, amino, cyano, -O(C₁₋₃ alkyl), - C(O)OH, -C(O)O(C₁₋₃ alkyl), C₁₋₃ alkyl, C₁₋₃ haloalkyl, hydroxyl-substituted C₁₋₃ alkyl, and amino-substituted C₁₋₃ alkyl;
R^{d2} is independently selected from the group consisting of -S(C₁₋₃ alkyl), -C(O)NH₂, -C(O)NH(C₁₋₃ alkyl), - C(O)N(C₁₋₃ alkyl)₂, -C(O)(C₁₋₃ alkyl), -C(O)(C₃₋₆ cycloalkyl), -C(O)(3- to 6-membered heterocyclyl), -C(O)-(C₁₋₂ alkylene)-NH(C₁₋₃ alkyl), -C(O)-(C₁₋₂ alkylene)-N(C₁₋₃ alkyl)₂, -(C₁₋₂ alkylene)-C(O)O(C₁₋₃ alkyl), -(C₁₋₂ alkylene)-C(O)(C₁₋₃ alkyl), -(C₁₋₂ alkylene)-S(O)₂(C₁₋₃ alkyl), -(C₁₋₂ alkylene)-C(O)NH₂, -(C₁₋₂ alkylene)-C(O)NH(C₁₋₃ alkyl), -(C₁₋₂ alkylene)-C(O)N(C₁₋₃ alkyl)₂, -S(O)₂(C₁₋₃ alkyl), C₁₋₆ alkyl, C₃₋₆ cycloalkyl, 3- to 6-membered monocyclic heterocyclyl, 7-membered heterospirocyclyl, phenyl, and 5- to 6-membered heteroaryl, wherein each of the alkyl, the alkylene, the cycloalkyl, the monocyclic heterocyclyl, the heterospirocyclyl, the phenyl, and the 5- to 6-membered heteroaryl is optionally substituted with one or more substituents selected from the group consisting of deuterium, halogen, hydroxyl, amino, cyano, C₁₋₃ alkyl, C₁₋₃ alkoxy, C₃₋₆ cycloalkyl, and 3- to 6-membered heterocyclyl.

The present invention provides a compound represented by Formula (IV-1) or Formula (IV-2), or a tautomer, stereoisomer, isotopic derivative, or pharmaceutically acceptable salt thereof: 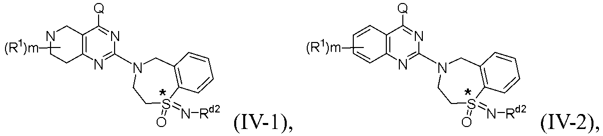 wherein Q, R¹, R^{d2}, and m are defined as in the compound represented by Formula (I), Formula (IV), or Formula (IV-a) of the present invention in the present application; "*" indicates that a chiral center is present at the S atom, with an R configuration, an S configuration, or a mixture of the two.

The present invention provides a compound represented by Formula (IV-3), or a tautomer, stereoisomer, isotopic derivative, or pharmaceutically acceptable salt thereof: 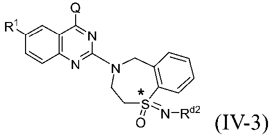 wherein Q, R¹, and R^{d2} are defined as in the compound represented by Formula (I), Formula (IV), Formula (IV-a), or Formula (IV-2) of the present invention in the present application; "*" indicates that a chiral center is present at the S atom, with an R configuration, an S configuration, or a mixture of the two.

The present invention provides a compound represented by Formula (IV-4) or Formula (IV-5), or a tautomer, stereoisomer, isotopic derivative, or pharmaceutically acceptable salt thereof: 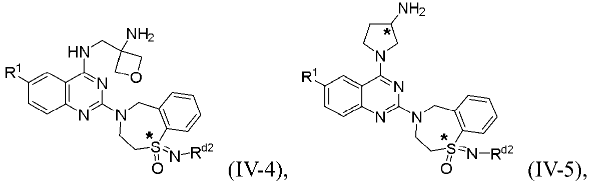 wherein R¹ and R^{d2} are defined as in the compound represented by Formula (I), Formula (IV), Formula (IV-a), Formula (IV-2), or Formula (IV-3) of the present invention in the present application; "*" indicates that a chiral center is present at the S atom, with an R configuration, an S configuration, or a mixture of the two.

The present invention provides a compound represented by Formula (IV-6) or Formula (IV-7), or a tautomer, stereoisomer, isotopic derivative, or pharmaceutically acceptable salt thereof: 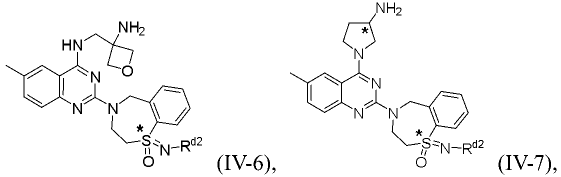 wherein R^{d2} is defined as in the compound represented by Formula (I), Formula (IV), Formula (IV-a), Formula (IV-2), or Formula (IV-3) of the present invention in the present application; "*" indicates that a chiral center is present at the S atom, with an R configuration, an S configuration, or a mixture of the two.

The above preferred embodiments may be arbitrarily combined.

Preferably, among the compounds, or the tautomers, stereoisomers, isotopic derivatives, or pharmaceutically acceptable salts thereof provided in the present invention, the compounds have the following structures:

| Compound structure and Number | Compound structure and Number | Compound structure and Number |
|---|---|---|
| 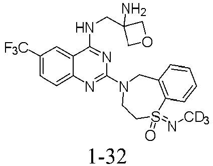 | 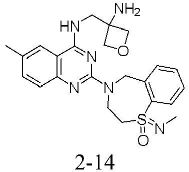 | 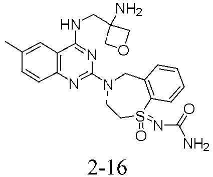 |
| 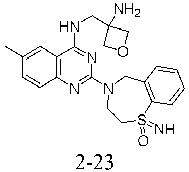 | 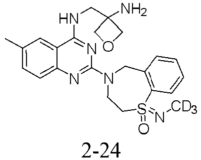 | 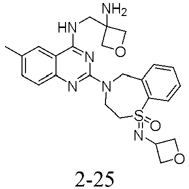 |
| 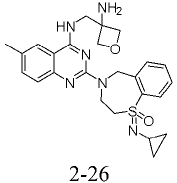 | 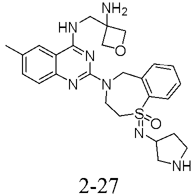 | 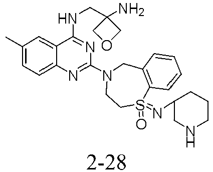 |
| 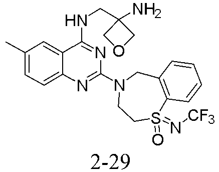 | 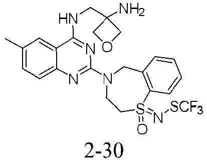 | 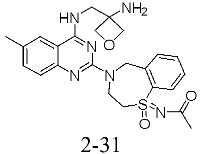 |
| 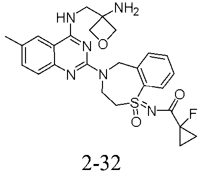 | 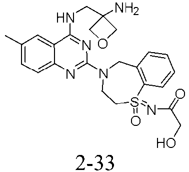 | 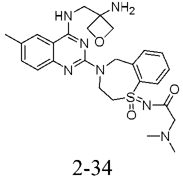 |
| 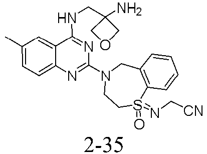 | 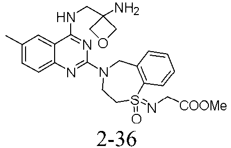 | 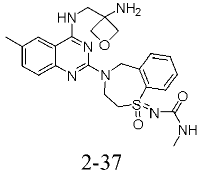 |
| 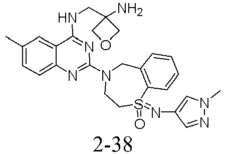 | 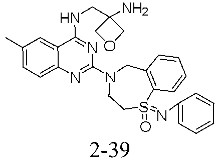 | 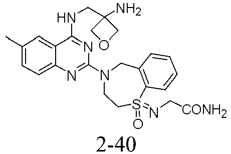 |
| 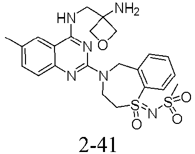 | 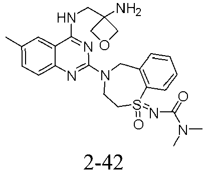 | 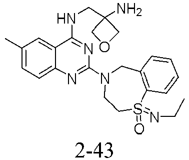 |
| 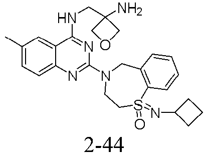 | 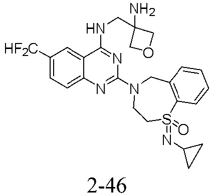 | 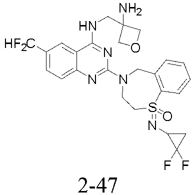 |
| 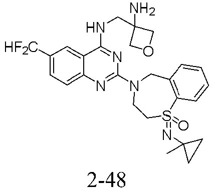 | 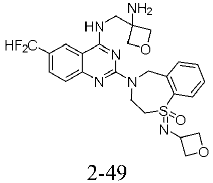 | 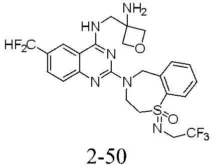 |
| 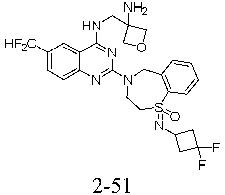 | 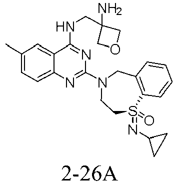 | 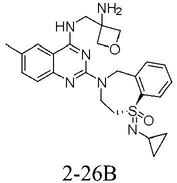 |
| 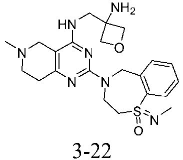 | 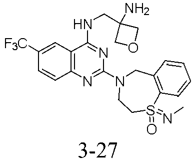 | 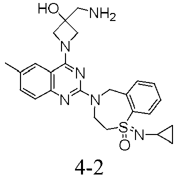 |
| 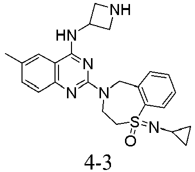 | 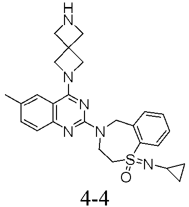 | 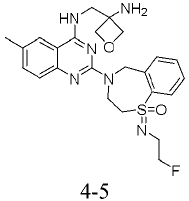 |
| 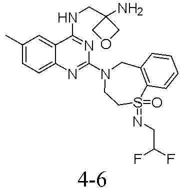 | 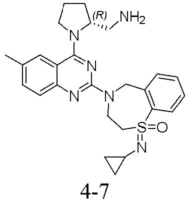 | 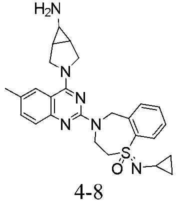 |
| 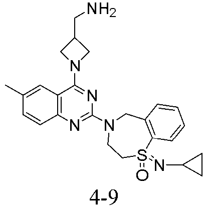 | 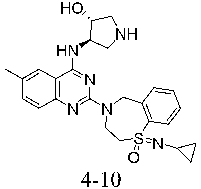 | 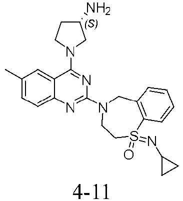 |
| 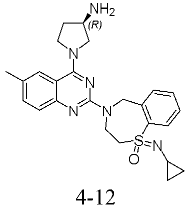 | 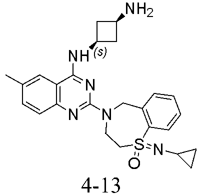 | 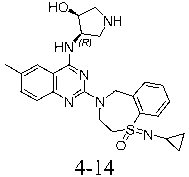 |
| 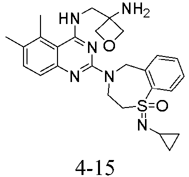 | 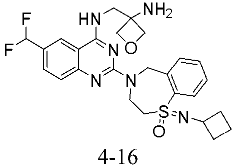 | 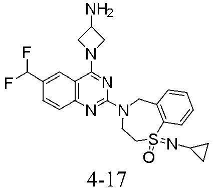 |
| 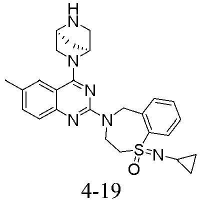 | 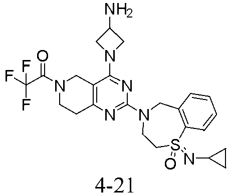 | 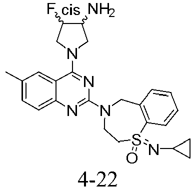 |
| 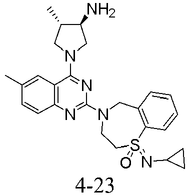 | 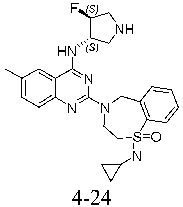 | 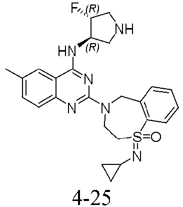 |
| 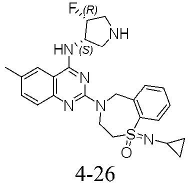 | 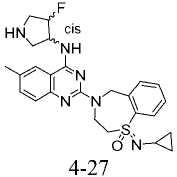 | 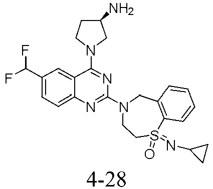 |
| 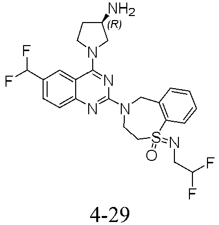 | 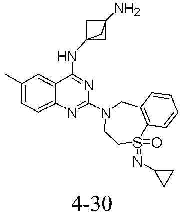 | 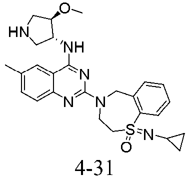 |
| 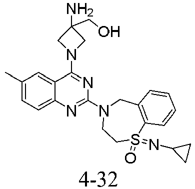 | 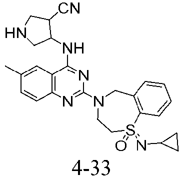 | 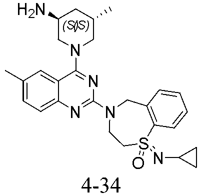 |
| 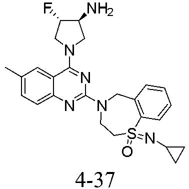 | 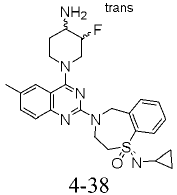 | 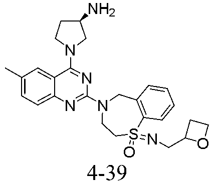 |
| 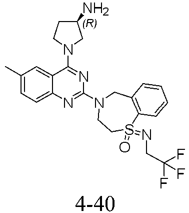 | 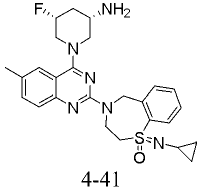 | 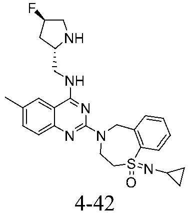 |
| 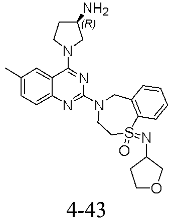 | 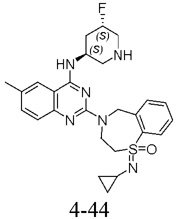 | 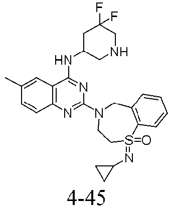 |
| 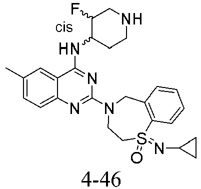 | 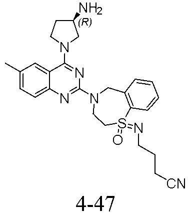 | 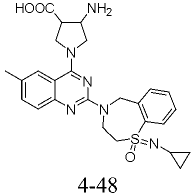 |
| 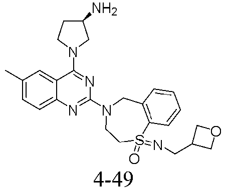 | 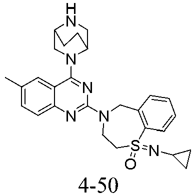 | 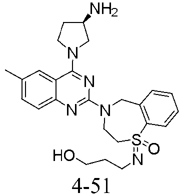 |
| 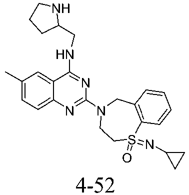 | 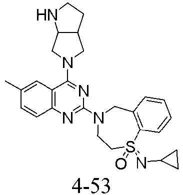 | 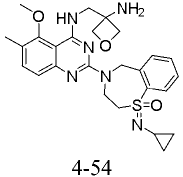 |
| 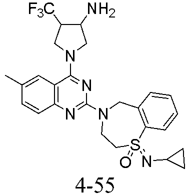 | 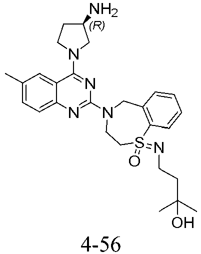 | 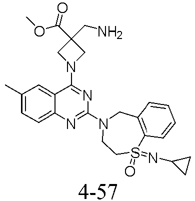 |
| 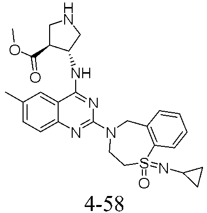 | 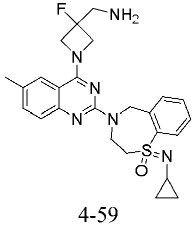 | 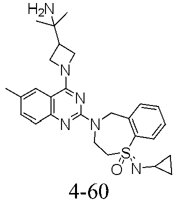 |
| 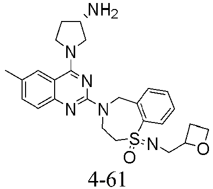 | 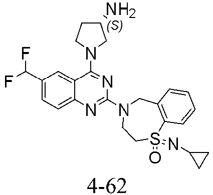 | 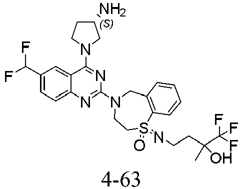 |
| 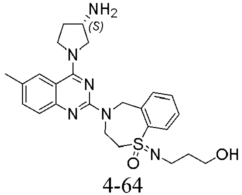 | 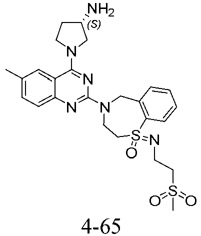 | 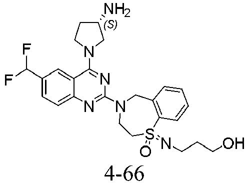 |
| 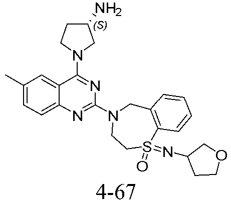 | 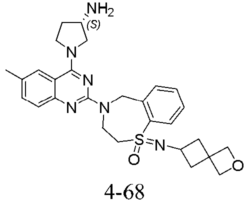 | 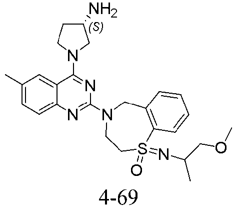 |
| 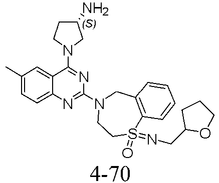 | 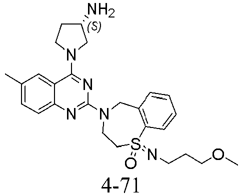 | 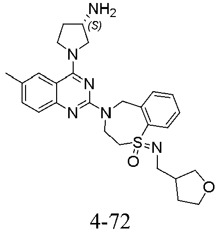 |
| 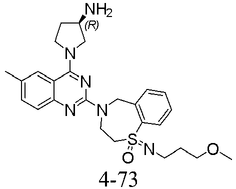 | 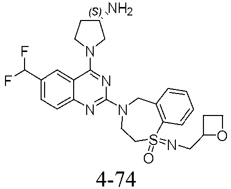 | 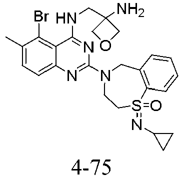 |
| 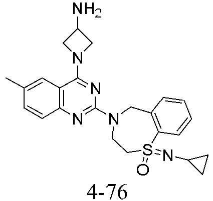 | 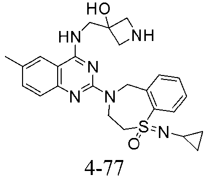 | 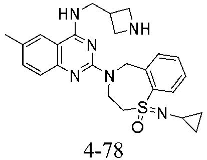 |
| 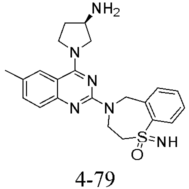 | 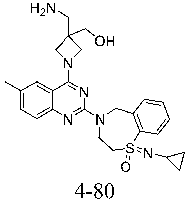 | 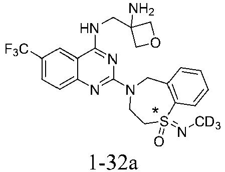 |
| 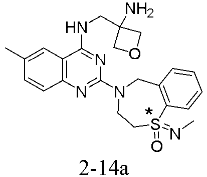 | 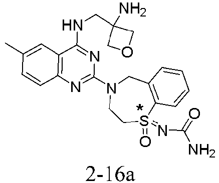 | 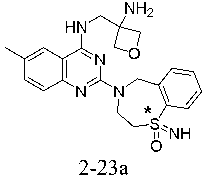 |
| 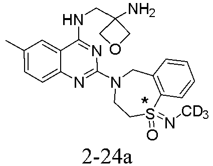 | 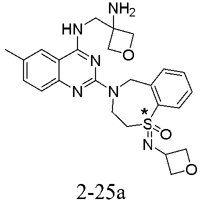 | 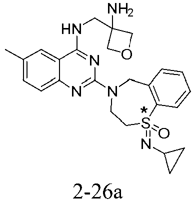 |
| 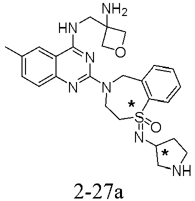 | 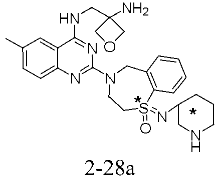 | 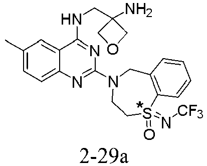 |
| 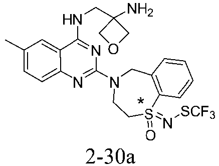 | 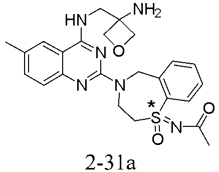 | 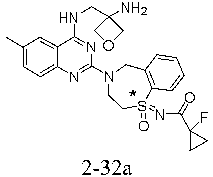 |
| 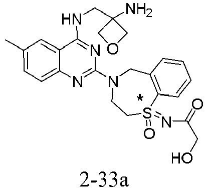 | 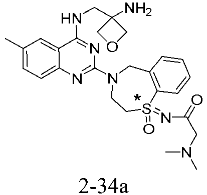 | 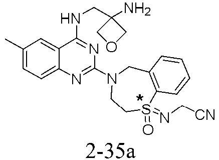 |
| 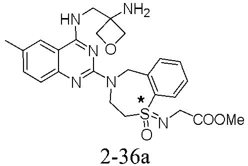 | 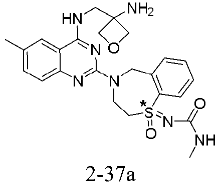 | 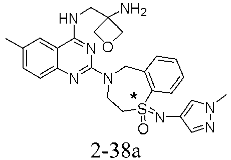 |
| 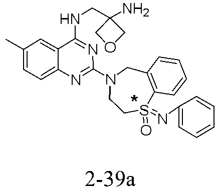 | 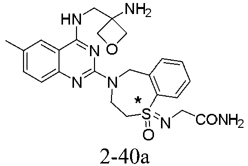 | 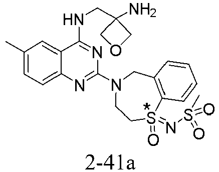 |
| 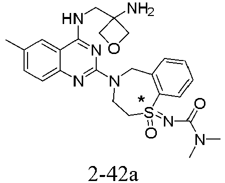 | 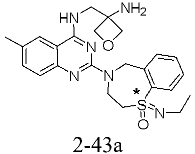 | 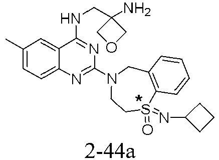 |
| 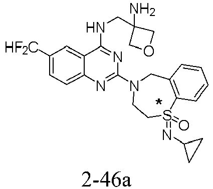 | 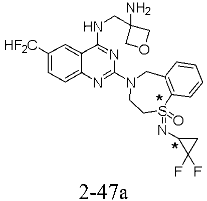 | 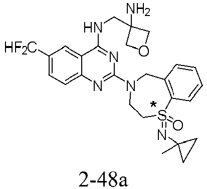 |
| 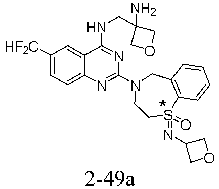 | 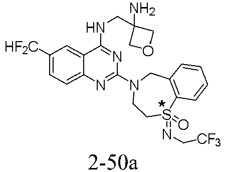 | 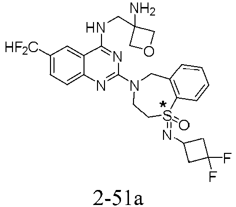 |
| 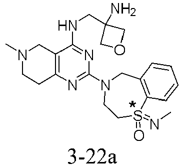 | 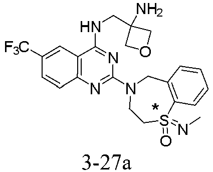 | 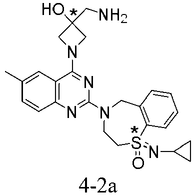 |
| 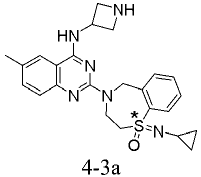 | 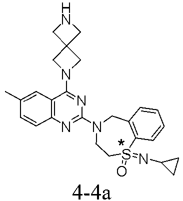 | 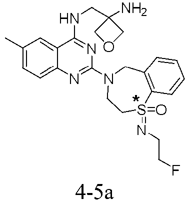 |
| 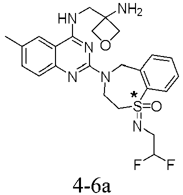 | 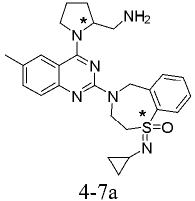 | 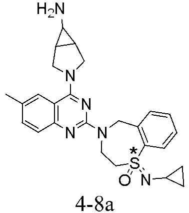 |
| 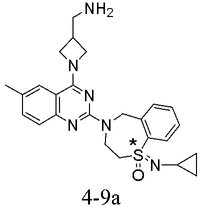 | 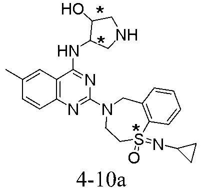 | 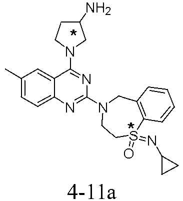 |
| 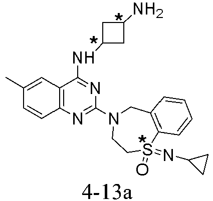 | 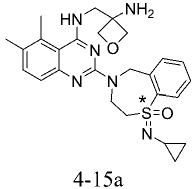 | 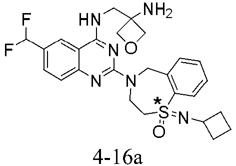 |
| 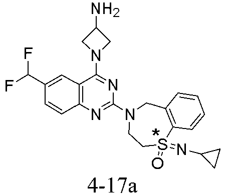 | 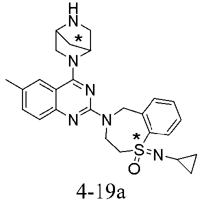 | 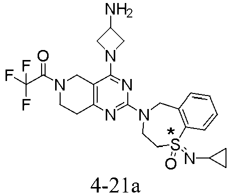 |
| 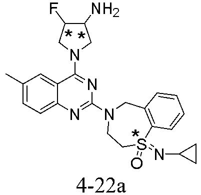 | 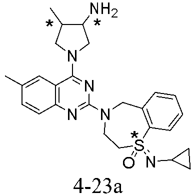 | 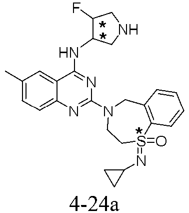 |
| 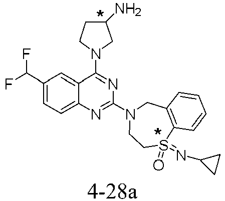 | 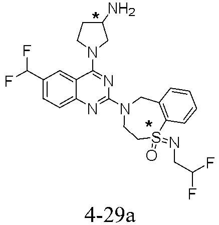 | 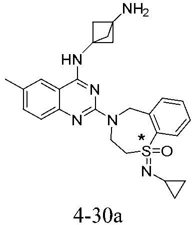 |
| 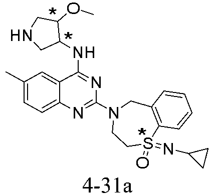 | 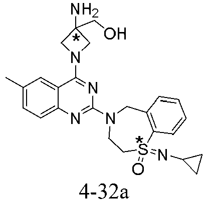 | 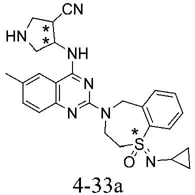 |
| 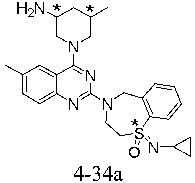 | 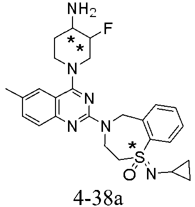 | 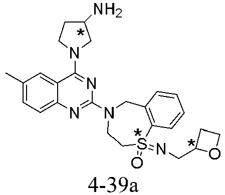 |
| 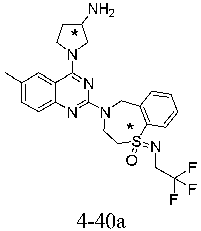 | 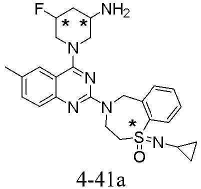 | 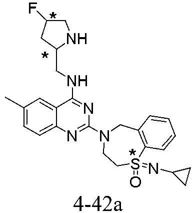 |
| 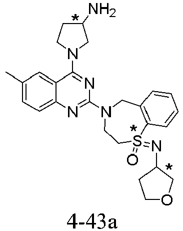 | 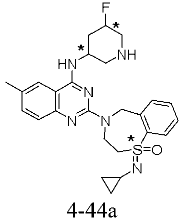 | 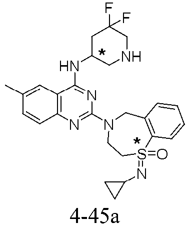 |
| 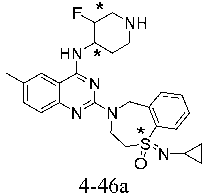 | 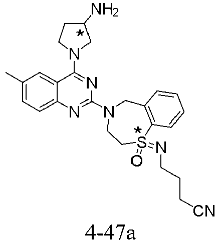 | 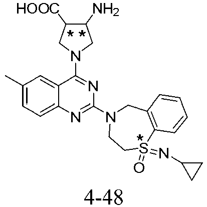 |
| 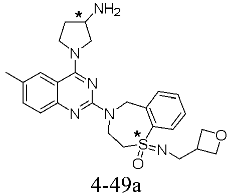 | 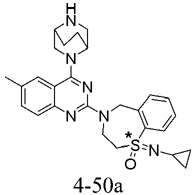 | 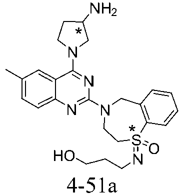 |
| 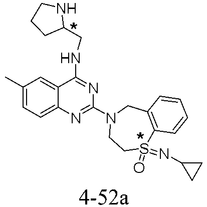 | 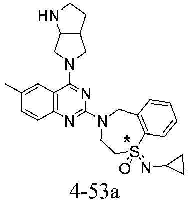 | 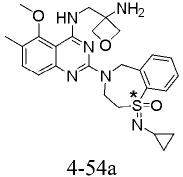 |
| 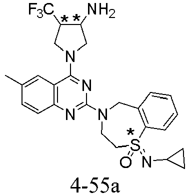 | 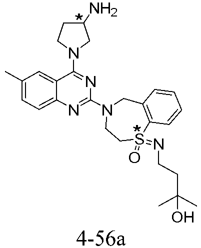 | 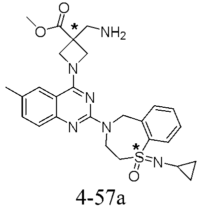 |
| 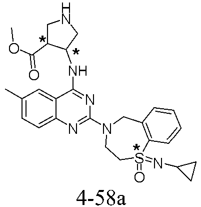 | 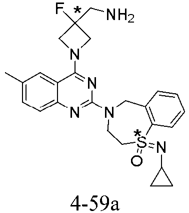 | 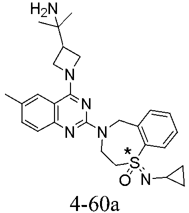 |
| 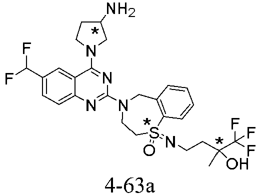 | 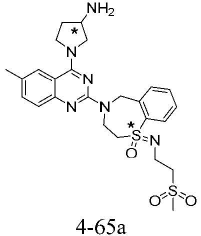 | 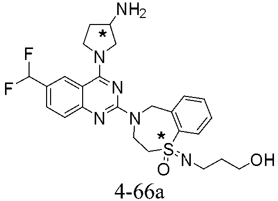 |
| 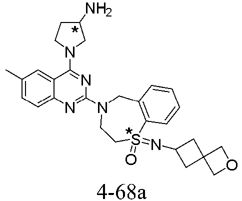 | 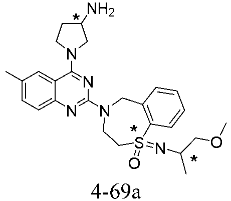 | 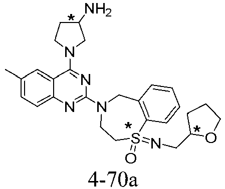 |
| 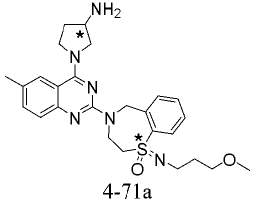 | 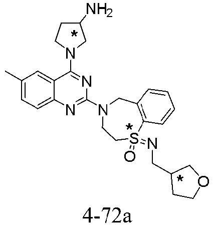 | 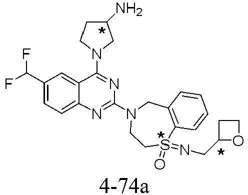 |
| 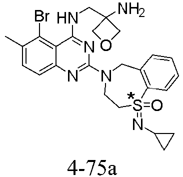 | 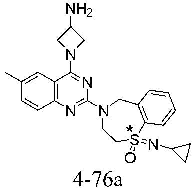 | 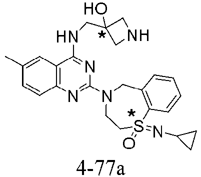 |
| 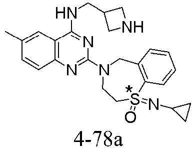 | 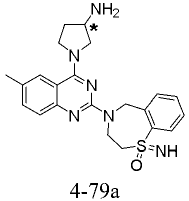 | 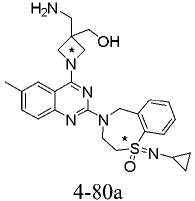 |

The present invention also provides an intermediate for preparing the compound represented by Formula (I), which has a structure represented by Formula (R-a): 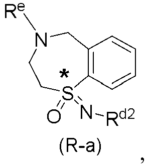 wherein R^{e} is selected from the group consisting of a protective group and hydrogen; R^{d2} is defined as in the compound represented by Formula (I), Formula (II), Formula (II-1), Formula (III), Formula (III-1), Formula (IV), Formula (IV-a), Formula (IV-1), Formula (IV-2), Formula (IV-3), Formula (IV-4), Formula (IV-5), Formula (IV-6), or Formula (IV-7), provided that R^{e} and R^{d2} are not hydrogen simultaneously.

In an embodiment of the present invention, the protective group is selected from the group consisting of tert-butoxycarbonyl (Boc), 9-fluorenylmethoxycarbonyl (FMOC), benzyloxycarbonyl (Cbz), p-methoxybenzyl (PMB), and acetyl (Ac); preferably tert-butoxycarbonyl (Boc).

In an embodiment of the present invention, the present invention also provides an intermediate for preparing the compound represented by Formula (I), which has a structure represented by Formula (R-a5) or Formula (R-a6):

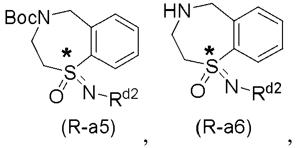

wherein R^{d2} is defined as in the compound represented by Formula (I), Formula (II), Formula (II-1), Formula (III), Formula (III-1), Formula (IV), Formula (IV-a), Formula (IV-1), Formula (IV-2), Formula (IV-3), Formula (IV-4), Formula (IV-5), Formula (IV-6), Formula (IV-7), or Formula (R-a).

The present invention also provides the following synthetic intermediates for preparing the target compounds:

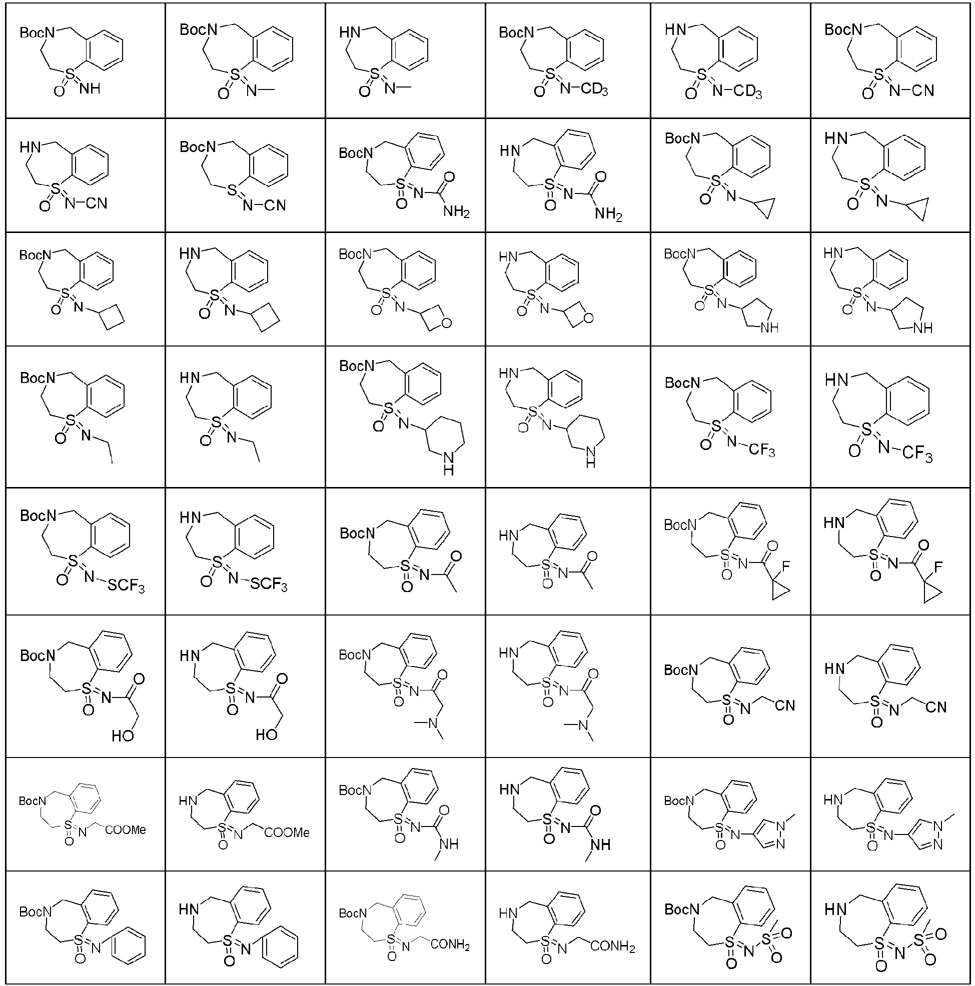

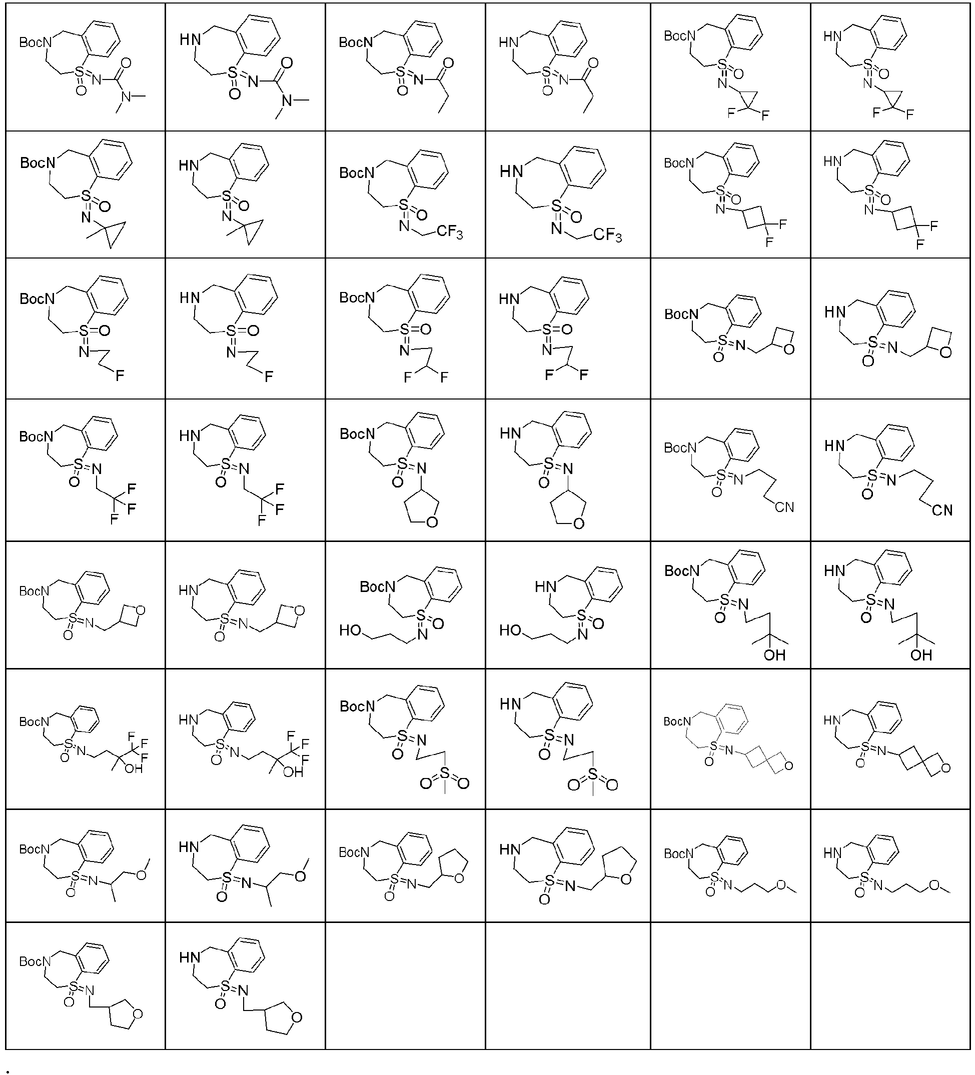

A further object of the present invention is to provide a method for preparing the compounds represented by the formulae, or tautomers, stereoisomers, isotopic derivatives, or pharmaceutically acceptable salts thereof.

The method may be implemented, for example, by using the method shown in the following scheme: 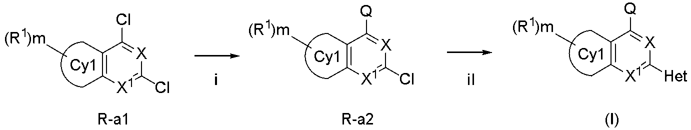
i) Compound R-a1 undergoes chemical transformation to yield Compound R-a2;
   for example, Compound R-a2 is obtained through a reaction using Compound R-a1, an amino derivative and others as basic raw materials;
ii) Compound R-a2 undergoes chemical transformation to yield Compound R-a;
   for example, the compound represented by Formula (I) is obtained through a reaction using Compound R-a2, an amino derivative and others as basic raw materials;
   in the above formulae, Cy1, X, X¹, Q, R¹, Het, and m are defined as in the compound represented by Formula (I) of the present invention.

In an embodiment of the present invention, the method may be implemented, for example, by using the method shown in the following scheme: 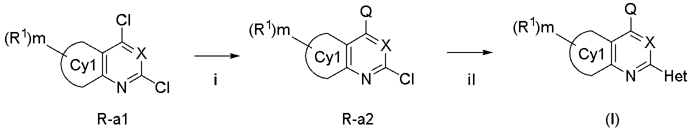
i) Compound R-a1 undergoes chemical transformation to yield Compound R-a2;
   for example, Compound R-a2 is obtained through a reaction using Compound R-a1, an amino derivative and others as basic raw materials;
ii) Compound R-a2 undergoes chemical transformation to yield Compound R-a;
   for example, the compound represented by Formula (I) is obtained through a reaction using Compound R-a2, an amino derivative and others as basic raw materials;
   in the above formulae, Cy1, X, Q, R¹, Het, and m are defined as in the compound represented by Formula (I) of the present invention.

In an embodiment of the present invention, the compound represented by Formula (IV) is obtained through a reaction using Compound R-a2, an amino derivative and others as basic raw materials, as shown below: 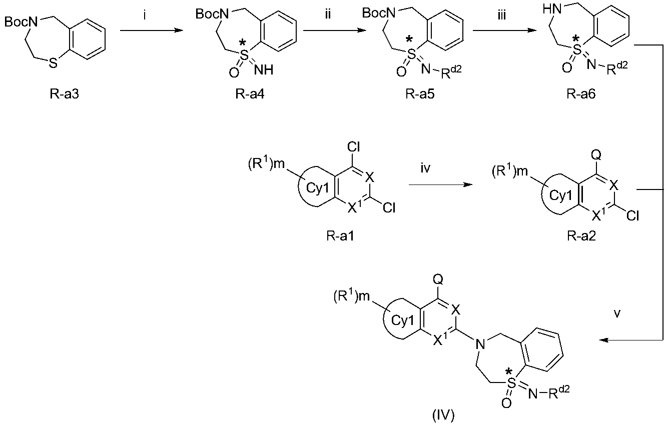
i) Compound R-a3 undergoes an oxidation reaction to yield Compound R-a4;
   for example, Compound R-a4 is obtained through a chemical reaction using Compound R-a3 together with iodobenzene diacetate, ammonium carbonate, and others, as basic raw materials;
ii) Compound R-a4 undergoes a substitution reaction to yield Compound R-a5;
   for example, Compound R-a5 is obtained through a chemical reaction using Compound R-a4, methanesulfonate derivatives and others as basic raw materials;
iii) Compound R-a5 undergoes a nitrogen atom deprotection reaction to yield Compound R-a6;
   for example, Compound R-a6 is obtained through a chemical reaction using Compound R-a5, trifluoroacetic acid and others as basic raw materials;
iv) Compound R-a1 undergoes chemical transformation to yield Compound R-a2;
   for example, Compound R-a2 is obtained through a reaction using Compound R-a1, an amino derivative and others as basic raw materials;
v) Compound R-a2 and Compound R-a6 undergo chemical transformation to yield the compound represented by Formula (IV);
   for example, the compound represented by Formula (IV) is obtained through a substitution reaction using Compound R-a2, Compound R-a6 and others as basic raw materials;
   in the above formulae, Cy1, X, X¹, Q, R¹, R^{d2}, and m are defined as in the compound represented by Formula (I) of the present invention.

In an embodiment of the present invention, the compound represented by Formula (IV-a) is obtained through a reaction using Compound R-a2, an amino derivative and others as basic raw materials, as shown below: 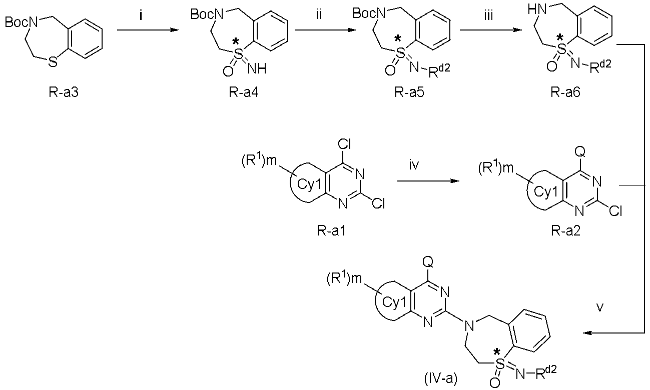
i) Compound R-a3 undergoes an oxidation reaction to yield Compound R-a4;
   for example, Compound R-a4 is obtained via a chemical reaction using Compound R-a3 together with iodobenzene diacetate, ammonium carbonate, and others as basic raw materials;
ii) Compound R-a4 undergoes a substitution reaction to yield Compound R-a5;
   for example, Compound R-a5 is obtained via a chemical reaction using Compound R-a4, methanesulfonate derivatives and others as basic raw materials;
iii) Compound R-a5 undergoes a nitrogen atom deprotection reaction to yield Compound R-a6;
   for example, Compound R-a6 is obtained via a chemical reaction using Compound R-a5, trifluoroacetic acid and others as basic raw materials;
iv) Compound R-a1 undergoes chemical transformation to yield Compound R-a2;
   for example, Compound R-a2 is obtained via a reaction using Compound R-a1, an amino derivative and others as basic raw materials;
v) Compound R-a2 and Compound R-a6 undergo chemical transformation to yield the compound represented by Formula (IV-a);
   for example, the compound represented by Formula (IV-a) is obtained via a substitution reaction using Compound R-a2, Compound R-a6 and others as basic raw materials;
   in the above formulae, Cy1, X, Q, R¹, R^{d2}, and m are defined as in the compound represented by Formula (I) of the present invention.

The present invention also provides a pharmaceutical composition comprising the compound of the present invention, or the tautomer, stereoisomer, isotopic derivative, or pharmaceutically acceptable salt thereof.

The present invention also provides a pharmaceutical composition comprising the compound of the present invention, or the tautomer, stereoisomer, isotopic derivative, or pharmaceutically acceptable salt thereof, and a pharmaceutically acceptable carrier.

The compound of the present invention, or the tautomer, stereoisomer, isotopic derivative, or pharmaceutically acceptable salt thereof may be administrated in a pure form or in the form of a suitable pharmaceutical composition via any acceptable administration route for drugs with similar uses. The pharmaceutical composition of the present invention may be prepared by combining the compound of the present invention with suitable pharmaceutically acceptable excipients. The pharmaceutical composition of the present invention may be formulated into solid, semisolid, liquid, or gaseous formulations. Generally, the above-mentioned pharmaceutical composition may be prepared by conventional preparation methods using conventional excipients in the field of formulations.

A further object of the present invention is to provide the use of the compound of the present invention, or the tautomer, stereoisomer, isotopic derivative, or pharmaceutically acceptable salt thereof, or the pharmaceutical composition thereof, in the preparation of a medicament for the prevention and/or treatment of diseases caused by RSV (Respiratory Syncytial Virus) infection.

In another aspect, the present application provides a method for preventing and/or treating diseases caused by RSV infection, or for inhibiting RSV in a patient or a biological sample, comprising administering the compound of the present invention, or the tautomer, stereoisomer, isotopic derivative, or pharmaceutically acceptable salt thereof, or the pharmaceutical composition of the present invention to an individual in need, or contacting the biological sample with the compound of the present invention or the pharmaceutical composition thereof.

In another aspect, the present application provides the compound of the present invention, or the tautomer, stereoisomer, isotopic derivative, or pharmaceutically acceptable salt thereof, or the pharmaceutical composition of the present invention, for use in preventing and/or treating diseases caused by RSV infection, or for inhibiting RSV in a patient or a biological sample.

Further, in the use or method provided by the present invention, the compound of the present invention, or the tautomer, stereoisomer, isotopic derivative, or pharmaceutically acceptable salt thereof, or the pharmaceutical composition, is used in combination with other drug(s).

A further object of the present invention is to provide a pharmaceutical composition comprising the compound of the present invention, or the tautomer, stereoisomer, isotopic derivative, or pharmaceutically acceptable salt thereof, or the pharmaceutical composition of the present invention, and an additional compound. The additional compound is selected from pharmaceutical active ingredients other than the compounds of the present invention.

### Definitions

"*" indicates that a chiral center is present at the atom, with an R configuration, an S configuration, or a mixture of the two.

The term "more" in the phrase "optionally substituted with one or more substituents each independently selected from..." refers to 2, 3, 4, 5, 6, 7, 8, 9, or 10; preferably 2, 3, or 4; more preferably 2.

The term "optional", "arbitrary", "optionally" or "arbitrarily" means that the event or circumstance described subsequently may but is not mandatory to occur, and the description includes both a case where the event or circumstance occurs and a case where the event or circumstance does not occur.

The term "oxo" means that two hydrogen atoms at the same substitution position are replaced by the same oxygen atom to form a double bond.

The term "thio" means that two hydrogen atoms at the same substitution position are replaced by the same sulfur atom to form a double bond.

Unless otherwise specified, the term "alkyl" refers to a monovalent saturated aliphatic hydrocarbon group, including a linear or branched group containing 1-20 (e.g., 1, 2, 3, 4, 5, 6, 7, 8, 9, 10, 11, 12, 13, 14, 15, 16, 17, 18, 19, or 20) carbon atoms, preferably 1-18 carbon atoms (i.e., C₁₋₁₈ alkyl) or 1-10 carbon atoms (i.e., C₁₋₁₀ alkyl), further preferably 1-8 carbon atoms (C₁₋₈ alkyl), more preferably 1-6 carbon atoms (i.e., C₁₋₆ alkyl), and more preferably 1-4 carbon atoms (i.e., C₁₋₄ alkyl). For example, "C₁₋₆ alkyl" means that the group is an alkyl containing 1-6 (specifically 1, 2, 3, 4, 5, or 6) carbon atoms in the carbon chain. Examples include, but are not limited to, methyl, ethyl, n-propyl, isopropyl, n-butyl, isobutyl, tert-butyl, sec-butyl, n-pentyl, neopentyl, 1,1-dimethylpropyl, 1,2-dimethylpropyl, 2,2-dimethylpropyl, 1-ethylpropyl, 2-methylbutyl, 3-methylbutyl, n-hexyl, n-heptyl, n-octyl, etc.

Unless otherwise specified, the term "alkenyl" refers to a linear or branched unsaturated aliphatic hydrocarbon group composed of carbon and hydrogen atoms and containing at least one double bond. The alkenyl may contain 2-20 carbon atoms, preferably 2-10 carbon atoms (i.e., C₂₋₁₀ alkenyl), more preferably 2-8 carbon atoms (C₂₋₈ alkenyl), more preferably 2-6 carbon atoms (i.e., C₂₋₆ alkenyl), 2-5 carbon atoms (i.e., C₂₋₅ alkenyl), 2-4 carbon atoms (i.e., C₂₋₄ alkenyl), 2-3 carbon atoms (i.e., C₂₋₃ alkenyl), or 2 carbon atoms (i.e., C₂ alkenyl). For example, "C₂₋₆ alkenyl" means that the group is an alkenyl containing 2-6 (specifically 2, 3, 4, 5, or 6) carbon atoms in the carbon chain. Non-limiting examples of the alkenyl include vinyl, 1-propenyl, 2-propenyl, 1-butenyl, isobutenyl, 1,3-butadienyl, etc.

Unless otherwise specified, the term "alkynyl" refers to a linear or branched unsaturated aliphatic hydrocarbon group composed of carbon and hydrogen atoms and containing at least one triple bond. The alkynyl may contain 2-20 carbon atoms, preferably 2-10 carbon atoms (i.e., C₂₋₁₀ alkynyl), further preferably 2-8 carbon atoms (C₂₋₈ alkynyl), more preferably 2-6 carbon atoms (i.e., C₂₋₆ alkynyl), 2-5 carbon atoms (i.e., C₂₋₅ alkynyl), 2-4 carbon atoms (i.e., C₂₋₄ alkynyl), 2-3 carbon atoms (i.e., C₂₋₃ alkynyl), or 2 carbon atoms (i.e., C₂ alkynyl). For example, "C₂₋₆ alkynyl" means that the group is an alkynyl containing 2-6 (specifically 2, 3, 4, 5, or 6) carbon atoms in the carbon chain. Non-limiting examples of the alkynyl include ethynyl, 1-propynyl, 2-propynyl, 1-butynyl, etc.

Unless otherwise specified, the term "alkoxy" refers to -O-alkyl, where the alkyl is defined as above, containing 1-20 carbon atoms, preferably 1-10 carbon atoms, more preferably 1-8 carbon atoms, and even more preferably 1-6 carbon atoms (specifically 1, 2, 3, 4, 5, or 6 carbon atoms). Representative examples include, but are not limited to, methoxy, ethoxy, propoxy, isopropoxy, butoxy, 1-methylpropoxy, 2-methylpropoxy, tert-butoxy, pentyloxy, 1-methylbutoxy, 2-methylbutoxy, 3-methylbutoxy, 1,1-dimethylpropoxy, 1,2-dimethylpropoxy, 2,2-dimethylpropoxy, 1-ethylpropoxy, etc.

Unless otherwise specified, the term "halogen" or "halo" refers to F, Cl, Br, or I. The term "haloalkyl" refers to an alkyl group as defined above where one, two, more, or all hydrogen atoms are replaced by halogen. Representative examples of the haloalkyl include CCl₃, CF₃, CHCl₂, CH₂Cl, CH₂Br, CH₂I, CH₂CF₃, CF₂CF₃, etc.

Unless otherwise specified, the term "carbocyclyl" or "carbocycle" refers to a non-aromatic cyclic hydrocarbon group with 3 to 20 (e.g., 3, 4, 5, 6, 7, 8, 9, 10, 11, 12, 13, 14, 15, 16, 17, 18, 19, 20) ring carbon atoms ("C₃₋₂₀ carbocyclyl"), and with no heteroatoms in the non-aromatic ring system. In some examples, the carbocyclyl has 3-12 ring carbon atoms ("C₃₋₁₂ carbocyclyl"), 4-12 ring carbon atoms ("C₄₋₁₂ carbocyclyl"), or 3-10 ring carbon atoms ("C₃₋₁₀ carbocyclyl"). In some examples, the carbocyclyl has 3-8 ring carbon atoms ("C₃₋₈ carbocyclyl"). In some examples, the carbocyclyl has 3-7 ring carbon atoms ("C₃₋₇ carbocyclyl"). In some examples, the carbocyclyl has 4-6 ring carbon atoms ("C₄₋₆ carbocyclyl"). In some examples, the carbocyclyl has 5-10 ring carbon atoms ("C₅₋₁₀ carbocyclyl") or 5-7 ring carbon atoms ("C₅₋₇ carbocyclyl"). Exemplary C₃₋₆ carbocyclyl groups include, but are not limited to, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), etc. Exemplary C₃₋₈ carbocyclyl groups include, but are not limited to, the aforementioned C₃₋₆ carbocyclyl groups as well as cycloheptyl (C₇), cycloheptenyl (C₇), cycloheptadienyl (C₇), cycloheptatrienyl (C₇), cyclooctyl (C₈), cyclooctenyl (C₈), bicyclo[2.2.1]heptyl (C₇), bicyclo[2.2.2]octyl (C₈), etc. Exemplary C₃₋₁₀ carbocyclyl groups include, but are not limited to, the aforementioned C₃₋₈ carbocyclyl groups as well as cyclononyl (C₉), cyclononenyl (C₉), cyclodecyl (C₁₀), cyclodecenyl (C₁₀), octahydro-1H-indenyl (C₉), decahydronaphthyl (C₁₀), spiro[4.5]decyl (C₁₀), etc. As illustrated in the above examples, in certain examples, the carbocyclyl is monocyclic ("monocyclic carbocyclyl") or a fused (fused carbocyclyl), bridged (bridged cyclyl), or spiro (spirocyclyl) ring system, such as a bicyclic system ("bicyclic carbocyclyl"), and may be saturated or partially unsaturated. "Carbocyclyl" also includes ring systems wherein the carbocyclyl ring as defined above is fused with one or more aryl or heteroaryl groups, where the point of attachment is on the carbocyclyl ring, and in such cases, the number of members of the carbocyclyl ring system is the number of carbons in the resulting carbocyclic system after fusion. In certain examples, each example of the carbocyclyl groups is independently optionally substituted, e.g., unsubstituted ("unsubstituted carbocyclyl") or substituted with one or more substituents ("substituted carbocyclyl"). In certain examples, the carbocyclyl group is unsubstituted C₃₋₁₀ carbocyclyl. In certain examples, the carbocyclyl group is substituted with C₃₋₁₀ carbocyclyl.

Unless otherwise specified, the term "cycloalkyl" refers to a monocyclic saturated aliphatic hydrocarbon group with a specific number of carbon atoms, preferably 3-12 (e.g., 3, 4, 5, 6, 7, 8, 9, 10, 11, or 12) carbon atoms (i.e., C₃₋₁₂ cycloalkyl), more preferably 3-10 carbon atoms (C₃₋₁₀ cycloalkyl), and further preferably 3-7 carbon atoms (C₃₋₇ cycloalkyl), 4-6 carbon atoms (C₄₋₆ cycloalkyl), or 5-6 carbon atoms (C₅₋₆ cycloalkyl). Examples include, but are not limited to, cyclopropyl, cyclobutyl, cyclopentyl, cyclohexyl, methylcyclopropyl, 2-ethyl-cyclopentyl, dimethylcyclobutyl, etc.

Unless otherwise specified, the term "heterocyclyl" or "heterocycle" refers to a saturated or partially unsaturated monocyclic or polycyclic cyclic non-aromatic substituent having ring carbon atoms and 1 to 4 ring heteroatoms, containing 3-20 (e.g., 3, 4, 5, 6, 7, 8, 9, 10, 11, 12, 13, 14, 15, 16, 17, 18, 19, or 20) ring atoms, among which 1, 2, 3, or more ring atoms are selected from the group consisting of N, O, and S, and the remaining ring atoms are C. It contains preferably 3-18 ring atoms (3- to 18-membered heterocyclyl), more preferably 3-15 ring atoms (3- to 15-membered heterocyclyl), 3-12 ring atoms (3- to 12-membered heterocyclyl), 4-12 ring atoms (4- to 12-membered heterocyclyl), 3-10 ring atoms (3- to 10-membered heterocyclyl), 3-8 ring atoms (3- to 8-membered heterocyclyl), 3-7 ring atoms (3- to 7-membered heterocyclyl), 4-7 ring atoms (4- to 7-membered heterocyclyl), 3-6 ring atoms (3- to 6-membered heterocyclyl), 4-6 ring atoms (4- to 6-membered heterocyclyl), or 5-6 ring atoms (5- to 6-membered heterocyclyl). The number of heteroatoms is preferably 1-4, more preferably 1-3 (i.e., 1, 2, or 3). Examples of the monocyclic heterocyclyl include pyrrolidinyl, imidazolidinyl, tetrahydrofuranyl, dihydropyrrolyl, piperidinyl, piperazinyl, pyranyl, etc. The polycyclic heterocyclyl includes fused, spiro, and bridged heterocyclyl. "Heterocyclyl" may be monocyclic ("monocyclic heterocyclyl") or fused (fused heterocyclyl), bridged ("bridged heterocyclyl" or "bridged-ring heterocyclyl"), or spiro ("heterospirocyclyl", "spiroheterocyclyl", or "spirocyclic heterocyclyl") ring systems, such as a bicyclic system ("bicyclic heterocyclyl"), and may be saturated or partially unsaturated. The heterocyclyl bicyclic system may contain one or more heteroatoms in one or both rings. "Heterocyclyl" also includes ring systems wherein the heterocyclyl ring as defined above is fused with one or more carbocyclyl groups with the point of attachment on the carbocyclyl or heterocyclyl ring, or ring systems wherein the heterocyclyl ring as defined above is fused with one or more aryl or heteroaryl groups, or ring systems wherein the cycloalkyl ring as defined above is fused with one or more heteroaryl groups with the point of attachment on the heterocyclyl or cycloalkyl ring). In such cases, the number of members of the heterocyclyl ring system is the number of atoms in the resulting ring system after fusion. In certain examples, each example of the heterocyclyl groups is independently optionally substituted, e.g., unsubstituted ("unsubstituted heterocyclyl") or substituted with one or more substituents ("substituted heterocyclyl"). Exemplary 3-membered heterocyclyl groups containing 1 heteroatom include, but are not limited to, aziridinyl, oxiranyl, and thiiranyl. Exemplary 4-membered heterocyclyl groups containing 1 heteroatom include, but are not limited to, azetidinyl, oxetanyl, and thietanyl. Exemplary 5-membered heterocyclyl groups containing 1 heteroatom include, but are not limited to, tetrahydrofuranyl, dihydrofuranyl, tetrahydrothiophenyl, dihydrothiophenyl, pyrrolidinyl, dihydropyrrolyl, and pyrrolyl-2,5-dione. Exemplary 5-membered heterocyclyl groups containing 2 heteroatoms include, but are not limited to, dioxolanyl, oxathiolanyl, dithiolanyl, and oxazolidin-2-one. Exemplary 5-membered heterocyclyl groups containing 3 heteroatoms include, but are not limited to, triazolinyl, oxadiazolinyl, and thiadiazolinyl. Exemplary 6-membered heterocyclyl groups containing 1 heteroatom include, but are not limited to, piperidinyl, tetrahydropyranyl, dihydropyridinyl, and thianyl. Exemplary 6-membered heterocyclyl groups containing 2 heteroatoms include, but are not limited to, piperazinyl, morpholinyl, dithianyl, and dioxanyl. Exemplary 6-membered heterocyclyl groups containing 3 heteroatoms include, but are not limited to, triazinanyl, oxadiazinanyl, thiadiazinanyl, oxathiazinanyl, and dioxazinanyl. Exemplary 7-membered heterocyclyl groups containing 1 heteroatom include, but are not limited to, azepanyl, oxepanyl, and thiepanyl. Exemplary 8-membered heterocyclyl groups containing 1 heteroatom include, but are not limited to, azocanyl, oxocanyl, and thiocanyl. Exemplary 5-membered heterocyclyl groups fused to a C₆ aryl ring (also referred to herein as a 5,6-bicyclic heterocycle) include, but are not limited to, indolinyl, isoindolinyl, dihydrobenzofuranyl, dihydrobenzothiophenyl, benzoxazolinonyl, etc. Exemplary 6-membered heterocyclyl groups fused to one aryl ring (also referred to herein as a 6,6-bicyclic heterocycle) include, but are not limited to, tetrahydroquinolinyl, tetrahydroisoquinolinyl, etc.

Unless otherwise specified, the term "spirocyclyl" or "spirocycle" refers to a saturated, monovalent aliphatic hydrocarbon group containing only one spiro carbon atom, which may contain 6-20 ring carbon atoms, preferably 7-10 ring carbon atoms. The spirocyclyl includes 3-membered/5-membered, 4-membered/4-membered, 4-membered/5-membered, 4-membered/6-membered, 5-membered/5-membered, and 5-membered/6-membered spirocyclyl groups, etc., where the spiro atom is counted in the number of members of each ring. Exemplary examples of the spirocyclyl include (but are not limited to) 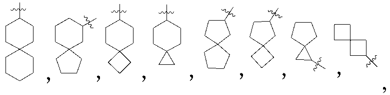 etc.

Unless otherwise specified, the term "heterospirocyclyl" or "spiroheterocyclyl" refers to a saturated, monovalent aliphatic group containing only one spiro carbon atom, which may contain 6-20 ring atoms, preferably 7-10 ring atoms, including 1-4 ring heteroatoms, preferably 1-3 (i.e., 1, 2, or 3) ring heteroatoms, wherein the heteroatoms are independently selected from the group consisting of N, O, and S. The heterospirocyclyl includes 3-membered/5-membered, 4-membered/4-membered, 4-membered/5-membered, 4-membered/6-membered, 5-membered/5-membered, and 5-membered/6-membered heterospirocyclyl groups, etc, where the spiro atom is counted in the number of members of each ring. Exemplary examples of the heterospirocyclyl include (but are not limited to) 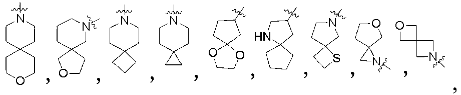 etc.

The fused carbocyclyl may contain 5-14 ring atoms, preferably 6-12 ring atoms, and more preferably 7-10 ring atoms. The fused carbocyclyl includes bicyclic, tricyclic, tetracyclic, or polycyclic fused carbocyclyl, preferably bicyclic, tricyclic, or tetracyclic fused carbocyclyl, and more preferably bicyclic or tricyclic fused carbocyclyl. Exemplary examples of the fused carbocyclyl include (but are not limited to) 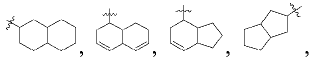 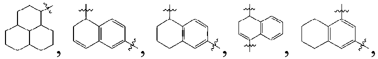 etc.

The fused heterocyclyl may contain 5-14 ring atoms, preferably 6-12 ring atoms, and more preferably 7-10 ring atoms, including 1-4 ring heteroatoms, preferably 1-3 (i.e., 1, 2, or 3) ring heteroatoms, where the heteroatoms are independently selected from the group consisting of N, O, and S. The fused heterocyclyl includes bicyclic, tricyclic, tetracyclic, or polycyclic fused heterocyclyl, preferably bicyclic, tricyclic, or tetracyclic fused heterocyclyl, and more preferably bicyclic or tricyclic fused heterocyclyl. Exemplary examples of the fused heterocyclyl include (but are not limited to) 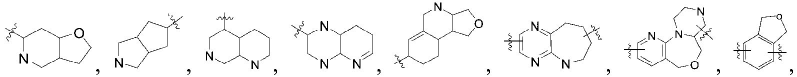 etc.

Unless otherwise specified, the term "bridged cyclyl" refers to a polycyclic, monovalent aliphatic hydrocarbon group wherein any two rings share two ring carbon atoms that are not directly connected, and it may contain 5-20 ring carbon atoms, preferably 6-14 ring carbon atoms, and more preferably 7-10 ring carbon atoms, and may contain one or more double bonds, but none of the rings has a fully conjugated π-electron system. The bridged cyclyl includes bicyclic, tricyclic, tetracyclic, or polycyclic bridged cyclyl, preferably bicyclic, tricyclic, or tetracyclic bridged cyclyl, and more preferably bicyclic or tricyclic bridged cyclyl. Exemplary examples of the bridged cyclyl include (but are not limited to) 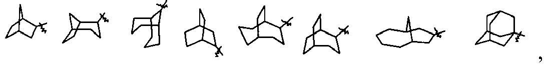 etc.

Unless otherwise specified, the term "bridged heterocyclyl" refers to a polycyclic, monovalent aliphatic group wherein any two rings share two ring atoms that are not directly connected, and it may contain 5-14 ring atoms, preferably 6-14 ring atoms, and more preferably 7-10 ring atoms, including 1-4 ring heteroatoms, preferably 1-3 (i.e., 1, 2, or 3) ring heteroatoms, wherein the heteroatoms are independently selected from the group consisting of N, O, and S, and may contain one or more double bonds, but none of the rings has a fully conjugated π-electron system. The bridged heterocyclyl includes bicyclic, tricyclic, tetracyclic, or polycyclic bridged heterocyclyl, preferably bicyclic, tricyclic, or tetracyclic bridged heterocyclyl, and more preferably bicyclic or tricyclic bridged heterocyclyl. Exemplary examples of the bridged heterocyclyl include (but are not limited to) 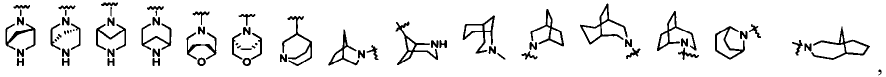 etc.

Unless otherwise specified, the term "aryl" or "aromatic cyclyl" refers to monocyclic, bicyclic, and tricyclic aromatic carbocyclic systems containing 6-16 (e.g., 6, 7, 8, 9, 10, 11, 12, 13, 14, 15, or 16) carbon atoms, or 6-14 carbon atoms, or 6-12 carbon atoms, or 6-10 carbon atoms, preferably 6-10 carbon atoms, more preferably 6-8 carbon atoms, more preferably phenyl. The term "aryl" can be used interchangeably with the term "aromatic ring". Examples of the aryl group include, but are not limited to, phenyl, naphthyl, anthracenyl, phenanthrenyl, or pyrenyl, etc.

Unless otherwise specified, the term "heteroaryl" or "heteroaromatic cyclyl" refers to an aromatic monocyclic or polycyclic ring system containing a 5- to 20-membered (e.g., 5, 6, 7, 8, 9, 10, 11, 12, 13, 14, 15, 16, 17, 18, 19, or 20-membered) structure, preferably a 5- to 18-membered, 5- to 12-membered, 5- to 10-membered, or 5- to 8-membered structure, more preferably a 5- to 6-membered structure, wherein 1, 2, 3, or more ring atoms are heteroatoms independently selected from the group consisting of O, N, and S and the remaining ring atoms are carbon atoms; and the number of heteroatoms is preferably 1, 2, or 3. The polycyclic heteroaryl is fused heteroaryl. Examples of the heteroaryl include, but are not limited to, furanyl, thienyl, oxazolyl, thiazolyl, isoxazolyl, oxadiazolyl, thiadiazolyl, pyrrolyl, pyrazolyl, imidazolyl, triazolyl, tetrazolyl, pyridyl, pyrimidinyl, pyrazinyl, pyridazinyl, thiadiazolyl, triazinyl, phthalazinyl, quinolinyl, isoquinolinyl, pteridinyl, purinyl, indolyl, isoindolyl, indazolyl, benzofuranyl, benzothienyl, benzopyridyl, benzopyrimidinyl, benzopyrazinyl, benzimidazolyl, benzophthalazinyl, pyrrolo[2,3-b]pyridyl, imidazo[1,2-a]pyridyl, pyrazolo[1,5-a]pyridyl, pyrazolo[1,5-a]pyrimidinyl, imidazo[1,2-b]pyridazinyl, [1,2,4]triazolo[4,3-b]pyridazinyl, [1,2,4]triazolo[1,5-a]pyrimidinyl, [1,2,4]triazolo[1,5-a]pyridyl, etc.

Linking substituents are described in various sections of the present invention. When the structure clearly requires a linking group, the Markush variables listed for that group should be understood as linking groups. For example, if the structure requires a linking group and the Markush group definition for that variable lists "alkyl" or "aryl", it should be understood that the "alkyl" or "aryl" represents an alkylene group or arylene group to be linked, respectively. The definition of such linking groups (e.g., alkylene) is the same as that of the term "alkyl", referring to divalent alkyl as defined above. The definition of C₀₋₆ alkylene includes C₀ (i.e., the linking group is merely a bond) and C₁₋₆ alkylene. The definition of C₁₋₆ alkylene is the same as that of the aforementioned C₁₋₆ alkyl.

Unless otherwise specified, the term "pharmaceutically acceptable salt" or "pharmaceutical salt" refers to those salts that are, within the scope of sound medical judgment, suitable for use in contact with mammalian tissues, particularly human tissues, without excessive toxicity, irritation, allergic responses, and the like, and are commensurate with a reasonable benefit/risk ratio. For example, medically acceptable salts of amines, carboxylic acids, and other types of compounds are well-known in the art. The salts may be prepared in situ during the final isolation and purification of the compounds of the present invention, or may be prepared separately by reacting the free base or free acid with an appropriate reagent.

Unless otherwise specified, the term "isotopic derivative" means that the compounds of the present invention may exist in isotopically labelled or enriched forms, containing one or more atoms whose atomic weight or mass number is different from that of the most abundant atom found in the nature. Isotopes may be radioactive or nonradioactive isotopes. Isotopes commonly used as isotopic labelling are: hydrogen isotopes, ²H and ³H; carbon isotopes: ¹³C and ¹⁴C; chlorine isotopes: ³⁵Cl and ³⁷Cl; fluorine isotope: ¹⁸F; iodine isotopes: ¹²³I and ¹²⁵I; nitrogen isotopes: ¹³N and ¹⁵N; oxygen isotopes: ¹⁵O, ¹⁷O and ¹⁸O; and sulfur isotope: ³⁵S. These isotopically labeled compounds can be used to study the distribution of pharmaceutical molecules in tissues. In particular, ³H and ¹³C are more widely used due to their ease of labeling and ease of detection. The substitution with certain heavy isotopes, such as heavy hydrogen (²H), can enhance metabolic stability, and prolong half-life, thereby achieving the purpose of reducing dosage and providing therapeutic advantages. The isotopically labeled compounds are generally synthesized starting from labeled starting materials in the same way as non-isotopically labeled compounds using known synthetic techniques.

Unless otherwise specified, the compounds of the present invention also comprise a "solvate" thereof. The term "solvate" means a physical association of a compound of the present invention with one or more solvent molecules, whether organic or inorganic. This physical association includes hydrogen bond. In certain instances, the solvate will be capable of isolation, for example, when one or more solvent molecules are incorporated into the crystal lattice of a crystalline solid. The solvent molecules in the solvate may be present in a regular arrangement and/or disordered arrangement. The solvate may comprise either a stoichiometric or non-stoichiometric number of solvent molecule(s). "Solvate" encompasses both solution phase and isolable solvates. Exemplary solvates include, but are not limited to, hydrates, ethanolates, methanolates, and isopropanolates. Methods of solvation are well known in the art. The term "hydrate" refers to a substance formed either by water molecules bound to cations or anions in a compound via coordinate bonds or covalent bonds, or by water molecules (not directly bound to cations or anions) existing in a certain proportion at specific positions in the solid crystal lattice.

Unless otherwise specified, the term "stereoisomer" refers to a compound that has the identical chemical constitution, but differs in the arrangement of atom(s) or group(s) in space. Stereoisomers include enantiomers, diastereomers, conformers (rotamers), geometric isomers (cis/trans), atropisomers, etc. A mixture of the resulting any stereoisomers can be separated into pure or substantially pure geometric isomers, enantiomers, or diastereomers, for example, by chromatography and/or fractional crystallization, on the basis of a difference in a physicochemical property of the components. Unless otherwise specified, the term "geometric (cis/trans) isomers" may include carbon-carbon double bonds or carbon-nitrogen double bonds with E or Z configuration, wherein the term "E" denotes the higher-order substituent on the opposite side of the carbon-carbon or carbon-nitrogen double bond, and the term "Z" denotes the higher-order substituent on the same side of the carbon-carbon or carbon-nitrogen double bond (determined using the Cahn-Ingold-Prelog priority rules). The compounds of the present invention may also exist as a mixture of "E" and "Z" isomers.

Unless otherwise specified, the term "tautomer" refers to structural isomers with different energies which are interconvertible via a low energy barrier. If tautomerism is possible (such as, in solution), a chemical equilibrium of tautomers can be achieved. For example, proton tautomers (also known as prototropic tautomers) include interconversions via migration of a proton, such as keto-enol isomerization and imine-enamine isomerization. Valence tautomers include interconversions by reorganization of some bonding electrons.

Unless indicated otherwise, the structural formulae depicted in the present invention include all isomeric (e.g., enantiomeric, diastereomeric, and geometric (or conformational) forms: e.g., R and S configurations containing an asymmetric center, (Z) and (E) isomers of double bond, and (Z) and (E) conformational isomers. Therefore, individual stereochemical isomers of the compounds of the present invention or mixtures of enantiomers, diastereomers, or geometrical isomers (or conformational isomers) thereof are within the scope of the present invention.

Unless otherwise specified, the compounds of the present invention also comprise a "prodrug" thereof. The term "prodrug" refers to a drug that is converted to its parent drug *in vivo.* Prodrugs are usually useful, and may improve some identified and undesirable physical or biological properties. Physical properties are generally relevant solubility (excessive or insufficient solubility in lipid or water) or stability, while problematic biological properties include too rapid metabolism or poor bioavailability, which may be related to physicochemical properties. For example, they may be bioavailable by oral administration, while the parent drug is not. The solubility of a prodrug in a pharmaceutical composition is also improved compared to a parent drug thereof. One example of a prodrug may be, but is not limited to, any one of the compounds of the present invention administered as an ester ("prodrug") to facilitate delivery through cell membranes, where the water solubility thereof is detrimental to migration, but beneficial once the prodrug enters a cell, and the prodrug is subsequently metabolized and hydrolyzed to a carboxylic acid, i.e., the active entity. Another example of a prodrug may be a short peptide (polyamino acid) bound to an acid group, in which the peptide is metabolized to present an active moiety.

Unless otherwise specified, the term "treatment/treating" encompasses any treatment of a disease, disorder, or condition in a patient, including: (a) inhibiting the symptoms of the disease, disorder, or condition, i.e., preventing its progression; or (b) alleviating the symptoms of the disease, disorder, or condition, i.e., causing regression of the disease or symptoms; or (c) ameliorating or eliminating the disease, disorder, or condition, or one or more symptoms associated with the disease.

Abbreviations used in Preparation Examples, Examples, and elsewhere herein are:
DCM: dichloromethane; TEA: triethylamine; DIEA: *N,N-*diisopropylethylamine; DMF: *N,N-*dimethylformamide; EtOAc: ethyl acetate; H: Hour; mL: milliliter; MeOH: methanol; TFA: trifluoroacetic acid; NBS: N-bromosuccinimide; NIS: N-iodosuccinimide; THF: tetrahydrofuran; Pd(dppf)Cl₂: [1,1'-bis(diphenylphosphino)ferrocene]dichloropalladium(II); DMSO-*d₆*: deuterated dimethyl sulfoxide; Toluene: methylbenzene; mCPBA: m-chloroperoxybenzoic acid; PE: petroleum ether; EA: ethyl acetate; LAH: lithium aluminum hydride.

The beneficial effects of the present invention are as follows:
The present invention designs a class of compounds with novel structures. *In vitro* cell studies show that the compounds of the present invention have good inhibitory effects on RSV. The hERG test indicates that the compounds of the present invention have low risk of cardiotoxicity and good safety. *In vivo* experiments demonstrate that the compounds of the present invention possess favorable pharmacokinetic properties and good *in vivo* antiviral efficacy. In addition, the present invention explores a specific synthesis method, which features simple processes and convenient operations, facilitating large-scale industrial production and application.

### DETAILED DESCRIPTION

The present invention will be further described below in conjunction with specific examples. It should be understood that these examples are merely used to illustrate the present invention, and are not intended to limit the scope of the present invention. For the experimental methods in the following examples where specific conditions are not indicated, conventional conditions or conditions recommended by the manufacturer are generally followed. Unless otherwise defined, all professional and scientific terms used herein have the same meaning as commonly understood by one of ordinary skill in the art. In addition, any methods and materials similar or equivalent to those described herein can be applied to the methods of the present invention. The preferred implementation methods and materials shown herein are illustrative only.

The chiral compounds involved in the present invention can be isolated and purified to obtain optically pure monomeric compounds using supercritical chromatography equipment, employing chiral columns with conventional solvents as the mobile phase.

The following are Preparation Examples of the exemplary compounds of the present invention.

### Intermediate Preparation Example 1-1: Synthesis of 2,4-dichloro-6-methyl-5,6,7,8-tetrahydropyrido[4,3-d]pyrimidine

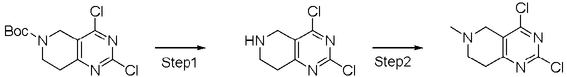

### Step 1: Synthesis of 2,4-dichloro-5,6,7,8-tetrahydropyrido[4,3-d]pyrimidine

tert-Butyl 2,4-dichloro-7,8-dihydropyrido[4,3-*d*]pyrimidin-6(5*H*)-carboxylate (0.5 g, 1.64 mmol), DCM (5 mL) and trifluoroacetic acid (2 mL) were sequentially added to a 100 mL reaction flask. The resulting reaction mixture was stirred at room temperature for 3 hours. LCMS indicated that the reaction was complete. For post-treatment, the resulting mixture was concentrated to dryness under reduced pressure to obtain crude 2,4-dichloro-5,6,7,8-tetrahydropyrido[4,3-*d*]pyrimidine, which was used directly in the next step of the reaction without further purification. ESI-MS(m/z): 204.00[M+H]⁺.

### Step 2: Synthesis of 2,4-dichloro-6-methyl-5,6,7,8-tetrahydropyrido[4,3-d]pyrimidine

DCM (4 mL) and methanol (1 mL) were added to the crude 2,4-dichloro-5,6,7,8-tetrahydropyrido[4,3-d]pyrimidine obtained in Step 1. The pH of the resulting reaction liquid was adjusted to 9 with triethylamine, and then adjusted to 5 with the dropwise addition of glacial acetic acid. Paraformaldehyde (0.45 g) was added with stirring at room temperature, the stirring was continued further for 1 hour, sodium cyanoborohydride (0.31 g, 4.9 mmol) was then added, and the resulting mixture was stirred at room temperature overnight to complete the reaction. LCMS indicated that the reaction was complete, and the reaction mixture was diluted with dichloromethane, washed with water, and concentrated to dryness under reduced pressure. The resulting concentrate was purified by column chromatography (DCM: MeOH = 40 : 1(v/v)) to obtain 2,4-dichloro-6-methyl-5,6,7,8-tetrahydropyrido[4,3-*d*]pyrimidine (0.4 g). ESI-MS(m/z): 218.02[M+H]⁺.

### Intermediate Preparation Example 2-3: Synthesis of 2,3,4,5-tetrahydrobenzo[f][1,4]thiazepin-1,1-dioxide

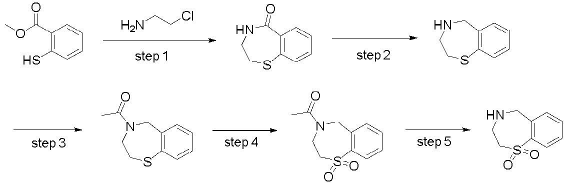

### Step 1: Synthesis of 3,4-dihydrobenzo[f][1,4]thiazepin-5(2H)-one

Methyl 2-mercaptobenzoate (3.00 g, 17.8 mmol), 2-chloroethylamine hydrochloride (2.07 g, 17.8 mmol), DMF (10 mL), and THF (10 mL) were added to a 100 mL reaction flask. Sodium hydride (2.22 g, 55.5 mmol) was slowly added at 0 °C. After LCMS indicated that the reaction was complete, water was added to the reaction mixture, and extraction was performed with ethyl acetate. The organic phase was concentrated under reduced pressure, slurried with a mixed solution (EA:PE = 1:1), and filtered to obtain 3,4-dihydrobenzo[*f*][1,4]thiazepin-5(2H)-one (1.30 g). ESI-MS(m/z): 180.05[M+H]⁺.

### Step 2: Synthesis of 2,3,4,5-tetrahydrobenzo[f][1,4]thiazepine

3,4-Dihydrobenzo[*f*][1,4]thiazepin-5(2*H*)-one (1.30 g, 7.3 mmol) and THF (20 mL) were added to a 100 mL reaction flask. Lithium aluminum hydride was slowly added at 0 °C, and the reaction was carried out at 60 °C for 7 hours. After LCMS indicated that the reaction was complete, water, a 15% sodium hydroxide solution, and water were slowly added at 0 °C to quench the reaction. The resulting mixture was filtered, and the filtrate was concentrated under reduced pressure to obtain 2,3,4,5-tetrahydrobenzo[*f*][1,4]thiazepine (1.10 g). ESI-MS(m/z): 166.06[M+H]⁺.

### Step 3: Synthesis of 1-(2,3-dihydrobenzo[f][1,4]thiazepin-4(5H)-yl)ethan-1-one

2,3,4,5-Tetrahydrobenzo[*f*][1,4]thiazepine (1.10 g, 5.3 mmol), triethylamine (1.11 mL, 6.3 mmol), and dichloromethane (10 mL) were added to a 50 mL reaction flask. Acetic anhydride (0.75 mL, 6.3 mmol) was added dropwise at 0°C. The reaction was conducted at room temperature for 1 hour. After LCMS indicated that the reaction was complete, the reaction mixture was washed with a saturated sodium chloride solution and dried over sodium sulfate. The organic phase was concentrated under reduced pressure to obtain 1-(2,3-dihydrobenzo[*f*][1,4]thiazepin-4(5*H*)-yl)ethan-1-one (1.08 g). ESI-MS(m/z): 208.08[M+H]⁺.

### Step 4: Synthesis of 1-(1,1-dioxido-2,3-dihydrobenzo[f][1,4]thiazepin-4(5H)-yl)ethan-1-one

1-(2,3-Dihydrobenzo[*f*][1,4]thiazepin-4(5*H*)-yl)ethan-1-one (1.08 g, 5.2 mmol) and dichloromethane (10 mL) were added to a 50 mL reaction flask. mCPBA (2.69 g, 15.6 mmol) dissolved in dichloromethane was slowly added dropwise at 0 °C, and the reaction was carried out at room temperature for 2 hours. After LCMS indicated that the reaction was complete, the reaction mixture was washed with a saturated aqueous sodium carbonate solution, sodium thiosulfate, and then an aqueous sodium chloride solution. The organic phase was dried over anhydrous sodium sulfate, concentrated under reduced pressure, washed with diethyl ether, and filtered to obtain 1-(1,1-dioxido-2,3-dihydrobenzo[*f*][1,4]thiazepin-4(5*H*)-yl)ethan-1-one (1.00 g). ESI-MS(m/z): 240.18[M+H]⁺.

### Step 5: Synthesis of 2,3,4,5-tetrahydrobenzo[f][1,4]thiazepine-1,1-dioxide

1-(1,1-Dioxido-2,3-dihydrobenzo[*f*][1,4]thiazepin-4(5*H*)-yl)ethan-1-one (1.00 g, 4.2 mmol), sodium hydroxide (0.84 g, 21.0 mmol), ethanol (10 mL), and water (7 mL) were added to a 50 mL reaction flask. The reaction was conducted at 80 °C for 16 hours. After LCMS indicated that the reaction was complete, the organic solvent was removed by rotary evaporation. The residue was slurried with water and filtered to obtain 2,3,4,5-tetrahydrobenzo[*f*][1,4]thiazepine-1,1-dioxide (0.66 g). ESI-MS(m/z): 198.11[M+H]⁺.

### Intermediate Preparation Example 2-5: Synthesis of 2,3,4,5-tetrahydropyrido[2,3-f][1,4]thiazepine-1,1-dioxide

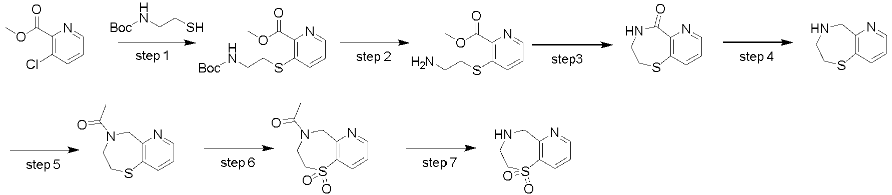

### Step 1: Synthesis of methyl 3-((2-((tert-butoxycarbonyl)amino)ethyl)thio)picolinate

Methyl 3-chloropicolinate (8 g, 46.63 mmol) was added to DMF (80 mL), followed by the addition of K₂CO₃ (8 g, 46.63 mmol, 3 eq) and tert-butyl (2-mercaptoethyl)carbamate (8.26 g, 46.63 mmol) sequentially. The resulting reaction mixture was stirred at 110 °C for 16 hours. LCMS monitored the formation of the product, the reaction mixture was extracted with water and ethyl acetate, and the organic phase was washed with semi-saturated brine, dried over anhydrous sodium sulfate, filtered, and concentrated under reduced pressure to obtain a crude product. The crude product was purified by column chromatography (PE:EA = 1:1) to obtain methyl 3-((2-((tert-butoxycarbonyl)amino)ethyl)thio)picolinate (2.8 g). ESI-MS(m/z): 313.38[M+H]⁺.

### Step 2: Synthesis of methyl 3-((2-aminoethyl)thio)picolinate

Methyl 3-((2-((tert-butoxycarbonyl)amino)ethyl)thio)picolinate (2.8 g, 8.96 mmol) was dissolved in DCM (28 mL) and HCl/1,4-dioxane (14 mL), and the mixed solution was stirred for reaction at 20°C for 2 hours. TLC detected the complete consumption of the starting material. The reaction mixture was concentrated under reduced pressure to obtain methyl 3-((2-aminoethyl)thio)picolinate hydrochloride (2.5 g). ESI-MS(m/z): 213.27[M+H]⁺.

### Step 3: Synthesis of 3,4-dihydropyrido[2,3-f][1,4]thiazepin-5(2H)-one

Methyl 3-((2-aminoethyl)thio)picolinate hydrochloride (2.5 g, 10.05 mmol) was dissolved in THF (125 mL) and MeOH (125 mL). CH₃ONa (3.26 g, 60.31 mmol) was added to the reaction solution at 0°C, and the mixed solution was stirred for reaction at 48 °C for 16 hours. LCMS detected the complete consumption of the starting material. After the solvent was removed from the reaction solution by vacuum distillation, the residue was extracted with water and dichloromethane. The organic phase was dried over anhydrous sodium sulfate and distilled under reduced pressure to obtain 3,4-dihydropyrido[2,3-f][1,4]thiazepin-5(2*H*)-one (1.3 g). ESI-MS(m/z):181.23[M+H]⁺.

### Step 4: Synthesis of 2,3,4,5-tetrahydropyrido[2,3-f][1,4]thiazepine

3,4-Dihydropyrido[2,3-*f*][1,4]thiazepin-5(2H)-one (1.1 g, 6.10 mmol) was dissolved in THF (22 mL). LAH (463.30 mg, 12.21 mmol) was added to the reaction solution at 0°C, and the mixed solution was stirred for reaction at 60°C for 4 hours. LCMS detected the complete consumption of the starting material. The reaction mixture was purified by column chromatography (DCM:MeOH = 10:1) to obtain 2,3,4,5-tetrahydropyrido[2,3-*f*][1,4]thiazepine (577.7 mg). ESI-MS(m/z):167.24 [M+H]⁺.

### Step 5: Synthesis of 1-(2,3-dihydropyrido[2,3-f][1,4]thiazepin-4(5H)-yl)ethan-1-one

2,3,4,5-Tetrahydropyrido[2,3-f][1,4]thiazepine (577.7 mg, 3.48 mmol) was dissolved in DCM (20 mL). Triethylamine (0.6 mL, 4.8 mmol) and acetic anhydride (0.3 mL, 4.2 mmol) were added at 0°C, and the resulting mixture was heated to room temperature and stirred for 2 hours. LCMS detected the complete consumption of the starting material. After the solvent was removed from the reaction mixture by vacuum distillation, the residue was extracted with water and dichloromethane. The organic phase was spin-dried and purified by silica gel column chromatography (DCM:MeOH = 30:1) to obtain 1-(2,3-dihydropyrido[2,3-f][1,4]thiazepin-4(5*H*)-yl)ethan-1-one (347.4 mg). ESI-MS(m/z):209.28 [M+H]⁺.

### Step 6: Synthesis of 1-(1,1-dioxo-2,3-dihydropyrido[2,3-f][1,4]thiazepin-4(5H)-yl)ethan-1-one

1-(2,3-Dihydropyrido[2,3-f][1,4]thiazepin-4(5*H*)-yl)ethan-1-one (347.4 mg, 1.67 mmol) was dissolved in DCM (20 mL). mCPBA (0.86 g, 4.9 mmol) was added at 0°C, and the resulting mixture was heated to room temperature and stirred for 2 hours. LCMS detected the complete consumption of the starting material. After the solvent was removed from the reaction mixture by vacuum distillation, the residue was extracted with water and dichloromethane. The organic phase was spin-dried and purified by silica gel column chromatography (DCM:MeOH = 30:1) to obtain 1-(1,1-dioxo-2,3-dihydropyrido[2,3-f][1,4]thiazepin-4(5*H*)-yl)ethan-1-one (289.1 mg). ESI-MS(m/z):241.28 [M+H]⁺.

### Step 7: Synthesis of 2,3,4,5-tetrahydropyrido[2,3-f][1,4]thiazepine-1,1-dioxide

1-(1,1-Dioxo-2,3-dihydropyrido[2,3-f][1,4]thiazepin-4(5*H*)-yl)ethan-1-one (289.1 mg, 1.20 mmol) was dissolved in EtOH/H₂O (20 mL, v/v=1:1). Sodium hydroxide (0.48 g, 12 mmol) was added, and the resulting mixture was heated to 78°C and reacted for 20 hours. LCMS detected the complete consumption of the starting material. After the solvent was removed from the reaction mixture by vacuum distillation, the residue was purified by silica gel column chromatography (DCM:MeOH = 30:1) to obtain 2,3,4,5-tetrahydropyrido[2,3-f][1,4]thiazepine-1,1-dioxide (110.2 mg). ESI-MS(m/z):199.24 [M+H]⁺.

### Intermediate Preparation Example 2-6: Synthesis of 2,3,4,5-tetrahydropyrido[3,4-f][1,4]thiazepine-1,1-dioxide

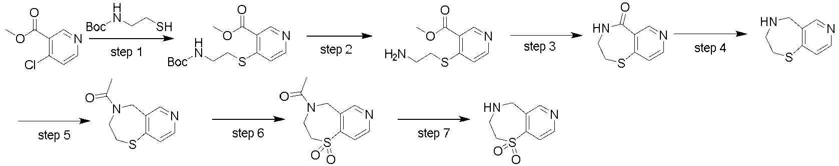

The synthesis method was the same as that of Intermediate Preparation Example 2-5, except that methyl 4-chloronicotinate was used instead of methyl 3-chloropicolinate as the starting material, to obtain 2,3,4,5-tetrahydropyrido[3,4-*f*][1,4]thiazepine-1,1-dioxide (98.2 mg).

### Intermediate Preparation Example 2-14: Synthesis of 1-imino-2,3,4,5-tetrahydro-1H-1λ⁴-benzo[f][1,4]thiazepine1-oxide

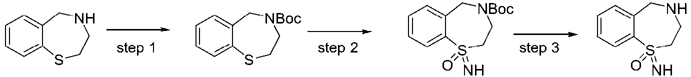

### Step 1: Synthesis of tert-butyl 2,3-dihydrobenzo[f][1,4]thiazepine-4(5H)-carboxylate

2,3,4,5-Tetrahydrobenzo[*f*][1,4]thiazepine (1 g, 6 mmol), DCM (10 ml), TEA (1.2 g, 12 mmol), and Boc anhydride (1.6 g, 7.2 mmol) were added to a 50 mL reaction flask. After the reaction was complete, H₂O was added, and the resulting mixture was extracted with DCM. The organic phase was washed with a saturated sodium chloride solution, collected, dried over anhydrous sodium sulfate, and concentrated under reduced pressure to obtain tert-butyl 2,3-dihydrobenzo[*f*][1,4]thiazepine-4(5*H*)-carboxylate (1.6 g, crude product). ESI-MS(m/z): 266.11[M+H]⁺.

### Step 2: Synthesis of tert-butyl 1-imino-1,2,3,5-tetrahydro-4H-1λ⁴-benzo[f][1,4]thiazepine-4-carboxylate 1-oxide

tert-Butyl 2,3-dihydrobenzo[*f*][1,4]thiazepine-4(5*H*)-carboxylate (1.6 g, 6 mmol) was added to MeOH (20 ml) in a 50 mL reaction flask, followed by the addition of ammonium carbonate (864 mg, 9 mmol). After stirring for 5 minutes, iodobenzene diacetate (4.4 g, 13.8 mmol) was added, and the reaction was carried out at room temperature overnight. The reaction mixture was concentrated under reduced pressure to obtain a crude product, which was purified by silica gel chromatography (EA:PE = 1:1) to obtain tert-butyl 1-imino-1,2,3,5-tetrahydro-4*H*-1λ⁴-benzo[*f*][1,4]thiazepine-4-carboxylate 1-oxide (1.3 g). ESI-MS(m/z): 297.12[M+H]⁺.

### Step 3: Synthesis of 1-imino-2,3,4,5-tetrahydro-1H-1λ⁴-benzo[f][1,4]thiazepine-1-oxide

tert-Butyl 1-imino-1,2,3,5-tetrahydro-4*H*-1λ⁴-benzo[*f*][1,4]thiazepine-4-carboxylate 1-oxide (1.3 g, 4.4 mmol) was added to a 50 mL reaction flask, followed by the addition of HCl/1,4-dioxane (15 mL, 4M). The reaction was conducted at room temperature for 14 hours. After the reaction was complete, the reaction mixture was concentrated under reduced pressure to obtain a crude product. The crude product was added into water, and the pH was adjusted to 9-10 with a saturated potassium carbonate solution. The resulting mixture was extracted with ethyl acetate, and the organic phase was washed with a saturated sodium chloride solution, collected, dried over anhydrous sodium sulfate, and concentrated under reduced pressure to obtain 1-imino-2,3,4,5-tetrahydro-1*H*-1λ⁴-benzo[*f*][1,4]thiazepine-1-oxide (560 mg). ESI-MS(m/z): 197.07[M+H]⁺.

### Intermediate Preparation Example 2-15: Synthesis of 1-(methylimino)-2,3,4,5-tetrahydro-1H-1λ⁴-benzo[f][1,4]thiazepine-1-oxide

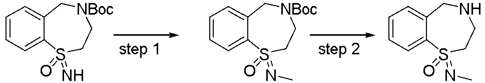

### Step 1: Synthesis of tert-butyl 1-(methylimino)-1,2,3,5-tetrahydro-4H-1λ⁴-benzo[f][1,4]thiazepine-4-carboxylate 1-oxide

tert-Butyl 1-imino-1,2,3,5-tetrahydro-4*H*-1λ⁴-benzo[*f*][1,4]thiazepine-4-carboxylate 1-oxide (2 g, 6.7 mmol) and THF (20 mL) were added to a 50 mL reaction flask. Sodium hydride (400 mg, 10 mmol) was slowly added at 0 °C. The reaction was carried out at room temperature for 0.5 hour, then iodomethane (1.05 g, 7.4 mmol) was added. The reaction was continued at room temperature for 2 hours. After the reaction was complete, water was added to the reaction mixture, and the resulting mixture was extracted with ethyl acetate. The organic phase was concentrated under reduced pressure to obtain a crude product, which was purified by silica gel chromatography (EA:PE = 1:1) to obtain tert-butyl 1-(methylimino)-1,2,3,5-tetrahydro-4*H*-1λ⁴-benzo[*f*][1,4]thiazepine-4-carboxylate 1-oxide (1.7 g). ESI-MS(m/z): 311.14[M+H]⁺.

### Step 2: Synthesis of 1-(methylimino)-2,3,4,5-tetrahydro-1H-1λ⁴- benzo[f][1,4]thiazepine-1-oxide

tert-Butyl 1-(methylimino)-1,2,3,5-tetrahydro-4*H*-1λ⁴- benzo[*f*][1,4]thiazepine-4-carboxylate 1-oxide (1.7 g, 5.5 mmol) was added to a 50 mL reaction flask, followed by the addition of HCl/1,4-dioxane (20 mL, 4M). The reaction was conducted at room temperature for 14 hours. After the reaction was complete, the reaction mixture was concentrated under reduced pressure to obtain a crude product. The crude product was added into water, and the pH was adjusted to 9-10 with a saturated potassium carbonate solution. The resulting mixture was extracted with ethyl acetate, and the organic phase was washed with a saturated sodium chloride solution, collected, dried over anhydrous sodium sulfate, and concentrated under reduced pressure to obtain 1-(methylimino)-2,3,4,5-tetrahydro-1*H*-1λ⁴-benzo[*f*][1,4]thiazepine-1-oxide (630 mg). ESI-MS(m/z): 211.08 [M+H]⁺.

### Intermediate Preparation Example 2-16: Synthesis of N-(1-oxido-2,3,4,5-tetrahydro-1λ⁴-benzo[f][1,4] thiazepin-1-ylidene)cyanamide

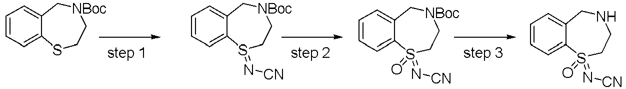

### Step 1: Synthesis of tert-butyl (E)-1-(cyanoimino)-1,2,3,5-tetrahydro-4H-1λ⁴-benzo[f][1,4]thiazepine-4-carboxylate

tert-Butyl 2,3-dihydrobenzo[*f*][1,4]thiazepine-4(5*H*)-carboxylate (1.7 g, 6.4 mmol) was added into MeOH (20 ml) in a 50 mL reaction flask, followed by the addition of ammonium cyanide (422 mg, 9.6 mmol). After stirring for 5 minutes, iodobenzene diacetate (2.3 g, 7 mmol) was added, and the reaction was carried out at room temperature overnight. The reaction mixture was concentrated under reduced pressure to obtain a crude product, which was purified by silica gel chromatography (EA:PE = 2:1) to obtain tert-butyl (*E*)-1-(cyanoimino)-1,2,3,5-tetrahydro-4*H*-1λ⁴-benzo[*f*][1,4]thiazepine-4-carboxylate (1.4 g). ESI-MS(m/z): 306.12[M+H]⁺.

### Step 2: Synthesis of tert-butyl 1-(cyanoimino)-1,2,3,5-tetrahydro-4H-1λ⁴-benzo[f][1,4]thiazepine-4-carboxylate 1-oxide

tert-Butyl (*E*)-1-(cyanoimino)-1,2,3,5-tetrahydro-4*H*-1λ⁴-benzo[*f*][1,4]thiazepine-4-carboxylate (1.4 g, 4.6 mmol) was added into ACN (70 ml) and DCM (7 ml) in a 50 mL reaction flask, followed by the addition of ruthenium chloride (9.5 mg, 0.046 mmol) and sodium periodate (1.5 g, 6.9 mmol). The reaction was carried out at room temperature overnight. The reaction mixture was concentrated under reduced pressure to obtain a crude product, which was purified by silica gel chromatography (EA:PE = 2:1) to obtain tert-butyl 1-(cyanoimino)-1,2,3,5-tetrahydro-4*H*-1λ⁴-benzo[*f*][1,4]thiazepine-4-carboxylate 1-oxide (1.1 g). ESI-MS(m/z): 322.11[M+H]⁺.

### Step 3: Synthesis of N-(1-oxido-2,3,4,5-tetrahydro-1λ⁴-benzo[f][1,4]thiazepin-1-ylidene)cyanamide

tert-Butyl 1-(cyanoimino)-1,2,3,5-tetrahydro-4*H*-1λ⁴-benzo[*f*][1,4]thiazepine-4-carboxylate 1-oxide (1.1 g, 3.4 mmol) was added into a 50 mL reaction flask, followed by the addition of HCl/1,4-dioxane (15 mL, 4M). The reaction was conducted at room temperature for 14 hours. After the reaction was complete, the reaction mixture was concentrated under reduced pressure to obtain a crude product. The crude product was added into water, and the pH was adjusted to 9-10 with a saturated potassium carbonate solution. The resulting mixture was extracted with ethyl acetate, and the organic phase was washed with a saturated sodium chloride solution, collected, dried over anhydrous sodium sulfate, and concentrated under reduced pressure to obtain *N*-(1-oxido-2,3,4,5-tetrahydro-1λ⁴-benzo[*f*][1,4]thiazepin-1-ylidene)cyanamide (300 mg). ESI-MS(m/z): 222.06[M+H]⁺.

### Intermediate Preparation Example 2-19: Synthesis of 1-(cyclopropylimino)-2,3,4,5-tetrahydro-1H-1λ⁴-benzo[f][1,4]thiazepine-1-oxide

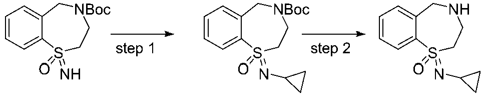

### Step 1: Synthesis of tert-butyl 1-(cyclopropylimino)-1,2,3,5-tetrahydro-4H-1λ⁴-benzo[f][1,4]thiazepine-4-carboxylate 1-oxide

tert-Butyl 1-imino-1,2,3,5-tetrahydro-4*H*-1λ⁴-benzo[*f*][1,4]thiazepine-4-carboxylate 1-oxide (200 mg, 0.7 mmol), Cu(OAc)₂ (200 mg, 1.1 mmol), cyclopropylboronic acid (120 mg, 1.4 mmol), pyridine (134 mg, 1.7 mmol), and 1,4-dioxane (20 mL) were added into a 50 mL reaction flask. The reaction was carried out at 100°C for 8 hours. After the reaction was completed, water was added into the reaction mixture, and the resulting mixture was extracted with ethyl acetate. The organic phase was concentrated under reduced pressure to obtain a crude product, which was purified by silica gel chromatography (EA:PE = 1:1) to obtain tert-butyl 1-(cyclopropylimino)-1,2,3,5-tetrahydro-4*H*-1λ⁴-benzo[*f*][1,4]thiazepine-4-carboxylate 1-oxide (150 mg). ESI-MS(m/z): 337.15[M+H]⁺.

### Step 2: Synthesis of 1-(cyclopropylimino)-2,3,4,5-tetrahydro-1H-1λ⁴-benzo[f][1,4]thiazepine-1-oxide

tert-Butyl 1-(cyclopropylimino)-1,2,3,5-tetrahydro-4*H*-1λ⁴-benzo[*f*][1,4]thiazepine-4-carboxylate 1-oxide (150 mg) was added into a 50 mL reaction flask, followed by the addition of HCl/1,4-dioxane (20 mL, 4M). The reaction was conducted at room temperature for 14 hours. After the reaction was completed, the reaction mixture was concentrated under reduced pressure to obtain a crude product. The crude product was added into water, and the pH was adjusted to 9-10 with a saturated potassium carbonate solution. The resulting mixture was extracted with ethyl acetate, and the organic phase was washed with a saturated sodium chloride solution, collected, dried over anhydrous sodium sulfate, and concentrated under reduced pressure to obtain 1-(cyclopropylimino)-2,3,4,5-tetrahydro-1*H*-1λ⁴- benzo[*f*][1,4]thiazepine-1-oxide (100 mg). ESI-MS(m/z): 237.10 [M+H]⁺.

### Intermediate Preparation Example 3-1: Synthesis of 4-methoxybenzyl (3-(aminomethyl)oxetan-3-yl)carbamate

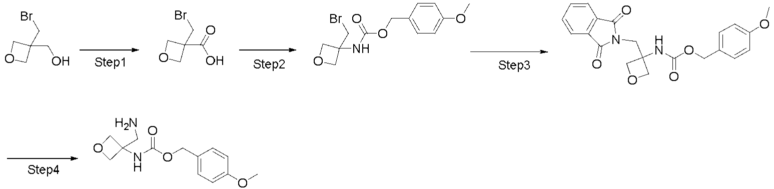

### Step 1: Synthesis of 3-(bromomethyl)oxetane-3-carboxylic acid

(3-(Bromomethyl)oxetan-3-yl)methanol (8.5 g, 46.95 mmol), acetonitrile (25 mL), water (130 mL), and KBr (0.57 g, 4.79 mmol) were sequentially added into a 500 mL reaction flask. A 7% aqueous sodium hypochlorite solution (120 mL) was added dropwise to the reaction mixture at a temperature that was not higher than 30°C. After the dropwise addition was completed, the reaction was conducted at room temperature for 4 hours. LCMS indicated that the reaction was complete. An aqueous sodium bicarbonate solution was added to adjust the pH of the system to 10, and the system was then extracted with ethyl acetate. The obtained aqueous phase was adjusted to pH 2 with 5N sulfuric acid, then extracted with dichloromethane, and concentrated under reduced pressure to dryness to obtain 3-(bromomethyl)oxetane-3-carboxylic acid (5.6 g).

### Step 2: Synthesis of 4-methoxybenzyl (3-(bromomethyl)oxetan-3-yl)carbamate

3-(Bromomethyl)oxetane-3-carboxylic acid (5 g, 25.77 mmol), toluene (50 mL), N-methylmorpholine (3.1 g, 30.65 mmol), (4-methoxyphenyl)methanol (3.6 g, 26.06 mmol), and DPPA (7.8 g, 28.34 mmol) were sequentially added into a 500 mL reaction flask. The reaction mixture was heated to 80°C and reacted for 4 hours. LCMS indicated that the reaction was complete. The resulting mixture was cooled to room temperature and diluted with ethyl acetate. The resulting system was washed with a saturated aqueous sodium bicarbonate solution, water, and saturated brine respectively. The organic phase was concentrated under reduced pressure to obtain 4-methoxybenzyl (3-(bromomethyl)oxetan-3-yl)carbamate (7.8 g). ESI-MS(m/z): 330.03[M+H]⁺.

### Step 3: Synthesis of 4-methoxybenzyl (3-((1,3-dioxoisoindolin-2-yl)methyl)oxetan-3-yl)carbamate

4-Methoxybenzyl (3-(bromomethyl)oxetan-3-yl)carbamate (7.8 g, 23.71 mmol), DMF (50 mL), and potassium phthalimide (4.4 g, 23.78 mmol) were sequentially added into a 250 mL reaction flask. The reaction mixture was heated to 60°C and reacted for 3 hours. LCMS indicated that the reaction was complete. The resulting mixture was extracted with ethyl acetate, and the organic phase was concentrated under reduced pressure to dryness. The concentrate was slurried with methyl tert-butyl ether to obtain 4-methoxybenzyl (3-((1,3-dioxoisoindolin-2-yl)methyl)oxetan-3-yl)carbamate (3.1 g). ESI-MS(m/z): 397.13[M+H]⁺.

### Step 4: Synthesis of 4-methoxybenzyl (3-(aminomethyl)oxetan-3-yl)carbamate

4-Methoxybenzyl (3-((1,3-dioxoisoindolin-2-yl)methyl)oxetan-3-yl)carbamate (3 g, 7.57 mmol), ethanol (30 mL), and 80% hydrazine hydrate (2.3 g, 37.88 mmol) were sequentially added into a 250 mL reaction flask. The resulting mixture was heated to 80°C and reacted for 2 hours. LCMS indicated that the reaction was complete. The reaction mixture was filtered, and the filtrate was concentrated under reduced pressure to obtain 4-methoxybenzyl (3-(aminomethyl)oxetan-3-yl)carbamate (2.0 g). ESI-MS(m/z): 267.13[M+H]⁺.

### Intermediate Preparation Example 3-2: Synthesis of 3-(aminomethyl)-N,N-dibenzyloxetan-3-amine

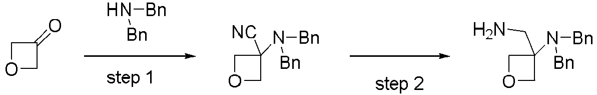

### Step 1: Synthesis of 3-(dibenzylamino)oxetane-3-carbonitrile

Oxetan-3-one (2 g, 27.6 mmol), AcOH (80 mL), dibenzylamine (9.9 g, 55.2 mmol), and TMSCN (5.5 g, 55.2 mmol) were sequentially added into a 250 mL reaction flask. The reaction was conducted at room temperature for 3 hours, and LCMS indicated that the reaction was complete. Acetic acid was removed by rotary evaporation, the residue was diluted with water and adjusted to pH 8 with sodium bicarbonate. Ethyl acetate (200 mL) was then added for extraction, and the resulting organic phase was isolated and washed with water and saturated brine respectively, and purified by preparative chromatography (PE:EA = 3:1) to obtain 3-(dibenzylamino)oxetane-3-carbonitrile (5.2 g). ESI-MS(m/z): 279.14[M+H]⁺.

### Step 2: Synthesis of 3-(aminomethyl)-N,N-dibenzyloxetan-3-amine

3-(Dibenzylamino)oxetane-3-carbonitrile (5.2 g, 18.7 mmol) was added into a 250 mL reaction flask, and then dissolved with 100 mL of tetrahydrofuran, LiAlH₄ (0.85 g, 22.4 mmol) was slowly added in batches at room temperature, and the reaction was conducted at room temperature for 4 hours. LCMS indicated that the reaction was complete. Under ice bath, water (0.9 mL), 15% NaOH (0.9 mL), and water (2.7 mL) were slowly added. Anhydrous sodium sulfate was then added for drying, and the resulting mixture was filtered, concentrated under reduced pressure, and then separated by preparative chromatography (DCM:MeOH = 20:1) to obtain 3-(aminomethyl)-N,N-dibenzyloxetan-3-amine (3 g, 10.6 mmol). ESI-MS(m/z):283.17[M+H]⁺.

### Intermediate Preparation Example 4-1: Synthesis of 2,4-dichloro-6-(difluoromethyl)quinazoline

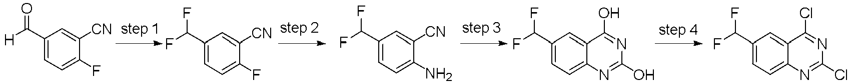

### Step 1: Synthesis of 5-(difluoromethyl)-2-fluorobenzonitrile

2-Fluoro-5-formylbenzonitrile (1.49 g) was added into a 100 mL reaction flask, and then dissolved with DCM. Diethylaminosulfur trifluoride (1.77 g) was slowly added at 0°C. After the addition was completed, the reaction was conducted at room temperature, and LCMS detected that the reaction was complete. Ice water was added, and the resulting mixture was extracted with ethyl acetate. The organic phases were combined, and spin-dried under reduced pressure to obtain 5-(difluoromethyl)-2-fluorobenzonitrile (1.5 g). ESI-MS(m/z): 172.03[M+H]⁺.

### Step 2: Synthesis of 5-(difluoromethyl)-2-aminobenzonitrile

5-(Difluoromethyl)-2-fluorobenzonitrile (1.49 g) was added into a 20 mL reaction flask, and then dissolved with THF. Ammonia water (1.77 g) was slowly added. After the addition was completed, the reaction was carried out at 100°C. LCMS detected that the reaction was complete. The reaction mixture was extracted with ethyl acetate, and the organic phases were combined and spin-dried under reduced pressure to obtain 5-(difluoromethyl)-2-aminobenzonitrile (0.3 g). ESI-MS(m/z): 169.03[M+H]⁺.

### Step 3: Synthesis of 6-(difluoromethyl)quinazoline-2,4-diol

5-(Difluoromethyl)-2-aminobenzonitrile (0.3 g) and DBU (2 mL) were sequentially added into a 20 mL reaction flask, and then dissolved with DMSO. The atmosphere was replaced with CO₂ three times, and the reaction was carried out at 100°C. LCMS detected that the reaction was terminated. Water was added and the resulting mixture was extracted with ethyl acetate, and the organic phases were combined, spin-dried under reduced pressure and purified by preparative chromatography to obtain 6-(difluoromethyl)quinazoline-2,4-diol (0.1 g). ESI-MS(m/z): 213.04[M+H]⁺.

### Step 4: Synthesis of 2,4-dichloro-6-(difluoromethyl)quinazoline

6-(Difluoromethyl)quinazoline-2,4-diol (0.1 g), phosphorus trichloride (2 mL), and phosphorus pentachloride (2 mL) were sequentially added into a 20 mL reaction flask. The reaction was carried out at 100°C. LCMS detected that the reaction was terminated. Water was added and the resulting mixture was extracted with ethyl acetate, and the organic phases were combined, concentrated under reduced pressure, and purified by preparative chromatography to obtain 2,4-dichloro-6-(difluoromethyl)quinazoline (0.05 g). ESI-MS(m/z): 248.97[M+H]⁺.

### Example 1-1: Synthesis of 4-(4-(((3-aminooxetan-3-yl)methyl)amino)-6-methyl-5,6,7,8-tetrahydropyrido[4,3-d]pyrimidin-2-yl)-2,3,4,5-tetrahydrobenzo[f][1,4]thiazepine-1,1-dioxide

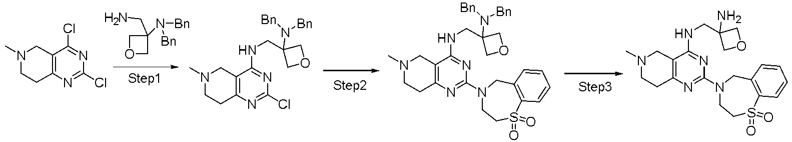

### Step 1: Synthesis of 2-chloro-N-((3-(dibenzylamino)oxetan-3-yl)methyl)-6-methyl-5,6,7,8-tetrahydropyrido[4,3-d]pyrimidin-4-amine

3-(Aminomethyl)-*N,N*-dibenzyloxetan-3-amine (517 mg, 1.83 mmol) and methanol (10 mL) were added into a 100 mL reaction flask, followed by the addition of 2,4-dichloro-6-methyl-5,6,7,8-tetrahydropyrido[4,3-*d*]pyrimidine (400 mg, 1.83 mmol) and triethylamine (270 mg, 2.67 mmol). The reaction mixture reacted at room temperature for 3 hours. LCMS indicated that the reaction was complete. For post-treatment, the reaction mixture was concentrated under reduced pressure to dryness, and the concentrate was purified by column chromatography (DCM: MeOH = 30:1) to obtain 2-chloro-*N*-((3-(dibenzylamino)oxetan-3-yl)methyl)-6-methyl-5,6,7,8-tetrahydropyrido[4,3-*d*]pyrimidin-4-amine (130 mg). ESI-MS(m/z): 464.21[M+H]⁺.

### Step 2: Synthesis of 4-(4-(((3-(dibenzylamino)oxetan-3-yl)methyl)amino)-6-methyl-5,6,7,8-tetrahydropyrido[4,3-d]pyrimidin-2-yl)-2,3,4,5-tetrahydrobenzo[f][1,4]thiazepine-1,1-dioxide

2-Chloro-*N*-((3-(dibenzylamino)oxetan-3-yl)methyl)-6-methyl-5,6,7,8-tetrahydropyrido[4,3-*d*]pyrimidin-4-amine (100 mg, 0.22 mmol), 2,3,4,5-tetrahydrobenzo[*f*][1,4]thiazepine-1,1-dioxide (47 mg, 0.24 mmol), p-toluenesulfonic acid monohydrate (8.4 mg, 0.044 mmol), and n-butanol (2 mL) were sequentially added into a 25 mL reaction flask. The reaction mixture reacted at 120°C for 4 hours. LCMS indicated that the reaction was complete. For post-treatment, ethyl acetate was added for dilution, and the resulting organic phase was washed with a saturated aqueous sodium bicarbonate solution and water respectively. The organic phase was concentrated under reduced pressure to dryness, and the concentrate was purified by TLC (DCM: MeOH = 10:1) to obtain 4-(4-(((3-(dibenzylamino)oxetan-3-yl)methyl)amino)-6-methyl-5,6,7,8-tetrahydropyrido[4,3-d]pyrimidin-2-yl)-2,3,4,5-tetrahydrobenzo[*f*][1,4]thiazepine-1,1-dioxide (60 mg). ESI-MS(m/z): 625.29[M+H]⁺.

### Step 3: Synthesis of 4-(4-(((3-aminooxetan-3-yl)methyl)amino)-6-methyl-5,6,7,8-tetrahydropyrido[4,3-d]pyrimidin-2-yl)-2,3,4,5-tetrahydrobenzo[f][1,4]thiazepine-1,1-dioxide

4-(4-(((3-(Dibenzylamino)oxetan-3-yl)methyl)amino)-6-methyl-5,6,7,8-tetrahydropyrido[4,3-*d*]pyrimidin-2-yl)-2,3,4,5-tetrahydrobenzo[f][1,4]thiazepine-1,1-dioxide (60 mg, 0.096 mmol) was dissolved in methanol (5 mL) in a 25 mL reaction flask, and 20% Pd(OH)₂ loaded on carbon (40 mg) was added. The reaction was conducted overnight at room temperature under a hydrogen atmosphere. LCMS indicated that the reaction was complete. The resulting mixture was filtered, and the filtrate was collected, concentrated under reduced pressure, and purified by TLC (DCM: MeOH = 10:1) to obtain 4-(4-(((3-aminooxetan-3-yl)methyl)amino)-6-methyl-5,6,7,8-tetrahydropyrido[4,3-*d*]pyrimidin-2-yl)-2,3,4,5-tetrahydrobenzo[f][1,4]thiazepine-1,1-dioxide (20 mg). ESI-MS(m/z): 445.19[M+H]⁺; ¹H NMR (600 MHz, CDCl₃) δ 8.05 (d, *J =* 7.7 Hz, 1H), 7.72 (d, *J =* 7.4 Hz, 1H), 7.52 (t, *J =* 7.3 Hz, 1H), 7.40 (t, *J =* 7.6 Hz, 1H), 5.12 (s, 2H), 4.80 (s, 1H), 4.51 (dd, *J =* 26.3, 6.0 Hz, 4H), 3.88 (s, 2H), 3.43 (s, 2H), 3.23 (s, 2H), 3.12 (q, *J =* 7.3 Hz, 2H), 2.81 - 2.61 (m, 4H), 2.51 (s, 3H), 1.43 (t, *J =* 7.3 Hz, 2H).

### Example 1-32: Synthesis of 4-(4-(((3-aminooxetan-3-yl)methyl)amino)-6-(trifluoromethyl)quinazolin-2-yl)-1-((methyl-D3)imino)-2,3,4,5-tetrahydro-1H-1λ⁴-benzo[f][1,4]thiazepine-1-oxide

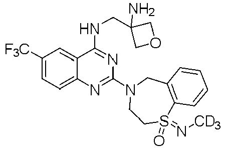

The synthesis method was the same as Example 2-5, except that 2,4-dichloro-6-(trifluoromethyl)quinazoline was used instead of 2,4-dichloro-6-methylquinazoline in Step 1, and 1-((methyl-D3)imino)-2,3,4,5-tetrahydro-1*H-*1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain the target compound (19 mg). ESI-MS(m/z): 510.57[M+H]⁺; ¹H NMR (600 MHz, CD₃OD) δ 8.32 (s, 1H), 7.95 (s, 1H), 7.88 (d, *J* = 5.9 Hz, 1H), 7.67 (d, *J* = 8.0 Hz, 1H), 7.58 (s, 1H), 7.42 (s, 2H), 5.51 - 5.39 (m, 1H), 4.76 (d, *J =* 14.5 Hz, 1H), 4.63 (d, *J =* 5.9 Hz, 2H), 4.55 (s, 1H), 3.94 (s, 1H), 3.62 (d, *J =* 14.1 Hz, 1H), 3.49 (s, 1H), 1.32 - 1.23 (m, 3H), 0.89 (t, *J =* 6.6 Hz, 2H).

### Example 2-5: Synthesis of 4-(4-(((3-aminooxetan-3-yl)methyl)amino)-6-methylquinazolin-2-yl)-2,3,4,5-tetrahydropyrido[3,4-f][1,4]thiazepine-1,1-dioxide

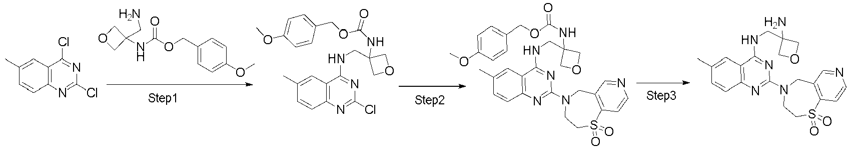

### Step 1: Synthesis of 4-methoxybenzyl (3-(((2-chloro-6-methylquinazolin-4-yl)amino)methyl)oxetan-3-yl)carbamate

4-Methoxybenzyl (3-(aminomethyl)oxetan-3-yl)carbamate (260 mg, 0.98 mmol) and methanol (10 mL) were added into a 100 mL reaction flask, followed by the addition of 2,4-dichloro-6-methylquinazoline (210 mg, 0.98 mmol) and triethylamine (200 mg, 1.98 mmol). The reaction mixture reacted at room temperature for 4 hours. LCMS indicated that the reaction was complete. For post-treatment, the reaction mixture was directly concentrated under reduced pressure to dryness. The concentrate was purified by silica gel column (DCM: MeOH = 30:1) to obtain 4-methoxybenzyl (3-(((2-chloro-6-methylquinazolin-4-yl)amino)methyl)oxetan-3-yl)carbamate (260 mg). ESI-MS(m/z): 443.90[M+H]⁺.

### Step 2: Synthesis of 4-methoxybenzyl (3-((2-(1,1-dioxo-2,3-dihydropyrido[3,4-f][1,4]thiazepin-4(5H)-yl)-6-methylquinazolin-4-yl)amino)methyl)oxetan-3-yl)carbamate

4-Methoxybenzyl (3-(((2-chloro-6-methylquinazolin-4-yl)amino)methyl)oxetan-3-yl)carbamate (260 mg, 0.60 mmol), 2,3,4,5-tetrahydropyrido[3,4-*f*][1,4]thiazepine-1,1-dioxide (120 mg, 0.60 mmol), NH₄Cl (8 mg, 0.15 mmol), and ethanol (10 mL) were sequentially added into a 25 mL reaction flask. The reaction mixture was heated to 78°C and reacted for 30 hours. LCMS indicated that the reaction was complete. The reaction mixture was concentrated under reduced pressure, and the concentrate was purified by silica gel column chromatography (DCM: MeOH = 30:1 to 20:1) to obtain 4-methoxybenzyl (3-((2-(1,1-dioxo-2,3-dihydropyrido[3,4-*f*][1,4]thiazepin-4(5*H*)-yl)-6-methylquinazolin-4-yl)amino)methyl)oxetan-3-yl)carbamate (100 mg). ESI-MS(m/z): 605.68[M+H]⁺.

### Step 3: Synthesis of 4-(4-(((3-aminooxetan-3-yl)methyl)amino)-6-methylquinazolin-2-yl)-2,3,4,5-tetrahydropyrido[3,4-f][1,4]thiazepine-1,1-dioxide

4-Methoxybenzyl (3-((2-(1,1-dioxo-2,3-dihydropyrido[3,4-*f*][1,4]thiazepin-4(5*H*)-yl)-6-methylquinazolin-4-yl)amino)methyl)oxetan-3-yl)carbamate (100 mg, 0.16 mmol), DCM (4 mL), and trifluoroacetic acid (1 mL) were added into a 100 mL reaction flask. The reaction mixture was stirred at room temperature for 1 hour. LCMS indicated that the reaction was complete. For post-treatment, the resulting mixture was concentrated under reduced pressure to dryness, the concentrate was dissolved in methanol, and the resulting solution was adjusted to pH 10 with ammonia-methanol. The resulting solution was concentrated under reduced pressure to dryness and purified by preparative HPLC to obtain 4-(4-(((3-aminooxetan-3-yl)methyl)amino)-6-methylquinazolin-2-yl)-2,3,4,5-tetrahydropyrido[3,4-*f*][1,4]thiazepine-1,1-dioxide (15 mg). ESI-MS(m/z): 441.52[M+H]⁺; ¹H NMR (600 MHz, CD₃OD) δ 9.01 (s, 1H), 8.76 (s, 1H), 7.96 (d, *J =* 4.8 Hz, 1H), 7.73 (s, 1H), 7.44 (d, *J =* 7.8 Hz, 1H), 7.38 (d, *J =* 8.5 Hz, 1H), 5.52 (s, 1H), 5.21 (d, *J* = 19.4 Hz, 2H), 4.74 - 4.63 (m, 3H), 4.59 (d, *J =* 6.8 Hz, 3H), 4.12 (s, 2H), 3.70 - 3.65 (m, 2H), 3.09 - 3.05 (m, 2H), 2.42 (s, 3H).

### Example 2-14: Synthesis of 4-(4-(((3-aminooxetan-3-yl)methyl)amino)-6-methylquinazolin-2-yl)-1-(methylimino)-2,3,4,5-tetrahydro-1H-1λ⁴-benzo[f][1,4]thiazepine-1-oxide

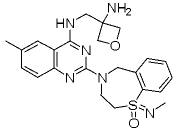

The synthesis method was the same as Example 2-5, except that 1-(methylimino)-2,3,4,5-tetrahydro-1*H*-1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methylquinazolin-2-yl)-1-(methylimino)-2,3,4,5-tetrahydro-1*H*-1λ⁴-benzo[*f*][1,4]thiazepine-1-oxide (52 mg). ESI-MS(m/z): 453.2[M+H]⁺. ¹H NMR (600 MHz, DMSO-*d₆*) δ 7.85 (dd, *J* = 20.1, 7.2 Hz, 4H), 7.56 (t, *J =* 7.1 Hz, 1H), 7.46 (s, 1H), 7.36 (d, *J =* 8.2 Hz, 1H), 7.22 (s, 1H), 5.31 (s, 1H), 4.97 (s, 1H), 4.69 (d, *J =* 13.7 Hz, 1H), 4.50 (s, 2H), 4.37 (s, 2H), 3.91 (d, *J =* 42.8 Hz, 3H), 3.68 (d, *J* = 58.2 Hz, 2H), 2.92 (s, 2H), 2.44 (s, 3H), 2.34 (s, 3H).

### Example 2-16: Synthesis of 1-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)urea

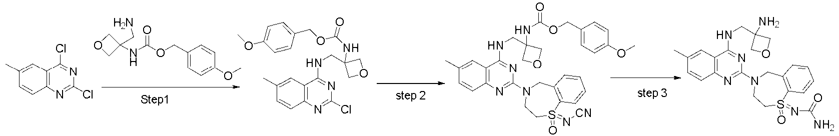

The synthesis method was the same as Example 2-5, except that *N*-(1-oxido-2,3,4,5-tetrahydro-1λ⁴-benzo[*f*][1,4]thiazepin-1-ylidene)cyanamide was used instead of 2,3,4,5-tetrahydropyrido[3,4-*f*][1,4]thiazepine-1,1-dioxide in Step 2, to obtain 1-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)urea (43 mg). ESI-MS(m/z): 482.18[M+H]⁺. ¹H NMR (600 MHz, DMSO-*d₆*) δ 8.07 (s, 1H), 7.93 (s, 1H), 7.89 - 7.80 (m, 2H), 7.60 (t, *J =* 7.2 Hz, 1H), 7.49 (s, 1H), 7.39 (s, 1H), 7.31 (s, 1H), 6.60 (s, 1H), 6.14 (s, 1H), 5.34 (s, 1H), 5.08 (s, 1H), 4.87 (s, 1H), 4.57 (d, *J =* 5.9 Hz, 2H), 4.44 (s, 2H), 4.07 (d, *J* = 39.7 Hz, 3H), 3.84 (s, 1H), 3.52 (s, 1H), 2.38 (d, *J* = 24.8 Hz, 3H).

### Example 2-23: Synthesis of 4-(4-(((3-aminooxetan-3-yl)methyl)amino)-6-methylquinazolin-2-yl)-1-imino-2,3,4,5-tetrahydro-1H-1λ⁴-benzo[f][1,4]thiazepine-1-oxide

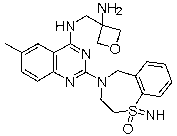

The synthesis method was the same as Example 2-5, except that 1-imino-2,3,4,5-tetrahydro-1*H*-1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methylquinazolin-2-yl)-1-imino-2,3,4,5-tetrahydro-1*H*-1λ⁴-benzo[*f*][1,4]thiazepine-1-oxide (32 mg). ESI-MS(m/z): 439.18[M+H]⁺. ¹H NMR (600 MHz, DMSO-*d₆*) δ 7.94 (d, *J =* 12.9 Hz, 1H), 7.86 - 7.76 (m, 3H), 7.52 (t, *J =* 6.9 Hz, 1H), 7.42 (s, 1H), 7.36 (d, *J =* 7.9 Hz, 1H), 7.23 (s, 1H), 5.08 (s, 2H), 4.62 (s, 1H), 4.49 (s, 2H), 4.36 (s, 3H), 3.89 (d, *J =* 95.3 Hz, 3H), 3.38 (s, 1H), 2.89 (s, 1H), 2.73 (s, 1H), 2.34 (s, 3H).

### Example 2-24: Synthesis of 4-(4-(((3-aminooxetan-3-yl)methyl)amino)-6-methylquinazolin-2-yl)-1-((deuteromethyl)imino)-2,3,4,5-tetrahydro-1H-1λ⁴-benzo[f][1,4]thiazepine-1-oxide

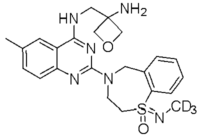

The synthesis method was the same as Example 2-5, except that 1-((deuteromethyl)imino)-2,3,4,5-tetrahydro-1*H*-1λ⁴-benzo[*f*][1,4]thiazepine-1-oxide was used instead of 2,3,4,5-tetrahydropyrido[3,4-f][1,4]thiazepine-1,1-dioxide in Step 2, to obtain 4-(4-(((3-aminooxetan-3-yl)methyl)amino)-6-methylquinazolin-2-yl)-1-((deuteromethyl)imino)-2,3,4,5-tetrahydro-1*H*-1λ⁴-benzo[*f*][1,4]thiazepine-1-oxide (32 mg). ESI-MS(m/z): 456.22[M+H]⁺. ¹H NMR (600 MHz, CD₃OD) *δ* 7.97 (d, *J* = 7.7 Hz, 1H), 7.89 (d, *J* = 7.4 Hz, 1H), 7.69 (s, 1H), 7.58 (t, *J* = 7.3 Hz, 1H), 7.44 (t, *J* = 7.6 Hz, 1H), 7.38 (d, *J =* 8.5 Hz, 1H), 7.32 (d, *J =* 8.5 Hz, 1H), 5.53 - 5.39 (m, 1H), 4.78 (d, *J =* 14.9 Hz, 1H), 4.67 (d, *J =* 5.7 Hz, 1H), 4.64 (d, *J =* 6.2 Hz, 1H), 4.55 (s, 2H), 4.12 (d, *J =* 12.0 Hz, 1H), 4.06 - 3.93 (m, 2H), 3.62 (d, *J =* 14.7 Hz, 1H), 3.52 (t, *J =* 12.5 Hz, 1H), 3.33 (s, 2H), 2.38 (s, 3H).

### Example 2-25: Synthesis of 4-(4-(((3-aminooxetan-3-yl)methyl)amino)-6-methylquinazolin-2-yl)-1-(oxetan-3-ylimino)-2,3,4,5-tetrahydro-1H-1λ⁴-benzo[f][1,4]thiazepine-1-oxide

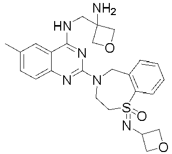

The synthesis method was the same as Example 2-5, except that 1-(oxetanylimino)-2,3,4,5-tetrahydro-1*H*-1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methylquinazolin-2-yl)-1-(oxetan-3-ylimino)-2,3,4,5-tetrahydro-1*H*-1λ⁴-benzo[*f*][1,4]thiazepine-1-oxide (32 mg). ESI-MS(m/z): 495.21[M+H]⁺. ¹H NMR (600 MHz, Methanol-*d*₄) δ 7.99 (dd, *J* = 7.9*,* 1.3 Hz, 1H), 7.92 - 7.88 (m, 1H), 7.71 - 7.68 (m, 1H), 7.64 - 7.58 (m, 1H), 7.48 (td, *J =* 7.7, 1.3 Hz, 1H), 7.38 (dd, *J =* 8.6, 1.9 Hz, 1H), 7.32 (d, *J =* 8.6 Hz, 1H), 5.45 (d, *J =* 15.2 Hz, 1H), 5.08 (s, 1H), 4.98 - 4.84 (m, 2H), 4.83 - 4.73 (m, 2H), 4.64 (dd, *J =* 20.2, 6.3 Hz, 2H), 4.53 (dd, *J =* 6.3, 3.5 Hz, 2H), 4.05 (dd, *J =* 63.1, 12.8 Hz, 2H), 3.68 - 3.48 (m, 2H), 3.24 - 2.86 (m, 2H), 2.73 - 2.60 (m, 1H), 2.37 (s, 3H).

### Example 2-26: Synthesis of 4-(4-(((3-aminooxetan-3-yl)methyl)amino)-6-methylquinazolin-2-yl)-1-(cyclopropylimino)-2,3,4,5-tetrahydro-1H-1λ⁴-benzo[f][1,4]thiazepine-1-oxide

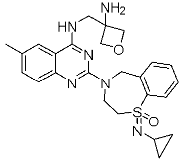

The synthesis method was the same as Example 2-5, except that 1-(cyclopropylimino)-2,3,4,5-tetrahydro-1*H-*1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methylquinazolin-2-yl)-1-(cyclopropylimino)-2,3,4,5-tetrahydro-1*H*-1λ⁴-benzo[*f*][1,4]thiazepine-1-oxide (80 mg). ESI-MS(m/z): 479.45[M+H]⁺; ¹H NMR (600 MHz, Methanol-*d*₄) δ 8.03 (d, *J* = 7.9 Hz, 1H), 7.87 (d, *J =* 7.9 Hz, 1H), 7.70 (s, 1H), 7.59 (t, *J =* 7.5 Hz, 1H), 7.45 (t, *J =* 7.1 Hz, 1H), 7.39 (dd, *J* = 8.6, 1.9 Hz, 1H), 7.33 (d, *J =* 8.5 Hz, 1H), 4.82 (d, *J =* 15.1 Hz, 2H), 4.66 (dd, *J =* 20.6, 6.4 Hz, 2H), 4.57 - 4.53 (m, 2H), 4.19 - 3.92 (m, 4H), 3.63 - 3.43 (m, 2H), 2.38 (s, 3H), 2.38 - 2.32 (m, 1H), 0.55 - 0.39 (m, 2H), 0.29 - 0.18 (m, 2H).

### Example 2-29: Synthesis of 4-(4-(((3-aminooxetan-3-yl)methyl)amino)-6-methylquinazolin-2-yl)-1-((trifluoromethyl)imino)-2,3,4,5-tetrahydro-1H-1λ⁴-benzo[f][1,4]thiazepine-1-oxide

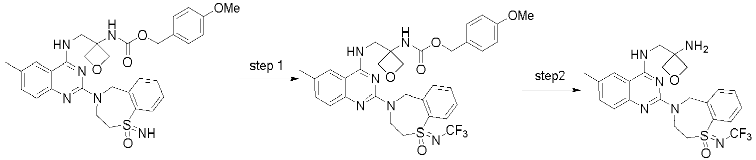

### Step 1: Synthesis of (4-methoxy)benzyl 3-(((6-methyl-2-(1-oxido-1-((trifluoromethyl)imino)-1,2,3,5-tetrahydro-4H-1λ⁴-benzo[f][1,4]thiazepin-4-yl)quinazolin-4-yl)amino)methyl)oxetan-3-ylcarbamate

(4-Methoxy)benzyl 3-(((2-(1-imino-1-oxido)-1,2,3,5-tetrahydro-4H-1λ⁴-benzo[*f*][1,4]thiazepin-4-yl)-6-methylquinazolin-4-yl)amino)methyl)oxetan-3-ylcarbamate (120 mg, 0.2 mmol), TMSCF₃ (142 mg, 1.0 mmol), Ag₂CO₃ (11 mg, 0.04 mmol), 1,10-phenanthroline (14 mg, 0.08 mmol), and 1,4-dioxane (4.0 mL) were added into a 25 mL flask. The atmosphere was replaced with oxygen three times, and the mixture was stirred at 60°C for 12 h. LCMS detection indicated that the reaction was complete. The solvent was removed by rotary exportation, and the residue was purified by thin-layer chromatography (DCM:MeOH = 10:1) to obtain (4-methoxy)benzyl 3-(((6-methyl-2-(1-oxido-1-((triffuoromethyl)imino)-1,2,3,5-tetrahydro-4H-1λ⁴-benzo[*f*][1,4]thiazepin-4-yl)quinazolin-4-yl)amino)methyl)oxetan-3-ylcarbamate (28 mg).

### Step 2: Synthesis of 4-(4-(((3-aminooxetan-3-yl)methyl)amino)-6-methylquinazolin-2-yl)-1-((trifluoromethyl)imino)-2,3,4,5-tetrahydro-1H-1λ⁴-benzo[f][1,4]thiazepine-1-oxide

(4-Methoxy)benzyl 3-(((6-methyl-2-(1-oxido-1-((trifluoromethyl)imino)-1,2,3,5-tetrahydro-4H-1λ⁴-benzo[f][1,4]thiazepin-4-yl)quinazolin-4-yl)amino)methyl)oxetan-3-ylcarbamate (134 mg, 0.2 mmol) and DCM (8 mL) were added into a 25 mL flask. TFA (1 mL) was added under stirring, and the resulting mixture was stirred at room temperature for 2 hours. LCMS detection indicated that the reaction was complete. The solvent was removed by rotary evaporation, and the residue was purified by thin-layer chromatography (DCM:MeOH = 10:1) to obtain 4-(4-(((3-aminooxetan-3-yl)methyl)amino)-6-methylquinazolin-2-yl)-1-((trifluoromethyl)imino)-2,3,4,5-tetrahydro-1*H*-1λ⁴-benzo[*f*][1,4]thiazepine-1-oxide (20 mg). ESI-MS(m/z): 507.17[M+H]⁺.

### Example 2-30: Synthesis of 4-(4-(((3-aminooxetan-3-yl)methyl)amino)-6-methylquinazolin-2-yl)-1-((trifluoromethylthio)imino)-2,3,4,5-tetrahydro-1H-1λ⁴-benzo[f][1,4]thiazepine-1-oxide

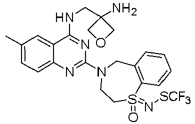

The synthesis method was the same as Example 2-5, except that 1-((trifluoromethylthio)imino)-2,3,4,5-tetrahydro-1*H*-1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methylquinazolin-2-yl)-1-((trifluoromethylthio)imino)-2,3,4,5-tetrahydro-1*H*-1λ⁴-benzo[*f*][1,4]thiazepine-1-oxide (32 mg). ESI-MS(m/z): 539.14[M+H]⁺.

### Example 2-31: Synthesis of N-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)acetamide

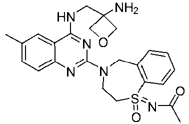

The synthesis method was the same as Example 2-5, except that *N*-(1-oxido-2,3,4,5-tetrahydro-1λ⁴-benzo[*f*][1,4]thiazepin-1-ylidene)acetamide was used instead of 2,3,4,5-tetrahydropyrido[3,4-*f*][1,4]thiazepine-1,1-dioxide in Step 2, to obtain *N*-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)acetamide (32 mg). ESI-MS(m/z): 481.19[M+H]⁺. ¹H NMR (600 MHz, Methanol-*d*₄) *δ* 8.07 (d, *J* = 7.9 Hz, 1H), 7.89 (d, *J* = 7.6 Hz, 1H), 7.70 (s, 1H), 7.65-7.59 (m, 1H), 7.48 (t, *J =* 7.7 Hz, 1H), 7.40 (dd, *J =* 8.6, 1.9 Hz, 1H), 7.34 (d, *J =* 8.5 Hz, 1H), 5.04-4.97 (m, 2H), 4.65 (dd, *J =* 13.3, 6.4 Hz, 2H), 4.54 (t, *J =* 6.7 Hz, 2H), 4.23-3.98 (m, 4H), 3.21 (q, *J =* 7.3 Hz, 2H), 2.39 (s, 3H), 2.08 (s, 3H).

### Example 2-32: Synthesis of N-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)-1-fluorocyclopropane-1-carboxamide

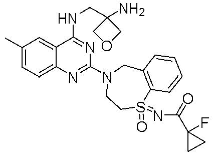

The synthesis method was the same as Example 2-5, except that *N*-(1-oxido-2,3,4,5-tetrahydro-1λ⁴-benzo[*f*][1,4]thiazepin-1-ylidene)-1-fluorocyclopropane-1-carboxamide was used instead of 2,3,4,5-tetrahydropyrido[3,4-*f*][1,4]thiazepine-1,1-dioxide in Step 2, to obtain *N*-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)-1-fluorocyclopropane-1-carboxamide (32 mg). ESI-MS(m/z): 525.21[M+H]⁺. 1H NMR (600 MHz, CD3OD) δ 8.11 (d, J = 7.7 Hz, 1H), 7.92 (d, J = 7.4 Hz, 1H), 7.72 (s, 1H), 7.65 (t, J = 7.2 Hz, 1H), 7.51 (t, J = 7.4 Hz, 1H), 7.44 - 7.40 (m, 1H), 7.35 (d, J = 8.5 Hz, 1H), 5.55 - 5.40 (m, 3H), 4.66 (dd, J = 13.2, 6.2 Hz, 2H), 4.55 (t, J = 6.7 Hz, 2H), 4.12 - 4.03 (m, 4H), 3.81 (s, 1H), 2.41 (s, 3H), 0.93 (s, 2H), 0.91 (s, 2H).

### Example 2-33: Synthesis of N-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)-2-hydroxyacetamide

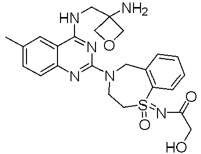

The synthesis method was the same as Example 2-5, except that *N*-(1-oxido-2,3,4,5-tetrahydro-1λ⁴-benzo[*f*][1,4]thiazepin-1-ylidene)-2-hydroxyacetamide was used instead of 2,3,4,5-tetrahydropyrido[3,4-*f*][1,4]thiazepine-1,1-dioxide in Step 2, to obtain *N*-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)-2-hydroxyacetamide (32 mg). ESI-MS(m/z): 497.19[M+H]⁺.

### Example 2-34: Synthesis of N-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)-2-(dimethylamino)acetamide

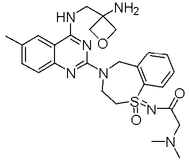

The synthesis method was the same as Example 2-5, except that *N*-(1-oxido-2,3,4,5-tetrahydro-1λ⁴-benzo[*f*][1,4]thiazepin-1-ylidene)-2-(dimethylamino)acetamide was used instead of 2,3,4,5-tetrahydropyrido[3,4-*f*][1,4]thiazepine-1,1-dioxide in Step 2, to obtain *N*-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)-2-(dimethylamino)acetamide (32 mg). ESI-MS(m/z): 524.24[M+H]⁺. ¹H NMR (600 MHz, Methanol-*d*₄) *δ* 8.10 (d, *J* = 8.4 Hz, 1H), 7.89 (d, *J =* 7.5 Hz, 1H), 7.70 (s, 1H), 7.65-7.60 (t, *J =* 7.5 Hz, 1H), 7.49 (t, *J =* 7.5 Hz, 1H), 7.40 (dd, *J* = 8.6, 1.9 Hz, 1H), 7.33 (d, *J* = 8.6 Hz, 1H), 5.42 (d, *J =* 15.5 Hz,2H), 4.97 (d, *J =* 15.5 Hz, 2H), 4.63 (dd, *J* =13.1, 6.2 Hz, 2H), 4.55 (t,*J =* 6.9 Hz, 2H), 4.21-3.96 (m, 4H), 3.76 (s, 2H), 3.27 (s, 3H), 2.39 (s, 3H), 2.33 (s, 3H).

### Example 2-37: Synthesis of 1-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)-3-methylurea

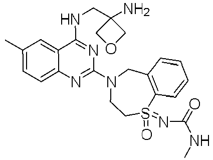

The synthesis method was the same as Example 2-5, except that 1-methyl-3-(1-oxido-2,3,4,5-tetrahydro-1λ⁴-benzo[*f*][1,4]thiazepin-1-ylidene)urea was used instead of 2,3,4,5-tetrahydropyrido[3,4-*f*][1,4]thiazepine-1,1-dioxide in Step 2, to obtain 1-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)-3-methylurea (32 mg). ESI-MS(m/z): 496.22[M+H]⁺. ¹H NMR (600 MHz, CD₃OD) *δ* 8.08 (d, *J* = 7.8 Hz, 1H), 7.88 (d, *J* = 7.4 Hz, 1H), 7.72 (s, 1H), 7.59 (t, *J* = 7.3 Hz, 1H), 7.49 - 7.41 (m, 2H), 7.36 (d, *J =* 8.5 Hz, 1H), 5.53 - 5.41 (m, 1H), 5.06 (s, 1H), 4.96 (s, 1H), 4.68 (dd, *J =* 14.3, 6.3 Hz, 2H), 4.57 (t, *J =* 6.2 Hz, 2H), 4.18 - 3.97 (m, 4H), 3.65 (s, 1H), 2.66 (s, 3H), 2.41 (s, 3H).

### Example 2-38: Synthesis of 4-(4-(((3-aminooxetan-3-yl)methyl)amino)-6-methylquinazolin-2-yl)-1-((1-methyl-1H-pyrazol-4-yl)imino)-2,3,4,5-tetrahydro-1H-1λ⁴-benzo[f][1,4]thiazepine-1-oxide

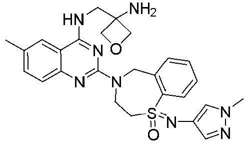

The synthesis method was the same as Example 2-5, except that 1-((1-methyl-1*H*-pyrazol-4-yl)imino)-2,3,4,5-tetrahydro-1*H*-1λ⁴-benzo[*f*][1,4]thiazepine-1-oxide was used instead of 2,3,4,5-tetrahydropyrido[3,4-f][1,4]thiazepine-1,1-dioxide in Step 2, to obtain 4-(4-(((3-aminooxetan-3-yl)methyl)amino)-6-methylquinazolin-2-yl)-1-((1-methyl-1*H*-pyrazol-4-yl)imino)-2,3,4,5-tetrahydro-1*H*-1λ⁴-benzo[*f*][1,4]thiazepine-1-oxide (32 mg). ESI-MS(m/z): 519.22[M+H]⁺. ¹H NMR (600 MHz, CD₃OD) *δ* 7.99 (d, *J =* 7.6 Hz, 1H), 7.83 (d, *J =* 7.3 Hz, 1H), 7.70 (s, 1H), 7.54 (t, *J =* 7.2 Hz, 1H), 7.39 (t*, J=* 7.4 Hz, 2H), 7.33 (d, *J =* 8.5 Hz, 1H), 7.20 (s, 1H), 7.08 (s, 1H), 5.55-5.36 (m, 1H), 4.94 (d, *J =* 14.0 Hz, 2H), 4.65 (dd, *J =* 19.2, 5.8 Hz, 2H), 4.54 (s, 2H), 4.11 (d, *J =* 12.7 Hz, 2H), 4.02 (d, *J* = 13.4 Hz, 1H), 3.80 (d, *J* = 14.2 Hz, 1H), 3.69 (s, 3H), 3.62 *(d, J=* 12.1 Hz, 1H), 2.39 (s, 3H).

**The preparation methods of the target compounds of Examples 2-27, 2-28, 2-35, 2-36, 2-39 and 2-40 are listed in the table below.**

| **Example No.** | **Chemical Name** | **Structural Formula** | **Reference Example** | **Raw Material Used** | **Characterization Data** |
|---|---|---|---|---|---|
| **Example 2-27** | 4-(4-(((3-aminooxetan-3-yl)methyl)amino)-6-methylquinazolin-2-yl)-1-(pyrrolidin-3-ylimino)-2,3,4,5-tetrahydro-1*H*-1λ⁴-benzo[*f*][1,4]thiazepine-1-oxide | 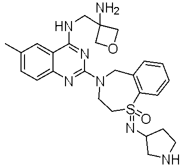 | Example 2-5 | In Step 2, 1-(pyrrolidin-3-ylimino)-2,3,4,5-tetrahydro-1*H*-1λ⁴-benzo[*f*] [1,4] thiazepine-1-oxide was used instead of 2,3,4,5-tetrahydropyrido[3,4 -*f*][1,4]thiazepine-1,1-dioxide. | ESI-MS(m/z): 507.24[M+ H]⁺ |
| **Example 2-28** | 4-(4-(((3-aminooxetan-3-yl)methyl)amino)-6-methylquinazolin-2-yl)-1-(piperidin-3-ylimino)-2,3,4,5-tetrahydro-1*H-*1λ⁴-benzo[*f*][1,4]thiazepine-1-oxide | 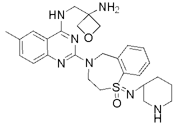 | Example 2-5 | In Step 2, 1-(piperidin-3-ylimino)-2,3,4,5-tetrahydro-1*H*-1λ⁴-benzo[f] [1,4] thiazepine-1-oxide was used instead of 2,3,4,5-tetrahydropyrido[3,4 -f][1,4]thiazepine-1,1-dioxide. | ESI-MS(m/z): 521.26[M+ H]⁺ |
| **Example 2-35** | 2-((4-(4-(((3-aminooxetan-3-yl)methyl)amino)-6-methylquinazolin-2-yl)-1-oxido-2,3,4,5-tetrahydro-1λ⁴- | 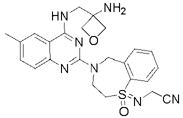 | Example 2-5 | In Step 2, 2-((1-oxido-2,3,4,5-tetrahydro-1λ⁴-benzo[*f*][1,4] thiazepin-1-ylidene) amino)acetonitrile | ESI-MS(m/z): 478.19[M+ H]⁺ |
| | benzo[*f*][1,4]thiazepin-1-ylidene)amino) acetonitrile | | | was used instead of 2,3,4,5-tetrahydropyrido[3,4 -f][1,4]thiazepine-1,1-dioxide. | |
| **Example 2-36** | methyl 2-((4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)amino)acetate | 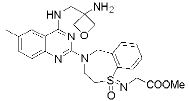 | Example 2-5 | In Step 2, methyl 2-((1-oxido-2,3,4,5-tetrahydro-1λ⁴-benzo[*f*][1,4] thiazepin-1-ylidene) amino)acetate was used instead of 2,3,4,5-tetrahydropyrido[3,4 -f][1,4]thiazepine-1,1-dioxide. | ESI-MS(m/z): 511.22[M+ H]⁺ |
| **Example 2-39** | 4-(4-(((3-aminooxetan-3-yl)methyl)amino)-6-methylquinazolin-2-yl)-1-(phenylimino)-2,3,4,5-tetrahydro-1*H-*1λ⁴-benzo[*f*][1,4] thiazepine-1-oxide | 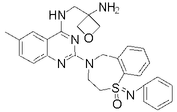 | Example 2-5 | In Step 2, 1-(phenylimino)-2,3,4,5-tetrahydro-1*H*-1λ⁴-benzo[*f*][1,4] thiazepine-1-oxide was used instead of 2,3,4,5-tetrahydropyrido[3,4 -*f*][1,4]thiazepine-1,1-dioxide. | ESI-MS(m/z): 515.22[M+ H]⁺ |
| **Example 2-40** | 2-((4-(4-(((3-aminooxetan-3-yl)methyl)amino)-6-methylquinazolin-2-yl)-1-oxido-2,3,4,5-tetrahydro-1*H*-1λ⁴-benzo[*f*][1,4]thiazepin-1-ylidene)amino) acetamide | 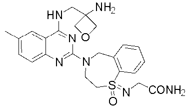 | Example 2-5 | In Step 2, 2-((1-oxido-2,3,4,5-tetrahydro-1*H*-1λ⁴-benzo[*f*][1,4] thiazepin-1-ylidene)amino) acetamide was used instead of 2,3,4,5-tetrahydropyrido[3,4 -f][1,4]thiazepine-1,1-dioxide. | ESI-MS(m/z): 496.21[M+ H]⁺ |

### Example 2-41: Synthesis of N-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)methanesulfonamide

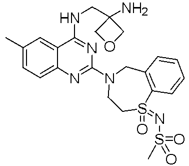

The synthesis method was the same as Example 2-5, except that *N*-(1-oxido-2,3,4,5-tetrahydro-1λ⁴-benzo[*f*][1,4]thiazepin-1-ylidene)methanesulfonamide was used instead of 2,3,4,5-tetrahydropyrido[3,4-*f*][1,4]thiazepine-1,1-dioxide in Step 2, to obtain *N*-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)methanesulfonamide (32 mg). ESI-MS(m/z): 517.16[M+H]⁺. ¹H NMR (600 MHz, CD₃OD) *δ* 8.05 (d, *J =* 7.5 Hz, 1H), 7.94 (d, *J =* 7.4 Hz, 1H), 7.71 (s, 1H), 7.66 (t, *J =* 7.3 Hz, 1H), 7.49 (t, *J =* 7.6 Hz, 1H), 7.40 (d, *J =* 8.4 Hz, 1H), 7.34 (d, *J =* 8.5 Hz, 1H), 5.50 (s, 1H), 5.02 (s, 2H), 4.65 (dd, *J =* 15.1, 6.1 Hz, 2H), 4.54 (t, *J =* 5.6 Hz, 2H), 4.11 (dd, *J =* 49.8, 35.4 Hz, 4H), 3.84 (s, 1H), 3.10 (s, 3H), 2.40 (s, 3H).

### Example 2-42: Synthesis of 3-(4-(4-(((3-aminooxetan-3-yl)methyl)amino)-6-methylquinazolin-2-yl)-1-oxido-2,3,4,5-tetrahydro-1λ⁴-benzo[f][1,4]thiazepin-1-ylidene)-1,1-dimethylurea

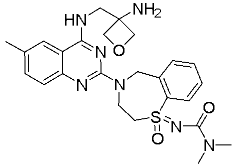

The synthesis method was the same as Example 2-5, except that 1,1-dimethyl-3-(1-oxido-2,3,4,5-tetrahydro-1λ⁴-benzo[*f*][1,4]thiazepin-1-ylidene)urea was used instead of 2,3,4,5-tetrahydropyrido[3,4-f][1,4]thiazepine-1,1-dioxide in Step 2, to obtain 3-(4-(4-(((3-aminooxetan-3-yl)methyl)amino)-6-methylquinazolin-2-yl)-1-oxido-2,3,4,5-tetrahydro-1λ⁴-benzo[*f*][1,4]thiazepin-1-ylidene)-1,1-dimethylurea (32 mg). ESI-MS(m/z): 510.22[M+H]⁺. H NMR (600 MHz, Methanol-d4) δ 8.06 (d, J = 7.9 Hz, 1H), 7.86 (d, J = 7.5 Hz, 1H), 7.69 (s, 1H), 7.58 (t, J = 7.5 Hz, 1H), 7.45 (t, J = 7.7 Hz, 1H), 7.39 (d, J = 8.6 Hz, 1H), 7.33 (d, J = 8.5 Hz, 1H), 5.43 (d, J = 14.5 Hz, 2H), 5.00 (d, J = 14.5 Hz, 2H), 4.64 (dd, J = 14.5, 6.3 Hz, 2H), 4.52 (t,J = 6.9 Hz, 2H), 4.07 (m, 4H), 3.07 (s, 3H), 2.83 (s, 3H), 2.38 (s, 3H).

**The preparation methods of the target compounds of Examples 2-43 and 2-44 are listed as below.**

| **Example No.** | **Chemical Name** | **Structural Formula** | **Characterization Data** |
|---|---|---|---|
| **Example 2-43** | 4-(4-(((3-aminooxetan-3-yl)methyl)amino)-6-methylquinazolin-2-yl)-1-(ethylimino)-2,3,4,5-tetrahydro-1*H*-1λ⁴-benzo[*f*][1,4]thiazepine-1-oxide | 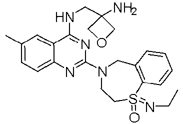 | ESI-MS(m/z): 467.22[M+H]⁺ |
| **Example 2-44** | 4-(4-(((3-aminooxetan-3-yl)methyl)amino)-6-methylquinazolin-2-yl)-1-(cyclobutylimino)-2,3,4,5-tetrahydro-1*H*-1λ⁴-benzo[f][1,4]thiazepine-1-oxide | 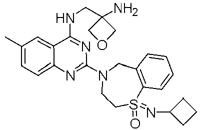 | ESI-MS(m/z): 493.23[M+H]⁺ |

### Example 2-46: Synthesis of 4-(4-(((3-aminooxetan-3-yl)methyl)amino)-6-(difluoromethyl)quinazolin-2-yl)-1-(cyclopropylimino)-2,3,4,5-tetrahydro-1H-1λ⁴-benzo[f][1,4]thiazepine-1-oxide

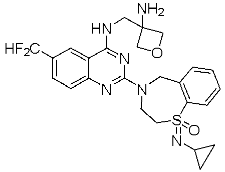

The synthesis method was the same as Example 2-5. ESI-MS(m/z): 515.20[M+H]⁺. ¹H NMR (600 MHz, DMSO-*d6*) δ 8.35 (d, J = 29.5 Hz, 1H), 8.28 (s, 1H), 7.94 (d, *J =* 13.2 Hz, 1H), 7.86 (s, 1H), 7.67 (d, *J =* 11.8 Hz, 1H), 7.59 (s, 1H), 7.45 (dd, *J =* 40.9, 34.2 Hz, 2H), 7.02 (t, *J* = 56.2 Hz, 1H), 5.51 - 5.28 (m, 1H), 4.99 (s, 1H), 4.75 (d, *J =* 13.0 Hz, 1H), 4.56 (dd, *J* = 45.8, 31.5 Hz, 3H), 4.33 - 4.19 (m, 1H), 4.10 - 3.84 (m, 2H), 3.60 (dd, *J* = 57.4, 37.4 Hz, 2H), 2.20 (s, 1H), 1.17 (t, *J =* 7.3 Hz, 1H), 0.39 (s, 2H), 0.25 - 0.04 (m, 2H).

**The preparation methods of the target compounds of Examples 2-47 to 2-51, 2-26A and 2-26B are listed as below.**

| **Example No.** | **Chemical Name** | **Structural Formula** | **Characterization Data** |
|---|---|---|---|
| **Example 2-47** | 4-(4-(((3-aminooxetan-3-yl)methyl)amino)-6-(difluoromethyl)quinazolin-2-yl)-1-((2,2-difluorocyclopropyl)imino)-2,3,4,5-tetrahydro-1*H*-1λ⁴-benzo[*f*][1,4]thiazepine-1-oxide | 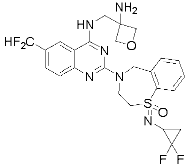 | ESI-MS(m/z): 551.18[M+H]⁺ |
| **Example 2-48** | 4-(4-(((3-aminooxetan-3-yl)methyl)amino)-6-(difluoromethyl)quinazolin-2-yl)-1-((1-methylcyclopropyl)imino)-2,3,4,5-tetrahydro-1*H*-1λ⁴-benzo[*f*][1,4]thiazepine-1-oxide | 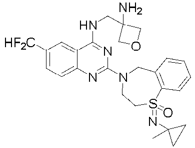 | ESI-MS(m/z): 529.21[M+H]⁺ |
| **Example 2-49** | 4-(4-(((3-aminooxetan-3-yl)methyl)amino)-6-(difluoromethyl)quinazolin-2-yl)-1-(oxetan-3-ylimino)-2,3,4,5-tetrahydro-1*H*-1λ⁴-benzo[*f*][1,4]thiazepine-1-oxide | 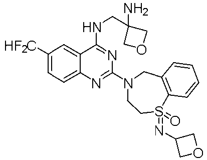 | ESI-MS(m/z): 531.19[M+H]⁺ |
| **Example 2-50** | 4-(4-(((3-aminooxetan-3-yl)methyl)amino)-6-(difluoromethyl)quinazolin-2-yl)-1-((2,2,2-trifluoroethyl)imino)-2,3,4,5-tetrahydro-1*H*-1λ⁴-benzo[*f*][1,4]thiazepine-1-oxide | 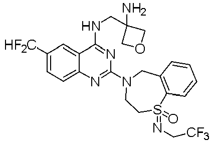 | ESI-MS(m/z): 557.17[M+H]⁺ |
| **Example 2-51** | 4-(4-(((3-aminooxetan-3-yl)methyl)amino)-6-(difluoromethyl)quinazolin-2-yl)-1-((3,3-difluorocyclobutyl)imino)-2,3,4,5-tetrahydro-1*H*-1λ⁴-benzo[*f*][1,4]thiazepine-1-oxide | 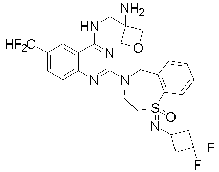 | ESI-MS(m/z): 565.19[M+H]⁺ |
| **Example 2-26A** | (S)-4-(4-(((3-aminooxetan-3-yl)methyl)amino)-6-(difluoromethyl)quinazolin-2-yl)-1-(cyclopropylimino)-2,3,4,5-tetrahydro-1*H*-1λ⁴-benzo[*f*][1,4]thiazepine-1-oxide | 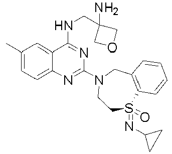 | ESI-MS(m/z): 479.45[M+H]⁺ |
| **Example 2-26B** | (R)-4-(4-(((3-aminooxetan-3-yl)methyl)amino)-6-(difluoromethyl)quinazolin-2-yl)-1-(cyclopropylimino)-2,3,4,5-tetrahydro-1*H*-1λ⁴-benzo[*f*][1,4]thiazepine-1-oxide | 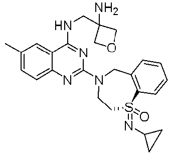 | ESI-MS(m/z): 479.45[M+H]⁺ |

### Example 3-22: Synthesis of 4-(4-(((3-aminooxetan-3-yl)methyl)amino)-6-methyl-5,6,7,8-tetrahydropyrido[4,3-d]pyrimidin-2-yl)-1-(methylimino)-2,3,4,5-tetrahydro-1H-1λ⁴-benzo[f][1,4]thiazepine-1-oxide

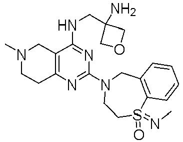

The synthesis method of this compound was the same as Example 1-1, except that 1-(methylimino)-2,3,4,5-tetrahydro-1*H*-1λ⁴-benzo[*f*][1,4]thiazepine-1-oxide was used instead of 2,3,4,5-tetrahydropyrido[3,4-*f*][1,4]thiazepine-1,1-dioxide in Step 2, to obtain 4-(4-(((3-aminooxetan-3-yl)methyl)amino)-6-methyl-5,6,7,8-tetrahydropyrido[4,3-*d*]pyrimidin-2-yl)-1-(methylimino)-2,3,4,5-tetrahydro-1*H*-1λ⁴-benzo[*f*][1,4]thiazepine-1-oxide. ESI-MS(m/z): 458.23[M+H]⁺. ¹H NMR (600 MHz, CD₃OD) *δ* 7.96 (d, *J =* 7.6 Hz, 1H), 7.78 (d, *J =* 6.2 Hz, 1H), 7.56 (t, *J* = 7.2 Hz, 1H), 7.45 (t, *J =* 7.4 Hz, 1H), 5.29 (d, *J =* 14.9 Hz, 1H), 4.92 (s, 1H), 4.71 (d, *J =* 14.9 Hz, 1H), 4.56 (dd, *J =* 19.8, 5.7 Hz, 2H), 4.47 (s, 2H), 3.97 - 3.83 (m, 2H), 3.81 (d, *J =* 13.6 Hz, 1H), 3.55 (d, *J =* 14.6 Hz, 1H), 3.42 (t, *J =* 12.3 Hz, 1H), 3.22 (s, 2H), 2.68 (s, 2H), 2.67 (s, 1H), 2.64 (d, *J =* 7.9 Hz, 1H), 2.53 (s, 3H), 2.45 (s, 3H).

### Example 3-27: Synthesis of 4-(4-(((3-aminooxetan-3-yl)methyl)amino)-6-(trifluoromethyl)quinazolin-2-yl)-2,3,4,5-tetrahydropyrido[2,3-f][1,4]thiazepine-1,1-dioxide

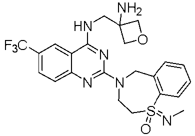

The synthesis method of this compound was the same as Example 1-1, except that 2,4-dichloro-6-(trifluoromethyl)quinazoline was used instead of 2,4-dichloro-6-methyl-5,6,7,8-tetrahydropyrido[4,3-*d*]pyrimidine in Step 1, and 1-(methylimino)-2,3,4,5-tetrahydro-1*H*-1λ⁴-benzo[*f*][1,4]thiazepine-1-oxide was used instead of 2,3,4,5-tetrahydropyrido[3,4-f][1,4]thiazepine-1,1-dioxide in Step 2, to obtain 4-(4-(((3-aminooxetan-3-yl)methyl)amino)-6-(trifluoromethyl)quinazolin-2-yl)-2,3,4,5-tetrahydropyrido[2,3-f][1,4]thiazepine-1,1-dioxide (23 mg). ESI-MS(m/z): 507.17[M+H]⁺.

### Example 4-2: Synthesis of 4-(4-(3-(aminomethyl)-3-hydroxyazetidin-1-yl)-6-methylquinazolin-2-yl)-1-(cyclopropylimino)-2,3,4,5-tetrahydro-benzo[f][1,4]thiazepine-1-oxide

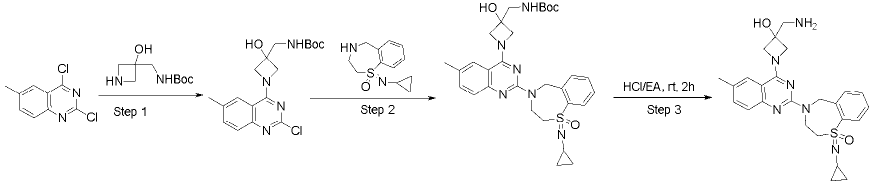

### Step 1: tert-Butyl (((1-(2-chloro-6-methylquinazolin-4-yl)-3-hydroxyazetidin-3-yl)methyl)carbamate

2,4-Dichloro-6-methylquinazoline (100 mg), methanol (13 mL), tert-butyl ((3-hydroxyazetidin-3-yl)methyl)carbamate (87 mg), and triethylamine (130 µL) were sequentially added into a 25 mL reaction flask. The resulting mixture reacted at room temperature for 1 h. After the reaction was completed, water was added to quench the reaction, methanol was removed by rotary evaporation, and the residue was extracted twice with DCM. The organic phases were combined, washed with saturated brine, dried, and spin-dried to obtain a crude product, which was slurried with petroleum ether/ethyl acetate (1:3) to yield the product (125 mg).

### Step 2: tert-Butyl ((1-(2-(1-(cyclopropylimino)-1-oxido-1,2,3,5-tetrahydro-benzo[f][1,4]thiazepin-4-yl)-6-methylquinazolin-4-yl)-3-hydroxyazetidin-3-yl)methyl)carbamate

tert-Butyl ((1-(2-chloro-6-methylquinazolin-4-yl)-3-hydroxyazetidin-3-yl)methyl)carbamate (76 mg), 1-(cyclopropylimino)-2,3,4,5-tetrahydro-benzo[*f*][1,4]thiazepine-1-oxide (52 mg), ammonium chloride (10 mg), and ethanol (10 mL) were sequentially added into a 50 mL reaction flask. The resulting mixture was refluxed until the starting materials disappeared. A small amount of water was added to quench the reaction, ethanol was removed by rotary evaporation, the aqueous phase was extracted twice with DCM, and the organic phases were combined, washed with saturated brine, and spin-dried to obtain a crude product (130 mg).

### Step 3: 4-(4-(3-(aminomethyl)-3-hydroxyazetidin-1-yl)-6-methylquinazolin-2-yl)-1-(cyclopropylimino)-2,3,4,5-tetrahydro-benzo[f][1,4]thiazepine-1-oxide

tert-Butyl ((1-(2-(1-(cyclopropylimino)-1-oxido-1,2,3,5-tetrahydro-benzo[*f*][1,4]thiazepin-4-yl)-6-methylquinazolin-4-yl)-3-hydroxyazetidin-3-yl)methyl)carbamate (100 mg) was dissolved in ethyl acetate (12 mL), and a 4M hydrogen chloride/ethyl acetate solution (10 mL) was added. The reaction was conducted at room temperature until the starting materials disappeared. Suction filtration was performed, the filter cake was collected, and ethyl acetate was removed under reduced pressure to obtain a hydrochloride salt crude product. The crude product was dissolved in water, the pH was adjusted to 8-9 with saturated sodium bicarbonate, followed by suction filtration and drying to obtain the product (60 mg). ESI-MS(m/z): 479.22 [M+H]+;¹H NMR (600 MHz, CD₃OD) *δ* 8.03 (d, *J =* 7.4 Hz, 1H), 7.80 (d, *J =* 7.4 Hz, 1H), 7.60 (t, *J =* 7.3 Hz, 1H), 7.51 (s, 1H), 7.46 (t, *J =* 7.5 Hz, 1H), 7.36 (dt, *J =* 19.9, 5.0 Hz, 2H), 5.42 (d, *J* = 15.0 Hz, 1H), 5.00 (s, 1H), 4.79 (d, *J =* 15.0 Hz, 1H), 4.53 (dd, *J* = 21.9, 8.8 Hz, 2H), 4.33 (d, *J =* 9.2 Hz, 1H), 4.27 (d, *J =* 9.3 Hz, 1H), 3.94 (s, 1H), 3.63 - 3.47 (m, 2H), 3.01 (s, 2H), 2.36 (s, 3H), 2.28 (dd, *J =* 13.6, 10.3 Hz, 1H), 0.60 - 0.50 (m, 1H), 0.50 - 0.39 (m, 1H), 0.33 - 0.17 (m, 2H).

### Example 4-3: Synthesis of 4-(4-(azetidin-3-ylamino)-6-methylquinazolin-2-yl)-1-(cyclopropylimino)-2,3,4,5-tetrahydro-benzo[f][1,4]thiazepine-1-oxide

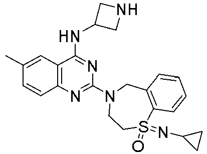

The synthesis method of this compound was the same as Example 4-2. ESI-MS(m/z): 449.20 [M+H]⁺, ¹H NMR (600 MHz, CDCl₃) δ 8.05 (d, *J =* 7.0 Hz, 1H), 7.80 (s, 1H), 7.67 (s, 1H), 7.51 (t, *J =* 7.4 Hz, 1H), 7.36 (dd, *J* = 19.2, 7.9 Hz, 3H), 5.37 (s, 2H), 4.82 (d, *J =* 14.7 Hz, 2H), 4.51 (s, 2H), 4.27 (d, *J =* 35.7 Hz, 2H), 4.01 (s, 1H), 3.57 (d, *J =* 14.2 Hz, 1H), 3.41 (s, 1H), 2.34 (s, 2H), 2.28 (s, 1H), 2.07 (s, 1H), 0.57 (d, *J =* 4.4 Hz, 1H), 0.46 (dd, *J* = 15.5, 5.8 Hz, 1H), 0.33 - 0.19 (m, 2H).

### Example 4-4: Synthesis of 1-(cyclopropylimino)-4-(6-methyl-4-(2,6-diazaspiro[3.3]heptan-2-yl)quinazolin-2-yl)-2,3,4,5-tetrahydro-benzo[f][1,4]thiazepine-1-oxide

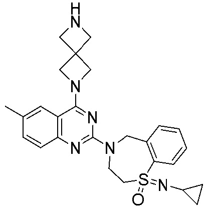

The synthesis method of this compound was the same as Example 4-2. ESI-MS(m/z): 475.22 [M+H]⁺, ¹H NMR (600 MHz, CDCl₃) δ 8.11 (s, 1H), 7.56 (s, 2H), 7.44 (s, 1H), 7.38 (s, 1H), 7.31 (s, 1H), 4.72 (s, 4H), 4.39 (s, 3H), 4.11 - 3.96 (m, 2H), 3.81 - 3.68 (m, 2H), 3.44 (dd, *J =* 44.7, 37.7 Hz, 4H), 2.27 (s, 3H), 0.58 (s, 1H), 0.47 (s, 1H), 0.28 (d, *J =* 28.9 Hz, 2H).

### Example 4-5: Synthesis of 4-(4-(((3-aminooxetan-3-yl)methyl)amino)-6-methylquinazolin-2-yl)-1-((2-fluoroethyl)imino)-2,3,4,5-tetrahydrobenzo[f][1,4]thiazepine-1-oxide

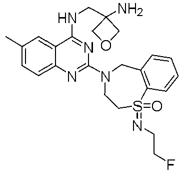

The synthesis method of this compound was the same as Example 4-2. ESI-MS(m/z): 485.23[M+H]⁺; ¹H NMR (600 MHz, Methanol-*d*₄) δ 7.99 - 7.95 (m, 1H), 7.87 (d, *J =* 7.5 Hz, 1H), 7.68 (s, 1H), 7.58 (t, *J =* 7.5 Hz, 1H), 7.46 - 7.41 (m, 1H), 7.38 (dd, *J* = 8.6, 1.9 Hz, 1H), 7.31 (d, *J =* 8.5 Hz, 1H), 5.44 (d, *J =* 15.1 Hz, 1H), 5.07 (s, 1H), 4.64 (dd, *J =* 20.3, 6.3 Hz, 2H), 4.53 (dd, *J =* 6.3, 3.7 Hz, 2H), 4.51 - 4.32 (m, 2H), 4.00 (d, *J =* 13.8 Hz, 2H), 3.63 (ddd, *J =* 14.7, 3.7, 2.0 Hz, 1H), 3.52 (t, *J =* 13.1 Hz, 1H), 3.14 (ddt, *J =* 28.7, 13.9, 4.8 Hz, 1H), 3.01 - 2.87 (m, 1H), 2.38 (s, 3H), 1.29 (t, *J =* 4.1 Hz, 2H).

### Example 4-6: Synthesis of 4-(4-(((3-aminooxetan-3-yl)methyl)amino)-6-methylquinazolin-2-yl)-1-((2,2-difluoroethyl)imino)-2,3,4,5-tetrahydrobenzo[f][1,4]thiazepine-1-oxide

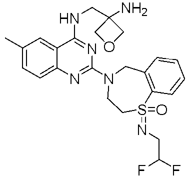

The synthesis method of this compound was the same as Example 4-2. ESI-MS(m/z): 503.23[M+H]⁺; ¹H NMR (600 MHz, Methanol-*d*₄) δ 7.97 (dd, *J* = 7.9, 1.3 Hz, 1H), 7.90 - 7.86 (m, 1H), 7.70 - 7.67 (m, 1H), 7.62 - 7.56 (m, 1H), 7.44 (td, *J =* 7.7, 1.3 Hz, 1H), 7.37 (dd, *J =* 8.6, 1.9 Hz, 1H), 7.31 (d, *J =* 8.6 Hz, 1H), 6.00 - 5.67 (m, 1H), 5.45 (d, *J* = 15.2 Hz, 1H), 5.08 (s, 1H), 4.64 (dd, *J =* 20.2, 6.3 Hz, 2H), 4.53 (dd, *J =* 6.3, 3.5 Hz, 2H), 4.05 (dd, *J =* 63.1, 12.8 Hz, 2H), 3.68 - 3.48 (m, 2H), 3.24 - 2.86 (m, 2H), 2.37 (s, 3H), 1.35 - 1.26 (m, 2H).

### Example 4-7: Synthesis of 4-(4-(((R)-2-(aminomethyl)pyrrolidin-1-yl)-6-methylquinazolin-2-yl)-1-(cyclopropylimino)-2,3,4,5-tetrahydrobenzo[f][1,4] thiazepine-1-oxide

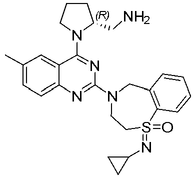

The synthesis method of this compound was the same as Example 4-2. ESI-MS(m/z): 477.28[M+H]⁺; ¹H NMR (600 MHz, Methanol-*d*₄) δ 8.00 - 7.96 (m, 1H), 7.80 (d, *J =* 7.5 Hz, 1H), 7.73 (s, 1H), 7.58 - 7.53 (m, 1H), 7.43 - 7.37 (m, 1H), 7.35 (d, *J =* 1.2 Hz, 2H), 5.40 (d, *J =* 15.0 Hz, 1H), 4.80 (d, *J =* 15.1 Hz, 1H), 4.65 (s, 1H), 4.02 (td, *J =* 9.6, 6.5 Hz, 1H), 3.85 (ddd, *J* = 10.4, 7.7, 3.1 Hz, 1H), 3.63 (dt, *J =* 15.1, 2.6 Hz, 1H), 3.55 (ddd, *J =* 14.4, 10.9, 3.0 Hz, 1H), 3.09 (dd, *J* = 13.0, 3.4 Hz, 1H), 2.80 (dd, *J =* 12.7, 7.6 Hz, 1H), 2.36 (s, 3H), 2.28 (dq, *J =* 7.4, 3.8 Hz, 1H), 2.22 - 2.14 (m, 1H), 2.05 (ddq, *J* = 13.9, 7.4, 4.8, 3.9 Hz, 1H), 1.97 - 1.88 (m, 1H), 1.85 - 1.75 (m, 1H), 1.37 - 1.24 (m, 2H), 0.92 - 0.85 (m, 1H), 0.55 - 0.48 (m, 1H), 0.43 (dtd, *J* = 9.8, 6.8, 4.7 Hz, 1H), 0.30 - 0.19 (m, 1H).

### Example 4-8: Synthesis of 4-(4-(6-amino-3-azabicyclo[3.1.0]hexan-3-yl)-6-methylquinazolin-2-yl)-1-(cyclopropylimino)-2,3,4,5-tetrahydro-benzo[f][1,4]thiazepine-1-oxide

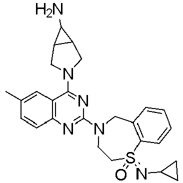

The synthesis method of this compound was the same as Example 4-2. ESI-MS(m/z):475.63 [M+H]⁺; ¹H NMR (600 MHz, CD₃OD) δ 7.96 (d, *J =* 7.7 Hz, 1H), 7.75 (d, *J =* 7.4 Hz, 1H), 7.68 (s, 1H), 7.54 (t, *J =* 7.3 Hz, 1H), 7.38 (t, *J =* 7.6 Hz, 1H), 7.31 - 7.26 (m, 2H), 5.34 (d, *J =* 15.1 Hz, 1H), 4.91 (s, 1H), 4.72 (d, *J =* 15.0 Hz, 1H), 4.19 (t, *J* = 12.1 Hz, 2H), 3.86 (d, *J =* 10.9 Hz, 3H), 3.55 (dd, *J =* 12.4, 3.0 Hz, 1H), 3.51 - 3.46 (m, 1H), 3.33 (dt, *J =* 3.2, 1.6 Hz, 1H), 2.30 (s, 3H), 2.28 - 2.24 (m, 1H), 1.92 (s, 1H), 1.73 (s, 1H), 0.52 (dq, *J =* 6.5, 4.5 Hz, 1H), 0.43 (ddd, *J =* 9.5, 8.3, 4.3 Hz, 1H), 0.29 - 0.19 (m, 2H).

### Example 4-9: Synthesis of 4-(4-(3-(aminomethyl)azetidin-1-yl)-6-methylquinazolin-2-yl)-1-(cyclopropylimino)-2,3,4,5-tetrahydro-benzo[f][1,4]thiazepine-1-oxide

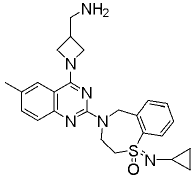

The synthesis method of this compound was the same as Example 4-2. ESI-MS(m/z): 463.22 [M+H]+;¹H NMR (600 MHz, CD₃OD) *δ* 8.01 (d, *J* = 7.3 Hz, 1H), 7.79 (d, *J* = 7.4 Hz, 1H), 7.58 (t, *J* = 7.3 Hz, 1H), 7.48 (s, 1H), 7.44 (t, *J* = 7.5 Hz, 1H), 7.33 (dt, *J* = 15.9, 5.1 Hz, 2H), 5.40 (d, *J* = 15.0 Hz, 1H), 4.99 (s, 1H), 4.77 (d, *J* = 14.9 Hz, 1H), 4.56 (d, *J* = 31.1 Hz, 2H), 4.20 (d, *J* = 25.1 Hz, 2H), 3.89 (d, *J* = 40.0 Hz, 1H), 3.54 (dd, *J* = 26.2, 7.2 Hz, 2H), 2.99 (d, *J* = 7.2 Hz, 2H), 2.87 (dt, *J* = 7.6, 5.4 Hz, 1H), 2.34 (s, 3H), 2.31 - 2.24 (m, 1H), 0.54 (td, *J* = 5.7, 2.0 Hz, 1H), 0.49 - 0.37 (m, 1H), 0.32 - 0.13 (m, 2H).

### Example 4-10: Synthesis of 1-(cyclopropylimino)-4-(4-(((3R,4R)-4-hydroxypyrrolidin-3-yl)amino)-6-methylquinazolin-2-yl)-2,3,4,5-tetrahydro-benzo[f][1,4]thiazepine-1-oxide

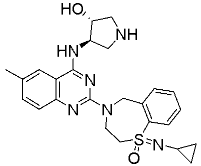

The synthesis method of this compound was the same as Example 4-2. ESI-MS(m/z): 479.22 [M+H]⁺, ¹H NMR (600 MHz, CDCl₃) δ 7.87 (dd, *J =* 72.0, 36.2 Hz, 4H), 7.51 (d, *J =* 7.3 Hz, 1H), 7.30 (s, 3H), 5.44 (s, 1H), 5.04 (s, 1H), 4.79 (s, 2H), 4.51 (s, 1H), 3.92 (s, 2H), 3.70 (s, 2H), 3.47 (s, 3H), 2.29 (d, *J =* 32.3 Hz, 4H), 0.52 (s, 1H), 0.43 (s, 1H), 0.22 (d, *J =* 29.5 Hz, 2H).

### Example 4-11: Synthesis of 4-(4-(((S)-3-aminopyrrolidin-1-yl)-6-methylquinazolin-2-yl)-1-(cyclopropylimino)-2,3,4,5-tetrahydrobenzo[f][1,4] thiazepine-1-oxide

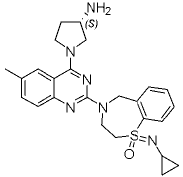

The synthesis method of this compound was the same as Example 4-2. ESI-MS(m/z): 463.24[M+H]⁺; ¹H NMR (600 MHz, Methanol-*d*₄) δ 7.97 (dd, *J* = 7.8, 1.4 Hz, 1H), 7.78 - 7.75 (m, 2H), 7.59 - 7.54 (m, 1H), 7.38 (td, *J =* 7.6, 1.3 Hz, 1H), 7.30 (d, *J =* 1.2 Hz, 2H), 5.39 (d, *J* = 15.1 Hz, 1H), 5.02 - 4.90 (m, 1H), 4.77 - 4.72 (m, 1H), 4.10 - 3.99 (m, 2H), 3.94 - 3.84 (m, 2H), 3.70 - 3.62 (m, 2H), 3.58 - 3.47 (m, 2H), 2.30 (s, 3H), 2.27 - 2.15 (m, 2H), 1.86 (dt, *J =* 13.2, 6.6 Hz, 1H), 0.50 - 0.43 (m, 1H), 0.40 (dtd, *J* = 9.8, 6.8, 4.8 Hz, 1H), 0.23 (dddd, *J =* 23.2, 10.4, 6.2, 4.4 Hz, 2H).

### Example 4-12: Synthesis of 4-(4-(((R)-3-aminopyrrolidin-1-yl)-6-methylquinazolin-2-yl)-1-(cyclopropylimino)-2,3,4,5-tetrahydrobenzo[f][1,4]thiazepine-1-oxide

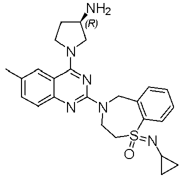

The synthesis method of this compound was the same as Example 4-2. ESI-MS(m/z): 463.27[M+H]⁺; ¹H NMR (600 MHz, Methanol-*d*₄) δ 7.99 (dd, *J =* 7.8, 1.4 Hz, 1H), 7.79 - 7.76 (m, 2H), 7.58 - 7.53 (m, 1H), 7.41 (td, *J =* 7.6, 1.3 Hz, 1H), 7.33 (d, *J =* 1.2 Hz, 2H), 5.41 (d, *J =* 15.1 Hz, 1H), 5.05 - 4.93 (m, 1H), 4.79 - 4.74 (m, 1H), 4.11 - 4.00 (m, 2H), 3.95 - 3.85 (m, 2H), 3.71 - 3.64 (m, 2H), 3.59 - 3.47 (m, 2H), 2.34 (s, 3H), 2.28 - 2.16 (m, 2H), 1.89 (dt, *J* = 13.2, 6.6 Hz, 1H), 0.55 - 0.48 (m, 1H), 0.42 (dtd, *J =* 9.8, 6.8, 4.8 Hz, 1H), 0.24 (dddd, *J =* 23.2, 10.4, 6.2, 4.4 Hz, 2H).

### Example 4-13: Synthesis of 4-(4-(((1R,3S)-3-aminocyclobutyl)amino)-6-methylquinazolin-2-yl)-1-(cyclopropylimino)-2,3,4,5-tetrahydro-benzo[f][1,4]thiazepine-1-oxide

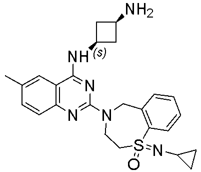

ESI-MS(m/z): 463.2[M+H]+. 1H NMR (600 MHz, DMSO-*d₆*) δ 7.92 (d, *J =* 6.8 Hz, 1H), 7.86 (d, *J =* 6.3 Hz, 1H), 7.84 (s, 1H), 7.57 (s, 1H), 7.46 (s, 1H), 7.33 (d, *J* = 8.2 Hz, 1H), 7.20 (s, 1H), 5.30 (d, *J =* 12.1 Hz, 1H), 4.90 (s, 1H), 4.70 (d, *J =* 13.4 Hz, 1H), 3.83 (s, 1H), 3.65 (d, *J =* 13.2 Hz, 2H), 2.65 (d, *J =* 4.2 Hz, 2H), 2.52 - 2.50 (m, 2H), 2.33 (s, 3H), 2.20 (d, *J* = 22.3 Hz, 1H), 1.82 (dd, *J =* 12.3, 5.9 Hz, 2H), 0.43 - 0.34 (m, 2H), 0.19 (s, 1H), 0.11 (s, 1H).

### Example 4-14: Synthesis of 1-(cyclopropylimino)-4-(4-(((3R,4S)-4-hydroxypyrrolidin-3-yl)amino)-6-methylquinazolin-2-yl)-2,3,4,5-tetrahydro-benzo[f][1,4]thiazepine-1-oxide

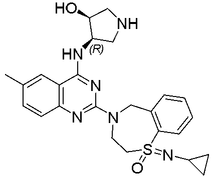

The synthesis method of this compound was the same as Example 4-2. ESI-MS(m/z): 479.2[M+H]⁺. ¹H NMR (600 MHz, CD₃OD) δ 8.01 (dd, *J* = 7.6, 3.9 Hz, 1H), 7.78 (d, *J =* 7.4 Hz, 1H), 7.65 (s, 0.5H), 7.61 (s, 0.5H), 7.57 (dd, *J* = 8.5, 5.3 Hz, 1H), 7.44 (dd, *J* = 11.9, 7.6 Hz, 1H), 7.37 (t, *J* = 8.7 Hz, 1H), 7.31 (d, *J =* 8.6 Hz, 1H), 5.42 (d, *J* = 15.0 Hz, 1H), 5.01 (s, 1H), 4.79 (d, *J =* 14.8 Hz, 1H), 4.69 (s, 1H), 4.44 (d, *J =* 41.1 Hz, 1H), 3.95 (s, 1H), 3.60 (dd, *J =* 14.6, 2.0 Hz, 1H), 3.56 - 3.46 (m, 1H), 3.38 - 3.34 (m, 2H), 3.07 - 2.98 (m, 2H), 2.37 (d, *J =* 6.6 Hz, 3H), 2.27 (s, 1H), 0.52 (dd, *J* = 6.1, 4.0 Hz, 1H), 0.46 - 0.40 (m, 1H), 0.29 - 0.19 (m, 2H).

### Example 4-15: Synthesis of 4-(4-(((3-aminooxetan-3-yl)methyl)amino)-5,6-dimethylquinazolin-2-yl)-1-(cyclopropylimino)-2,3,4,5-tetrahydro-benzo[f][1,4]thiazepine-1-oxide

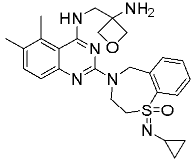

The synthesis method of this compound was the same as Example 4-2. ESI-MS(m/z): 493.23 [M+H]+;¹H NMR (600 MHz, CD₃OD) *δ* 8.04 (d, *J* = 7.7 Hz, 1H), 7.87 (d, *J =* 7.4 Hz, 1H), 7.60 (t, *J =* 7.2 Hz, 1H), 7.48 (t, *J* = 7.4 Hz, 1H), 7.34 (d, *J* = 8.5 Hz, 1H), 7.20 (d*, J =* 8.5 Hz, 1H), 5.46 (d, *J =* 14.8 Hz, 1H), 5.02 (s, 1H), 4.83 (d, *J* = 15.2 Hz, 1H), 4.72 - 4.49 (m, 4H), 4.08 (d, *J =* 13.7 Hz, 1H), 3.97 (d, *J =* 13.4 Hz, 2H), 3.63 (d, *J* = 15.9 Hz, 1H), 3.57 - 3.47 (m, 1H), 2.66 (s, 3H), 2.33 (s, 3H), 2.28 (dd, *J =* 14.6, 11.0 Hz, 1H), 0.61 - 0.50 (m, 1H), 0.50 - 0.39 (m, 1H), 0.33 - 0.18 (m, 2H).

### Example 4-16: Synthesis of 4-(4-(((3-aminooxetan-3-yl)methyl)amino)-6-(difluoromethyl)quinazolin-2-yl)-1-(cyclobutylimino)-2,3,4,5-tetrahydro-benzo[f][1,4]thiazepine-1-oxide

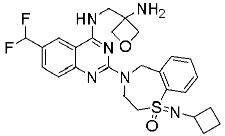

The synthesis method of this compound was the same as Example 4-2. ESI-MS(m/z): 529.21 [M+H]⁺, ¹H NMR (600 MHz, DMSO-d6) δ 8.31 (s, 1H), 8.20 (d, *J* = 25.8 Hz, 1H), 7.92 - 7.79 (m, 2H), 7.61 (dd, *J* = 37.4, 7.4 Hz, 2H), 7.41 (dd, *J* = 59.4, 16.6 Hz, 2H), 7.00 (t, *J =* 56.2 Hz, 1H), 5.36 (d, *J =* 13.6 Hz, 1H), 4.97 (dd, *J =* 20.9, 13.5 Hz, 1H), 4.79 (d, *J* = 12.5 Hz, 1H), 4.54 - 4.27 (m, 4H), 4.07 - 3.82 (m, 3H), 3.80 - 3.52 (m, 2H), 3.42 (d, *J =* 21.9 Hz, 1H), 2.07 - 1.90 (m, 2H), 1.69 (d*, J* = 36.6 Hz, 2H), 1.53 (d, *J* = 9.2 Hz, 1H), 1.42 (dd, *J* = 18.1, 8.9 Hz, 1H).

### Example 4-17: Synthesis of 4-(4-(3-aminoazetidin-1-yl)-6-(difluoromethyl)quinazolin-2-yl)-1-(cyclopropylimino)-2,3,4,5-tetrahydro-benzo[f][1,4]thiazepine-1-oxide

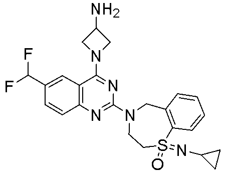

The synthesis method of this compound was the same as Example 4-2. ESI-MS(m/z): 485.18[M+H]+; ¹H NMR (600 MHz,Methanol-d4) δ 8.04 (d, *J* = 7.8 Hz, 1H), 7.82 (dd, *J =* 14.8, 4.8 Hz, 2H), 7.73 - 7.57 (m, 2H), 7.48 (t, *J* = 7.7 Hz, 2H), 6.77 (t*, J* = 56.3 Hz, 1H), 5.42 (d, *J* = 15.0 Hz, 1H), 5.06 (s, 1H), 4.81 (d, *J =* 15.2 Hz, 3H), 4.10 (dd, *J* = 139.9, 32.5 Hz, 4H), 3.68 - 3.42 (m, 2H), 2.34 - 2.23 (m, 1H), 0.64 - 0.40 (m, 2H), 0.33 - 0.16 (m, 2H).

### Example 4-19: Synthesis of 4-(4-((1S,4S)-2,5-diazabicyclo[2.2.1]heptan-2-yl)-6-methylquinazolin-2-yl)-1-(cyclopropylimino)-2,3,4,5-tetrahydro-benzo[f][1,4]thiazepine-1-oxide

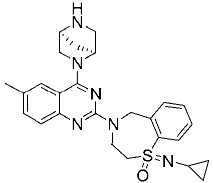

The synthesis method of this compound was the same as Example 4-2. ESI-MS(m/z): 475.22 [M+H]⁺, ¹H NMR (600 MHz, CDCl3) δ 8.08 (d*, J* = 8.2 Hz, 1H), 7.49 (dd, *J* = 15.6, 7.7 Hz, 1H), 7.44 (d, *J =* 14.0 Hz, 2H), 7.41 - 7.36 (m, 3H), 5.39 (d*, J* = 13.3 Hz, 1H), 5.22 (s, 1H), 4.99 (s, 1H), 4.86 (d, *J* = 14.8 Hz, 1H), 4.35 (s, 1H), 4.14 - 4.01 (m, 4H), 3.56 - 3.42 (m, 3H), 2.37 (s, 3H), 2.30 (s, 1H), 2.20 - 2.08 (m, 2H), 1.91 (s, 1H), 0.64 - 0.57 (m, 1H), 0.49 - 0.44 (m, 1H), 0.32 - 0.22 (m, 2H).

### Example 4-21: Synthesis of 1-(4-(3-aminoazetidin-1-yl)-2-(1-(cyclopropylimino)-1-oxido-1,2,3,5-tetrahydro-benzo[f][1,4]thiazepin-4-yl)-7,8-dihydropyrido[4,3-d]pyrimidin-6(5R)-yl)-2,2,2-trifluoroethan-1-one

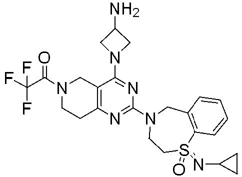

The synthesis method of this compound was the same as Example 4-2. ESI-MS(m/z):536.20 [M+H]+; ¹H NMR (600 MHz, CD₃OD) *δ* 8.02 (d, *J =* 7.3 Hz, 1H), 7.71 (d, *J* = 7.4 Hz, 1H), 7.59 (t, *J* = 7.4 Hz, 1H), 7.48 (t,*J* = 7.5 Hz, 1H), 5.27 (d, *J =* 14.9 Hz, 1H), 4.89 (s, 1H), 4.75 (d, *J =* 15.0 Hz, 1H), 4.63 - 4.28 (m, 4H), 4.06 - 3.68 (m, 6H), 3.54 (d, *J =* 14.7 Hz, 1H), 3.49 - 3.37 (m, 1H), 2.80 - 2.57 (m, 2H), 2.27 (s, 1H), 0.53 (ddd, *J =* 10.2, 6.5, 4.6 Hz, 1H), 0.48 - 0.38 (m, 1H), 0.34 - 0.15 (m, 2H).

### Example 4-22: Synthesis of 4-(4-(3-amino-4-fluoropyrrolidin-1-yl)-6-methylquinazolin-2-yl)-1-(cyclopropylimino)-2,3,4,5-tetrahydro-benzo[f][1,4]thiazepine-1-oxide

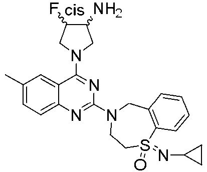

The synthesis method of this compound was the same as Example 4-2. ESI-MS(m/z): 481.21 [M+H]+;¹H NMR (600 MHz, CD₃OD) *δ* 8.01 (dd, *J =* 7.3, 3.1 Hz, 1H), 7.81 (t, *J* = 7.5 Hz, 1H), 7.76 (d, *J =* 9.0 Hz, 1H), 7.58 (dd, *J* = 13.6, 6.5 Hz, 1H), 7.44 (t, *J =* 7.6 Hz, 1H), 7.39 - 7.33 (m, 2H), 5.49 - 5.34 (m, 1H), 5.10 - 4.89 (m, 2H), 4.80 (dd, *J* = 14.7, 7.3 Hz, 1H), 4.35 - 4.00 (m, 3H), 3.95 (s, 1H), 3.86 - 3.72 (m, 1H), 3.69 - 3.46 (m, 3H), 2.37 (s, 3H), 2.28 (s, 1H), 0.64 - 0.49 (m, 1H), 0.49 - 0.37 (m, 1H), 0.36 - 0.13 (m, 2H).

### Example 4-23: 4-(4-((3R,4S)-3-amino-4-methylpyrrolidin-1-yl)-6-methylquinazolin-2-yl)-1-(cyclopropylimino)-2,3,4,5-tetrahydro-benzo[f][1,4]thiazepine-1-oxide

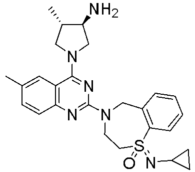

The synthesis method of this compound was the same as Example 4-2. ESI-MS(m/z): 477.24[M+H]⁺; ¹H NMR (600 MHz, CD₃OD) δ 8.01 (s, 1H), 7.81 (d, *J =* 7.5 Hz, 2H), 7.58 (s, 1H), 7.45 (s, 1H), 7.35 (d, *J =* 2.0 Hz, 2H), 5.43 (dd, *J* = 14.9, 6.6 Hz, 1H), 5.01 (s, 1H), 4.25 - 4.10 (m, 2H), 3.95 (s, 1H), 3.59 (d, *J* = 2.2 Hz, 4H), 3.18 - 3.05 (m, 1H), 2.28 (dd, *J* = 6.8, 3.4 Hz, 1H), 2.07 (tt, *J =* 13.1, 6.7 Hz, 1H), 1.31 (d, *J =* 11.7 Hz, 3H), 1.20 (t, *J =* 6.8 Hz, 3H), 0.92 (s, 1H), 0.53 (dt*, J =* 8.8, 4.9 Hz, 1H), 0.47 - 0.42 (m, 1H), 0.30 - 0.20 (m, 2H).

### Example 4-24: Synthesis of 1-(cyclopropylimino)-4-(4-(((3S,4S)-4-fluoropyrrolidin-3-yl)amino)-6-methylquinazolin-2-yl)-2,3,4,5-tetrahydrobenzo[f][1,4]thiazepine-1-oxide

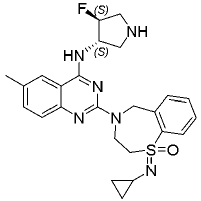

The synthesis method of this compound was the same as Example 4-2. ESI-MS(m/z): 481.20[M+H]⁺; ¹H NMR (600 MHz, Methanol-*d*₄) δ 8.04 - 7.99 (m, 1H), 7.88 - 7.83 (m, 1H), 7.68 (tt, *J =* 1.9, 0.9 Hz, 1H), 7.60 - 7.52 (m, 1H), 7.44 (tt, *J* = 7.9, 2.9 Hz, 1H), 7.38 (dd, *J* = 8.6, 1.9 Hz, 1H), 7.32 (d, *J =* 8.5 Hz, 1H), 5.50 - 5.43 (m, 1H), 5.09 - 5.06 (m, 2H), 4.79 (d*, J =* 16.4 Hz, 2H), 3.95 (s, 1H), 3.61 - 3.48 (m, 2H), 3.23 (dd, *J =* 24.3, 18.3 Hz, 1H), 3.01 (td, *J* = 12.3, 4.4 Hz, 1H), 2.37 (t, *J =* 1.2 Hz, 3H), 2.27 (tt, *J =* 7.0, 3.6 Hz, 1H), 1.35 - 1.27 (m, 2H), 0.56 - 0.50 (m, 1H), 0.43 (dddd, *J* = 9.7, 6.8, 5.7, 1.9 Hz, 1H), 0.25 (dddd, *J =* 17.1, 14.5, 10.8, 5.8 Hz, 2H).

### Example 4-25: Synthesis of 1-(cyclopropylimino)-4-(4-(((3R,4R)-4-fluoropyrrolidin-3-yl)amino)-6-methylquinazolin-2-yl)-2,3,4,5-tetrahydrobenzo[f][1,4]thiazepine-1-oxide

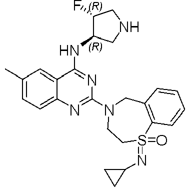

The synthesis method of this compound was the same as Example 4-2. ESI-MS(m/z): 481.18[M+H]⁺; ¹H NMR (600 MHz, Methanol-*d*₄) δ 8.01 (d, *J* = 7.6 Hz, 1H), 7.88 - 7.83 (m, 1H), 7.70 - 7.67 (m, 1H), 7.60 - 7.52 (m, 1H), 7.44 (td, *J* = 7.8, 3.6 Hz, 1H), 7.37 (dd, *J* = 8.6, 1.9 Hz, 1H), 7.32 (d, *J=* 8.5 Hz, 1H), 5.47 (t, *J=* 10.9 Hz, 1H), 5.07 (s, 2H), 4.80 (t, *J =* 14.2 Hz, 2H), 3.95 (s, 1H), 3.61 - 3.48 (m, 2H), 3.26 - 3.07 (m, 1H), 3.00 (td, *J* = 12.7, 4.4 Hz, 1H), 2.37 (t, *J* = 1.1 Hz, 3H), 2.27 (dq, *J* = 6.8, 3.7 Hz, 1H), 1.38 - 1.11 (m, 2H), 0.53 (dddd, *J* = 10.0, 8.1, 5.3, 3.0 Hz, 1H), 0.43 (dtdd, *J* = 8.9, 6.8, 4.7, 1.9 Hz, 1H), 0.31 - 0.14 (m, 2H).

### Example 4-26: Synthesis of 1-(cyclopropylimino)-4-(4-(((3S,4R)-4-fluoropyrrolidin-3-yl)amino)-6-methylquinazolin-2-yl)-2,3,4,5-tetrahydro-benzo[f][1,4]thiazepine-1-oxide

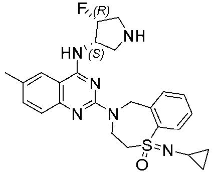

The synthesis method of this compound was the same as Example 4-2. ESI-MS(m/z): 481.21 [M+H]+;¹H NMR (600 MHz, CD₃OD) *δ* 8.02 (d, *J =* 7.7 Hz, 1H), 7.77 (d, *J =* 7.0 Hz, 1H), 7.74 (s, 1H), 7.59 (dt, *J =* 14.7, 7.5 Hz, 1H), 7.45 (t, *J* = 7.6 Hz, 1H), 7.39 (dt, *J* = 8.5, 2.1 Hz, 1H), 7.33 (dd, *J* = 8.5, 3.6 Hz, 1H), 5.43 (d*, J* = 15.1 Hz, 1H), 5.35 - 5.18 (m, 1H), 5.08 (d, *J =* 60.0 Hz, 2H), 4.81 (d, *J =* 13.5 Hz, 1H), 3.97 (s, 1H), 3.61 (dd, *J =* 14.6, 1.5 Hz, 1H), 3.57 - 3.48 (m, 1H), 3.39 (ddd, *J* = 18.9, 15.9, 7.8 Hz, 2H), 3.28 (ddd, *J =* 26.7, 13.9, 4.1 Hz, 1H), 3.07 (dd, *J* = 19.0, 10.2 Hz, 1H), 2.39 (s, 3H), 2.28 (dd*, J =* 6.6, 3.4 Hz, 1H), 0.62 - 0.47 (m, 1H), 0.48 - 0.37 (m, 1H), 0.34 - 0.15 (m, 2H).

### Example 4-27: Synthesis of 1-(cyclopropylimino)-4-(4-((cis-4-fluoropyrrolidin-3-yl)amino)-6-methylquinazolin-2-yl)-2,3,4,5-tetrahydro-benzo[f][1,4]thiazepine-1-oxide

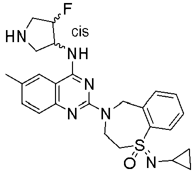

The synthesis method of this compound was the same as Example 4-2. ESI-MS(m/z): 481.21[M+H]⁺ ;¹H NMR (600 MHz, CD₃OD) δ 8.03 (d, *J* = 7.7 Hz, 1H), 7.78 (d, *J =* 6.8 Hz, 1H), 7.74 (s, 1H), 7.64 - 7.56 (m, 1H), 7.46 (s, 1H), 7.40 (d, *J =* 8.5 Hz, 1H), 7.36 - 7.31 (m, 1H), 5.46 (t, *J* = 23.6 Hz, 1H), 3.98 (s, 1H), 3.61 (s, 1H), 3.50 (d, *J =* 33.6 Hz, 2H), 3.38 (dd, *J =* 21.9, 11.0 Hz, 2H), 3.10 (dd, *J =* 18.9, 10.0 Hz, 1H), 2.29 (d, *J =* 2.7 Hz, 1H), 1.38 - 1.26 (m, 6H), 0.94 - 0.84 (m, 1H), 0.53 (s, 1H), 0.45 (dd, *J* = 9.8, 4.9 Hz, 1H), 0.26 (d, *J =* 24.3 Hz, 2H).

### Example 4-28: Synthesis of 4-(4-(((R)-3-aminopyrrolidin-1-yl)-6-(difluoromethyl)quinazolin-2-yl)-1-(cyclopropylimino)-2,3,4,5-tetrahydro-benzo[f][1,4]thiazepine-1-oxide

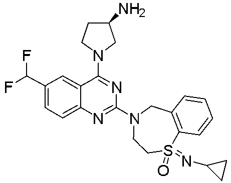

The synthesis method of this compound was the same as Example 4-2. ESI-MS(m/z): 499.2[M+H]⁺. ¹H NMR (600 MHz, Methanol-*d*₄) δ 8.09 (d, *J =* 83.1 Hz, 2H), 7.82 (s, 1H), 7.54 (d, *J =* 87.1 Hz, 4H), 6.78 (t, *J =* 55.4 Hz, 1H), 5.45 (s, 1H), 5.05 (s, 1H), 4.02 (d, *J* = 76.9 Hz, 4H), 3.64 (d, *J* = 40.2 Hz, 4H), 2.27 (s, 2H), 1.91 (s, 1H), 1.30 (s, 1H), 0.49 (d, *J* = 53.9 Hz, 2H), 0.26 (s, 2H).

### Example 4-29: Synthesis of 4-(4-(((R)-3-aminopyrrolidin-1-yl)-6-(difluoromethyl)quinazolin-2-yl)-1-((2,2-difluoroethyl)imino)-2,3,4,5-tetrahydrobenzo[f][1,4]thiazepine-1-oxide

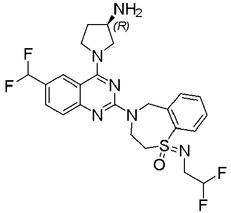

The synthesis method of this compound was the same as Example 4-2. ESI-MS(m/z): 523.13[M+H]⁺; ¹H NMR (600 MHz, Methanol-*d*₄) δ 8.15 (s, 1H), 7.97 (d*, J =* 7.8 Hz, 1H), 7.82 (d*, J =* 7.5 Hz, 1H), 7.64 - 7.57 (m, 2H), 7.50 - 7.42 (m, 2H), 6.77 (t, *J* = 56.4 Hz, 1H), 5.84 (tt, *J* = 56.5, 4.1 Hz, 1H), 5.43 (d, *J* = 15.1 Hz, 1H), 4.04 (q, *J* = 39.9, 38.7 Hz, 4H), 3.86 - 3.45 (m, 4H), 3.24 - 3.14 (m, 1H), 2.93 (d, *J =* 15.0 Hz, 1H), 2.27 (s, 1H), 1.98 (d, *J =* 45.1 Hz, 1H), 1.42 - 1.29 (m, 2H).

### Example 4-30: Synthesis of 4-(4-((3-aminobicyclo[1.1.1]pentan-1-yl)amino)-6-methylquinazolin-2-yl)-1-(cyclopropylimino)-2,3,4,5-tetrahydrobenzo[f][1,4]thiazepine-1-oxide

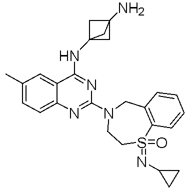

The synthesis method of this compound was the same as Example 4-2. ESI-MS(m/z): 475.24[M+H]⁺; ¹H NMR (600 MHz, Methanol-*d*₄) δ 7.96 (d, *J =* 7.8 Hz, 1H), 7.80 (d, *J* = 7.5 Hz, 1H), 7.61 (s, 1H), 7.50 (t, *J =* 7.5 Hz, 1H), 7.36 (t, *J* = 7.7 Hz, 1H), 7.30 (t, *J =* 6.8 Hz, 2H), 5.40 (d, *J =* 14.9 Hz, 1H), 4.99 (s, 1H), 4.77 (d, *J =* 15.0 Hz, 1H), 3.98 (s, 1H), 3.65 - 3.59 (m, 1H), 3.44 (t, *J =* 13.0 Hz, 1H), 3.31 (d, *J =* 2.9 Hz, 1H), 2.31 (s, 3H), 2.23 (q, *J* = 9.5 Hz, 6H), 0.52 (dt, *J* = 10.1, 5.3 Hz, 1H), 0.42 (ddd, *J* = 11.6, 7.2, 4.7 Hz, 1H), 0.26 (tt*, J* = 11.1, 5.7 Hz, 2H).

### Example 4-31: Synthesis of 1-(cyclopropylimino)-4-(4-(((3R,4R)-4-methoxypyrrolidin-3-yl)amino)-6-methylquinazolin-2-yl)-2,3,4,5-tetrahydro-benzo[f][1,4]thiazepine-1-oxide

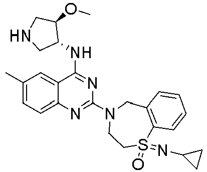

The synthesis method of this compound was the same as Example 4-2. ESI-MS(m/z): 493.23 [M+H]⁺, ¹H NMR (600 MHz, Methanol-*d*₄) δ 8.02 (s, 1H), 7.86 (dd, *J* = 28.0, 6.3 Hz, 1H), 7.72 (d, *J* = 4.5 Hz, 1H), 7.59 (s, 1H), 7.48 - 7.39 (m, 2H), 7.37 (s, 1H), 5.46 (t, *J =* 13.4 Hz, 1H), 5.06 (s, 1H), 4.24 (s, 1H), 3.99 (s, 1H), 3.79 (s, 1H), 3.64 - 3.40 (m, 10H), 2.40 (s, 3H), 2.29 (s, 1H), 0.50 (d, *J =* 54.2 Hz, 2H), 0.27 (d, *J =* 22.6 Hz, 2H).

### Example 4-32: Synthesis of 4-(4-(3-amino-3-(hydroxymethyl)azetidin-1-yl)-6-methylquinazolin-2-yl)-1-(cyclopropylimino)-2,3,4,5-tetrahydro-benzo[f][1,4]thiazepine-1-oxide

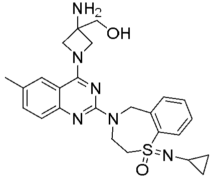

The synthesis method of this compound was the same as Example 4-2. ESI-MS(m/z): 479.22 [M+H]⁺.

### Example 4-33: 4-((2-(1-(cyclopropylimino)-1-oxido-1,2,3,5-tetrahydro-4H-benzo[f][1,4]thiazolin-4-yl)-6-methylquinazolin-4-yl)amino)pyrrolidine-3-carbonitrile

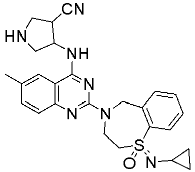

The synthesis method of this compound was the same as Example 4-2. ESI-MS(m/z): 488.22[M+H]⁺; ¹H NMR (600 MHz, CD₃OD) δ 8.05 (d, *J =* 7.6 Hz, 1H), 7.86 - 7.79 (m, 2H), 7.62 (dd, *J =* 14.2, 7.2 Hz, 1H), 7.48 (t*, J =* 7.5 Hz, 1H), 7.44 - 7.42 (m, 1H), 7.37 (dd, *J =* 8.5, 5.9 Hz, 1H), 5.51 (s, 1H), 5.47 (d, *J =* 14.8 Hz, 1H), 5.09 (s, 2H), 4.01 (s, 1H), 3.69 - 3.63 (m, 1H), 3.61 (d, *J* = 9.7 Hz, 1H), 3.55 (s, 2H), 3.46 (d, *J* = 7.2 Hz, 1H), 3.14 (s, 1H), 2.42 (s, 3H), 2.30 (dd, *J =* 6.6, 3.1 Hz, 1H), 2.07 - 2.02 (m, 1H), 0.55 (d, *J =* 4.5 Hz, 1H), 0.47 - 0.43 (m, 1H), 0.32 - 0.19 (m, 2H).

### Example 4-34: Synthesis of 4-(4-((3S,SS)-3-amino-5-methylpiperidin-1-yl)-6-methylquinazolin-2-yl)-1-(cyclopropylimino)-2,3,4,5-tetrahydro-benzo[f][1,4]thiazepine-1-oxide

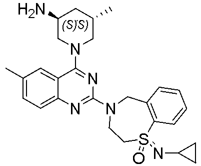

The synthesis method of this compound was the same as Example 4-2. ESI-MS(m/z): 491.25 [M+H]+;¹H NMR (600 MHz, CD₃OD) *δ* 8.03 (d, *J* = 7.7 Hz, 1H), 7.83 (d, *J =* 7.4 Hz, 1H), 7.62 (t, *J =* 10.9 Hz, 2H), 7.46 (t, *J* = 7.4 Hz, 1H), 7.41 (s, 2H), 5.44 (d, *J =* 14.9 Hz, 1H), 5.03 (s, 1H), 3.99 (s, 1H), 3.83 (dd, *J =* 28.9, 12.5 Hz, 2H), 3.62 (d, *J* = 14.6 Hz, 2H), 3.53 (dd, *J =* 22.5, 11.6 Hz, 2H), 3.34 (s, 1H), 3.09 - 2.92 (m, 1H), 2.41 (s, 3H), 2.33 - 2.20 (m, 2H), 1.84 (t, *J =* 13.1 Hz, 1H), 1.64 (t, *J =* 9.6 Hz, 1H), 1.03 (td, *J =* 18.7, 6.5 Hz, 3H), 0.60 - 0.50 (m, 1H), 0.50 - 0.40 (m, 1H), 0.34 - 0.17 (m, 2H).

### Example 4-37: Synthesis of 4-(4-((3S,4S)-3-amino-4-fluoropyrrolidin-1-yl)-6-methylquinazolin-2-yl)-1-(cyclopropylimino)-2,3,4,5-tetrahydro-benzo[f][1,4]thiazepine-1-oxide

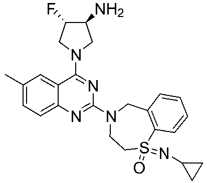

The synthesis method of this compound was the same as Example 4-2. ESI-MS(m/z): 481.2[M+H]⁺. ¹H NMR (600 MHz, Methanol-*d*₄) δ 8.01 (s, 1H), 7.80 (s, 2H), 7.58 (s, 1H), 7.44 (s, 1H), 7.37 (s, 2H), 5.44 (s, 1H), 5.10 (s, 1H), 5.01 (s, 1H), 4.45-4.22 (m, 3H), 4.09-3.96 (m, 2H), 3.86-3.74 (m, 2H), 3.59 (s, 2H), 2.38 (s, 3H), 2.28 (s, 1H), 0.54 (s, 1H), 0.45 (s, 1H), 0.25 (s, 2H).

### Example 4-38: 4-(4-(trans-4-amino-3-fluoropiperidin-1-yl)-6-methylquinazolin-2-yl)-1-(cyclopropylimino)-2,3,4,5-tetrahydro-benzo[f][1,4]thiazepine-1-oxide

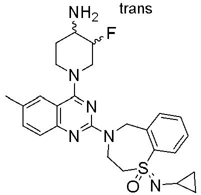

The synthesis method of this compound was the same as Example 4-2. ESI-MS(m/z): 495.23[M+H]⁺ ;¹H NMR (600 MHz, CD₃OD) δ 8.04 (d, *J* = 7.7 Hz, 1H), 7.82 (d, *J* = 6.9 Hz, 1H), 7.61 (s, 1H), 7.51 (s, 1H), 7.48 (d, *J* = 7.5 Hz, 1H), 7.42 (s, 2H), 5.51 (s, 1H), 5.42 (d, *J* = 13.4 Hz, 1H), 5.05 (s, 1H), 4.63 - 4.47 (m, 1H), 4.42 (s, 2H), 4.14 (dd, *J* = 25.2, 12.9 Hz, 1H), 4.00 (s, 1H), 3.62 (s, 1H), 3.54 (d, *J* = 11.5 Hz, 1H), 3.23 (s, 1H), 3.16 - 3.03 (m, 2H), 2.40 (s, 3H), 2.29 (s, 1H), 2.04 (d, *J =* 12.1 Hz, 1H), 1.68 - 1.57 (m, 2H), 0.59 - 0.51 (m, 1H), 0.45 (s, 1H), 0.24 (s, 2H).

### Example 4-39: Synthesis of 4-(4-(((R)-3-aminopyrrolidin-1-yl)-6-methylquinazolin-2-yl)-1-((oxetan-2-ylmethyl)imino)-2,3,4,5-tetrahydro-benzo[f][1,4]thiazepine-1-oxide

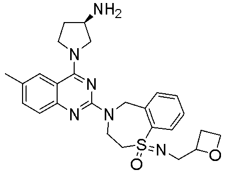

The synthesis method of this compound was the same as Example 4-2. ESI-MS(m/z): 493.2[M+H]⁺.

### Example 4-40: Synthesis of 4-(4-(((R)-3-aminopyrrolidin-1-yl)-6-methylquinazolin-2-yl)-1-((2,2,2-trifluoroethyl)imino)-2,3,4,5-tetrahydrobenzo[f][1,4]thiazepine-1-oxide

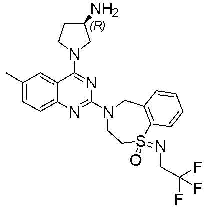

The synthesis method of this compound was the same as Example 4-2. ESI-MS(m/z): 505.20[M+H]⁺; ¹H NMR (600 MHz, Methanol-*d*₄) δ 7.94 (d, *J* = 7.8 Hz, 1H), 7.79 (d, *J* = 7.5 Hz, 1H), 7.76 (s, 1H), 7.57 (td, *J* = 7.5, 3.1 Hz, 1H), 7.40 (t, *J* = 7.6 Hz, 1H), 7.32 (s, 2H), 5.40 (d, *J =* 15.1 Hz, 1H), 5.01 (s, 1H), 4.78 (d, *J =* 15.2 Hz, 1H), 4.10 - 4.00 (m, 2H), 3.94 (s, 2H), 3.72 - 3.54 (m, 4H), 3.43 (dq, *J =* 14.0, 9.4 Hz, 1H), 3.18 (s, 1H), 2.33 (s, 3H), 2.21 (p, *J* = 7.3, 6.8 Hz, 1H), 1.89 (dq, *J* = 13.3, 6.8 Hz, 1H).

### Example 4-41: Synthesis of 4-(4-((3S,SR)-3-amino-5-fluoropiperidin-1-yl)-6-methylquinazolin-2-yl)-1-(cyclopropylimino)-2,3,4,5-tetrahydro-benzo[f][1,4]thiazepine-1-oxide

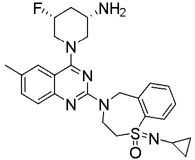

The synthesis method of this compound was the same as Example 4-2. ESI-MS(m/z): 495.23 [M+H]⁺, ¹H NMR (600 MHz, Methanol-*d*₄) δ 7.98 (t, *J* = 6.9 Hz, 1H), 7.84 - 7.79 (m, 1H), 7.58 (d, *J =* 9.4 Hz, 2H), 7.44 - 7.36 (m, 3H), 5.38 (dt, *J* = 20.7, 10.4 Hz, 1H), 5.08 - 4.92 (m, 1H), 4.83 - 4.78 (m, 1H), 4.14 - 3.87 (m, 3H), 3.60 (d, *J* = 14.6 Hz, 1H), 3.54 - 3.36 (m, 2H), 3.26 - 3.16 (m, 1H), 3.16 - 3.01 (m, 2H), 2.40 (d, *J =* 14.5 Hz, 1H), 2.38 (s, 3H), 2.27 (s, 1H), 1.81 - 1.68 (m, 1H), 0.55 - 0.40 (m, 2H), 0.30 - 0.19 (m, 2H).

### Example 4-42: Synthesis of 1-(cyclopropylimino)-4-(4-((((2S,4R)-4-fluoropyrrolidin-2-yl)methyl)amino)-6-methylquinazolin-2-yl)-2,3,4,5-tetrahydro-benzo[f][1,4]thiazepine-1-oxide

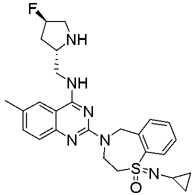

The synthesis method of this compound was the same as Example 4-2. ESI-MS(m/z): 495.23 [M+H]⁺, ¹H NMR (600 MHz, Methanol-*d*₄) δ 8.07 - 8.01 (m, 1H), 7.82 (t, *J* = 7.5 Hz, 1H), 7.68 (s, 1H), 7.61 (t, *J* = 7.5 Hz, 1H), 7.50 - 7.43 (m, 2H), 7.38 (dd, *J* = 8.5, 1.8 Hz, 1H), 5.47 (ddd, *J* = 26.7, 12.3, 6.7 Hz, 2H), 4.95 (d, *J* = 34.6 Hz, 1H), 4.27 (ddd, *J =* 30.3, 18.4, 6.6 Hz, 1H), 4.15 - 3.90 (m, 3H), 3.66 - 3.50 (m, 4H), 3.23 (q, *J =* 7.3 Hz, 1H), 2.63 - 2.51 (m, 1H), 2.41 (s, 3H), 2.32 - 2.25 (m, 1H), 2.11 (ddd, *J =* 37.4, 26.1, 11.3 Hz, 1H), 0.47 (ddd, *J =* 24.9, 19.1, 14.2 Hz, 2H), 0.27 (ddd, *J =* 24.9, 14.3, 5.3 Hz, 2H).

### Example 4-43: Synthesis of 4-(4-(((R)-3-aminopyrrolidin-1-yl)-6-methylquinazolin-2-yl)-1-((tetrahydrofuran-3-yl)imino)-2,3,4,5-tetrahydrobenzo[f][1,4]thiazepine-1-oxide

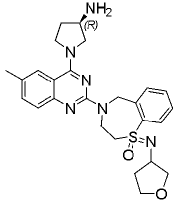

The synthesis method of this compound was the same as Example 4-2. ESI-MS(m/z): 493.17[M+H]⁺; ¹H NMR (600 MHz, Methanol-*d*₄) δ 8.13 - 7.98 (m, 1H), 7.97 - 7.84 (m, 2H), 7.72 - 7.60 (m, 2H), 7.60 - 7.44 (m, 2H), 5.51 (t, *J* = 16.5 Hz, 1H), 5.11 - 4.90 (m, 2H), 4.55 - 4.31 (m, 2H), 4.29 - 4.03 (m, 4H), 3.87 (dt, *J* = 15.5, 7.9 Hz, 1H), 3.78 - 3.60 (m, 4H), 3.56 (dd, *J =* 8.5, 4.9 Hz, 1H), 3.48 (s, 1H), 2.56 (dd, *J =* 13.6, 6.7 Hz, 1H), 2.43 (s, 3H), 2.34 (dt, *J* = 12.2, 5.5 Hz, 1H), 2.13 - 1.89 (m, 1H), 1.88 - 1.65 (m, 1H).

### Example 4-44: Synthesis of 1-(cyclopropylimino)-4-(4-(((3S,5S)-5-fluoropiperidin-3-yl)amino)-6-methylquinazolin-2-yl)-2,3,4,5-tetrahydrobenzo[f][1,4]thiazepine-1-oxide

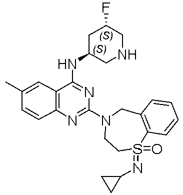

The synthesis method of this compound was the same as Example 4-2. ESI-MS(m/z): 495.19[M+H]⁺; ¹H NMR (600 MHz, Methanol-*d*₄) δ 8.00 (t*, J* = 7.0 Hz, 1H), 7.88 (dd, *J =* 49.8, 7.5 Hz, 1H), 7.67 - 7.64 (m, 1H), 7.58 (dt, *J* = 15.4, 7.6 Hz, 1H), 7.43 (t, *J =* 7.7 Hz, 1H), 7.35 (dt, *J =* 8.6, 2.0 Hz, 1H), 7.29 (dd, *J =* 8.5, 5.9 Hz, 1H), 5.56 - 5.35 (m, 1H), 5.11 - 4.93 (m, 2H), 4.76 (q, *J =* 19.6, 18.0 Hz, 2H), 4.03 - 3.80 (m, 1H), 3.62 - 3.46 (m, 2H), 3.29 - 3.22 (m, 2H), 2.86 (ddd, *J* = 37.6, 14.1, 3.2 Hz, 1H), 2.66 (t, *J* = 11.7 Hz, 1H), 2.35 (s, 3H), 2.26 (dp, *J=* 10.9, 4.4 Hz, 1H), 1.95 - 1.67 (m, 1H), 1.39 - 1.29 (m, 1H), 0.51 (ddd, *J =* 10.3, 6.4, 3.2 Hz, 1H), 0.46 - 0.39 (m, 1H), 0.30 - 0.20 (m, 2H).

### Example 4-45: Synthesis of 1-(cyclopropylimino)-4-(4-(((5,5-difluoropiperidin-3-yl)amino)-6-methylquinazolin-2-yl)-2,3,4,5-tetrahydrobenzo[f][1,4]thiazepine-1-oxide

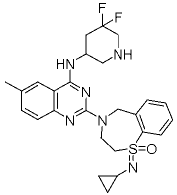

The synthesis method of this compound was the same as Example 4-2. ESI-MS(m/z): 513.16[M+H]⁺; ¹H NMR (600 MHz, Methanol-*d*₄) δ 8.04 (dd, *J =* 8.0, 3.5 Hz, 1H), 7.86 (dd, *J =* 45.2, 7.5 Hz, 1H), 7.67 (d, *J =* 2.6 Hz, 1H), 7.65 - 7.56 (m, 1H), 7.51 - 7.44 (m, 1H), 7.40 (dt, *J* = 8.6, 1.8 Hz, 1H), 7.33 (dd, *J* = 8.6, 4.1 Hz, 1H), 5.59 - 5.39 (m, 1H), 5.03 (d, *J =* 13.4 Hz, 1H), 4.81 (d, *J =* 15.6 Hz, 1H), 4.07 - 3.90 (m, 1H), 3.68 - 3.45 (m, 2H), 3.22 (d, *J* = 13.8 Hz, 2H), 3.02 - 2.81 (m, 1H), 2.71 - 2.55 (m, 1H), 2.39 (s, 3H), 2.28 (tt, *J* = 7.2*,* 3.8 Hz, 1H), 2.05 (d*, J =* 5.4 Hz, 1H), 1.63 - 1.60 (m, 1H), 1.42 - 1.39 (m, 1H), 0.54 (h, *J* = 4.1*,* 3.7 Hz, 1H), 0.45 (ddd, *J* = 11.5, 9.1, 5.7 Hz, 1H), 0.32 - 0.22 (m, 2H).

### Example 4-46: Synthesis of 1-(cyclopropylimino)-4-(4-((3-fluoropiperidin-4-yl)amino)-6-methylquinazolin-2-yl)-2,3,4,5-tetrahydro-benzo[f][1,4]thiazepine-1-oxide

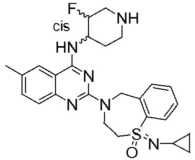

The synthesis method of this compound was the same as Example 4-2. ESI-MS(m/z): 495.23 [M+H]+;¹H NMR (600 MHz, CD₃OD) *δ* 8.03 (d, *J =* 7.7 Hz, 1H), 7.83 - 7.70 (m, 2H), 7.57 (dd, *J* = 14.4, 7.1 Hz, 1H), 7.46 (t, *J* = 7.5 Hz, 1H), 7.38 (d, *J =* 8.4 Hz, 1H), 7.32 (d*, J =* 8.4 Hz, 1H), 5.43 (dd, *J =* 14.8, 6.8 Hz, 1H), 4.96 (d, *J =* 49.1 Hz, 2H), 4.81 (d, *J =* 14.1 Hz, 1H), 4.63 (d, *J* = 30.3 Hz, 1H), 3.96 (s, 1H), 3.63 (dd, *J =* 12.0, 2.5 Hz, 1H), 3.58 - 3.46 (m, 1H), 3.43 - 3.34 (m, 1H), 3.20 (dd, *J =* 22.9, 13.4 Hz, 1H), 3.12 - 2.95 (m, 1H), 2.88 (dd, *J =* 24.7, 12.5 Hz, 1H), 2.39 (s, 3H), 2.29 (s, 1H), 2.11 - 1.99 (m, 1H), 1.82 (dd, *J* = 36.2, 11.7 Hz, 1H), 0.53 (s, 1H), 0.44 (dt, *J* = 11.5, 6.6 Hz, 1H), 0.32 - 0.16 (m, 2H).

### Example 4-47: Synthesis of 4-((4-(4-(((R)-3-aminopyrrolidin-1-yl)-6-methylquinazolin-2-yl)-1-oxido-2,3,4,5-tetrahydrobenzo[f][1,4]thiazepin-1-ylidene)amino)butanenitrile

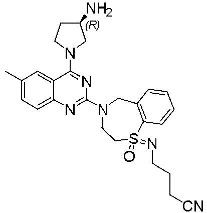

The synthesis method of this compound was the same as Example 4-2. ESI-MS(m/z): 490.18[M+H]⁺; ¹H NMR (600 MHz, Methanol-*d*₄) δ 7.96 (dd, *J* = 7.8, 1.3 Hz, 1H), 7.79 (d, *J =* 6.4 Hz, 2H), 7.56 (tdd, *J =* 7.6, 3.1, 1.4 Hz, 1H), 7.42 (t, *J* = 7.7 Hz, 1H), 7.34 (d*, J =* 1.9 Hz, 2H), 5.41 (d, *J* = 15.1 Hz, 1H), 4.99 (s, 1H), 4.82 - 4.77 (m, 1H), 4.15 - 3.86 (m, 4H), 3.69 (dddd, *J =* 20.2, 15.1, 11.4, 4.9 Hz, 2H), 3.64 - 3.49 (m, 2H), 2.97 (dt, *J =* 12.6, 6.3 Hz, 1H), 2.80 (dt, *J =* 13.0, 6.3 Hz, 1H), 2.56 (td, *J =* 7.0, 3.3 Hz, 2H), 2.35 (s, 3H), 2.21 (ddd, *J =* 25.5, 13.9, 6.9 Hz, 1H), 1.91 (dt, *J* = 13.2, 6.5 Hz, 1H), 1.79 (p*, J* = 6.7 Hz, 2H).

### Example 4-48: Synthesis of 4-amino-1-(2-(1-(cyclopropylimino)-1-oxido-1,2,3,5-tetrahydro-4H-benzo [f][1,4]thiazepin-4-yl)-6-methylquinazolin-4-yl)pyrrolidine-3-carboxylic acid

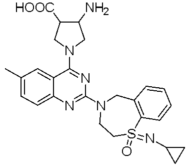

The synthesis method of this compound was the same as Example 4-2. ESI-MS(m/z): 507.2[M+H]⁺. ¹H NMR (600 MHz, DMSO-d6) δ 7.91 (s, 1H), 7.76 (s, 1H), 7.72-7.78 (m, 2H), 7.56 (s, 1H), 7.44 (s, 1H), 7.32 - 7.23 (m, 1H), 5.27 (s, 1H), 4.89 (s, 1H), 4.70 (s, 1H), 4.04-3.80 (m, 8H), 2.81-2.92(m,1H), 2.31 (s, 3H), 2.16 (s, 1H), 0.38 (d*, J =* 19.4 Hz, 2H), 0.14 (d, *J =* 42.9 Hz, 2H).

### Example 4-49: Synthesis of 4-(4-(((R)-3-aminopyrrolidin-1-yl)-6-methylquinazolin-2-yl)-1-((oxetan-3-ylmethyl)imino)-2,3,4,5-tetrahydro-benzo[f][1,4]thiazepine-1-oxide

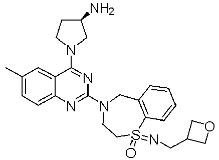

The synthesis method of this compound was the same as Example 4-2. ESI-MS(m/z): 493.2[M+H]⁺.

### Example 4-50: Synthesis of 4-(4-(2,5-diazabicyclo[2.2.2]octan-2-yl)-6-methylquinazolin-2-yl)-1-(cyclopropylimino)-2,3,4,5-tetrahydro-benzo[f][1,4]thiazepine-1-oxide

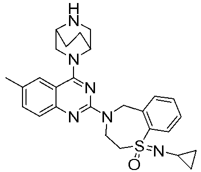

The synthesis method of this compound was the same as Example 4-2. ESI-MS(m/z): 489.24 [M+H]⁺, ¹H NMR (600 MHz, Methanol-*d*₄) δ 8.04 - 8.00 (m, 1H), 7.79 (d, *J =* 7.2 Hz, 1H), 7.65 (s, 1H), 7.58 (t, *J =* 7.4 Hz, 1H), 7.45 (dd*, J =* 12.1, 7.3 Hz, 1H), 7.40 (d*, J =* 8.1 Hz, 2H), 5.41 (d, *J* = 14.8 Hz, 1H), 5.00 (s, 1H), 4.81 (d*, J =* 15.0 Hz, 1H), 4.74 (s, 1H), 4.21 (dd, *J* = 18.7, 11.6 Hz, 1H), 4.05 (dd, *J =* 16.7, 11.8 Hz, 1H), 3.97 (s, 1H), 3.61 (d, *J* = 14.8 Hz, 1H), 3.56 - 3.46 (m, 2H), 3.40 (s, 1H), 3.29 (d, *J* = 11.8 Hz, 1H), 2.38 (d, *J* = 9.2 Hz, 3H), 2.28 (s, 2H), 2.11 - 2.00 (m, 2H), 1.91 (dd, *J =* 22.4, 12.2 Hz, 1H), 0.56 - 0.41 (m, 2H), 0.29 - 0.20 (m, 2H).

### Example 4-51: 4-(4-(((R)-3-aminopyrrolidin-1-yl)-6-methylquinazolin-2-yl)-1-((3-hydroxypropyl)imino)-2,3,4,5-tetrahydro-benzo[f][1,4]thiazepine-1-oxide

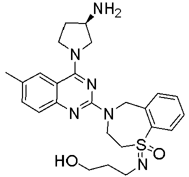

The synthesis method of this compound was the same as Example 4-2. ESI-MS(m/z): 481.23 [M+H]⁺. ¹H NMR (600 MHz, Methanol-*d*₄) δ 7.98 (d, *J* = 7.8 Hz, 1H), 7.81 (d, *J =* 9.2 Hz, 2H), 7.60 - 7.54 (m, 1H), 7.47 - 7.41 (m, 1H), 7.36 (d, *J =* 1.9 Hz, 2H), 5.44 (d, *J =* 15.0 Hz, 1H), 5.03 (s, 1H), 4.83 (d, *J =* 15.2 Hz, 1H), 4.23 - 4.05 (m, 2H), 4.04 - 3.89 (m, 2H), 3.80 - 3.40 (m, 6H), 2.99 (dd, *J* = 12.7, 6.4 Hz, 1H), 2.81 (dt, *J* = 12.3, 6.6 Hz, 1H), 2.38 (s, 3H), 2.26 (dq, *J* = 13.2, 6.8 Hz, 1H), 1.94 (dt, *J* = 13.2, 6.9 Hz, 1H), 1.81 - 1.67 (m, 2H).

### Example 4-52: Synthesis of 1-(cyclopropylimino)-4-(6-methyl-4-((pyrrolidin-2-ylmethyl)amino)quinazolin-2-yl)-2,3,4,5-tetrahydro-benzo[f][1,4]thiazepine-1-oxide

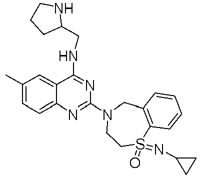

The synthesis method of this compound was the same as Example 4-2. ESI-MS(m/z): 477.2[M+H]⁺. ¹H NMR (600 MHz, Methanol-*d*₄) δ 8.02 (d, *J* = 7.7 Hz, 1H), 7.81 (d, *J* = 7.4 Hz, 1H), 7.63 (s, 1H), 7.58 (t, *J* = 7.1 Hz, 1H), 7.45 (t, *J* = 7.5 Hz, 1H), 7.35 (d, *J* = 8.4 Hz, 1H), 7.30 (d, *J =* 8.4 Hz, 1H), 5.44 (dd, *J =* 14.0, 10.0 Hz, 1H), 5.02 (s, 1H), 4.80 (d, *J =* 14.8 Hz, 1H), 3.96 (s, 1H), 3.70 (s, 1H), 3.61 (d, *J =* 14.4 Hz, 1H), 3.58 - 3.48 (m, 2H), 3.09-3.03 (m, 1H), 3.01 - 2.91 (m, 1H), 2.37 (s, 3H), 2.29 (s, 1H), 2.03 (s, 1H), 1.95 - 1.81 (m, 2H), 1.64 (s, 1H), 0.90 (dd, *J* = 17.2, 9.8 Hz, 1H), 0.54 (d, *J* = 4.4 Hz, 1H), 0.48 - 0.41 (m, 1H), 0.31 - 0.22 (m, 2H).

### Example 4-53: 1-(cyclopropylimino)-4-(4-(hexahydropyrrolo[3,4-b]pyrrol-5(1H)-yl)-6-methylquinazolin-2-yl)-2,3,4,5-tetrahydro-benzo[f][1,4]thiazepine-1-oxide

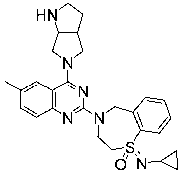

The synthesis method of this compound was the same as Example 4-2. ESI-MS(m/z): 489.24[M+H]⁺ ;¹H NMR (600 MHz, CD₃OD) δ 8.04 (d, *J =* 7.8 Hz, 1H), 7.82 (d, *J =* 7.7 Hz, 2H), 7.62 - 7.57 (m, 1H), 7.47 (t, *J =* 7.6 Hz, 1H), 7.41 - 7.36 (m, 2H), 5.51 (s, 2H), 5.44 (d, *J =* 14.6 Hz, 1H), 4.16 - 4.10 (m, 3H), 4.07 (s, 1H), 3.95 - 3.89 (m, 1H), 3.61 (d, *J =* 15.0 Hz, 1H), 3.55 (d, *J =* 10.9 Hz, 1H), 3.24 - 3.17 (m, 2H), 3.11 (s, 1H), 2.40 (s, 3H), 2.29 (s, 1H), 2.21 (dd, *J =* 14.5, 6.8 Hz, 1H), 1.99 (d, *J =* 3.8 Hz, 1H), 1.94 (s, 2H), 0.57 - 0.52 (m, 1H), 0.48 - 0.42 (m, 1H), 0.31 - 0.21 (m, 2H).

### Example 4-54: Synthesis of 4-(4-(((3-aminoxetan-3-yl)methyl)amino)-5-methoxy-6-methylquinazolin-2-yl)-1-(cyclopropylimino)-2,3,4,5-tetrahydro-benzo[f][1,4]thiazepine-1-oxide

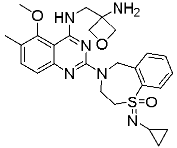

The synthesis method of this compound was the same as Example 4-2. ESI-MS(m/z): 509.23 [M+H]+;¹H NMR (600 MHz, CD₃OD) *δ* 8.04 (d, *J =* 7.8 Hz, 1H), 7.89 (d, J= 7.5 Hz, 1H), 7.60 (t, *J* = 7.4 Hz, 1H), 7.47 (t,*J* = 7.5 Hz, 1H), 7.29 (d, *J* = 8.0 Hz, 1H), 6.52 (d, *J* = 8.1 Hz, 1H), 5.47 (d, *J* = 14.9 Hz, 1H), 5.16 (s, 1H), 4.82 (d, *J=* 15.1 Hz, 1H), 4.75 - 4.46 (m, 4H), 4.06 (d, *J =* 15.3 Hz, 1H), 3.97 (d, *J* = 13.3 Hz, 2H), 3.81 (s, 1H), 3.63 (d, *J* = 15.2 Hz, 1H), 3.54 (s, 1H), 2.43 (s, 3H), 2.29 (s, 1H), 2.05 (dd, *J =* 12.2, 6.4 Hz, 1H), 1.62 (s, 1H), 0.61 - 0.51 (m, 1H), 0.45 (dt, *J* = 11.6, 6.7 Hz, 1H), 0.27 (ddd, *J* = 13.9, 10.8, 5.9 Hz, 2H).

### Example 4-55: 4-(4-(3-amino-4-(trifluoromethyl)pyrrolidin-1-yl)-6-methylquinazolin-2-yl)-1-(cyclopropylimino)-2,3,4,5-tetrahydro-benzo[f][1,4]thiazepine-l-oxide

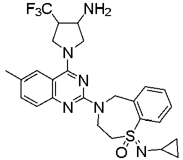

The synthesis method of this compound was the same as Example 4-2. ESI-MS(m/z): 531.21 [M+H]⁺ ;¹H NMR (600 MHz, CD₃OD) δ 8.05 (d, *J* = 5.7 Hz, 1H), 7.80 (d, *J =* 7.6 Hz, 1H), 7.61 - 7.57 (m, 1H), 7.50 - 7.46 (m, 2H), 7.42 (t, *J* = 7.5 Hz, 2H), 4.32 (dd, *J =* 12.7, 6.0 Hz, 3H), 3.88 (dd, *J* = 21.7, 8.4 Hz, 3H), 3.66 - 3.57 (m, 3H), 3.09 (s, 2H), 2.41 (s, 3H), 2.32 - 2.27 (m, 2H), 2.05 (dd, *J =* 12.7, 6.5 Hz, 3H), 1.62 (s, 3H), 0.57 - 0.52 (m, 1H), 0.48 - 0.42 (m, 1H), 0.31 - 0.21 (m, 2H).

### Example 4-56: Synthesis of 4-(4-(((R)-3-aminopyrrolidin-1-yl)-6-methylquinazolin-2-yl)-1-((3-hydroxy-3-methylbutyl)imino)-2,3,4,5-tetrahydrobenzo[f][1,4]thiazepine-1-oxide

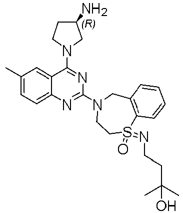

The synthesis method of this compound was the same as Example 4-2. ESI-MS(m/z): 509.27[M+H]⁺; ¹H NMR (600 MHz, Methanol-*d*₄) δ 7.91 (dd, *J* = 7.8, 2.7 Hz, 1H), 7.77 (d, *J =* 7.5 Hz, 1H), 7.72 (s, 1H), 7.53 (td, *J =* 7.4, 4.3 Hz, 1H), 7.36 (dt, *J* = 9.6, 4.7 Hz, 1H), 7.33 - 7.26 (m, 2H), 5.38 (d, *J* = 15.0 Hz, 1H), 4.98 (s, 1H), 4.78 (d*, J =* 15.5 Hz, 1H), 4.05 - 3.96 (m, 2H), 3.95 - 3.76 (m, 2H), 3.56 (dddd, *J =* 34.6, 25.7, 13.0, 4.6 Hz, 4H), 3.03 (ddd, *J* = 12.2, 9.4, 6.1 Hz, 1H), 2.85 - 2.69 (m, 1H), 2.30 (s, 3H), 2.16 (dt, *J =* 13.0, 6.4 Hz, 1H), 1.83 (dt, *J* = 13.3, 6.8 Hz, 1H), 1.74 (ddd, *J =* 13.6, 9.6, 6.1 Hz, 1H), 1.65 (ddd, *J =* 14.0, 9.6, 5.6 Hz, 1H), 1.11 (d*, J =* 12.7 Hz, 6H).

### Example 4-57: Synthesis of methyl 3-(aminomethyl)-1-(2-(1-(cyclopropylimino)-1-oxido-1,2,3,5-tetrahydro-benzo[f][1,4]thiazepin-4-yl)-6-methylquinazolin-4-yl)azetidine-3-carboxylate

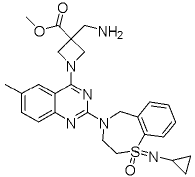

The synthesis method of this compound was the same as Example 4-2. ESI-MS(m/z): 521.23 [M+H]⁺, ¹H NMR (600 MHz, Methanol-*d*₄) δ 8.01 (d, *J* = 7.5 Hz, 1H), 7.79 (d*, J =* 7.4 Hz, 1H), 7.58 (t*, J =* 7.3 Hz, 1H), 7.46 - 7.42 (m, 2H), 7.36 - 7.32 (m, 2H), 5.39 (d, *J =* 15.0 Hz, 1H), 4.99 (s, 1H), 4.77 (d, *J =* 15.0 Hz, 1H), 4.68 (dd, *J =* 18.7, 8.4 Hz, 2H), 4.41 (s, 2H), 3.92 (s, 1H), 3.59 - 3.55 (m, 1H), 3.53 - 3.48 (m, 1H), 3.36 (d, *J =* 11.5 Hz, 3H), 3.25 (s, 2H), 2.34 (d, *J =* 4.3 Hz, 3H), 2.29 - 2.26 (m, 1H), 0.55 - 0.43 (m, 2H), 0.28 - 0.22 (m, 2H).

### Example 4-58: Synthesis of methyl (3S,4R)-4-((2-(1-(cyclopropylimino)-1-oxido-1,2,3,5-tetrahydro-benzo[f][1,4]thiazepyridin-4-yl)-6-methylquinazolin-4-yl)amino)pyrrolidine-3-carboxylate

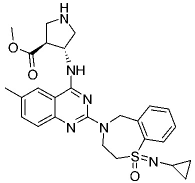

The synthesis method of this compound was the same as Example 4-2. ESI-MS(m/z): 521.23 [M+H]⁺, ¹H NMR (600 MHz, Methanol-*d*₄) δ 8.02 (t, *J* = 7.8 Hz, 1H), 7.86 (dd, *J =* 22.6, 7.4 Hz, 1H), 7.69 (d, *J* = 4.7 Hz, 1H), 7.60 (d, *J* = 7.2 Hz, 1H), 7.45 (dd, *J* = 16.0, 7.9 Hz, 1H), 7.39 (d, *J =* 8.5 Hz, 1H), 7.36 - 7.30 (m, 1H), 5.53 - 5.38 (m, 1H), 5.06 (d, *J =* 45.8 Hz, 2H), 4.80 (dd, *J =* 28.2, 15.4 Hz, 1H), 3.95 (s, 1H), 3.72 (d, *J =* 19.0 Hz, 3H), 3.57 (dd, *J* = 20.3, 17.2 Hz, 2H), 3.49 - 3.40 (m, 2H), 3.30 - 3.20 (m, 1H), 3.14 - 3.00 (m, 2H), 2.39 (s, 3H), 2.28 (d, *J* = 3.1 Hz, 1H), 0.56 - 0.42 (m, 2H), 0.31 - 0.21 (m, 2H).

### Example 4-59: Synthesis of 4-(4-(3-(aminomethyl)-3-fluoroazetidin-1-yl)-6-methylquinazolin-2-yl)-1-(cyclopropylimino)-2,3,4,5-tetrahydrobenzo[f][1,4]thiazepine-1-oxide

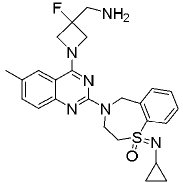

The synthesis method of this compound was the same as Example 4-2. ESI-MS(m/z): 481.20[M+H]⁺; ¹H NMR (600 MHz, Methanol-*d*₄) δ 7.99 (d, *J =* 7.8 Hz, 1H), 7.77 (d, *J =* 8.0 Hz, 1H), 7.57 (t*, J =* 7.5 Hz, 1H), 7.42 (d, *J =* 8.0 Hz, 2H), 7.36 - 7.29 (m, 2H), 5.38 (d, *J =* 15.1 Hz, 1H), 4.98 (d, *J =* 14.8 Hz, 1H), 4.76 (d, *J =* 15.1 Hz, 1H), 4.54 (dddt, *J* = 51.1, 31.5, 20.9, 10.8 Hz, 3H), 3.90 (d, *J =* 13.4 Hz, 1H), 3.56 (dt, *J =* 14.8, 2.8 Hz, 1H), 3.49 (ddd, *J* = 14.5, 11.1, 3.0 Hz, 1H), 3.14 (d*, J* = 22.2 Hz, 2H), 2.35 (d, *J* = 9.1 Hz, 1H), 2.33 (s, 3H), 2.26 (dt, *J* = 7.1, 3.5 Hz, 1H), 0.55 - 0.48 (m, 1H), 0.42 (ddd, *J* = 11.5, 9.1, 5.7 Hz, 1H), 0.29 - 0.19 (m, 2H).

### Example 4-60: 4-(4-(3-(2-aminopropan-2-yl)azetidin-1-yl)-6-methylquinazolin-2-yl)-1-(cyclopropylimino)-2,3,4,5-tetrahydro-benzo[f][1,4]thiazepine-1-oxide

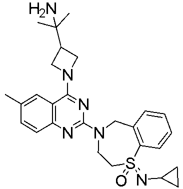

The synthesis method of this compound was the same as Example 4-2. ESI-MS(m/z): 491.25[M+H]⁺ ;¹H NMR (600 MHz, CD₃OD) δ 8.04 (d, *J* = 7.8 Hz, 1H), 7.82 (d, *J* = 7.2 Hz, 1H), 7.60 (t, *J* = 7.9 Hz, 1H), 7.54 (s, 1H), 7.47 (d, *J* = 7.4 Hz, 1H), 7.40 (d*, J =* 8.6 Hz, 1H), 7.35 (d, *J =* 8.6 Hz, 1H), 4.57 (d, *J =* 36.7 Hz, 3H), 4.41 (s, 2H), 3.57 (dd, *J =* 35.5, 13.1 Hz, 3H), 2.38 (s, 3H), 2.04 (d, *J =* 8.2 Hz, 1H), 1.32 (s, 6H), 0.92 (t, *J =* 7.0 Hz, 1H), 0.55 (d, *J* = 4.7 Hz, 1H), 0.49 - 0.42 (m, 2H), 0.28 (d, *J =* 17.9 Hz, 1H).

### Example 4-61: 4-(4-(((S)-3-aminopyrrolidin-1-yl)-6-methylquinazolin-2-yl)-1-((oxetan-2-ylmethyl)imino)-2,3,4,5-tetrahydro-benzo[f][1,4]thiazepine- 1-oxide

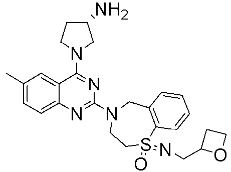

The synthesis method of this compound was the same as Example 4-2. ESI-MS(m/z): 493.23 [M+H]⁺, ¹H NMR (600 MHz, Methanol-*d*₄) δ 7.99 - 7.93 (m, 1H), 7.82 - 7.75 (m, 2H), 7.60 - 7.53 (m, 1H), 7.41 (dh, *J* = 10.2, 2.6 Hz, 1H), 7.34 (s, 2H), 5.39 (d*, J* = 15.1 Hz, 1H), 5.01 (s, 1H), 4.95 - 4.88 (m, 1H), 4.83 (ddd, *J* = 12.1, 5.3, 3.4 Hz, 1H), 4.66 - 4.59 (m, 1H), 4.50 (ddt, *J =* 47.3, 9.1, 5.9 Hz, 1H), 4.17 - 4.02 (m, 3H), 3.93 (ddt, *J =* 33.4, 12.3, 7.5 Hz, 1H), 3.78 - 3.49 (m, 4H), 3.09 (ddd, *J* = 26.6, 13.1, 5.0 Hz, 1H), 2.90 (ddd, *J* = 19.2, 13.1, 5.7 Hz, 1H), 2.64 (dtd, *J* = 14.3, 7.9, 4.0 Hz, 1H), 2.48 (dddt, *J =* 65.0, 11.1, 9.0, 7.0 Hz, 1H), 2.35 (s, 3H), 2.23 (dp, *J* = 9.9, 5.2, 3.9 Hz, 1H), 1.91 (dt*, J =* 13.3, 6.8 Hz, 1H).

### Example 4-62: Synthesis of 4-(4-((S)-3-aminopyrrolidin-1-yl)-6-(difluoromethyl)quinazolin-2-yl)-1-(cyclopropylimino)-2,3,4,5-tetrahydro-benzo[f][1,4]thiazepine-1-oxide

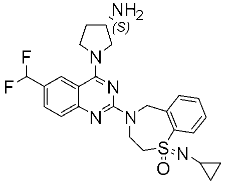

The synthesis method of this compound was the same as Example 4-2. ESI-MS(m/z): 499.20 [M+H]⁺, ¹H NMR (600 MHz, Methanol-*d*₄) δ 8.16 (s, 1H), 8.03 (d, *J =* 7.7 Hz, 1H), 7.82 (d, *J =* 7.3 Hz, 1H), 7.65 - 7.57 (m, 2H), 7.51 - 7.43 (m, 2H), 6.78 (t, *J =* 56.4 Hz, 1H), 5.45 (d, *J =* 14.9 Hz, 1H), 5.06 (s, 1H), 4.82 (d, *J =* 14.8 Hz, 1H), 4.11 (dd, *J =* 26.7, 9.0 Hz, 2H), 3.95 (s, 2H), 3.79 - 3.67 (m, 2H), 3.61 (d, *J =* 13.3 Hz, 1H), 3.57 - 3.48 (m, 1H), 2.29 (d, *J =* 3.2 Hz, 2H), 2.00 - 1.90 (m, 1H), 0.56 - 0.42 (m, 2H), 0.27 (ddd, *J =* 17.7, 11.2, 5.6 Hz, 2H).

### Example 4-63: Synthesis of 4-(4-((S)-3-aminopyrrolidin-1-yl)-6-(difluoromethyl)quinazolin-2-yl)-1-((4,4,4-trifluoro-3-hydroxy-3-methylbutyl)imino)-2,3,4,5-tetrahydro-benzo[f] [1,4]thiazepine-1-oxide

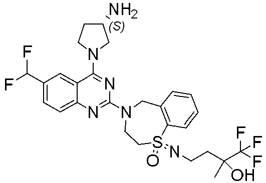

The synthesis method of this compound was the same as Example 4-2. ESI-MS(m/z): 599.21 [M+H]+;¹H NMR (600 MHz, CD₃OD) *δ* 8.16 (s, 1H), 7.99 (d, *J* = 7.5 Hz, 1H), 7.83 (d, *J* = 7.1 Hz, 1H), 7.69 - 7.54 (m, 2H), 7.52 - 7.39 (m, 2H), 6.78 (t, *J =* 54.0 Hz, 1H), 5.43 (t, *J =* 16.2 Hz, 1H), 5.07 (s, 1H), 4.83 (s, 1H), 4.28 - 4.04 (m, 2H), 3.96 (d, *J* = 19.2 Hz, 2H), 3.83 - 3.67 (m, 2H), 3.64 (d, *J* = 14.0 Hz, 1H), 3.53 (td, *J =* 14.8, 7.6 Hz, 1H), 3.10 (dd, *J* = 9.7, 6.0 Hz, 1H), 2.91 (s, 1H), 2.28 (s, 1H), 2.05 - 1.91 (m, 2H), 1.86 (ddd, *J* = 20.8, 15.8, 14.3 Hz, 1H), 1.83 - 1.67 (m, 1H), 1.25 *(d, J=* 7.2 Hz, 3H).

### Example 4-64: Synthesis of 4-(4-(((S)-3-aminopyrrolidin-1-yl)-6-methylquinazolin-2-yl)-1-((3-hydroxypropyl)imino)-2,3,4,5-tetrahydro-benzo[f][1,4]thiazepine-1-oxide

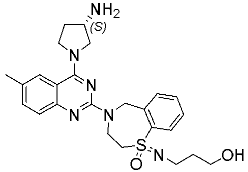

The synthesis method of this compound was the same as Example 4-2. ESI-MS(m/z): 481.23 [M+H]+;¹H NMR (600 MHz, CD₃OD) *δ* 7.97 (d, *J =* 7.7 Hz, 1H), 7.80 (d, *J =* 8.5 Hz, 2H), 7.57 (t, *J* = 5.5 Hz, 1H), 7.43 (t, *J* = 7.5 Hz, 1H), 7.35 (s, 2H), 5.43 (d, *J* = 14.9 Hz, 1H), 5.02 (s, 1H), 4.82 (d, *J* = 15.4 Hz, 1H), 4.20 - 3.82 (m, 4H), 3.75 - 3.47 (m, 6H), 3.07 - 2.93 (m, 1H), 2.87 - 2.71 (m, 1H), 2.37 (s, 3H), 2.23 (dd, *J* = 12.1, 5.8 Hz, 1H), 1.89 (dd, *J* = 11.9, 6.1 Hz, 1H), 1.81 - 1.65 (m, 2H).

### Example 4-65: Synthesis of 4-(4-(((S)-3-aminopyrrolidin-1-yl)-6-methylquinazolin-2-yl)-1-((2-(methylsulfonyl)ethyl)imino)-2,3,4,5-tetrahydrobenzo[f][1,4]thiazepine-1-oxide

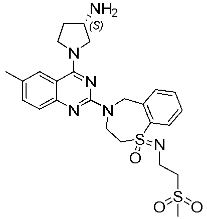

The synthesis method of this compound was the same as Example 4-2. ESI-MS(m/z): 529.17[M+H]⁺; ¹H NMR (600 MHz, Methanol-*d*₄) δ 8.00 - 7.96 (m, 2H), 7.82 - 7.78 (m, 2H), 7.60 - 7.55 (m, 1H), 7.47 - 7.42 (m, 1H), 7.41 - 7.32 (m, 1H), 5.40 (d, *J =* 15.2 Hz, 1H), 4.19 - 3.92 (m, 4H), 3.74 (ddd, *J =* 23.4, 11.0, 5.1 Hz, 2H), 3.67 - 3.62 (m, 2H), 3.60 - 3.52 (m, 2H), 3.29 - 3.17 (m, 1H), 2.99 (s, 3H), 2.37 (s, 3H), 2.32 - 2.13 (m, 2H), 1.92 - 2.08 (m, 2H), 1.57 - 1.65 (m, 1H).

### Example 4-66: Synthesis of 4-(4-(((S)-3-aminopyrrolidin-1-yl)-6-(difluoromethyl)quinazolin-2-yl)-1-((3-hydroxypropyl)imino)-2,3,4,5-tetrahydro-benzo[f][1,4]thiazepine-1-oxide

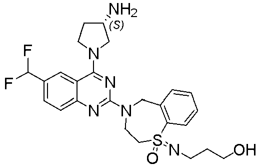

The synthesis method of this compound was the same as Example 4-2. ESI-MS(m/z): 517.21 [M+H]+;¹H NMR (600 MHz, CD₃OD) *δ* 8.17 (s, 1H), 7.99 (d*, J* = 7.7 Hz, 1H), 7.83 (d, *J* = 7.1 Hz, 1H), 7.64 (d, *J* = 8.6 Hz, 1H), 7.59 (s, 1H), 7.50 (d, *J* = 8.5 Hz, 1H), 7.45 (t, *J =* 7.4 Hz, 1H), 6.79 (t, *J* = 60.0 Hz, 1H), 5.45 (d, *J* = 15.0 Hz, 1H), 5.09 (s, 1H), 4.84 (s, 2H), 4.05 (dd, *J* = 87.4, 28.2 Hz, 4H), 3.84 - 3.58 (m, 4H), 3.56 - 3.42 (m, 1H), 3.02 (ddd, *J* = 18.7, 13.7, 7.0 Hz, 1H), 2.82 (d, *J* = 5.9 Hz, 1H), 2.25 (d, *J* = 33.5 Hz, 1H), 1.96 (d, *J* = 5.1 Hz, 1H), 1.81 - 1.68 (m, 2H).

### Example 4-67: Synthesis of 4-(4-(((S)-3-aminopyrrolidin-1-yl)-6-methylquinazolin-2-yl)-1-((tetrahydrofuran-3-yl)imino)-2,3,4,5-tetrahydro-benzo[f][1,4]thiazepine-1-oxide

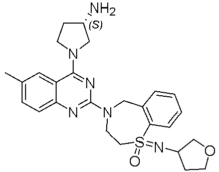

The synthesis method of this compound was the same as Example 4-2. ESI-MS(m/z): 493.23 [M+H]⁺, ¹H NMR (600 MHz, Methanol-*d*₄) δ 7.97 (dd, *J* = 14.2, 7.7 Hz, 1H), 7.82 - 7.78 (m, 2H), 7.58 (t, *J=* 7.0 Hz, 1H), 7.43 (dd, *J =* 13.3, 7.2 Hz, 1H), 7.34 (s, 2H), 5.47 - 5.39 (m, 1H), 5.01 (s, 1H), 4.78 (dd, *J =* 48.6, 15.1 Hz, 1H), 4.07 (ddd, *J* = 16.6, 13.6, 5.3 Hz, 2H), 3.99 - 3.87 (m, 3H), 3.79 - 3.73 (m, 1H), 3.70 (dd, *J =* 14.5, 7.8 Hz, 1H), 3.65 (t, *J= 9.0* Hz, 1H), 3.58 (ddd, *J =* 20.6, 8.1, 3.9 Hz, 2H), 3.55 - 3.51 (m, 1H), 3.50 - 3.46 (m, 1H), 2.36 (s, 3H), 2.22 (dt, *J* = 12.6, 6.9 Hz, 1H), 2.07 - 1.83 (m, 3H), 1.76 (dt, *J* = 19.1, 6.3 Hz, 1H).

### Example 4-68: Synthesis of 1-((2-oxaspiro[3.3]heptan-6-yl)imino)-4-(4-(((S)-3-aminopyrrolidin-1-yl)-6-methylquinazolin-2-yl)-2,3,4,5-tetrahydro-benzo[f][1,4]thiazepine-1-oxide

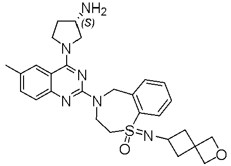

The synthesis method of this compound was the same as Example 4-2. ESI-MS(m/z): 519.2[M+H]⁺.

### Example 4-69: Synthesis of 4-(4-(((S)-3-aminopyrrolidin-1-yl)-6-methylquinazolin-2-yl)-1-((1-methoxypropan-2-yl)imino)-2,3,4,5-tetrahydro-benzo[f][1,4]thiazepine-1-oxide

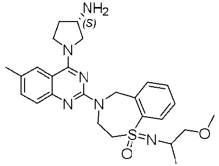

The synthesis method of this compound was the same as Example 4-2. ESI-MS(m/z): 495.2[M+H]⁺. ¹H NMR (600 MHz, Methanol-*d*₄) δ 7.96 (d, *J =* 7.2 Hz, 1H), 7.78 (d, *J =* 7.0 Hz, 1H), 7.76 (s, 1H), 7.55 (s, 1H), 7.39 (d, *J* = 5.8 Hz, 1H), 7.32 (s, 2H), 5.39 (dd*, J =* 32.7, 14.8 Hz, 1H), 5.06 (d, *J =* 15.2 Hz, 1H), 5.00 (s, 1H), 4.10 - 3.83 (m, 4H), 3.62 (dd, *J =* 26.8, 16.3 Hz, 3H), 3.51 (dd, *J =* 28.8, 16.8 Hz, 2H), 3.33 (s, 1H), 3.28 - 3.22 (m, 1H), 3.17 (s, 2H), 2.34 (s, 3H), 2.19 (d, *J* = 5.8 Hz, 1H), 1.85 (d, *J =* 5.4 Hz, 1H), 1.13 (d*, J* = 6.4 Hz, 2H), 1.01 (d, *J =* 6.2 Hz, 2H).

### Example 4-70: Synthesis of 4-(4-(((S)-3-aminopyrrolidin-1-yl)-6-methylquinazolin-2-yl)-1-(((tetrahydrofuran-2-yl)methyl)imino)-2,3,4,5-tetrahydro-benzo[f][1,4]thiazepine-1-oxide

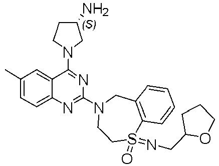

The synthesis method of this compound was the same as Example 4-2. ESI-MS(m/z): 507.2[M+H]⁺. ¹H NMR (600 MHz, Methanol-*d*₄) δ 7.94 (d, *J =* 7.6 Hz, 1H), 7.78 (d, *J* = 11.0 Hz, 2H), 7.55 (d, *J* = 2.8 Hz, 1H), 7.39 (t, *J =* 7.5 Hz, 1H), 7.33 (s, 2H), 5.38 (dd*, J =* 14.1, 7.8 Hz, 1H), 4.99 (s, 1H), 4.94 (d, *J =* 15.1 Hz, 1H), 4.10 - 3.99 (m, 3H), 3.99 - 3.96 (m, 0.5H), 3.93 (dt*, J =* 12.3, 6.2 Hz, 1H), 3.87-3.82 (m, 1H), 3.74 - 3.71 (m, 0.5H), 3.68 (t, *J =* 6.7 Hz, 1H), 3.63 (t*, J =* 8.5 Hz, 2H), 3.60 - 3.49 (m, 2H), 2.98 (dd, *J =* 12.5, 5.5 Hz, 0.5H), 2.90 (dd, *J =* 12.4, 4.8 Hz, 0.5H), 2.69 (dd, *J =* 14.7, 9.3 Hz, 1H), 2.34 (s, 3H), 2.25 - 2.16 (m, 1H), 2.02 - 1.94 (m, 1H), 1.92 - 1.79 (m, 3H), 1.67 (td, *J =* 15.3, 7.4 Hz, 0.5H), 1.59 (td, *J* = 14.6, 7.5 Hz, 0.5H).

### Example 4-71: Synthesis of 4-(4-(((S)-3-aminopyrrolidin-1-yl)-6-methylquinazolin-2-yl)-1-((3-methoxypropyl)imino)-2,3,4,5-tetrahydro-benzo[f][1,4]thiazepine-1-oxide

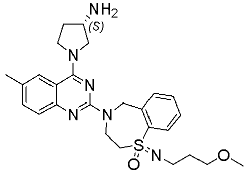

The synthesis method of this compound was the same as Example 4-2. ESI-MS(m/z): 495.25 [M+H]+;¹H NMR (600 MHz, CD₃OD) *δ* 7.95 (d, *J* = 7.7 Hz, 1H), 7.80 (d, *J* = 6.4 Hz, 2H), 7.61 - 7.52 (m, 1H), 7.42 (t, *J* = 7.5 Hz, 1H), 7.34 (s, 2H), 5.42 (d, *J =* 14.9 Hz, 1H), 5.01 (s, 1H), 4.80 (d, *J =* 14.7 Hz, 2H), 4.16 - 3.81 (m, 4H), 3.74 - 3.38 (m, 6H), 3.33 (dt, *J =* 3.1*,* 1.6 Hz, 2H), 2.94 (d, *J =* 6.2 Hz, 1H), 2.84 - 2.71 (m, 1H), 2.36 (s, 3H), 2.22 (dt, *J* = 11.9, 5.9 Hz, 1H), 1.88 (dd, *J* = 12.1, 6.1 Hz, 1H), 1.83 - 1.66 (m, 2H).

### Example 4-72: Synthesis of 4-(4-(((S)-3-aminopyrrolidin-1-yl)-6-methylquinazolin-2-yl)-1-(((tetrahydrofuran-3-yl)methyl)imino)-2,3,4,5-tetrahydrobenzo[f][1,4]thiazepine-1-oxide

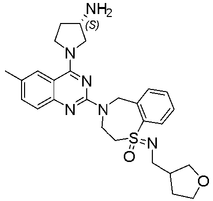

The synthesis method of this compound was the same as Example 4-2. ESI-MS(m/z): 507.17[M+H]⁺; ¹H NMR (600 MHz, Methanol-*d*₄) δ 7.92 (d, *J* = 7.8 Hz, 1H), 7.79 - 7.73 (m, 2H), 7.53 (td, *J =* 7.5, 3.0 Hz, 1H), 7.38 (ddd, *J =* 9.7, 7.0, 2.1 Hz, 1H), 7.31 (d*, J* = 1.9 Hz, 2H), 5.39 (d, *J* = 15.0 Hz, 1H), 4.98 (s, 1H), 4.80 - 4.61 (m, 1H), 4.11 - 3.88 (m, 2H), 3.89 - 3.72 (m, 2H), 3.71 - 3.46 (m, 6H), 2.86 (ddd, *J* = 26.0, 12.1, 7.3 Hz, 1H), 2.63 (ddd, *J =* 30.6, 11.9, 7.2 Hz, 1H), 2.38 (dq, *J* = 18.0, 7.1 Hz, 1H), 2.32 (s, 3H), 2.19 (dq, *J=* 13.0, 6.6 Hz, 1H), 1.97 (tdd, *J =* 13.6, 7.9, 5.8 Hz, 1H), 1.86 (dq, *J* = 13.2, 6.8 Hz, 1H), 1.63 - 1.50 (m, 1H), 1.37 - 1.25 (m, 2H).

### Example 4-73: Synthesis of 4-(4-(((R)-3-aminopyrrolidin-1-yl)-6-methylquinazolin-2-yl)-1-((3-methoxypropyl)imino)-2,3,4,5-tetrahydro-benzo[f][1,4]thiazepine-1-oxide

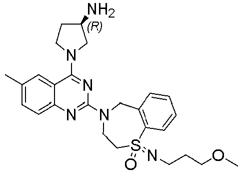

The synthesis method of this compound was the same as Example 4-2. ESI-MS(m/z): 495.25 [M+H]+;¹H NMR (600 MHz, CD₃OD) *δ* 7.98 (d, *J =* 7.8 Hz, 1H), 7.84 - 7.75 (m, 2H), 7.61 - 7.53 (m, 1H), 7.44 (t, *J =* 7.5 Hz, 1H), 7.39 - 7.33 (m, 2H), 5.43 (d, *J =* 14.9 Hz, 1H), 5.02 (s, 1H), 4.83 (d, *J* = 15.7 Hz, 1H), 4.19 - 3.71 (m, 5H), 3.69 - 3.41 (m, 5H), 3.04 - 2.91 (m, 1H), 2.86 - 2.72 (m, 1H), 2.53 (d, *J =* 3.2 Hz, 3H), 2.38 (s, 3H), 2.29 (dd, *J =* 11.5, 5.9 Hz, 1H), 2.01 (ddd, *J* = 19.2, 12.5, 6.2 Hz, 1H), 1.80 - 1.68 (m, 2H).

### Example 4-74: Synthesis of 4-(4-((S)-3-aminopyrrolidin-1-yl)-6-(difluoromethyl)quinazolin-2-yl)-1-((oxetan-2-ylmethyl)imino)-2,3,4,5-tetrahydro-benzo[f][1,4]thiazepine-1-oxide

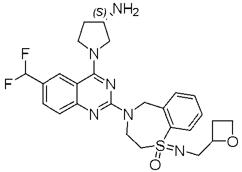

The synthesis method of this compound was the same as Example 4-2. ESI-MS(m/z): 529.21 [M+H]⁺, ¹H NMR (600 MHz, Methanol*d*₄) δ 8.15 (s, 1H), 7.99 (dd, *J =* 17.5, 10.0 Hz, 1H), 7.80 (t, *J =* 10.0 Hz, 1H), 7.61 (t, *J* = 17.5 Hz, 2H), 7.51 - 7.40 (m, 2H), 6.78 (t, *J =* 56.4 Hz, 1H), 5.38 (dd, *J =* 29.0, 10.0 Hz, 1H), 5.07 (s, 1H), 4.93 (dd, *J* = 22.3, 13.0 Hz, 1H), 4.83 (dd, *J* = 13.0, 6.1 Hz, 1H), 4.67 - 4.61 (m, 1H), 4.55 (dt, *J* = 9.1, 5.9 Hz, 1H), 4.07 (d, *J* = 6.1 Hz, 3H), 3.96 (d, *J* = 35.8 Hz, 1H), 3.76 - 3.59 (m, 3H), 3.58 - 3.49 (m, 1H), 3.11 (ddd, *J =* 27.0, 13.2, 4.8 Hz, 1H), 2.89 (d, *J* = 12.7 Hz, 1H), 2.70 - 2.61 (m, 1H), 2.55 (ddd, *J* = 10.9, 9.1, 7.1 Hz, 1H), 2.30 - 2.20 (m, 1H), 1.96 - 1.89 (m, 1H).

### Example 4-75: Synthesis of 4-(4-(((3-aminoxetan-3-yl)methyl)amino)-5-bromo-6-methylquinazolin-2-yl)-1-(cyclopropylimino)-2,3,4,5-tetrahydro-benzo[f][1,4]thiazepine-1-oxide

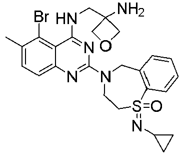

The synthesis method of this compound was the same as Example 4-2. ESI-MS(m/z): 557.13/559.13 [M+H]+; ¹H NMR (600 MHz, CD₃OD) *δ* 8.04 (d, *J =* 7.7 Hz, 1H), 7.86 (d, *J =* 7.4 Hz, 1H), 7.61 (t, *J =* 7.3 Hz, 1H), 7.47 (t, *J* = 7.6 Hz, 1H), 7.40 (d*, J =* 8.6 Hz, 1H), 7.31 (d*, J =* 8.4 Hz, 1H), 5.44 (d, *J* = 14.9 Hz, 1H), 5.04 (s, 1H), 4.82 (d, *J =* 15.1 Hz, 1H), 4.64 (d, *J =* 5.8 Hz, 1H), 4.59 (dd, *J =* 14.4, 6.1 Hz, 3H), 4.14 - 3.90 (m, 3H), 3.63 (d*, J =* 15.7 Hz, 1H), 3.52 (d, *J* = 11.8 Hz, 1H), 2.40 (s, 3H), 2.34 - 2.25 (m, 1H), 0.60 - 0.49 (m, 1H), 0.49 - 0.40 (m, 1H), 0.34 - 0.19 (m, 2H).

### Example 4-76: Synthesis of 4-(4-(3-aminoazetidin-1-yl)-6-methylquinazolin-2-yl)-1-(cyclopropylimino)-2,3,4,5-tetrahydrobenzo[f][1,4]thiazepine-1-oxide

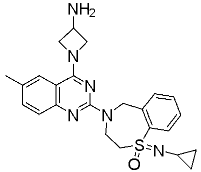

The synthesis method of this compound was the same as Example 4-2. ESI-MS(m/z): 449.2[M+H]⁺. ¹H NMR (600 MHz, CD₃OD) δ 8.02 (d, J = 7.8 Hz, 1H), 7.79 (d, J = 7.4 Hz, 1H), 7.59 (t, J = 7.1 Hz, 1H), 7.46 (dd, *J =* 14.2, 5.9 Hz, 2H), 7.35 (dt*, J* = 20.4, 5.1 Hz, 2H), 5.41 (d, *J* = 15.0 Hz, 1H), 4.99 (s, 1H), 4.81 - 4.66 (m, 3H), 4.15 (dd, *J* = 21.2, 16.5 Hz, 2H), 3.96 (ddd, *J =* 12.5, 7.3, 5.3 Hz, 2H), 3.59 (dd, *J =* 12.6, 3.2 Hz, 1H), 3.54 - 3.48 (m, 1H), 2.35 (s, 3H), 2.32 - 2.25 (m, 1H), 0.54 (ddd, *J* = 10.3, 6.5, 4.6 Hz, 1H), 0.48 - 0.42 (m, 1H), 0.31 - 0.21 (m, 2H).

### Example 4-77: Synthesis of 1-(cyclopropylimino)-4-(4-(((3-hydroxyazetidin-3-yl)methyl)amino)-6-methylquinazolin-2-yl)-2,3,4,5-tetrahydro-benzo[f][1,4]thiazepine-1-oxide

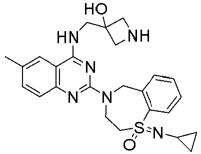

The synthesis method of this compound was the same as Example 4-2. ESI-MS(m/z): 479.2 [M+H]⁺. ¹H NMR (600 MHz, CD₃OD) δ 8.02 (d, *J =* 7.5 Hz, 1H), 7.86 (d, *J =* 7.3 Hz, 1H), 7.66 (s, 1H), 7.58 (dd, *J =* 19.6, 12.5 Hz, 1H), 7.45 (t, *J* = 7.3 Hz, 1H), 7.39 (d*, J =* 8.2 Hz, 1H), 7.33 (d, *J =* 8.5 Hz, 1H), 5.43 (d, *J =* 15.0 Hz, 1H), 5.02 (s, 1H), 4.81 (d, *J =* 14.8 Hz, 1H), 4.02 (d, *J =* 59.9 Hz, 2H), 3.83 (d, *J =* 13.9 Hz, 1H), 3.77 (d, *J =* 8.5 Hz, 1H), 3.71 (dd, *J* = 16.0, 8.9 Hz, 3H), 3.61 (d, *J =* 14.4 Hz, 1H), 3.50 (s, 1H), 2.41 - 2.35 (m, 3H), 2.32 - 2.25 (m, 1H), 0.54 (d, *J =* 4.1 Hz, 1H), 0.44 (d, *J =* 5.0 Hz, 1H), 0.26 (dd, *J =* 13.2, 7.1 Hz, 2H).

### Example 4-78: Synthesis of 4-(4-((azetidin-3-ylmethyl)amino)-6-methylquinazolin-2-yl)-1-(cyclopropylimino)-2,3,4,5-tetrahydro-benzo[f][1,4]thiazepine-1-oxide

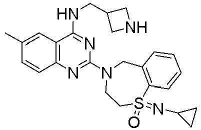

The synthesis method of this compound was the same as Example 4-2. ESI-MS(m/z): 463.2 [M+H]⁺. ¹H NMR (600 MHz, CD₃OD) δ 7.97 (dd, *J =* 19.5, 7.7 Hz, 1H), 7.76 (d, *J* = 7.1 Hz, 1H), 7.63 (d, *J =* 13.1 Hz, 1H), 7.59 - 7.52 (m, 1H), 7.41 (t*, J =* 7.4 Hz, 1H), 7.36 - 7.33 (m, 1H), 7.31 - 7.27 (m, 1H), 5.41 (d, *J =* 15.0 Hz, 1H), 5.01 (s, 1H), 4.75 (t*, J* = 19.0 Hz, 1H), 3.97 - 3.87 (m, 3H), 3.83 - 3.73 (m, 3H), 3.59 (t, *J =* 13.5 Hz, 1H), 3.54 - 3.46 (m, 1H), 3.28 (dt, *J* = 14.8, 7.5 Hz, 1H), 2.34 (d, *J* = 13.5 Hz, 3H), 2.30 - 2.23 (m, 2H), 0.55 - 0.48 (m, 1H), 0.45 - 0.38 (m, 1H), 0.28 - 0.17 (m, 2H).

### Example 4-79: Synthesis of 4-(4-(((R)-3-aminopyrrolidin-1-yl)-6-methylquinazolin-2-yl)-1-imino-2,3,4,5-tetrahydro-benzo[f][1,4]thiazepine-1-oxide

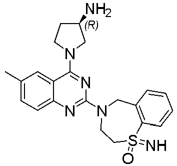

The synthesis method of this compound was the same as Example 4-2. ESI-MS(m/z): 423.19 [M+H]⁺.¹H NMR (600 MHz, DMSO-d6) δ 7.92 (d, *J =* 6.1 Hz, 1H), 7.75 (s, 1H), 7.71 (s, 1H), 7.52 (s, 1H), 7.40 (s, 1H), 7.32 (d, *J =* 7.3 Hz, 1H), 7.25 (s, 1H), 5.11 (d, *J =* 41.7 Hz, 2H), 4.62 (s, 2H), 3.95-3.80 (m, 3H), 3.70-3.38 (m, 4H), 2.33 (s, 3H), 2.04 (s, 1H), 1.75 (s, 1H).

### Example 4-80: Synthesis of 4-(4-(3-(aminomethyl)-3-(hydroxymethyl)azetidin-1-yl)-6-methylquinazolin-2-yl)-1-(cyclopropylimino)-2,3,4,5-tetrahydro-benzo[f][1,4]thiazepine-1-oxide

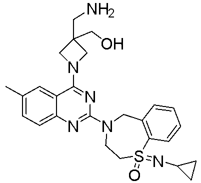

The synthesis method of this compound was the same as Example 4-2. ESI-MS(m/z): 493.22 [M+H]⁺, ¹H NMR (600 MHz, Methanol-*d*₄) δ 8.04 (d, *J =* 7.7 Hz, 1H), 7.80 (d, *J =* 7.4 Hz, 1H), 7.60 (t, *J =* 7.3 Hz, 1H), 7.52 (s, 1H), 7.48 (t, *J* = 7.6 Hz, 1H), 7.38 (dd, *J =* 23.2, 8.6 Hz, 2H), 5.43 (d, *J =* 14.9 Hz, 1H), 5.10 - 4.90 (m, 2H), 4.35 (d, *J =* 33.7 Hz, 4H), 3.95 (s, 3H), 3.61 - 3.49 (m, 2H), 3.39 (s, 2H), 2.37 (s, 3H), 2.29 (d, *J =* 3.4 Hz, 1H), 0.57 - 0.44 (m, 2H), 0.31 - 0.22 (m, 2H).

The following are the efficacy tests and data of the compounds of the present invention.

### Test Example 1: Antiviral detection test of small molecules against RSV strain A2 based on ELISA method

### 1. Cell line

Source of cell line: Nanjing Kebai Biotechnology Co., Ltd.
Cell type: human laryngeal carcinoma epithelial cells Hep-2

### 2. Reagents and consumables

96-well culture plate (Cat.No. 3610, Corning Costar);
Fetal bovine serum (Cat.No. 10099-141, GIBCO);
DMEM medium (Cat.No. 11995500BT, GIBCO);
Envision 2104 microplate reader.

### 3. Test method

1. Day 1: The Hep-2 cells were seeded into a 96-well plate at a density of 1×10⁴ cells/well;
2. Day 2:
   A. Compound dilution: The compound in the first well was diluted to 9 µM, and 3-fold gradient dilution was performed, with a total of 10 gradients;
   B. The Hep-2 cell plate prepared on Day 1 was taken out from the incubator, the supernatant was discarded, and 100 PFU/well/50 µL of the RSV A2 virus was added to each well. The plate was stood at 37°C in a 5% CO₂ incubator for 2 hours, the supernatant was aspirated and discarded, and then 100 µL of the gradient-diluted compound or DMEM maintenance medium containing 2% FBS was added to each well;
3. Day 5:
   A. Add fixative: The gradient-diluted compound or medium was discarded from the wells, and 100 µL/well of fixative (4% paraformaldehyde) was added. The plate was stood at room temperature for 10 minutes, and then was washed once with 250 µL PBS;
   B. 100 µL/well of a blocking solution was added, and the plate was incubated at 37°C in a 5% CO₂ incubator for 30 minutes;
   C. After incubation, the detection antibody (Anti RSV Fpro) was added at 0.3 µg/50 µL/well (6 µg/mL), and the plate was incubated at 37°C in a 5% CO₂ incubator for 1 hour;
   D. After incubation, the HRP secondary antibody was added (diluted 10,000-fold with 0.2% BSA), and the plate was incubated at 37°C in a 5% CO₂ incubator for 1 hour;
   E. After incubation, the wells were washed for 4 times with PBST, 250 µL/well, each washing was allowed to stand for 5 minutes, and the liquid in the wells was shaken off after completion of the washing;
   F. 100 µL/well of a development solution was added, the plate was incubated at room temperature in the dark for 20 minutes, and 50 µL/well of a reaction termination solution was added. The plate was tapped gently to mix the solutions, and the absorbance at 450 nm was measured using the microplate reader.
4. Data analysis
   Inhibition rate of the compound against the virus = 100% - (SC - CC)/(VC - CC) × 100%
   SC: OD450 value of the sample (cells + test compound + virus)
   CC: OD450 value of the normal growing cell control (cells)
   VC: OD450 value of the virus growth control (cells + virus)

The software Graphpad Prism 6 was used, and the calculation formula XY-analysis/Nonlinear regression (curve fit)/Dose response-Inhibition/log (inhibition) vs. response-Variable slope (four parameters) was adopted to fit the IC₅₀ curve and calculate the IC₅₀ value.

5. The experimental results demonstrated that the compounds of the present invention have good inhibitory effects against the RSV strain A2 *in vitro;* the inhibitory effect of the preferred compounds is better than that of the control compound AK0529 (i.e., AK0529 IC₅₀ / IC₅₀ of the compounds of the present invention > 1), more preferably AK0529 IC₅₀ / IC₅₀ of the compounds of the present invention ≥ 2, more preferably AK0529 IC₅₀ / IC₅₀ of the compounds of the present invention ≥ 3, more preferably AK0529 IC₅₀ / IC₅₀ of the compounds of the present invention ≥ 4, more preferably AK0529 IC₅₀ / IC₅₀ of the compounds of the present invention ≥ 5, more preferably ≥ 10, more preferably AK0529 IC₅₀ / IC₅₀ of the compounds of the present invention ≥ 20. For example, 1 < AK0529 IC₅₀ / IC₅₀ of the compounds of the present invention < 2, or 2 ≤ AK0529 IC₅₀ / IC₅₀ of the compounds of the present invention < 3, or 3 ≤ AK0529 IC₅₀ / IC₅₀ of the compounds of the present invention < 4, or 4 ≤ AK0529 IC₅₀ / IC₅₀ of the compounds of the present invention < 5, or 5 ≤ AK0529 IC₅₀ / IC₅₀ of the compounds of the present invention < 10, or 10 ≤ AK0529 IC₅₀ / IC₅₀ of the compounds of the present invention < 20, or AK0529 IC₅₀ / IC₅₀ of the compounds of the present invention ≥ 20. The IC₅₀ values of exemplary compounds for inhibiting the RSV strain A2 *in vitro* are shown in Table 1 below.

**Table 1 In vitro inhibitory activity of the compounds of the present invention against the RSV A2 virus strain**

| Compound No. | Antiviral Activity (IC₅₀, nM) | AK0529 IC₅₀ / IC₅₀ of the compounds of the present invention | Compound No. | Antiviral Activity (IC₅₀, nM) | AK0529 IC₅₀ / IC₅₀ of the compounds of the present invention |
|---|---|---|---|---|---|
| 2-14 | A | 5≤ ratio < 10 | 2-31 | A | 1 < ratio <2 |
| 2-16 | B | 1 < ratio <2 | 2-32 | A | 1 < ratio <2 |
| 2-23 | A | 2≤ ratio <5 | 2-37 | A | 1 < ratio <2 |
| 2-24 | A | ratio ≥20 | 2-38 | B | 1 < ratio <2 |
| 2-26 | A | 2≤ ratio <5 | 2-43 | A | 2≤ ratio <5 |
| 4-2 | A | 2≤ ratio <5 | 2-46 | A | ratio ≥20 |
| 4-5 | A | 2≤ ratio <5 | 4-56 | A | 2≤ ratio <5 |
| 4-6 | A | 10≤ ratio <20 | 4-57 | A | ratio ≥20 |
| 4-9 | A | 10≤ ratio <20 | 4-61 | A | 10≤ ratio <20 |
| 4-10 | A | 2≤ ratio <5 | 4-62 | A | 1 < ratio <2 |
| 4-11 | A | 5≤ ratio < 10 | 4-63 | A | 2≤ ratio <5 |
| 4-12 | A | 2≤ ratio <5 | 4-64 | A | 2≤ ratio <5 |
| 4-13 | A | 5≤ ratio < 10 | 4-65 | A | 5≤ ratio < 10 |
| 4-16 | A | ratio ≥20 | 4-66 | A | 2≤ ratio <5 |
| 4-17 | A | ratio ≥20 | 4-67 | A | 2≤ ratio <5 |
| 4-24 | A | ratio ≥20 | 4-69 | A | 2≤ ratio <5 |
| 4-28 | A | 2≤ ratio <5 | 4-70 | A | 5≤ ratio < 10 |
| 4-32 | A | 5≤ ratio <10 | 4-71 | A | 2≤ ratio <5 |
| 4-39 | A | 5≤ ratio <10 | 4-72 | A | 1 < ratio <2 |
| 4-43 | A | 2≤ ratio <5 | 4-73 | A | 2≤ ratio <5 |
| 4-47 | A | 5≤ ratio <10 | 4-74 | A | ratio ≥20 |
| 4-76 | A | ratio ≥20 | | | |

| | | | | | |
|---|---|---|---|---|---|
| Note: A represents IC₅₀ < 10 nM, and B represents IC₅₀ > 10 nM. AK0529 refers to the compound of Example 61-1 in Patent application publication No. WO2013020993A1, and its preparation method refers to Example 61-1 of Patent application publication No. WO2013020993A1. | | | | | |

### Test Example 2: Pharmacokinetic Experiment

The compounds of the present invention for intragastric administration were dissolved in a suitable solvent to form a clear solution or suspension.

Administration mode and dosage: The compounds of the present invention were administered intragastrically at 10 mg/kg or 20 mg/kg.

Number of animals: 3 ICR mice per group for intragastric administration.

Sample collection: For the intragastric administration group, 100 µL of blood was collected (using pre-heparinized 100 µL Capillary Blood Collection Tubes) from the orbital vein of mice at 0.25 h, 0.5 h, 1 h, 2 h, 4 h, and 6 h after administration and placed in heparin sodium anticoagulant tubes. Mice plasma samples were obtained by centrifugation at 3000 g for 10 minutes within 2 hours. For the intragastric administration group, clean blood was collected at 6-hour time point and then lung tissues were collected.

Sample treatment: Plasma and lung tissue homogenates were subjected to protein precipitation with methanol at a certain ratio, and the supernatant was taken and diluted with 50% methanol-water at a certain ratio for sample loading.

Sample detection: The drug concentration in plasma was detected by the LC/MS/MS method, and the drug-time curve was plotted. Pharmacokinetic parameters (such as T_{1/2}(h)/AUC₀₋ₜ) were calculated by non-compartmental model statistical moments to describe the pharmacokinetic behavior of the compounds in mice after administration.

Data processing: Pharmacokinetic parameters were calculated by WinNonlin.

The test results demonstrated that the compounds of the present invention have good *in vivo* pharmacokinetics and have potential for drug development. Exemplary compounds were shown in Table 2 below, and in particular, their T_{1/2} and lung tissue drug concentrations at 6 h are significantly superior to those of the control compound AK0529.

**Table 2 Pharmacokinetic parameters of mice after intragastric administration at 20 mg/kg**

| **Compound No.** | **T_{1/2}** | Lung tissue drug concentration at 6 h |
|---|---|---|
| | h | (ng/g) |
| AK0529 | 1.55 | 1206 |
| 4-8 | 10.8 | 25320 |
| 4-9 | 6.21 | 5920 |
| 4-11 | 6.91 | 22700 |
| 4-12 | 6.44 | 29700 |
| 4-15 | 1.61 | 5130 |
| 4-17 | 5.29 | 75800 |
| 4-24 | 4.48 | 15420 |
| 4-43 | 2.33 | 19220 |
| 4-62 | - | 53140 |
| 4-67 | 2.16 | 8220 |
| 4-69 | 2.28 | 6014 |
| 4-74 | 2.49 | 7174 |
| 4-76 | - | 28820 |

### Test Example 3: In vitro inhibition assay for human potassium ion channel (hERG)

Experimental equipment: Electrophysiological detection was performed using the fully automated patch clamp QPatch 48 X (Sophion) device.

Experimental procedure: The prepared cells were placed in the centrifuge chambers of the QPatch workbench, the cells were washed by the centrifugation/suspension method for multiple times to replace the cell culture medium with an extracellular solution. One MTP-96 plate was taken out and placed at the MTP source position. The QPlate chip was taken out and then the QPlate was placed at the QPlate source position. The robotic arm scanned the MTP-96 plate and the barcodes of the QPlate chip, and grabbed them to the measurement station. Intracellular and extracellular solutions were aspirated from the liquid pool and added to the intracellular solution pool and the cell and test substance pool of the QPlate chip, respectively. At the measurement station, all measurement sites on the QPlate underwent initial quality control. The quality control process included aspirating the cell suspension from the cell container of the centrifuge, positioning the cells into the chip wells through a pressure controller, establishing a high-resistance seal, and forming a whole-cell recording mode. Once a stable baseline of control current was obtained, the test substances were aspirated from the test substance MTP-96 plate according to concentration gradient and applied to the cells. The current detected in each cell in the extracellular solution without the compound was used as its own control group, and the detection was independently repeated on two cells. All QPatch electrophysiological experiments were performed at 24°C.

Compound treatment: The compounds were diluted into a gradient of 0.3 µM, 1 µM, 3 µM, 10µM, and 30 µM.

Data processing: The IC₅₀ values were calculated using GraphPad Prism software.

The test results demonstrated that the compounds of the present invention have no significant inhibitory effect on the hERG channel within the tested concentration range of this assay, indicating that the compounds of the present invention have a low risk of cardiotoxicity. The IC₅₀ of the compounds of the present invention is greater than that of AK0529, indicating that the hERG performance of the compounds of the present invention is significantly superior to that of the control compound AK0529. Exemplary compounds are shown in Table 3 below.

**Table 3**

| **Compound No.** | **IC₅₀ of the Compound of the present invention /AK0529 IC₅₀** |
|---|---|
| 4-10 | > 1 |
| 4-32 | > 1 |
| 4-51 | > 1 |
| 4-61 | > 1 |

### Test Example 4: In Vivo Pharmacodynamic Assay

The compound of the present invention was dissolved in a suitable solvent to form a clear solution or suspension for intragastric administration.
Animals: BALB/c mice, 6-weeks old, female.
Establishment of *in vivo* RSV infection model: Mice were inoculated intranasally with the virus on Day 0, with an inoculation dose of 3.5 × 10⁶ p.f.u. per mouse.
Grouping of animals: Animals were divided into a vehicle control group and an administration group, with 5 animals in each group.

Administration mode and dosage: The compound of the present invention was administered intragastrically at 100 mg/kg or 50 mg/kg, or the vehicle used for the compound of the present invention was administered intragastrically. The administration volume was 10 mL/kg.

Experimental procedure: Mice were inoculated intranasally with the virus on Day 0 at an inoculation dose of 3.5 × 10⁶ p.f.u. per mouse. Mice were continuously treated with the compound of the present invention from Day 0 to Day 4 for 5 days at a dose of 50 mg/kg, once daily, via intragastric administration, with an administration volume of 10 mL/kg; the first administration was conducted 1 hour before virus inoculation. Alternatively, mice were treated once with the compound of the present invention on Day 0 at a dose of 100 mg/kg, via intragastric administration, with an administration volume of 10 mL/kg; the first administration was conducted 1 hour before virus inoculation. On Day 5, mice were euthanized, and lung tissue samples were collected. Animals were observed daily from Day 0 to Day 5, and the body weight, health status, and survival status were recorded.

Sample processing: After lungs were collected, they were quickly frozen in the HBSS* solution at a 10-fold weight-to-volume ratio (w:v (g:mL) = 1:10), and stored in a -80°C refrigerator for plaque assay to detect viral load.

Sample detection: Viral load in lung tissues was detected by plaque assay.

Preparation of *HBSS solution: Per milliliter of Hanks Buffer stock solution, 25 µmol of HEPES, 0.21 mmol of sucrose, 5 µmol of sodium L-glutamate, and 2 µL of a triple-antibiotic mixture containing 20 units of penicillin, 20 µg of streptomycin, and 0.05 µg of amphotericin B were added.

Experimental results: On Day 5 after the first administration, the viral load in lung tissues of mice treated with the compound of the present invention was lower than that of the control compound AK0529, and the administration frequency was less than that of the control compound AK0529, indicating that the compound of the present invention has good *in vivo* antiviral effects. The exemplary compound is shown in Table 4 below.

**Table 4 Changes in pulmonary RSV viral load in mice after administration**

| **log (RSV Viral Load)** | **Vehicle Group** | **AK0529 (50 mpk, BID^{a}, 5 days administration)** | **Compound 4-11 (50 mpk, QD^{b}, 5 days administration)** | **Compound 4-11 (100 mpk, QD^{b}, administration once)** |
|---|---|---|---|---|
| Mean | 4.31 | 3.06 | 2.30° | 2.48 |

| | | | | |
|---|---|---|---|---|
| Note: a: BID indicates twice-daily administration; b: QD indicates once-daily administration; c: This value is the lower limit of detection for viral load. | | | | |

The embodiments of the present invention have been described above. However, the present invention is not limited to the above embodiments. Any modifications, equivalent substitutions, improvements, etc., made within the spirits and principles of the present invention shall be included in the protection scope of the present invention.

## Claims

1. A compound represented by Formula (I), or a tautomer, stereoisomer, isotopic derivative, or pharmaceutically acceptable salt thereof, having the following structure: 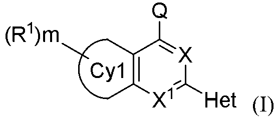 wherein,
Cy1 is independently selected from the group consisting of being absent, C₄₋₁₂ carbocyclyl, 4- to 12-membered heterocyclyl, C₆₋₁₂ aryl, and 5- to 12-membered heteroaryl; when Cy1 is absent, R¹ is connected to the heteroaromatic ring, i.e., 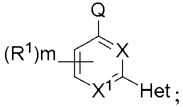
R¹ is independently selected from the group consisting of hydrogen, deuterium, halogen, oxo, hydroxyl, amino, nitro, cyano, -R^{a1}, -OR^{a1}, -SR^{a1}, -N(R^{a1})₂, -S(O)₂R^{a1}, -S(O)₂N(R^{a1})₂, -S(O)R^{a1}, -S(O)(NR^{a1})R^{a1}, - S(O)N(R^{a1})₂, -P(O)(OR^{a1})₂, -P(O)(N(R^{a1})₂)₂, -SC(R^{a1})₃, -C(R^{a1})₂OR^{a1}, -C(R^{a1})₂N(R^{a1})₂, -C(O)R^{a1}, -C(O)OR^{a1}, -C(O)N(R^{a1})₂, -C(O)OR^{a1}-OC(O)R^{a1}, -C(O)N(R^{a1})OR^{a1}, -OC(O)R^{a1}, -OC(O)N(R^{a1})₂, -N(R^{a1})C(O)OR^{a1}, - N(R^{a1})C(O)R^{a1}, -N(R^{a1})C(O)N(R^{a1})₂, -N(R^{a1})S(O)₂R^{a1}, -C(S)R^{a1}, -C(S)OR^{a1}, -C(S)N(R^{a1})₂, - C(S)N(R^{a1})OR^{a1}, -OC(S)R^{a1}, and -OC(S)N(R^{a1})₂;
R^{a1} is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{a2}; or
two R^{a1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocyclyl, C₆₋₁₄ aryl, and 5- to 12-membered heteroaryl;
R^{a2} is independently selected from the group consisting of deuterium, halogen, oxo, hydroxyl, amino, nitro, cyano, mercapto, C₁₋₆ alkyl, and C₁₋₆ alkoxy;
X¹ is independently selected from the group consisting of CR² and N;
X is independently selected from the group consisting of CR² and N;
R² is independently selected from the group consisting of hydrogen, deuterium, halogen, hydroxyl, amino, nitro, cyano, -OR^{b1}, -SR^{b1}, -N(R^{b1})₂, -S(O)₂R^{b1}, -S(O)₂N(R^{b1})₂, -S(O)R^{b1}, -S(O)(NR^{b1})R^{b1}, - S(O)N(R^{b1})₂, -P(O)(OR^{b1})₂, -P(O)(N(R^{b1})₂)₂, -C(R^{b1})₂(OR^{b1}), -C(R^{b1})₂N(R^{b1})₂, -C(O)R^{b1}, -C(O)OR^{b1}, - C(O)N(R^{b1})₂, -C(O)N(R^{b1})OR^{b1}, -OC(O)R^{b1}, -OC(O)N(R^{b1})₂, -N(R^{b1})C(O)OR^{b1}, -N(R^{b1})C(O)R^{b1}, - N(R^{b1})C(O)N(R^{b1})₂, -N(R^{b1})S(O)₂R^{b1}, C₁₋₆ alkyl, C₃₋₇ carbocyclyl, 3- to 7-membered heterocyclyl, C₆₋₁₂ aryl, and 5- to 12-membered heteroaryl, wherein each of the C₁₋₆ alkyl, the C₃₋₇ cycloalkyl, the 3- to 7-membered heterocyclyl, the C₆₋₁₂ aryl, and the 5- to 12-membered heteroaryl is optionally substituted with one or more R^{b2};
R^{b1} is independently selected from the group consisting of hydrogen, deuterium, C₁₋₆ alkyl, C₃₋₇ carbocyclyl, 4- to 7-membered heterocyclyl, phenyl, and 5- to 6-membered heteroaryl, wherein each of the C₁₋₆ alkyl, the C₃₋₇ carbocyclyl, the 4- to 7-membered heterocyclyl, the phenyl, and the 5- to 6-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl; or
two R^{b1}s connected to a same atom, together with the atom to which they are connected, form C₃₋₁₂ carbocyclyl, 3- to 12-membered heterocyclyl, or 5- to 12-membered heteroaryl, wherein each of the C₃₋₁₂ carbocyclyl, the 3- to 12-membered heterocyclyl, and the 5- to 12-membered heteroaryl is optionally substituted with one or more substituents selected from the group consisting of deuterium, halogen, oxo, hydroxyl, amino, nitro, cyano, mercapto, C₁₋₆ alkyl, C₁₋₆ alkoxy, C₃₋₁₀ cycloalkyl, 3- to 10-membered heterocycloalkyl, C₆₋₁₄ aryl, and 5- to 12-membered heteroaryl;
R^{b2} is independently selected from the group consisting of deuterium, halogen, oxo, hydroxyl, amino, nitro, cyano, mercapto, C₁₋₆ alkyl, and C₁₋₆ alkoxy;
m is independently selected from 0, 1, 2, 3, or 4;
Q is independently selected from the group consisting of deuterium, halogen, -R³, -OR³, -SR³, -N(R³)-(C₀₋₆ alkylene)-R³, -N(R³)-(C₀₋₆ alkylene)-N(R³)₂, -S(O)₂R³, -S(O)₂N(R³)₂, -S(O)R³, -S(O)(NR³)R³, - S(O)N(R³)₂, -P(O)(OR³)₂, -P(O)(N(R³)₂)₂, -C(R³)₂(OR³), -C(R³)₂N(R³)₂, -C(O)R³, -C(O)OR³, -C(O)N(R³)₂, -C(O)N(R³)OR³, -OC(O)R³, -OC(O)N(R³)₂, -N(R³)C(O)OR³, -N(R³)C(O)R³, -N(R³)C(O)N(R³)₂, and - N(R³)S(O)₂R³, wherein the alkylene is optionally substituted with one or more R^{c1};
R³ is independently selected from the group consisting of hydrogen, deuterium, C₁₋₆ alkyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₃₋₁₂ carbocyclyl, the 3- to 12-membered heterocyclyl, the C₆₋₁₂ aryl, and the 5- to 12-membered heteroaryl is optionally substituted with one or more R^{c2}; or
two R³s connected to a same nitrogen atom, together with the nitrogen atom to which they are connected, form 3- to 12-membered heterocyclyl or 5- to 12-membered heteroaryl, wherein each of the 3- to 12-membered heterocyclyl and the 5- to 12-membered heteroaryl is optionally substituted with one or more substituents selected from the group consisting of halogen, hydroxyl, amino, -C(O)NH₂, -(CH₂)₁₋₆NH₂, oxo, C₁₋₆ alkyl, C₁₋₆ alkoxy, oxetanylamino, and C₁₋₆ alkylpiperazinyl;
R^{c1} is independently selected from the group consisting of deuterium, halogen, hydroxyl, amino, nitro, cyano, mercapto, C₁₋₆ alkyl, C₁₋₆ alkoxy, C₃₋₆ cycloalkyl, 3- to 6-membered heterocyclyl, phenyl, and 5- to 6-membered heteroaryl;
Rc² is independently selected from the group consisting of deuterium, oxo, halogen, hydroxyl, amino, cyano, -OR^{c3}, -SR^{c3}, -N(R^{c3})₂, -S(O)₂R^{c3}, -S(O)₂N(R^{c3})₂, -S(O)R^{c3}, -S(O)(NR^{c3})R^{c3}, -P(O)(OR^{c3})₂, - P(O)(N(R^{c3})₂)₂, -C(R^{c3})₂(OR^{c3}), -C(R^{c3})₂N(R^{c3})₂, -C(O)R^{c3}, -C(O)OR^{c3}, -C(O)N(R^{c3})_{2,} -C(O)N(R^{c3})OR^{c3}, - OC(O)R^{c3}, -OC(O)N(R^{c3})₂, -N(R^{c3})C(O)OR^{c3}, -N(R^{c3})C(O)R^{c3}, -N(R^{c3})C(O)N(R^{c3})_{2,} - N(R^{c3})C(O)R^{c3}N(R^{c3})OC(O)R^{c3}, -N(R^{c3})S(O)₂R^{c3}, C₁₋₆ alkyl, C₃₋₆ cycloalkyl, 3- to 6-membered heterocyclyl, C₆₋₈ aryl, and 5- to 8-membered heteroaryl, wherein each of the C₁₋₆ alkyl, the C₃₋₆ cycloalkyl, the 3- to 6-membered heterocyclyl, the C₆₋₈ aryl, and the 5- to 8-membered heteroaryl is optionally substituted with one or more R^{c4};
each of R^{c3} and R^{c4} is independently selected from the group consisting of hydrogen, deuterium, oxo, halogen, hydroxyl, amino, C₁₋₁₈ alkyl, C₁₋₆ alkoxy, C₁₋₆ haloalkyl, C₁₋₆ alkyl optionally substituted with one or more hydroxyl groups, C₁₋₆ alkyl optionally substituted with one or more amino groups, C₃₋₆ cycloalkyl, 3- to 6-membered heterocyclyl, C₆₋₈ aryl, and 5- to 6-membered heteroaryl;
Het has a structure as below: 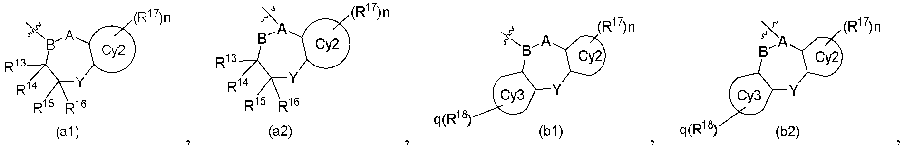 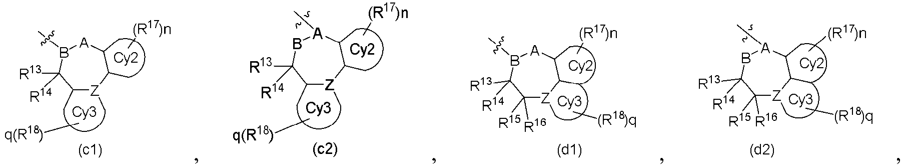 or 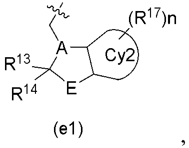
wherein,
when A or B is connected to 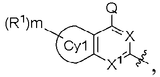 A and B are each independently selected from the group consisting of -C(R^{d1})-, -N-, 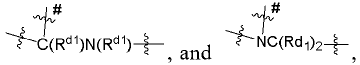 where "#" denotes the end connected to 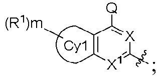
when A or B is not connected to 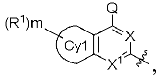 A and B are each independently selected from the group consisting of a bond, -C(R^{d1})₂-, -NR^{d1}-, -C(R^{d1})₂N(R^{d1})-, and -NR^{d1}C(R^{d1})₂-;
R^{d1} is independently selected from the group consisting of being absent, a bond, hydrogen, deuterium, hydroxyl, amino, nitro, cyano, and C₁₋₆ alkyl; or two R^{d1}s connected to a same carbon atom together form oxo;
when R^{d1} is absent, 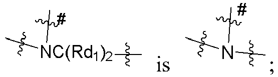
Y is independently selected from the group consisting of -O-, -N(R^{d2})-, -C(R^{d2})₂-, -S-, -C(O)-, -C(=NO(C₀₋₆ alkyl))-, -S(O)-, -S(O)₂-, and -S(O)(=N-R^{d2})-;
R^{d2} is independently selected from the group consisting of hydrogen, deuterium, halogen, hydroxyl, amino, nitro, cyano, -OR^{d3}, -SR^{d3}, -N(R^{d3})₂, -C(O)R^{d3}, -(C₁₋₆ alkylene)-C(O)R^{d3}, -(C₁₋₆ alkylene)-S(O)₂R^{d3}, - C(O)OR^{d3}, -(C₁₋₆ alkylene)-C(O)OR^{d3}, -C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, -S(O)₂N(R^{d3})₂, -S(O)R^{d3}, -C(O)N(R^{d3})OR^{d3}, -OC(O)R^{d3}, -OC(O)N(R^{d3})₂, - N(R^{d3})C(O)OR^{d3}, -N(R)^{d3}C(O)R^{d3} -N(R^{d3})C(O)N(R^{d3})₂, -N(R^{d3})S(O)₂R^{d3}, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₁₋₆ alkylene, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, nitro, cyano, C₁₋₆ alkyl, C₁₋₆ alkoxy, C₃₋₁₂ cycloalkyl, 3- to 12-membered heterocyclyl, C₆₋₁₂ aryl, and 5- to 12-membered heteroaryl;
R^{d3} is independently selected from the group consisting of hydrogen, deuterium, halogen, hydroxyl, amino, nitro, cyano, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy;
R¹³, R¹⁴, R¹⁵, and R¹⁶ are each independently selected from the group consisting of hydrogen, deuterium, oxo, halogen, hydroxyl, amino, imino, sulfinamido, nitro, cyano, C₁₋₆ alkyl, C₁₋₆ haloalkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₁₋₆ haloalkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more R^{d4}; when one of R¹³ and R¹⁴ is selected from the group consisting of oxo and imino, the other of R¹³ and R¹⁴ is absent; when one of R¹⁵ and R¹⁶ is selected from the group consisting of oxo and imino, the other of R¹⁵ and R¹⁶ is absent; or
R¹³ and R¹⁴, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy; or
R¹⁵ and R¹⁶, together with the carbon atom to which they are connected, form C₃₋₁₂ carbocyclyl or 3- to 12-membered heterocyclyl, wherein each of the C₃₋₁₂ carbocyclyl and the 3- to 12-membered heterocyclyl is optionally substituted with one or more substituents selected from the group consisting of halogen, hydroxyl, amino, cyano, C₁₋₆ alkyl, and C₁₋₆ alkoxy;
R^{d4} is independently selected from the group consisting of deuterium, halogen, oxo, hydroxyl, amino, cyano, sulfonyl, urea, C₂₋₆ alkenyl, C₂₋₆ alkynyl, C₁₋₆ alkyl, C₁₋₆ alkoxy, and C₃₋₈ cycloalkyl, wherein each of the C₁₋₆ alkyl, the C₁₋₆ alkoxy, and the C₃₋₈ cycloalkyl is optionally substituted with one or more halogen;
Cy2 is independently selected from the group consisting of phenyl, 5- to 6-membered heterocyclyl, and 5- to 6-membered heteroaryl;
Cy3 is independently selected from the group consisting of phenyl, 5- to 6-membered heterocyclyl, and 5- to 6-membered heteroaryl;
R¹⁷ and R¹⁸ are each independently selected from the group consisting of deuterium, halogen, hydroxyl, amino, cyano, nitro, carboxyl, oxo, -OR^{d5}, -SR^{d5}, -N(R^{d5})₂, -S(O)₂R^{d5}, -S(O)₂N(R^{d5})₂, -S(O)R^{d5},-S(O)(NR^{d5})R^{d5}, -S(O)N(R^{d5})₂, -P(O)(OR^{d5})₂, -P(O)(N(R^{d5})₂)₂, -C(R^{d5})₂(OR^{d5}), -C(R^{d5})₂N(R^{d5})₂, -C(O)R^{d5},-C(O)OR^{d5}, -C(O)N(R^{d5})₂, -C(O)N(R^{d5})OR^{d5}, -OC(O)R^{d5}, -OC(O)N(R^{d5})₂, -N(R^{d5})C(O)OR^{d5},-N(R^{d5})C(O)R^{d5}, -N(R^{d5})C(O)N(R^{d5})₂, -N(R^{d5})S(O)₂R^{d5}, -N(R^{d5})S(O)₂N(R^{d5}) ₂, -N(R^{d5})S(O)₂O(R^{d5}), C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, cyano, carboxyl, C₁₋₆ alkyl, C₁₋₆ haloalkyl, C₁₋₆ alkoxy, C₁₋₆ haloalkoxy, C₂₋₆ alkenyl, C₂₋₆ alkynyl, and C₃₋₈ cycloalkyl;
R^{d5} is independently selected from the group consisting of hydrogen, deuterium, halogen, hydroxyl, C₁₋₆ alkyl, C₂₋₆ alkenyl, C₂₋₆ alkynyl, C₃₋₁₂ cycloalkyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ cycloalkyl, the 3- to 12-membered heterocyclyl, the C₆₋₁₂ aryl, and the 5- to 12-membered heteroaryl is optionally substituted with one or more substituents selected from the group consisting of deuterium, halogen, hydroxyl, amino, cyano, and carboxyl;
Z is independently selected from the group consisting of C and N;
E is independently selected from the group consisting of -C(R^{d6})₂- and -N(R^{d6})-;
R^{d6} is independently selected from the group consisting of hydrogen, deuterium, halogen, hydroxyl, amino, cyano, nitro, sulfinamido, C₁₋₆ alkyl, C₂₋₆ alkenyl, C₂₋₆ alkynyl, C₃₋₁₂ carbocyclyl, 3- to 12-membered heterocyclyl, C₆₋₁₂ aryl, and 5- to 12-membered heteroaryl, wherein each of the C₁₋₆ alkyl, the C₂₋₆ alkenyl, the C₂₋₆ alkynyl, the C₃₋₁₂ carbocyclyl, the 3- to 12-membered heterocyclyl, the C₆₋₁₂ aryl, and the 5- to 12-membered heteroaryl is optionally substituted with one or more substituents selected from the group consisting of deuterium, halogen, oxo, hydroxyl, amino, carboxyl, and cyano; or two R^{d6}s connected to a same carbon atom together form oxo, thio, or imino; or two R^{d6}s connected to a same carbon atom, together with the carbon atom to which they are connected, form C₃₋₁₅ carbocyclyl or 3- to 15-membered heterocyclyl, wherein each of the C₃₋₁₅ carbocyclyl and the 3- to 15-membered heterocyclyl is optionally substituted with one or more substituents selected from the group consisting of deuterium, halogen, oxo, hydroxyl, amino, carboxyl, and cyano;
n and q are each independently selected from 0, 1, 2, 3, or 4;
when a plurality of R^{a1}s, a plurality of R^{a2}s, a plurality of R^{b1}s, a plurality of R^{b2}s, a plurality of R^{c1}s, a plurality of R^{c2}s, a plurality of R^{c3}s, a plurality of R^{c4}s, a plurality of R^{d1}s, a plurality of R^{d2}s, a plurality of R^{d3}s, a plurality of R^{d4}s, a plurality of R^{d5}s, a plurality of R^{d6}s, a plurality of R¹s, a plurality of R³s, a plurality of R¹⁷s, or a plurality of R¹⁸s are present simultaneously, each R^{a1}, each R^{a2}, each R^{b1}, each R^{b2}, each R^{c1}, each R^{c2}, each R^{c3}, each R^{d1}, each R^{d2}, each R^{d3}, each R^{d4}, each R^{d5}, each R^{d6}, each R¹, each R³, each R¹⁷, or each R¹⁸ may be the same or different.

2. The compound, or the tautomer, stereoisomer, isotopic derivative, or pharmaceutically acceptable salt thereof according to claim 1, **characterised in that** Cy1 is independently selected from the group consisting of 4- to 6-membered heterocyclyl, phenyl, and 5- to 6-membered heteroaryl;
preferably, Cy1 is independently selected from the group consisting of 4- to 12-membered heterocyclyl and C₅₋₁₂ aryl;
preferably, Cy1 is independently selected from the group consisting of 5- to 6-membered heterocyclyl and phenyl;
preferably, Cy1 is independently selected from phenyl;
preferably, Cy1 is independently selected from 5- to 6-membered heterocyclyl, wherein a heteroatom in the heterocyclyl is selected from the group consisting of N and S;
preferably, Cy1 is selected from the group consisting of the following groups: 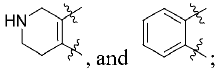
preferably, 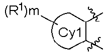 is selected from the group consisting of the following groups: 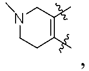 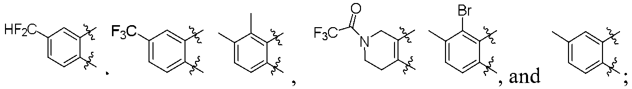
preferably, 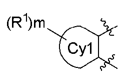 is selected from the group consisting of the following groups: 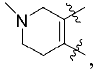 and

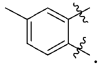

3. The compound, or the tautomer, stereoisomer, isotopic derivative, or pharmaceutically acceptable salt thereof according to any one of claims 1 and 2, **characterised in that** R¹ is independently selected from the group consisting of hydrogen, halogen, oxo, hydroxyl, amino, nitro, cyano, -R^{a1}, -OR^{a1}, -SR^{a1}, -N(R^{a1})₂,-S(O)₂R^{a1}, -S(O)₂N(R^{a1})₂, -C(O)R^{a1}, -C(O)OR^{a1}, -C(O)N(R^{a1})₂, -C(O)OR^{a1}-OC(O)R^{a1}, and -N(R^{a1})C(O)R^{a1};
preferably, R¹ is independently selected from the group consisting of hydrogen, halogen, oxo, hydroxyl, amino, cyano, -R^{a1}, -C(O)R^{a1}, and -C(O)ORa¹;
preferably, R^{a1} is independently selected from the group consisting of hydrogen, C₁₋₆ alkyl, and C₃₋₆ cycloalkyl, wherein each of the C₁₋₆ alkyl and the C₃₋₆ cycloalkyl is optionally substituted with one or more R^{a2};
preferably, R^{a2} is independently selected from the group consisting of deuterium, halogen, oxo, hydroxyl, amino, nitro, cyano, mercapto, C₁₋₄ alkyl, and C₁₋₄ alkoxy;
preferably, R^{a2} is independently selected from the group consisting of halogen (e.g., fluorine, chlorine, bromine) and hydroxyl;
preferably, R^{a2} is independently selected from the group consisting of fluorine and hydroxyl;
preferably, R^{a2} is independently selected from fluorine;
preferably, R¹ is independently selected from the group consisting of hydrogen, fluorine, chlorine, bromine, oxo, hydroxyl, amino, -R^{a1}, -OR^{a1}, -S(O)₂R^{a1}, -C(O)R^{a1}, -C(O)OR^{a1}, -C(O)N(R^{a1})₂, and -C(O)OR^{a1}-OC(O)R^{a1};
R^{a1} is independently selected from the group consisting of hydrogen, C₁₋₆ alkyl, and C₃₋₆ cycloalkyl, wherein each of the C₁₋₆ alkyl and the C₃₋₆ cycloalkyl is optionally substituted with one or more R^{a2};
R^{a2} is independently selected from the group consisting of fluorine, chlorine, bromine, oxo, hydroxyl, and amino;
preferably, R¹ is independently selected from the group consisting of hydrogen, halogen, oxo, C₁₋₄ alkyl, C₁₋₄ alkoxy, C₃₋₆ cycloalkyl, C₁₋₄ haloalkyl, C₁₋₄ alkyl optionally substituted with one or more hydroxyl groups, -S(O)₂(C₁₋₄ alkyl), -C(O)(C₁₋₄ alkyl), -C(O)(C₁₋₄ haloalkyl), -C(O)O(C₁₋₄ alkyl), -C(O)NH(C₁₋₄ alkyl), and-C(O)O-(C₁₋₄ alkyl)-OC(O)(C₁₋₄ alkyl);
preferably, R¹ is independently selected from the group consisting of C₁₋₂ alkyl and C₁₋₂ haloalkyl;
preferably, R¹ is independently selected from the group consisting of hydrogen, fluorine, chlorine, oxo, methyl, ethyl, n-propyl, isopropyl, n-butyl, methoxy, ethoxy, n-propoxy, isopropoxy, cyclopropyl, cyclobutyl, cyclopentyl, cyclohexyl, -CH₂F, -CF₂H, -CF₃, -C(O)CH₃, -C(O)CH₂CH₃, -S(O)₂CH₃, -CH₂OH, -C(O)CF₃,-C(O)CF₂H, -C(O)OCH₃, -C(O)OCH₂CH₃, -C(O)NHCH₃, and -C(O)O-CH(CH₃)-OC(O)CH(CH₃)₂;
preferably, R¹ is independently selected from the group consisting of methyl and -CF₂H;
preferably, R¹ is independently selected from C₁₋₄ alkyl;
preferably, R¹ is independently selected from methyl;
preferably, m is independently selected from 0, 1, 2, or 3;
preferably, m is independently selected from 0, 1, or 2;
preferably, m is independently selected from 1.

4. The compound, or the tautomer, stereoisomer, isotopic derivative, or pharmaceutically acceptable salt thereof according to any one of claims 1 to 3, **characterised in that** X¹ is selected from N, and X is selected from N;
preferably, X¹ is selected from N;
preferably, X is selected from N;
preferably, X is selected from CR²;
preferably, R² is independently selected from the group consisting of halogen (e.g., fluorine, chlorine, bromine, preferably fluorine) and cyano;
preferably, X is selected from CR²; and R² is independently selected from the group consisting of fluorine, cyano, and methyl.

5. The compound, or the tautomer, stereoisomer, isotopic derivative, or pharmaceutically acceptable salt thereof according to any one of claims 1 to 4, **characterised in that** Q is independently selected from the group consisting of -R³, -N(R³)-(C₀₋₆ alkylene)-R³, -N(R³)-(C₀₋₆ alkylene)-N(R³)₂, -N(R³)C(O)OR³, -N(R³)C(O)R³, -N(R³)C(O)N(R³)₂, and -N(R³)S(O)₂R³, wherein the alkylene is optionally substituted with one or more R^{c1};
preferably, Q is independently selected from the group consisting of -R³, -N(R³)-(C₀₋₆ alkylene)-R³, and -N(R³)-(C₀₋₆ alkylene)-N(R³)₂, wherein the alkylene is optionally substituted with one or more R^{c1};
preferably, Q is independently selected from the group consisting of -R³, -NH-(C₀₋₄ alkylene)-R³, and-NH-(C₀₋₄ alkylene)-NHR³, wherein the alkylene is optionally substituted with one or more R^{c1};
preferably, R³ is independently selected from the group consisting of hydrogen, C₁₋₆ alkyl, C₃₋₇ carbocyclyl, 3- to 7-membered heterocyclyl, phenyl, and 5- to 6-membered heteroaryl, wherein each of the C₁₋₆ alkyl, the C₃₋₇ carbocyclyl, the 3- to 7-membered heterocyclyl, the phenyl, and the 5- to 6-membered heteroaryl is optionally substituted with one or more R^{c2}; or
two R³s connected to a same nitrogen atom, together with the nitrogen atom to which they are connected, form 3- to 7-membered heterocyclyl or 5- to 6-membered heteroaryl, wherein each of the 3- to 7-membered heterocyclyl and the 5- to 6-membered heteroaryl is optionally substituted with one or more substituents selected from the group consisting of halogen, hydroxyl, amino, -C(O)NH₂, -(CH₂)₁₋₄NH₂, oxo, C₁₋₄ alkyl, C₁₋₄ alkoxy, oxetanylamino, and C₁₋₄ alkylpiperazinyl;
preferably, R³ is independently selected from the group consisting of hydrogen, C₁₋₆ alkyl, C₃₋₁₂ carbocyclyl, and 3- to 12-membered heterocyclyl, wherein each of the C₁₋₆ alkyl, the C₃₋₁₂ carbocyclyl, and the 3- to 12-membered heterocyclyl is substituted with one or more R^{c2};
preferably, R³ is independently selected from the group consisting of hydrogen, C₁₋₆ alkyl, C₃₋₁₀ carbocyclyl, and 3- to 10-membered heterocyclyl, wherein each of the C₁₋₆ alkyl, the C₃₋₁₀ carbocyclyl, and the 3- to 10-membered heterocyclyl is substituted with one or more R^{c2};
preferably, R³ is independently selected from the group consisting of hydrogen, C₁₋₄ alkyl, C₃₋₆ cycloalkyl, 3- to 6-membered heterocycloalkyl, and 6- to 9-membered heterospirocyclyl, wherein each of the C₁₋₄ alkyl, the C₃₋₆ cycloalkyl, the 3- to 6-membered heterocycloalkyl, and the 6- to 9-membered heterospirocyclyl is optionally substituted with one or more R^{c2};
preferably, R³ is independently selected from the group consisting of hydrogen, C₁₋₄ alkyl, C₄₋₆ cycloalkyl, 4- to 6-membered heterocycloalkyl, and 7-membered heterospirocyclyl, wherein the heteroatom in the 4- to 6-membered heterocycloalkyl and the 7-membered heterospirocyclyl is N or O, and the number of the heteroatom is 1 or 2; and each of the C₁₋₄ alkyl, the C₄₋₆ cycloalkyl, the 4- to 6-membered heterocycloalkyl, and the 7-membered heterospirocyclyl is optionally substituted with one or more R^{c2};
preferably, R³ is independently selected from the group consisting of hydrogen, C₁₋₄ alkyl, 4- to 6-membered heterocycloalkyl, and 7-membered heterospirocycloalkyl, wherein the heteroatom in the 4- to 6-membered heterocycloalkyl and 7-membered heterospirocycloalkyl is N or O, and the number of the heteroatom is 1; and each of the C₁₋₄ alkyl, the 4- to 6-membered heterocycloalkyl, and the 7-membered heterospirocycloalkyl is optionally substituted with one or more R^{c2};
preferably, R³ is independently selected from the group consisting of hydrogen, methyl, ethyl, n-propyl, isopropyl, 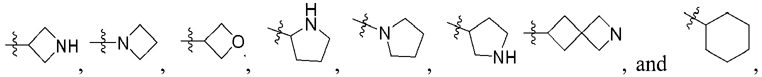 and each of the aforementioned groups is optionally substituted with one or more R^{c2};
preferably, R^{c1} is independently selected from the group consisting of deuterium, halogen, hydroxyl, amino, nitro, cyano, mercapto, C₁₋₄ alkyl, and C₁₋₄ alkoxy;
preferably, R^{c1} is independently selected from the group consisting of fluorine, chlorine, bromine, hydroxyl, amino, methyl, ethyl, n-propyl, and isopropyl;
preferably, R^{c1} is independently selected from fluorine;
preferably, R^{c2} is independently selected from the group consisting of deuterium, oxo, halogen, hydroxyl, amino, -OR^{c3}, -N(R^{c3})₂, -C(R^{c3})₂(OR^{c3}), -C(R^{c3})₂N(R^{c3})₂, -C(O)R^{c3}, -C(O)OR^{c3}, -C(O)N(R^{c3})₂,-C(O)N(R^{c3})OR^{c3}, -OC(O)R^{c3}, -OC(O)N(R^{c3})₂, -N(R^{c3})C(O)OR^{c3}, -N(R^{c3})C(O)R^{c3}, -N(R^{c3})C(O)N(R^{c3})₂,-N(R^{c3})S(O)₂R^{c3}, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more R^{c4};
preferably, R^{c2} is independently selected from the group consisting of halogen, hydroxyl, amino, and C₁₋₄ alkyl, wherein the C₁₋₄ alkyl is optionally substituted with one or more R^{c4};
preferably, R^{c2} is independently selected from the group consisting of halogen (e.g., fluorine, chlorine, bromine), hydroxyl, amino, methyl, ethyl, n-propyl, and isopropyl, each of the aforementioned groups is optionally substituted with one or more substituents selected from the group consisting of halogen (e.g., fluorine, chlorine, bromine), hydroxyl, and amino;
preferably, each of R^{c3} and R^{c4} is independently selected from the group consisting of hydrogen, halogen, hydroxyl, amino, C₁₋₆ alkyl, C₁₋₆ haloalkyl, and C₁₋₆ alkyl optionally substituted with one or more hydroxyl groups;
preferably, R^{c3} is independently selected from the group consisting of hydrogen, methyl, ethyl, n-propyl, and isopropyl;
preferably, R^{c4} is independently selected from the group consisting of halogen, hydroxyl, and amino;
preferably, R^{c4} is independently selected from the group consisting of hydroxyl and amino;
preferably, Q is independently selected from the group consisting of -R³, -N(R³)-(C₀₋₆ alkylene)-R³, and -N(R³)-(C₀₋₆ alkylene)-N(R³)₂, wherein the alkylene is optionally substituted with one or more substituents selected from the group consisting of halogen, hydroxyl, amino, and C₁₋₆ alkyl;
R³ is independently selected from the group consisting of hydrogen, C₁₋₆ alkyl, C₃₋₁₀ carbocyclyl, and 3- to 10-membered heterocyclyl, wherein each of the C₁₋₆ alkyl, the C₃₋₁₀ carbocyclyl, and the 3- to 10-membered heterocyclyl is optionally substituted with one or more R^{c2};
R^{c2} is independently selected from the group consisting of halogen, hydroxyl, amino, and C₁₋₆ alkyl, wherein the C₁₋₆ alkyl is optionally substituted with one or more substituents selected from the group consisting of halogen, hydroxyl, and amino;
preferably, Q is independently selected from the group consisting of C₃₋₆ cycloalkyl, 3- to 6-membered heterocyclyl, -NH-(C₀₋₆ alkylene)-R³, and -NH-(C₀₋₆ alkylene)-N(R³)₂, wherein the alkylene is optionally substituted with one or more substituents selected from the group consisting of halogen, hydroxyl, amino, methyl, and ethyl;
R³ is independently selected from the group consisting of hydrogen, C₁₋₆ alkyl, C₃₋₆ cycloalkyl, 3- to 6-membered heterocyclyl, and 6- to 9-membered heterospirocyclyl, wherein each of the C₁₋₆ alkyl, the C₃₋₆ cycloalkyl, the 3- to 6-membered heterocyclyl, and the 6- to 9-membered heterospirocyclyl is optionally substituted with one or more R^{c2};
R^{c2} is independently selected from the group consisting of halogen, hydroxyl, amino, C₁₋₆ alkyl, amino-substituted C₁₋₆ alkyl, and hydroxyl-substituted C₁₋₆ alkyl;
preferably, Q is independently selected from the group consisting of C₃₋₆ cycloalkyl, 3- to 6-membered heterocyclyl, -NH-(C₀₋₄ alkylene)-(C₃₋₆ cycloalkyl), -NH-(C₀₋₄ alkylene)-(3- to 6-membered heterocyclyl),-NH-(C₀₋₄ alkylene)-(7-membered heterospirocyclyl), -NH-(C₀₋₄ alkylene)-NH₂, -NH-(C₀₋₄ alkylene)-NH(C₁₋₄ alkyl), and -NH-(C₀₋₄ alkylene)-N(C₁₋₄ alkyl)₂, wherein the alkylene is optionally substituted with one or more substituents selected from halogen (e.g., fluorine, chlorine, bromine); and each of the C₁₋₄ alkyl, the C₃₋₆ cycloalkyl, the 3- to 6-membered heterocyclyl, and the 7-membered heterospirocyclyl is optionally substituted with one or more substituents selected from the group consisting of halogen (e.g., fluorine, chlorine, bromine), hydroxyl, amino, C₁₋₄ alkyl, amino-substituted C₁₋₄ alkyl, and hydroxyl-substituted C₁₋₄ alkyl;
preferably, Q is independently selected from the group consisting of 3- to 10-membered nitrogen-containing heterocyclyl (preferably 3- to 8-membered nitrogen-containing heterocyclyl), -NH-(C₀₋₂ alkylene)-(C₃₋₆ cycloalkyl)-NH₂, and -NH-(C₀₋₂ alkylene)-(3- to 6-membered heterocyclyl); the 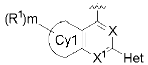 is connected to a nitrogen atom in the 3- to 10-membered nitrogen-containing heterocyclyl, where " 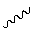 " denotes a connecting site; the heteroatom in the 3- to 6-membered heterocyclyl is N or O, and the number of the heteroatom is 1 or 2; and each of the 3- to 10-membered nitrogen-containing heterocyclyl, the C₃₋₆ cycloalkyl, and the 3- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl);
preferably, Q is independently selected from the group consisting of 3- to 7-membered nitrogen-containing heterocyclyl, -NH-(C₀₋₂ alkylene)-(C₃₋₆ cycloalkyl)-NH₂, and -NH-(C₀₋₂ alkylene)-(3- to 6-membered heterocyclyl); the 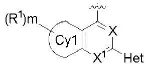 is connected to a nitrogen atom in the 3- to 7-membered nitrogen-containing heterocyclyl, where " 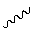 " denotes a connecting site; the heteroatom in the 3- to 6-membered heterocyclyl is N or O, and the number of the heteroatom is 1 or 2; and each of the 3- to 7-membered nitrogen-containing heterocyclyl, the C₃₋₆ cycloalkyl, and the 3- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl);
preferably, Q is independently selected from the group consisting of 4- to 6-membered nitrogen-containing heterocyclyl, -NH-(C₄₋₅ cycloalkyl)-NH₂, and -NH-(C₀₋₂ alkylene)-(4- to 6-membered heterocyclyl); the 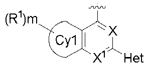 is connected to a nitrogen atom in the 4- to 6-membered nitrogen-containing heterocyclyl, where " 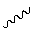 " denotes a connecting site; the heteroatom in the 4- to 6-membered heterocyclyl is N or O, and the number of the heteroatom is 1 or 2; and each of the 4- to 6-membered nitrogen-containing heterocyclyl and the 4- to 6-membered heterocyclyl is optionally substituted with one or more substituents selected from the group consisting of halogen (e.g., fluorine, chlorine, bromine), hydroxyl, amino, -(C₁₋₂ alkyl)-NH₂, -(C₁₋₂ alkyl)-OH, and -C(O)O(C₁₋₂ alkyl);
preferably, Q is independently selected from the group consisting of 4- to 6-membered nitrogen-containing heterocyclyl, -NH-(C₄₋₅ cycloalkyl)-NH₂, and -NH-(C₀₋₂ alkylene)-(4- to 6-membered heterocyclyl); the 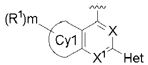 is connected to a nitrogen atom in the 4- to 6-membered nitrogen-containing heterocyclyl, where " 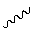 " denotes a connecting site; the heteroatom in the 4- to 6-membered heterocyclyl is N or O, and the number of the heteroatom is 1; and each of the 4- to 6-membered nitrogen-containing heterocyclyl and the 4- to 6-membered heterocyclyl is optionally substituted with one or more substituents selected from the group consisting of fluorine, chlorine, hydroxyl, amino, -CH₂NH₂, -CH₂OH, and -C(O)OCH₃;
preferably, Q is selected from the group consisting of the following groups: 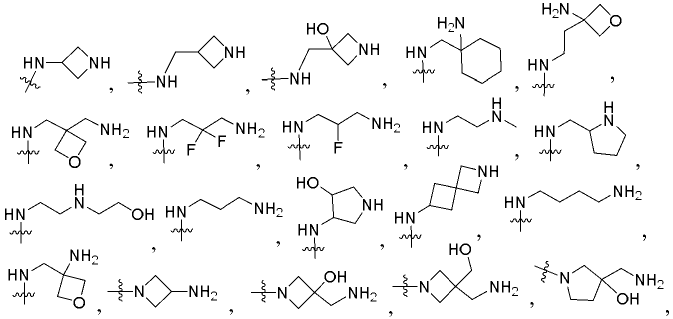 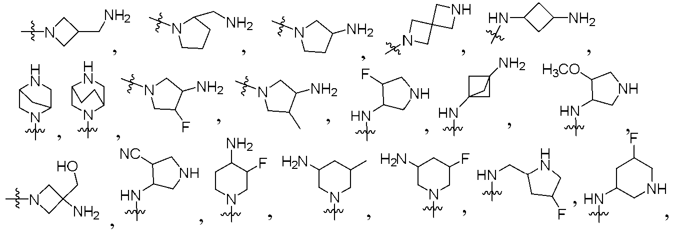 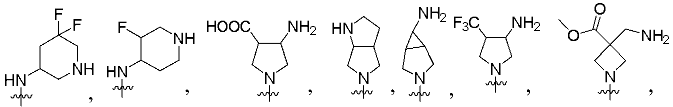 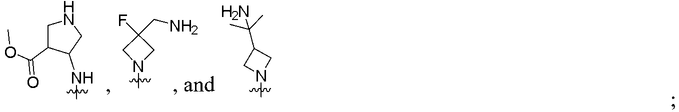
preferably, Q is selected from the group consisting of

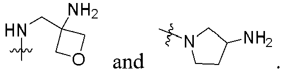

6. The compound, or the tautomer, stereoisomer, isotopic derivative, or pharmaceutically acceptable salt thereof according to any one of claims 1 to 5, **characterised in that** Het is selected from 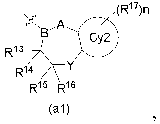 wherein B is -N-; and A is -CH₂-;
preferably, Het is selected from the following structure: 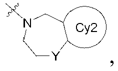 wherein Cy2 is selected from the group consisting of phenyl and pyridyl;
preferably, Cy2 is independently selected from the group consisting of phenyl and 6-membered heteroaryl;
preferably, Cy2 is independently selected from the group consisting of phenyl and pyridyl;
preferably, Cy2 is independently selected from the group consisting of phenyl, 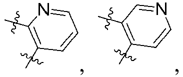 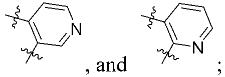
preferably, Cy2 is independently selected from phenyl;
preferably, R¹³, R¹⁴, R¹⁵ and R¹⁶ are each independently selected from hydrogen;
preferably, R¹⁷ is independently selected from the group consisting of fluorine and chlorine;
preferably, n and q are each independently selected from 0 or 1;
preferably, n and q are each independently selected from 0.

7. The compound, or the tautomer, stereoisomer, isotopic derivative, or pharmaceutically acceptable salt thereof according to any one of claims 1 to 6, **characterised in that** Y is independently selected from the group consisting of -O-, -N(R^{d2})-, -S(O)-, -S(O)₂-, and -S(O)(=N-R^{d2})-;
preferably, Y is independently selected from the group consisting of -S(O)-, -S(O)₂-, and -S(O)(=N-R^{d2})-;
preferably, Y is independently selected from the group consisting of -S(O)₂- and -S(O)(=N-R^{d2})-;
preferably, Y is independently selected from -S(O)₂-;
preferably, R^{d2} is independently selected from the group consisting of hydrogen, deuterium, halogen, hydroxyl, amino, nitro, cyano, -SR^{d3}, -N(R^{d3})₂, -C(O)R^{d3}, -(C₁₋₆ alkylene)-C(O)R^{d3}, -C(O)OR^{d3}, -(C₁₋₆ alkylene)-C(O)OR^{d3}, -C(O)N(R^{d3})₂, -(C₁₋₆ alkylene)-C(O)N(R^{d3})₂, -C(O)-(C₁₋₆ alkylene)-N(R^{d3})₂, -S(O)₂R^{d3}, -C(O)N(R^{d3})OR^{d3}, -OC(O)R^{d3}, -OC(O)N(R^{d3})₂, C₁₋₆ alkyl, C₃₋₁₀ carbocyclyl, 3- to 10-membered heterocyclyl, C₆₋₈ aryl, and 5- to 10-membered heteroaryl, wherein each of the C₁₋₆ alkyl, the C₁₋₆ alkylene, the C₃₋₁₀ carbocyclyl, the 3- to 10-membered heterocyclyl, the C₆₋₈ aryl, and the 5- to 10-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy;
preferably, R^{d2} is independently selected from the group consisting of hydrogen, deuterium, halogen, hydroxyl, amino, nitro, cyano, -SR^{d3}, -N(R^{d3})₂, -C(O)R^{d3}, -(C₁₋₄ alkylene)-C(O)R^{d3}, -C(O)OR^{d3}, -(C₁₋₄ alkylene)-C(O)OR^{d3}, -C(O)N(R^{d3})₂, -(C₁₋₄ alkylene)-C(O)N(R^{d3})₂, -C(O)-(C₁₋₄ alkylene)-N(R^{d3})₂, -S(O)₂R^{d3}, C₁₋₄ alkyl, C₃₋₆ carbocyclyl, 3- to 6-membered heterocyclyl, phenyl, and 5- to 6-membered heteroaryl, wherein each of the C₁₋₄ alkyl, the C₁₋₄ alkylene, the C₃₋₆ carbocyclyl, the 3- to 6-membered heterocyclyl, the phenyl, and the 5- to 6-membered heteroaryl is optionally substituted with one or more substituents selected from the group consisting of deuterium, halogen, oxo, hydroxyl, amino, nitro, cyano, C₁₋₄ alkyl, and C₁₋₄ alkoxy;
preferably, R^{d2} is independently selected from the group consisting of hydrogen, -SR^{d3}, -C(O)R^{d3}, -(C₁₋₄ alkylene)-C(O)R^{d3}, -(C₁₋₄ alkylene)-C(O)OR^{d3}, -C(O)N(R^{d3})₂, -(C₁₋₄ alkylene)-C(O)N(R^{d3})₂, -C(O)-(C₁₋₄ alkylene)-N(R^{d3})₂, -S(O)₂R^{d3}, C₁₋₄ alkyl, C₃₋₆ cycloalkyl, 3- to 6-membered heterocyclyl, phenyl, and 5- to 6-membered heteroaryl, wherein the heteroatom in the 3- to 6-membered heterocyclyl and the 5- to 6-membered heteroaryl is N or O, and the number of the heteroatom is 1 or 2; and each of the C₁₋₄ alkyl, the C₁₋₄ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen (e.g., fluorine, chlorine, bromine, preferably fluorine), oxo, hydroxyl, amino, cyano, methyl, ethyl, n-propyl, isopropyl, n-butyl, methoxy, ethoxy, n-propoxy, and isopropoxy;
preferably, R^{d3} is independently selected from the group consisting of hydrogen, deuterium, halogen, hydroxyl, amino, nitro, cyano, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen, oxo, hydroxyl, amino, nitro, cyano, C₁₋₆ alkyl, and C₁₋₆ alkoxy;
preferably, R^{d3} is independently selected from the group consisting of hydrogen, halogen, hydroxyl, amino, nitro, cyano, C₁₋₄ alkyl, C₃₋₆ cycloalkyl, and 3- to 6-membered heterocyclyl, wherein each of the C₁₋₄ alkyl, the C₃₋₆ cycloalkyl, and the 3- to 6-membered heterocyclyl is optionally substituted with one or more substituents selected from the group consisting of halogen, oxo, hydroxyl, amino, nitro, cyano, C₁₋₄ alkyl, and C₁₋₄ alkoxy;
preferably, R^{d3} is independently selected from the group consisting of hydrogen, fluorine, chlorine, bromine, hydroxyl, amino, cyano, methyl, ethyl, n-propyl, isopropyl, n-butyl, cyclopropyl, cyclobutyl, cyclopentyl, and cyclobutyl, and each of the aforementioned groups is optionally substituted with one or more substituents selected from the group consisting of fluorine, chlorine, bromine, hydroxyl, and amino;
preferably, R^{d2} is independently selected from the group consisting of hydrogen, -SR^{d3}, -C(O)R^{d3}, -(C₁₋₆ alkylene)-C(O)R^{d3}, -C(O)OR^{d3}, -(C₁₋₆ alkylene)-C(O)OR^{d3}, -C(O)N(R^{d3})₂, -(C₁₋₆ alkylene)-C(O)N(R^{d3})₂,-C(O)-(C₁₋₆ alkylene)-N(R^{d3})₂, -S(O)₂R^{d3}, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₁₋₆ alkylene, the C₃₋₆ cycloalkyl, the 3-to 6-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, and C₁₋₆ alkoxy;
R^{d3} is independently selected from the group consisting of hydrogen, halogen, hydroxyl, amino, cyano, C₁₋₆ alkyl, C₃₋₆ cycloalkyl, 3- to 6-membered heterocyclyl, phenyl, and 5- to 6-membered heteroaryl, wherein each of the C₁₋₆ alkyl, the C₃₋₆ cycloalkyl, the 3- to 6-membered heterocyclyl, the phenyl, and the 5- to 6-membered heteroaryl is optionally substituted with one or more substituents selected from the group consisting of deuterium, halogen, hydroxyl, amino, cyano, C₁₋₆ alkyl, and C₁₋₆ alkoxy;
preferably, R^{d2} is independently selected from the group consisting of hydrogen, -S(C₁₋₄ alkyl),-C(O)NH₂, -C(O)NH(C₁₋₄ alkyl), -C(O)N(C₁₋₄ alkyl)₂, -C(O)(C₁₋₄ alkyl), -C(O)(C₃₋₆ cycloalkyl), -C(O)(3- to 6-membered heterocyclyl), -C(O)-(C₁₋₄ alkylene)-NH(C₁₋₄ alkyl), -C(O)-(C₁₋₄ alkylene)-N(C₁₋₄ alkyl)₂, -(C₁₋₄ alkylene)-C(O)O(C₁₋₄ alkyl), -(C₁₋₄ alkylene)-C(O)(C₁₋₄ alkyl), -(C₁₋₄ alkylene)-C(O)NH₂, -(C₁₋₄ alkylene)-C(O)NH(C₁₋₄ alkyl), -(C₁₋₄ alkylene)-C(O)N(C₁₋₄ alkyl)₂, -S(O)₂(C₁₋₄ alkyl), C₁₋₄ alkyl, C₃₋₆ cycloalkyl, 3- to 6-membered heterocyclyl, phenyl, and 5- to 6-membered heteroaryl, wherein each of the C₁₋₄ alkyl, the C₁₋₄ alkylene, the C₃₋₆ cycloalkyl, the 3- to 6-membered heterocyclyl, the phenyl, and the 5- to 6-membered heteroaryl is optionally substituted with one or more substituents selected from the group consisting of deuterium, fluorine, chlorine, bromine, hydroxyl, amino, cyano, methyl, ethyl, n-propyl, isopropyl, and n-butyl;
preferably, R^{d2} is independently selected from the group consisting of hydrogen, -C(O)NH₂,-C(O)NH(C₁₋₃ alkyl), -C(O)(C₁₋₃ alkyl), -C(O)(C₃₋₆ cycloalkyl), -(C₁₋₃ alkylene)-S(O)₂(C₁₋₃ alkyl), C₁₋₆ alkyl (more preferably C₁₋₅ alkyl, more preferably C₁₋₃ alkyl), C₃₋₆ cycloalkyl (more preferably C₃₋₄ cycloalkyl), 3-to 7-membered heterocyclyl (more preferably 4- to 5-membered heterocyclyl), and 5- to 6-membered heteroaryl (more preferably 5-membered heteroaryl containing 2 nitrogen atoms), wherein each of the alkyl, the cycloalkyl, the heterocyclyl, and the heteroaryl is optionally substituted with one or more substituents selected from the group consisting of deuterium, halogen (more preferably fluorine, chlorine, bromine, more preferably fluorine), hydroxyl, amino, cyano, C₁₋₃ alkyl (more preferably methyl), C₁₋₃ alkoxy (more preferably methoxy), and 3- to 6-membered heterocyclyl (more preferably 4- to 5-membered oxygen-containing heterocyclyl); and the heteroatom in the heterocyclyl and the heteroaryl is O or N, and the number of the heteroatom is 1 or 2;
preferably, R^{d2} is independently selected from the group consisting of hydrogen, -C(O)NH₂,-C(O)NH(C₁₋₂ alkyl), -C(O)(C₁₋₂ alkyl), -C(O)(C₃₋₄ cycloalkyl), -(C₁₋₂ alkylene)-S(O)₂(C₁₋₂ alkyl), C₁₋₆ alkyl (more preferably C₁₋₅ alkyl, more preferably C₁₋₃ alkyl), -(C₁₋₃ alkylene)-(C₁₋₂ alkoxy), -(C₁₋₂ alkylene)-(3- to 6-membered heterocyclyl) (more preferably -(C₁₋₂ alkylene)-(4- to 5-membered heterocyclyl)), C₃₋₆ cycloalkyl (more preferably C₃₋₄ cycloalkyl), 4- to 6-membered heterocyclyl (more preferably 4- to 5-membered heterocyclyl), and 5- to 6-membered heteroaryl (more preferably 5-membered heteroaryl containing 2 nitrogen atoms), wherein the alkyl is optionally substituted with one or more substituents selected from the group consisting of deuterium, halogen (more preferably fluorine, chlorine, bromine, more preferably fluorine), hydroxyl, and cyano; the cycloalkyl is optionally substituted with one or more substituents selected from halogen (more preferably fluorine, chlorine, bromine, more preferably fluorine); the heteroaryl is optionally substituted with one or more substituents selected from C₁₋₂ alkyl (preferably methyl); and the heteroatom in the heterocyclyl and the heteroaryl is O or N, and the number of the heteroatom is 1 or 2;
preferably, R^{d2} is independently selected from the group consisting of -(C₁₋₃ alkylene)-S(O)₂(C₁₋₃ alkyl), C₁₋₆ alkyl (more preferably C₁₋₅ alkyl, more preferably C₁₋₃ alkyl), C₃₋₆ cycloalkyl (more preferably C₃₋₄ cycloalkyl), and 3- to 6-membered heterocyclyl (more preferably 4- to 5-membered heterocyclyl), wherein each of the alkyl, the cycloalkyl, and the heterocyclyl is optionally substituted with one or more substituents selected from the group consisting of deuterium, halogen (more preferably fluorine, chlorine, bromine, more preferably fluorine), hydroxyl, cyano, C₁₋₃ alkyl (more preferably methyl), C₁₋₃ alkoxy (more preferably methoxy), and 3- to 6-membered heterocyclyl (more preferably 4- to 5-membered heterocyclyl); and the heteroatom in the heterocyclyl is O, and the number of the heteroatom is 1;
preferably, R^{d2} is independently selected from the group consisting of -(C₁₋₂ alkylene)-S(O)₂(C₁₋₂ alkyl), C₁₋₆ alkyl (more preferably C₁₋₅ alkyl, more preferably C₁₋₃ alkyl), -(C₁₋₃ alkylene)-(C₁₋₂ alkoxy), -(C₁₋₂ alkylene)-(3- to 6-membered heterocyclyl) (more preferably -(C₁₋₂ alkylene)-(4- to 5-membered heterocyclyl)), C₃₋₆ cycloalkyl (more preferably C₃₋₄ cycloalkyl), and 4- to 6-membered heterocyclyl (more preferably 4- to 5-membered heterocyclyl), wherein the alkyl is optionally substituted with one or more substituents selected from the group consisting of deuterium, halogen (more preferably fluorine, chlorine, bromine; more preferably fluorine), hydroxyl, and cyano; and the heteroatom in the heterocyclyl is O, and the number of the heteroatom is 1;
preferably, R^{d2} is independently selected from the group consisting of -(C₁₋₂ alkylene)-(3- to 6-membered heterocyclyl) (more preferably -(C₁₋₂ alkylene)-(4- to 5-membered heterocyclyl)), C₃₋₆ cycloalkyl (more preferably C₃₋₄ cycloalkyl), and 3- to 6-membered heterocyclyl (more preferably 4- to 5-membered heterocyclyl), wherein the heteroatom in the heterocyclyl is O, and the number of the heteroatom is 1;
preferably, R^{d2} is independently selected from the group consisting of hydrogen, -C(O)NH₂, -CD₃, -CF₃, -SCF₃, -C(O)CH₃, -C(O)CH₂OH, -C(O)CH₂N(CH₃)₂, -CH₂CN, -CH₂CH₂CH₂CN, -CH₂CH₂C(CH₃)₂OH,-CH₂CH₂CH₂OH, -CH(CH₃)CH₂OCH₃, -CH₂CH₂CH₂OCH₃, -CH₂-C(O)OCH₃, -C(O)NHCH₃, -CH₂-C(O)NH₂, -S(O)₂CH₃, -C(O)N(CH₃)₂, -CH₂CF₃, -CH₂CH₂F, -CH₂CHF₂, -CH₂CH₂SO₂CH₃, methyl, ethyl, n-propyl, isopropyl, n-butyl, cyclopropyl, cyclobutyl, cyclopentyl, cyclohexyl, phenyl,

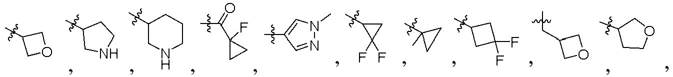

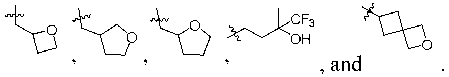

8. The compound, or the tautomer, stereoisomer, isotopic derivative, or pharmaceutically acceptable salt thereof according to any one of claims 1 to 7, **characterised in that** the compound has a structure represented by Formula (II), Formula (II-1), Formula (III), Formula (III-1), Formula (IV), Formula (IV-a), Formula (IV-1), Formula (IV-2), Formula (IV-3), Formula (IV-4), Formula (IV-5), Formula (IV-6), or Formula (IV-7): 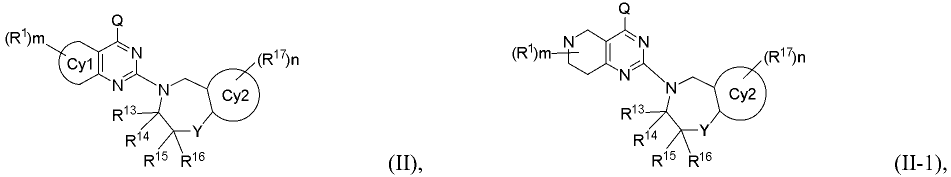 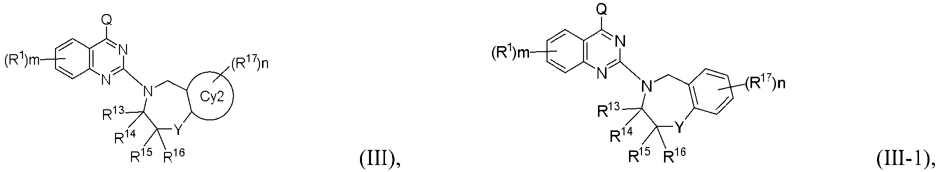 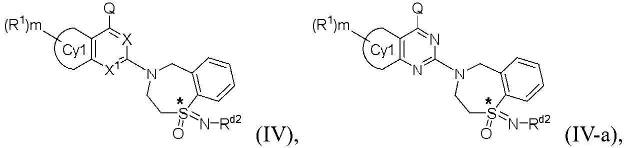 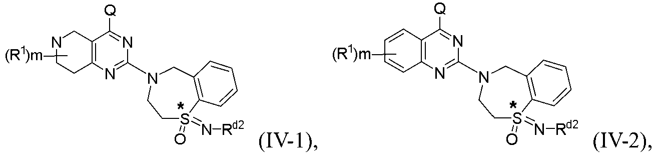 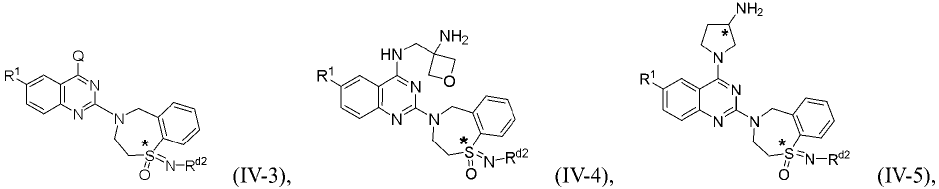 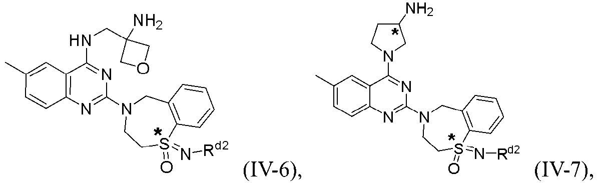 wherein Cy1, Cy2, R¹, Q, X, X¹, m, n, R^{d2}, R¹³, R¹⁴, R¹⁵ , R¹⁶, and R¹⁷ are as defined in any one of claims 1 to 7.

9. The compound, or the tautomer, stereoisomer, isotopic derivative, or pharmaceutically acceptable salt thereof according to claim 1, **characterised in that** the compound is selected from:
| Compound structure and numbering | Compound structure and numbering | Compound structure and numbering |
|---|---|---|
| 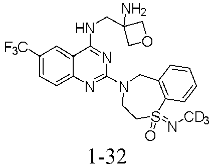 | 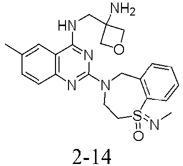 | 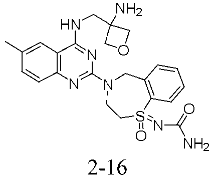 |
| 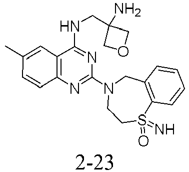 | 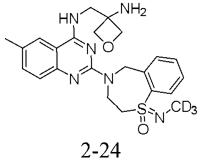 | 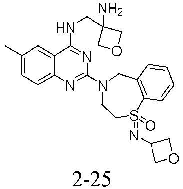 |
| 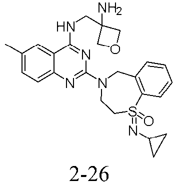 | 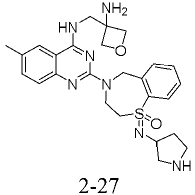 | 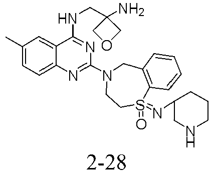 |
| 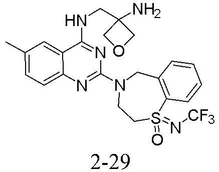 | 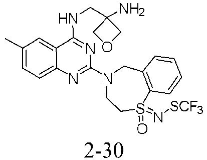 | 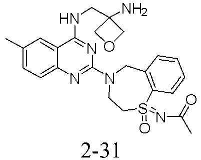 |
| 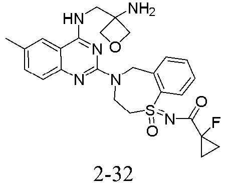 | 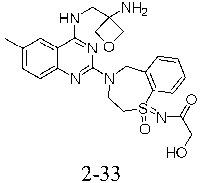 | 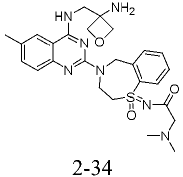 |
| 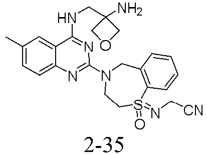 | 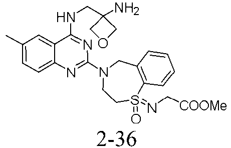 | 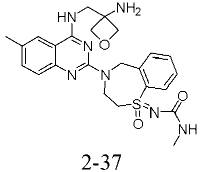 |
| 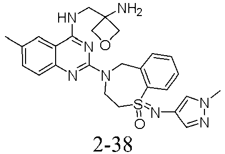 | 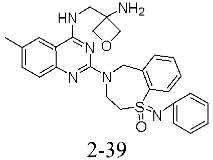 | 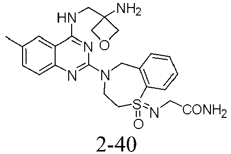 |
| 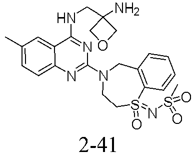 | 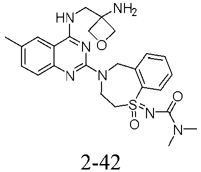 | 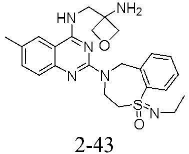 |
| 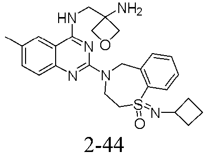 | 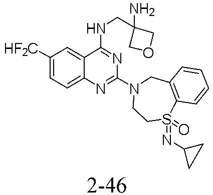 | 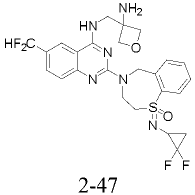 |
| 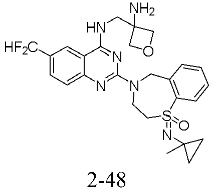 | 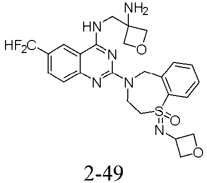 | 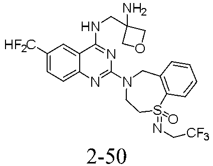 |
| 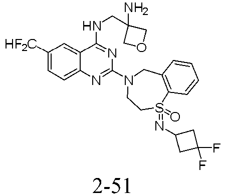 | 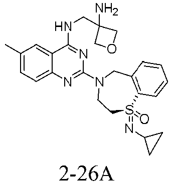 | 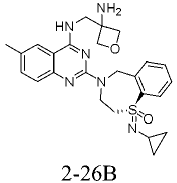 |
| 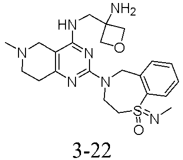 | 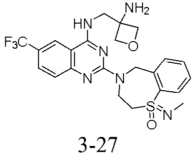 | 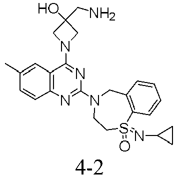 |
| 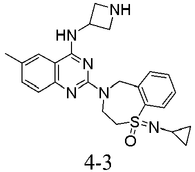 | 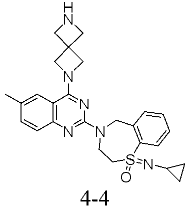 | 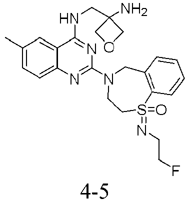 |
| 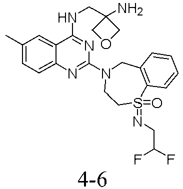 | 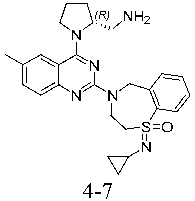 | 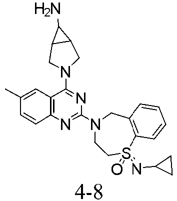 |
| 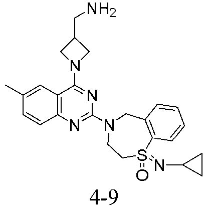 | 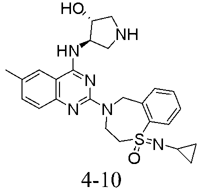 | 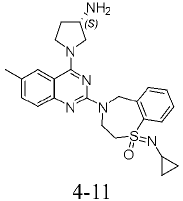 |
| 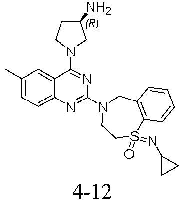 | 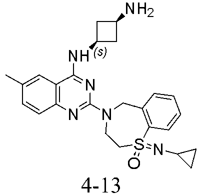 | 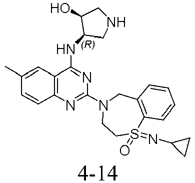 |
| 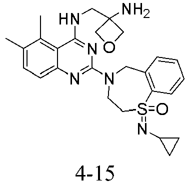 | 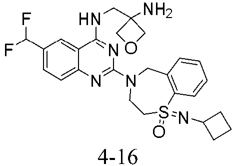 | 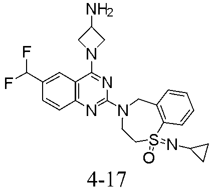 |
| 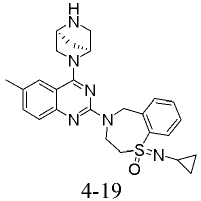 | 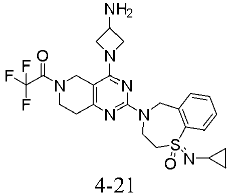 | 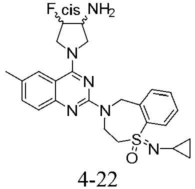 |
| 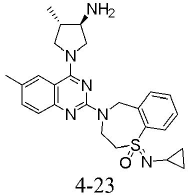 | 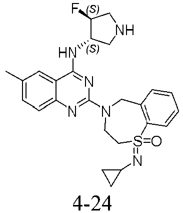 | 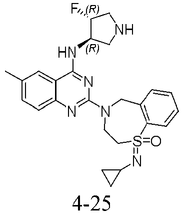 |
| 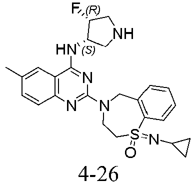 | 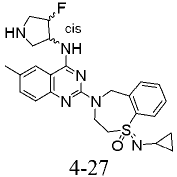 | 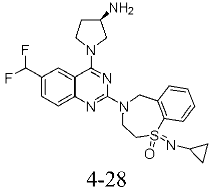 |
| 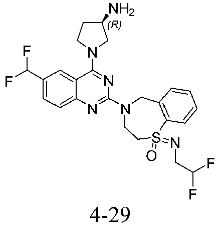 | 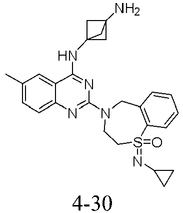 | 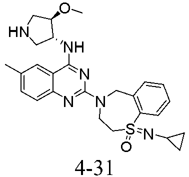 |
| 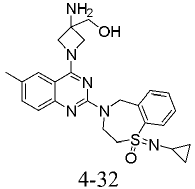 | 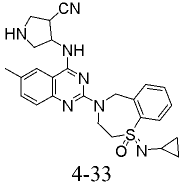 | 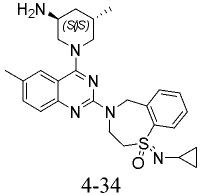 |
| 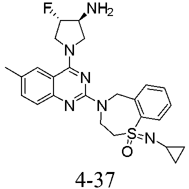 | 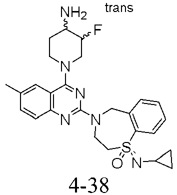 | 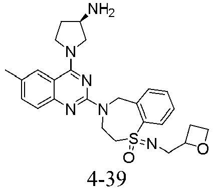 |
| 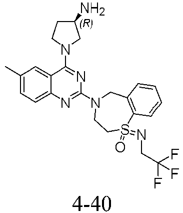 | 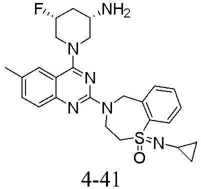 | 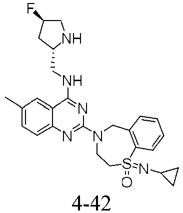 |
| 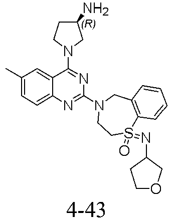 | 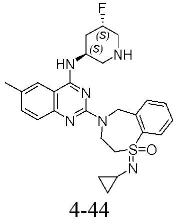 | 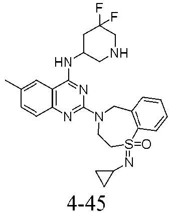 |
| 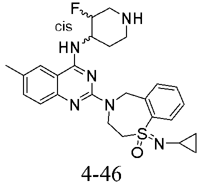 | 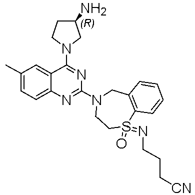 | 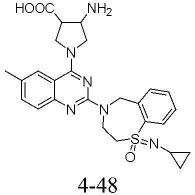 |
| | 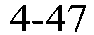 | |
| 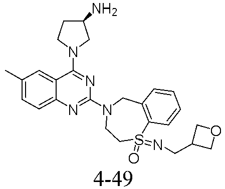 | 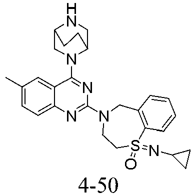 | 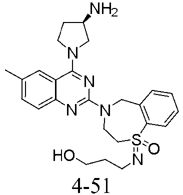 |
| 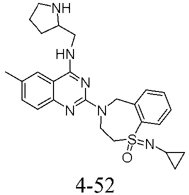 | 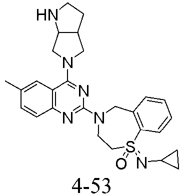 | 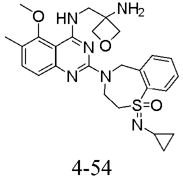 |
| 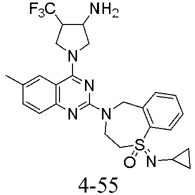 | 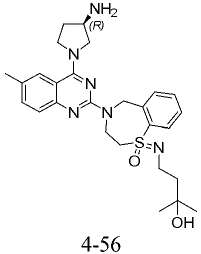 | 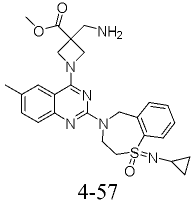 |
| 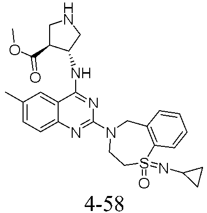 | 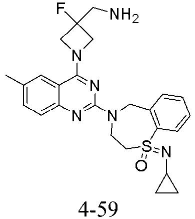 | 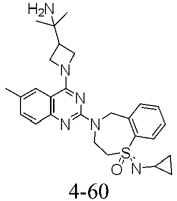 |
| 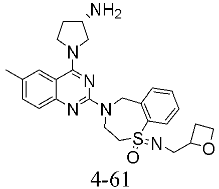 | 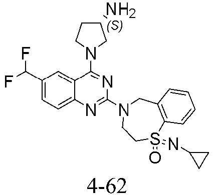 | 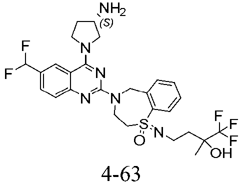 |
| 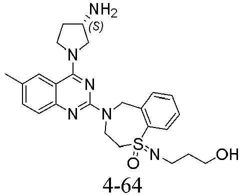 | 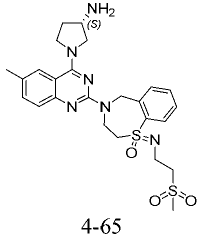 | 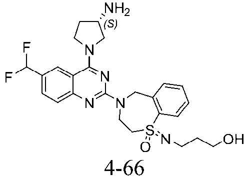 |
| 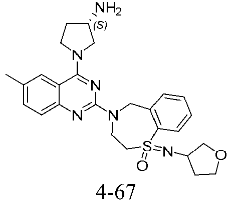 | 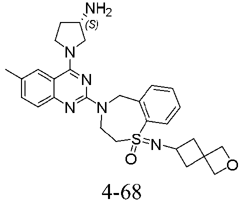 | 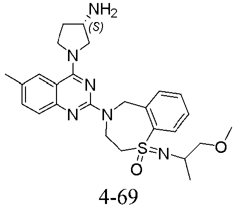 |
| 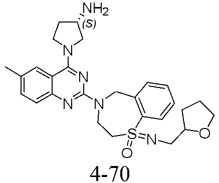 | 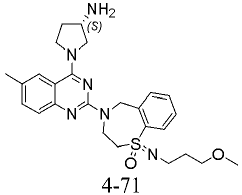 | 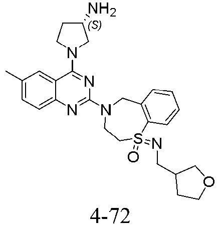 |
| 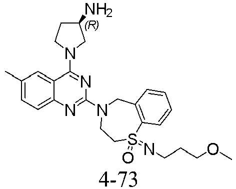 | 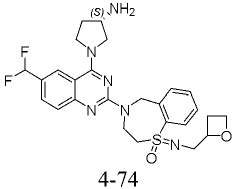 | 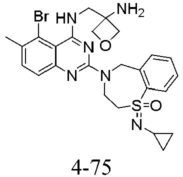 |
| 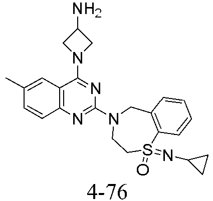 | 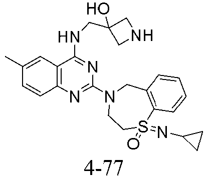 | 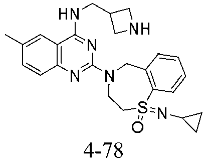 |
| 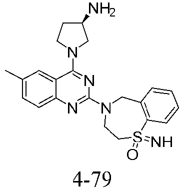 | 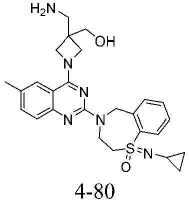 | 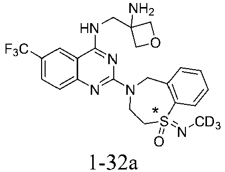 |
| 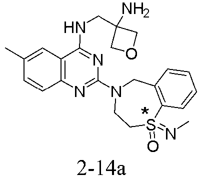 | 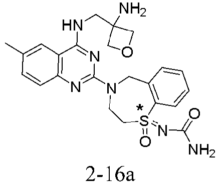 | 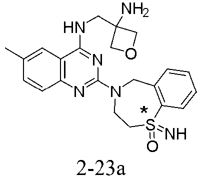 |
| 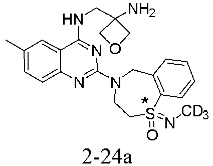 | 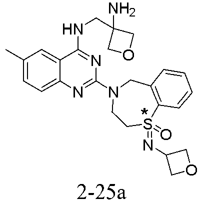 | 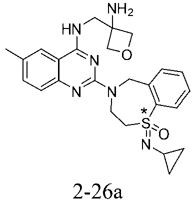 |
| 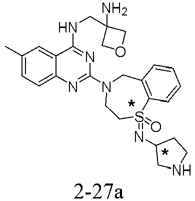 | 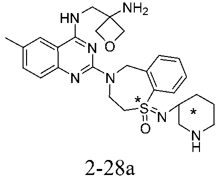 | 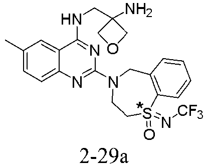 |
| 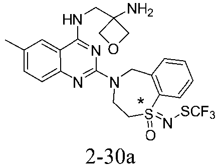 | 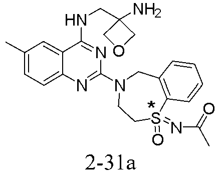 | 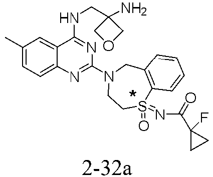 |
| 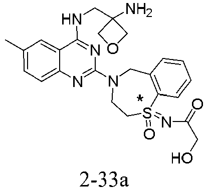 | 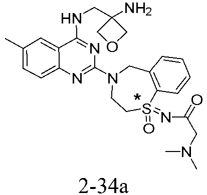 | 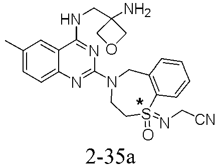 |
| 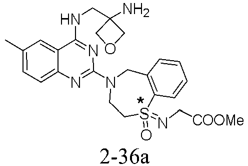 | 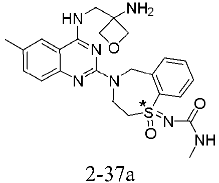 | 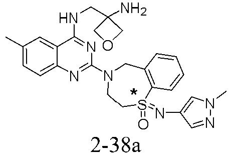 |
| 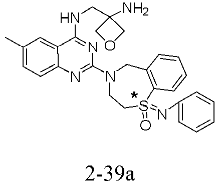 | 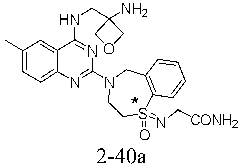 | 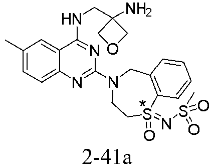 |
| 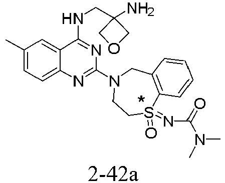 | 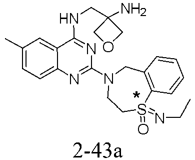 | 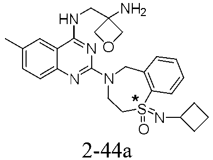 |
| 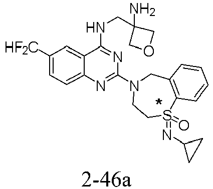 | 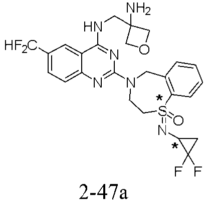 | 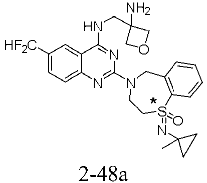 |
| 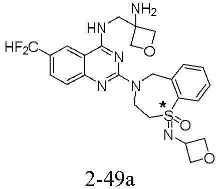 | 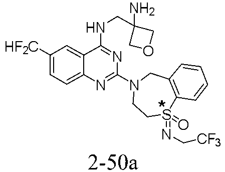 | 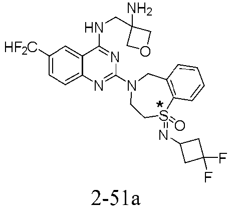 |
| 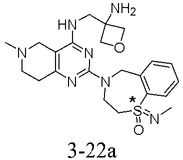 | 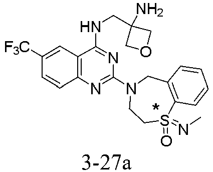 | 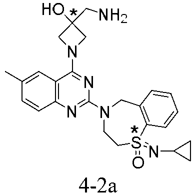 |
| 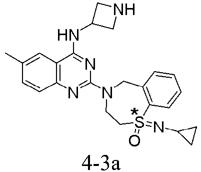 | 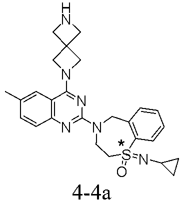 | 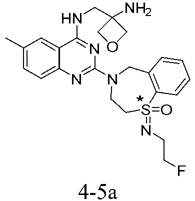 |
| 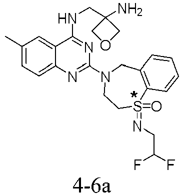 | 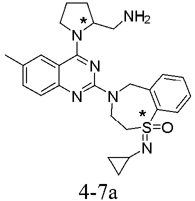 | 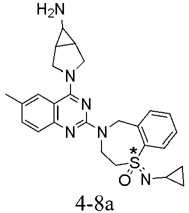 |
| 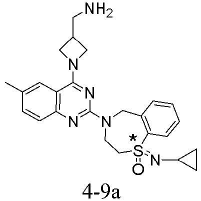 | 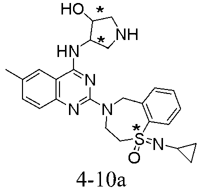 | 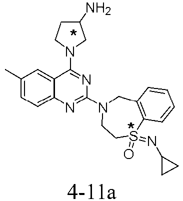 |
| 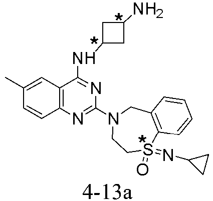 | 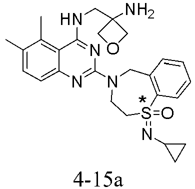 | 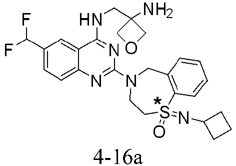 |
| 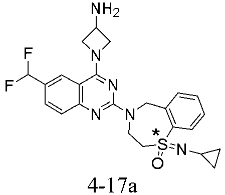 | 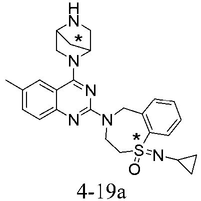 | 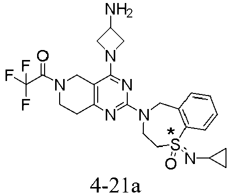 |
| 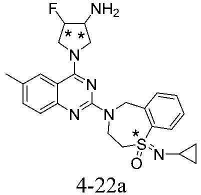 | 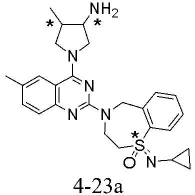 | 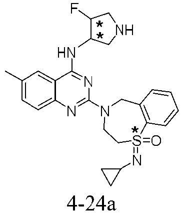 |
| 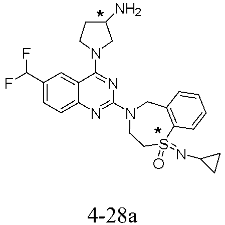 | 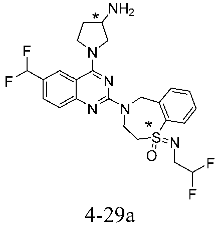 | 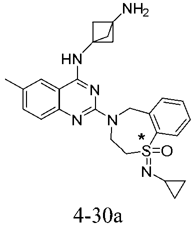 |
| 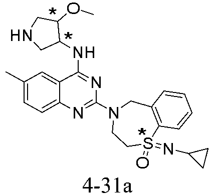 | 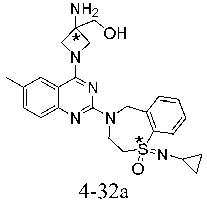 | 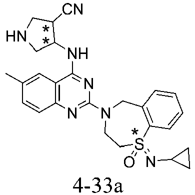 |
| 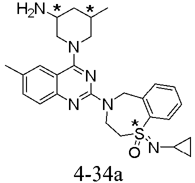 | 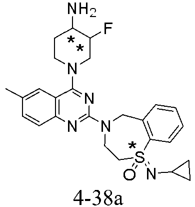 | 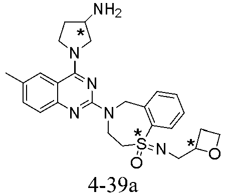 |
| 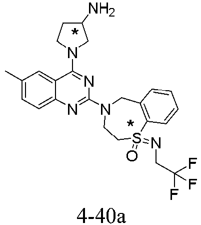 | 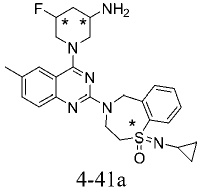 | 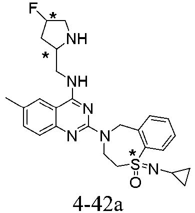 |
| 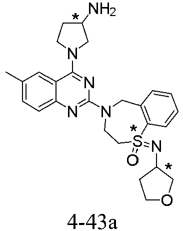 | 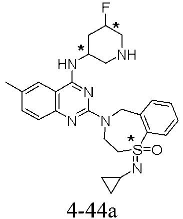 | 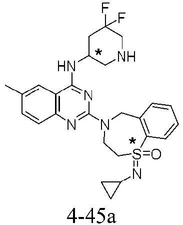 |
| 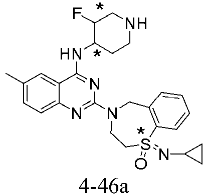 | 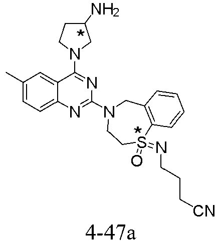 | 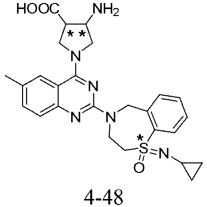 |
| 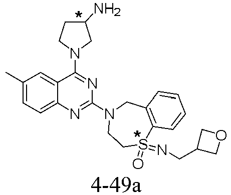 | 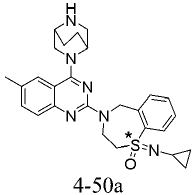 | 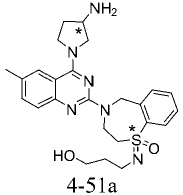 |
| 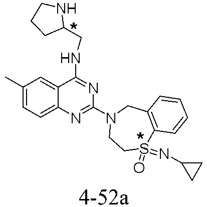 | 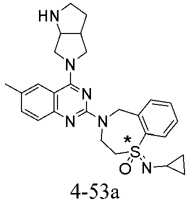 | 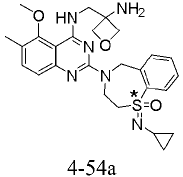 |
| 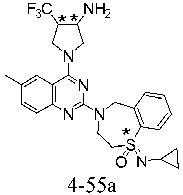 | 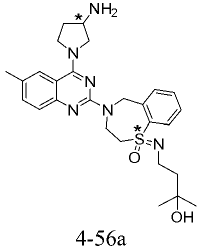 | 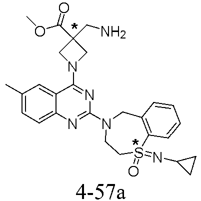 |
| 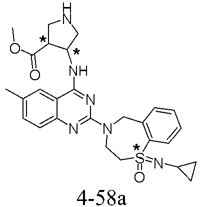 | 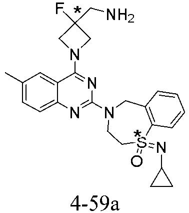 | 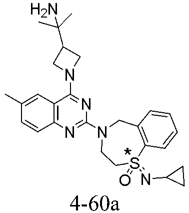 |
| 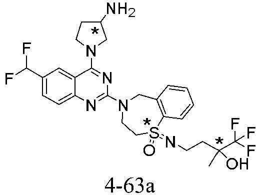 | 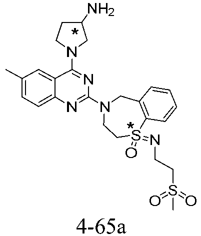 | 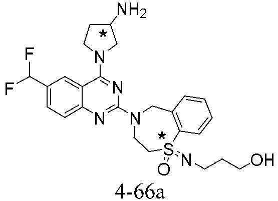 |
| 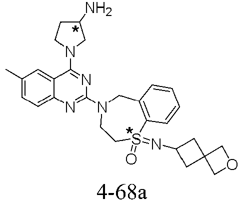 | 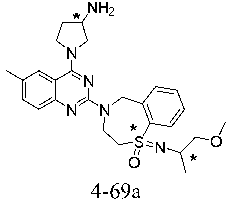 | 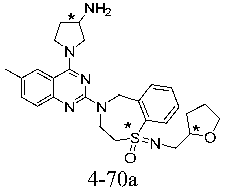 |
| 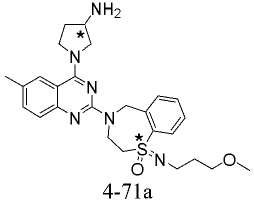 | 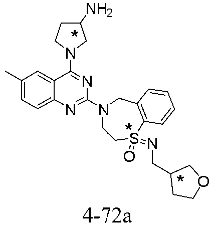 | 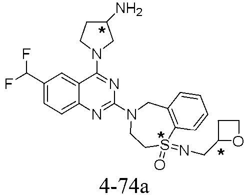 |
| 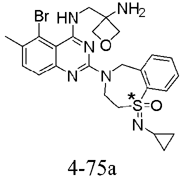 | 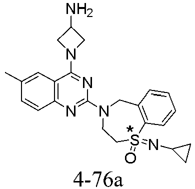 | 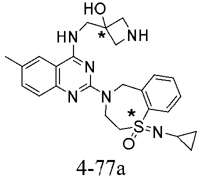 |
| 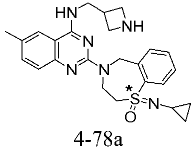 | 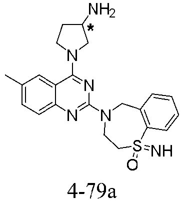 | 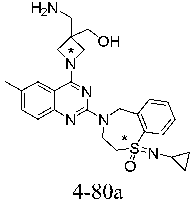 |

10. An intermediate for preparing the compound according to any one of claims 1 to 9, having a structure represented by Formula (R-a), Formula (R-a5), or Formula (R-a6): 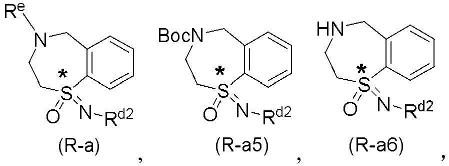 wherein R^{e} is selected from the group consisting of a protective group and hydrogen; and R^{d2} is as defined in any one of claims 1 to 9; with the proviso that R^{e} and R^{d2} are not simultaneously hydrogen.

11. An intermediate for preparing the compound according to any one of claims 1 to 10 is selected from:

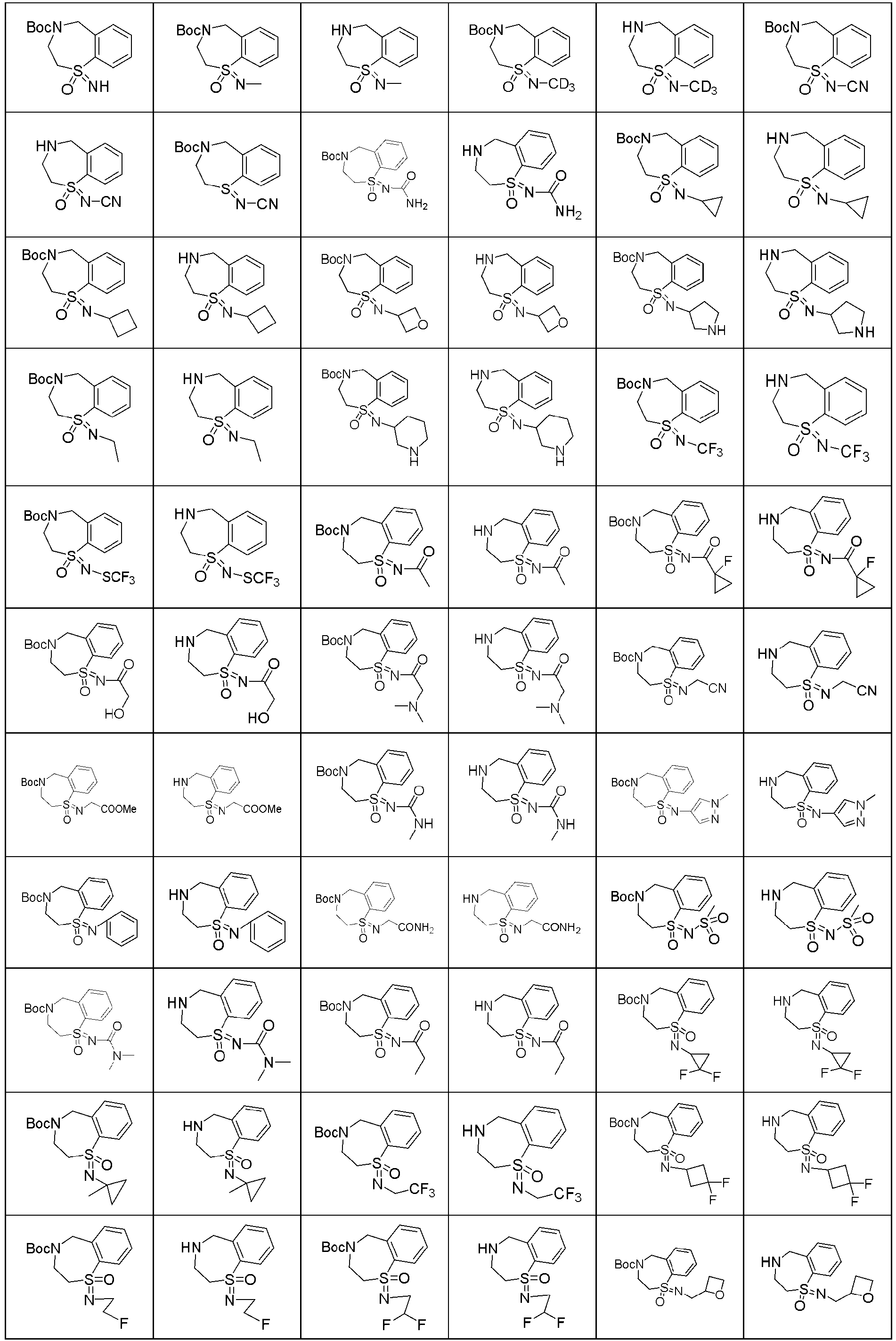

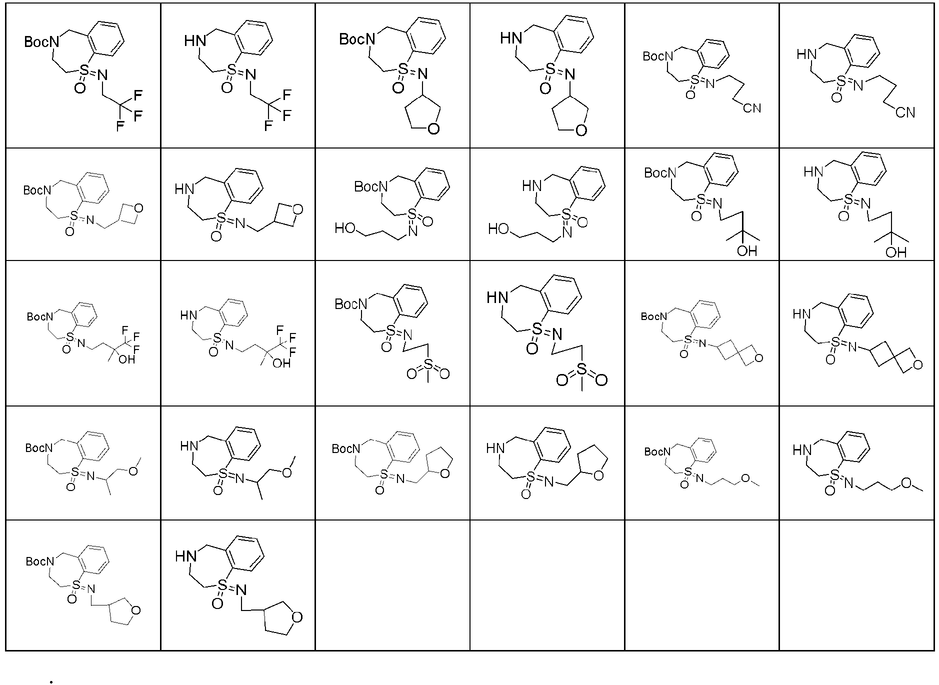

12. A pharmaceutical composition, **characterised by** comprising the compound, or the tautomer, stereoisomer, isotopic derivative, or pharmaceutically acceptable salt thereof according to any one of claims 1 to 9.

13. Use of the compound, or the tautomer, stereoisomer, isotopic derivative, or pharmaceutically acceptable salt thereof according to any one of claims 1 to 9, or the pharmaceutical composition according to claim 12, in the manufacture of a medicament for the prevention and/or treatment of diseases caused by RSV infection.

14. A pharmaceutical composition, **characterised by** comprising the compound, or the tautomer, stereoisomer, isotopic derivative, or pharmaceutically acceptable salt thereof according to any one of claims 1 to 9, or the pharmaceutical composition according to claim 12, and an additional compound.
